# EUROPÄISCHE PATENTSCHRIFT

(11) **EP 2 794 573 B1**
(45) Veröffentlichungstag und Bekanntmachung des Hinweises auf die Patenterteilung: **30.08.2017**
(21) Anmeldenummer: 12801753.0
(22) Anmeldetag: 17.12.2012
(51) Int. Cl.: A01N 43/56, A01N 43/713, A01N 43/80, C07D 231/10, C07D 403/12

(54) **NEUE INSEKTIZIDE AROMATISCHE AMIDE**
NOVEL INSECTICIDAL AROMATIC AMIDES
NOUVEAUX AMIDES AROMATIQUES INSECTICIDES

(30) Priorität: 20.12.2011 EP 11194602
(43) Veröffentlichungstag der Anmeldung: 29.10.2014
(73) Patentinhaber: Bayer Intellectual Property GmbH, 40789 Monheim (DE)
(72) Erfinder: MAUE, Michael, 40764 Langenfeld (DE); KAPFERER, Tobias, 4054 Basel (CH); MÜHLTHAU, Friedrich August, 65779 Kelkheim-Fischbach (DE); MALSAM, Olga, 51503 Rösrath (DE)
(74) Vertreter: BIP Patents
(86) Internationale Anmeldenummer: PCT/EP2012/075849
(87) Internationale Veröffentlichungsnummer: WO 2013/092522

(56) Entgegenhaltungen:
- WO-A2-2010/051926

## Beschreibung

Die vorliegende Anmeldung betrifft neue aromatische Amid-Derivate, Verfahren zu ihrer Herstellung und ihre Verwendung zur Bekämpfung von tierischen Schädlingen, vor allem von Arthropoden und insbesondere von Insekten, Spinnentieren und Nematoden.

Es ist bekannt, dass bestimmte Halogen-substituierte Verbindungen herbizid wirksam sind (vgl. J. Org. Chem. 1997, 62(17), 5908-5919, J. Heterocycl. Chem. 1998, 35(6), 1493-1499, WO 2004/035545, WO 2004/106324, US 2006/069132, WO 2008/029084).

Es ist bekannt, dass bestimme aromatische Amid-Derivate die Rho Kinase inhibieren (WO2008/086047), als Bradykinin-Antagonisten fungieren (WO1998/42672 oder zur Verringerung von Mastzellen bzw. zur Inhibierung deren Degranulierung eingesetzt werden können (WO2005/112920).

Ferner ist bekannt, dass bestimmte aromatische Amide zur Bekämpfung von tierischen Schädlingen, insbesondere als Pflanzenschutzmittel eingesetzt werden können (EP1911751, WO2010/051926).

Über die Verwendung der in der vorliegenden Erfindung beschriebenen aromatischen Amide zur Bekämpfung von tierischen Schädlingen, insbesondere als Pflanzenschutzmittel, ist jedoch nichts bekannt.

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nie als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Es wurde nun überraschenderweise gefunden, dass bestimmte aromatische Amide, sowie deren *N*-Oxide und Salze biologische Eigenschaften aufweisen und sich insbesondere zur Bekämpfung von tierischen Schädlingen eignen und deshalb besonders gut im agrochemischen Bereich und im Bereich der Tiergesundheit einsetzbar sind.

Gegenstand der Erfindung sind Verbindungen der Formel (Ia) 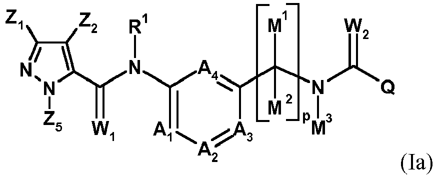 in denen
- R¹: für Wasserstoff oder den gegebenenfalls substituierten Gruppierungen C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Cyano-C₁-C₂-alkyl, Aryl(C₁-C₃)-alkyl, Heteroaryl(C₁-C₃)-alkyl steht;
- A¹: für CR²,
- A₂: für CR³,
- A₃: für CR⁴ oder Stickstoff, und
- A₄: für CR⁵ stehen, wobei
- R², R³, und R⁵: unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N,N* Di-C₂-C₆alkylamino, *N*-C₂-C₇-Alkylaminocarbonyl, *N*-C₂-C₇-Cycloalkylaminocarbonyl oder C₂-C₄-Alkoxycarbonyl, stehen;
- R⁴: für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfmyl, C₁-C₆-Halogenalkylsulfinyl, C₁-C₆-Alkylsulfonyl, C₁-C₆-Halogenalkylsulfonyl, C₁-C₆-Alkylamino, *N,N*-Di-C₂-C₆alkylamino, *N*-C₂-C₇-Alkylaminocarbonyl, *N*-C₂-C₇-Cycloalkylaminocarbonyl oder C₂-C₄-Alkoxycarbonyl oder *N*-Heteroaryl steht;
wenn die Gruppierung A₃ nicht für Stickstoff steht, können R³ und R⁴ gemeinsam mit dem Kohlenstoff, an den sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
R² und R³ können gemeinsam mit dem Kohlenstoff, an den sie gebunden sind, einen 6-gliedrigen Ring bilden, der 0, 1 oder 2 Stickstoffatome enthält und/oder 0 oder 1 Sauerstoffatom und/oder 0 oder 1 Schwefelatom enthält;
- M¹ und M²: jeweils unabhängig voneinander Wasserstoff, C₁-C₆-Alkyl, C₂-C₆-Alkenyl, C₂-C₆-Alkinyl, C₃-C₆-Cycloalkyl, C₂-C₇-Alkoxycarbonyl, C₁-C₆-Halogenalkyl, Cyano, oder Cyano-C₁-C₂-alkyl stehen, oder
- M¹ und M²: mit dem Kohlenstoffatom, an das sie gebunden sind, einen gegebenenfalls substituierten 3-, 4-, 5- oder 6-gliedrigen Ring bilden, der gegebenenfalls 0, 1 oder 2 Stickstoffatome und/oder 0, 1 oder 2 Sauerstoffatome und/oder 0, 1 oder 2 Schwefelatome enthält,
- M³: für Wasserstoff, gegebenenfalls substituiertes C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Cyano-C₁-C₂-alkyl, Aryl(C₁-C₃)-alkyl oder Heteroaryl(C₁-C₃)-alkyl steht,
- W¹ und W²: unabhängig voneinander für Sauerstoff oder Schwefel stehen;
- p: den Wert 1 annimmt,
- Q: für Wasserstoff oder die gegebenenfalls substituierten Gruppierungen C₁-C₆-Alkyl, C₂-C₆-Alkenyl, C₂-C₆-Alkinyl, C₃-C₆-Cycloalkyl, Cyano-C₁-C₂-alkyl, C₁-C₅-Heterocycloalkyl, C₁-C₄-Alkoxy, C₄-C₇-Alkylcycloalkyl, C₄-C₇-Cycloalkylalkyl, C₂-C₇-Alkylcarbonyl, C₁-C₆-Alkylaldehyd, C₁-C₆-Hydroxyalkyl, C₂-C₇-Alkoxycarbonyl, C₁-C₆-Halogenalkyl, für Formyl, Hydroxy, Halogen, Cyano, Aryl, Heteroaryl oder für eine Gruppierung OR⁷, SR⁷, NR⁶R⁸ steht, wobei
- R⁶: für Wasserstoff oder die gegebenenfalls substituierten Gruppierungen C₁-C₆-Alkyl, Aryl, Heteroaryl, C₂-C₆-Alkenyl, C₂-C₆-Alkinyl, C₃-C₆-Cycloalkyl, C₄-C₇-Alkylcycloalkyl und C₄-C₇-Cycloalkylalkyl, C₂-C₇-Alkylcarbonyl, C₂-C₇-Alkoxycarbonyl steht;
- R⁷: ausgewählt ist aus den gegebenenfalls substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
- R⁸: ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
wobei
- Z¹ und Z²: unabhängig voneinander für Wasserstoff, Halogen, Cyano, Nitro oder die gegebenenfalls substituierten Gruppierungen C₁-C₆-Alkyl, C₂-C₄-Alkenyl, C₂-C₄-Alkinyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfmyl, C₁-C₆-Halogenalkylsulfinyl, C₁-C₆-Alkylsulfonyl, C₁-C₆-Halogenalkylsulfonyl, *N,N*-Di-(C₁-C₆)alkylamino, -S(O)₂NR¹³R¹⁴, - S(O)ₙR¹⁵, -S(O)(=NR¹⁶)R¹⁷ oder für gegebenenfalls substituiertes Phenyl oder Pyridinyl steht;
- Z⁵: für Wasserstoff oder die gegebenenfalls substituierten Gruppierungen C₁-C₆-Alkyl, C₂-C₄-Alkenyl, C₂-C₄-Alkinyl, C₁-C₆-Halogenalkyl, C₃-C₆-Cycloalkyl, C₃-C₆-Halogencycloalkyl, oder für gegebenenfalls substituiertes Phenyl oder Pyridinyl steht;
- R¹³: ausgewählt ist aus Wasserstoff oder einer der gegebenfalls substituierten Gruppierungen C₁-C₆-Alkyl, C₁-C₆-Halogenalkyl, C₂-C₆-Alkenyl, C₂-C₆-Alkinyl, C₃-C₆-Cycloalkyl, C₄-C₇-Alkylcycloalkyl, C₄-C₇-Cycloalkylalkyl, C₂-C₇-Alkylcarbonyl C₂-C₇-Alkoxycarbonyl, Aryl oder Heteroaryl;
- R¹⁴: ausgewählt ist aus Wasserstoff oder einer der gegebenenfalls substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
- R¹⁵: ausgewählt ist aus gegebenenfalls substituiertem C₁-C₆-Alkyl, C₂-C₆-Alkenyl, C₂-C₆-Alkinyl, C₃-C₆-Cycloalkyl, C₄-C₇-Alkylcycloalkyl, C₄-C₇-Cycloalkylalkyl, C₁-C₄-Haloalkyl, Aryl oder Heteroaryl;
- R¹⁶: ausgewählt ist aus Wasserstoff oder einer der gegebenenfalls substituierten Gruppierungen C₁-C₆-Alkyl, C₁-C₆-Halogenalkyl, C₂-C₆-Alkenyl, C₂-C₆-Alkinyl, C₃-C₆-Cycloalkyl, C₄-C₇-Alkylcycloalkyl, C₄-C₇-Cycloalkylalkyl, C₂-C₇-Alkylcarbonyl, C₂-C₇-Alkoxycarbonyl, Cyano oder Nitro;
- R¹⁷: ausgewählt ist aus Wasserstoff, gegebenenfalls substituiertem C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl, Aryl oder Heteroaryl; wobei für den dass die R¹ R², R³, R⁴, R^{5,} R⁶, R⁷, R⁸, R¹³, R¹⁴, R¹⁵, R¹⁶, R¹⁷, M¹, Fall, Gruppen M², M³, Z¹, Z², Z⁵ und Q jeweils unabhängig voneinander substituiert sind, die Substituenten ein (1) Substituent oder mehrere Substituenten ausgewählt sind aus einer Gruppe bestehend aus Amino, Hydroxy, Halogen, Nitro, Cyano, Isocyano, Mercapto, Isothiocyanato, Carboxy, Carbonamid, SF₅, Aminosulfonyl, Alkyl, Cycloalkyl, Alkenyl, Cycloalkenyl, Alkinyl, *N-*Mono-alkyl-amino, *N,N*-Dialkylamino, *N*-Alkanoylamino, Alkoxy, Alkenyloxy, Alkinyloxy, Cycloalkoxy, Cycloalkenyloxy, Alkoxycarbonyl, Alkenyloxycarbonyl, Alkinyloxycarbonyl, Aryloxycarbonyl, Alkanoyl, Alkenylcarbonyl, Alkinylcarbonyl, Arylcarbonyl, Alkylthio, Cycloalkylthio, Alkenylthio, Cycloalkenylthio, Alkinylthio, Alkylsulfenyl und Alkylsulfinyl, wobei beide Enantiomere der Alkylsulfmylgruppe umfasst sind, Alkylsulfonyl, *N*-Mono-alkyl-aminosulfonyl, *N,N*-Dialkyl-aminosulfonyl, Alkylphosphinyl, Alkylphosphonyl, wobei für Alkylphosphinyl bzw. Alkylphosphonyl beide Enantiomere umfasst sind, *N*-Alkyl-aminocarbonyl, *N,N*-Dialkyl-amino-carbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl, Trialkylsilyl, Alkoxyalkyl, Alkylthioalkyl, Alkylthioalkoxy, Alkoxyalkoxy, Phenethyl, Benzyloxy, Halogenalkyl, Halogencycloalkyl, Halogenalkoxy, Halogenalkylthio, Halogenalkylsulfinyl, Halogenalkylsulfonyl, Halogenalkanoyl, Halogenalkylcarbonyl, Halogenalkoxycarbonyl, Halogenalkoxyalkoxy, Halogenalkoxyalkylthio, Halogenalkoxyalkanoyl und Halogenalkoxyalkyl.

Bevorzugt sind Verbindungen der Formeln (Ia) 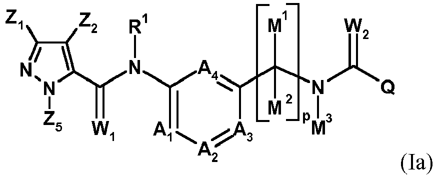 in denen
- R¹: für Wasserstoff, Methyl, Ethyl, n-Propyl, Isopropyl, n-Butyl, 2-Butinyl, Isobutyl, sec-Butyl, tert-Butyl, Methoxymethyl, Ethoxymethyl, Propoxymethyl, Methylcarbonyl, Ethylcarbonyl, n-Propylcarbonyl, Allyl, Propargyl, Isopropylcarbonyl, sec-Butylcarbonyl, tert-Butylcarbonyl, Methoxycarbonyl, Ethoxycarbonyl, n-Propoxycarbonyl, Isopropoxycarbonyl, sec-Butoxycarbonyl, tert-Butoxycarbonyl, Cyanomethyl, 2-Cyanoethyl steht;
- A₁: für CR²,
- A₂: für CR³,
- A₃: für CR⁴ und
- A₄ für CR⁵: stehen, wobei
- R², R³, und R⁵: unabhängig voneinander für Wasserstoff, Fluor, Chlor, Brom, CN, NO₂, Methyl, Ethyl, Fluormethyl, Difluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Methylsulfonyl, Methylsulfinyl, Trifluormethylsulfonyl, Trifluormethylsulfinyl und *N-*Cyclopropylaminocarbonyl stehen;
- R⁴: für Wasserstoff, Fluor, Chlor, Brom, CN, NO₂, Methyl, Ethyl, Fluormethyl, Difluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Methylsulfonyl, Methylsulfinyl, Trifluormethylsulfonyl, Trifluormethylsulfinyl, *N*-Cyclopropylaminocarbonyl stehen und *N*-Triazolyl;
- R³ und R⁴: können gemeinsam mit dem Kohlenstoff, an den sie gebunden sind, einen 6-gliedrigen Ring bilden, der 0, 1 oder 2 Stickstoffatome und/oder 0 oder 1 Sauerstoffatom und/oder 0 oder 1 Schwefelatom enthält, oder
- R² und R³: können gemeinsam mit dem Kohlenstoff, an den sie gebunden sind, einen 6-gliedrigen Ring bilden, der 0, 1 oder 2 Stickstoffatome enthält und/oder 0 oder 1 Sauerstoffatom und/oder 0 oder
- 1: Schwefelatom enthält;
- M¹: für Wasserstoff steht,
- M²: für Wasserstoff, C₁-C₃-Alkyl, C₂-C₃-Alkenyl, C₂-C₃-Alkoxycarbonyl, Cyano, oder Cyano-C₁-C₂-alkyl steht,
- M³: für Wasserstoff, Methyl, Ethyl, n-Propyl, Methoxymethyl, Allyl oder Cyanomethyl steht;
- W¹ und W²: jeweils für Sauerstoff stehen;
- p: den Wert 1 annimmt,
- Q: für Wasserstoff oder die gegebenenfalls substituierten Gruppierungen C₁-C₆-Alkyl, C₂-C₆-Alkenyl, C₂-C₆-Alkinyl, C₃-C₆-Cycloalkyl, Cyano-C₁-C₂-alkyl, C₁-C₅-Heterocycloalkyl, C₁-C₄-Alkoxy, C₄-C₇-Alkylcycloalkyl, C₄-C₇-Cycloalkylalkyl, C₂-C₇-Alkylcarbonyl, C₁-C₆-Alkylaldehyd, C₁-C₆-Hydroxyalkyl, C₂-C₇-Alkoxycarbonyl, C₁-C₆-Halogenalkyl, Cyano, Aryl, Heteroaryl, oder für eine Gruppierung OR⁷, SR⁷ oder NR⁶R⁸ steht, wobei
- R⁶: für Wasserstoff oder C₁-C₃-Alkyl steht;
- R⁷: ausgewählt ist aus den gegebenenfalls substituierten Gruppierungen C₁-C₄-Alkyl, C₂-C₄-Alkenyl, C₂-C₄-Alkinyl oder C₃-C₆-Cycloalkyl;
- R⁸: Wasserstoff ist;
wobei
- Z¹ und Z²: unabhängig voneinander für Wasserstoff, Chlor, Brom, Iod, Cyano, Nitro oder die gegebenenfalls substituierten Gruppierungen C₁-C₄-Alkyl, C₁-C₄-Alkenyl, C₁-C₄-Halogenalkyl, C₃-C₆-Halogencycloalkyl, C₁-C₄-Alkylthio, C₁-C₄-Alkylsulfinyl, C₁-C₄-Alkylsulfonyl steht.
- Z⁵: für Wasserstoff oder die gegebenenfalls substituierten Gruppierungen C₁-C₄-Alkyl, C₂-C₄-Alkenyl, C₂-C₄-Alkinyl, C₁-C₄-Halogenalkyl, C₃-C₄-Cycloalkyl oder C₁-C₃-Alkoxy steht;
wobei für den Fall, dass die Gruppen R⁷, Z¹, Z², Z⁵ und Q jeweils unabhängig voneinander substituiert sind, die Substituenten ein (1) Substituent oder mehrere Substituenten ausgewählt sind aus einer Gruppe bestehend aus Amino, Hydroxy, Halogen, Nitro, Cyano, Isocyano, Mercapto, Isothiocyanato, Carboxy, Carbonamid, SF₅, Aminosulfonyl, Alkyl, Cycloalkyl, Alkenyl, Cycloalkenyl, Alkinyl, *N*-Mono-alkyl-amino, *N,N*-Dialkylamino, *N*-Alkanoylamino, Alkoxy, Alkenyloxy, Alkinyloxy, Cycloalkoxy, Cycloalkenyloxy, Alkoxycarbonyl, Alkenyloxycarbonyl, Alkinyloxycarbonyl, Aryloxycarbonyl, Alkanoyl, Alkenylcarbonyl, Alkinylcarbonyl, Arylcarbonyl, Alkylthio, Cycloalkylthio, Alkenylthio, Cycloalkenylthio, Alkinylthio, Alkylsulfenyl und Alkylsulfinyl, wobei beide Enantiomere der Alkylsulfinylgruppe umfasst sind, Alkylsulfonyl, *N*-Mono-alkyl-aminosulfonyl, *N,N*-Dialkyl-aminosulfonyl, Alkylphosphinyl, Alkylphosphonyl, wobei für Alkylphosphinyl bzw. Alkylphosphonyl beide Enantiomere umfasst sind, *N*-Alkyl-aminocarbonyl, *N,N*-Dialkyl-amino-carbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl, Trialkylsilyl, Alkoxyalkyl, Alkylthioalkyl, Alkylthioalkoxy, Alkoxyalkoxy, Phenethyl, Benzyloxy, Halogenalkyl, Halogencycloalkyl, Halogenalkoxy, Halogenalkylthio, Halogenalkylsulfinyl, Halogenalkylsulfonyl, Halogenalkanoyl, Halogenalkylcarbonyl, Halogenalkoxycarbonyl, Halogenalkoxyalkoxy, Halogenalkoxyalkylthio, Halogenalkoxyalkanoyl und Halogenalkoxyalkyl.

Besonders bevorzugt sind Verbindungen der Formel (Ia) 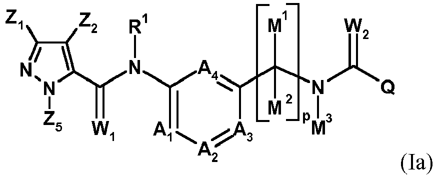 in denen
- R¹: für Wasserstoff, Methyl, Ethyl, n-Propyl, Isopropyl, n-Butyl, 2-Butinyl, Isobutyl, sec-Butyl, Methoxymethyl, Ethoxymethyl, Methylcarbonyl, Ethylcarbonyl, Allyl, Propargyl, Isopropylcarbonyl, Methoxycarbonyl, Ethoxycarbonyl, Isopropoxycarbonyl, Cyanomethyl, 2-Cyanoethyl steht;
- A₁: für CR²,
- A₂: für CR³,
- A₃: für CR⁴ und
- A₄: für CR⁵ stehen, und wobei
- R² für: Wasserstoff oder Chlor steht,
- R³ und R⁴ und R⁵: unabhängig voneinander für Wasserstoff, Fluor, Chlor, Brom, Methyl oder Ethyl stehen,
- M¹: für Wasserstoff steht;
- M²: für Wasserstoff oder Methyl steht;
- M³: für Wasserstoff steht;
- W¹ und W²: jeweils für Sauerstoff stehen;
- p: den Wert 1 annimmt,
- Q: für Wasserstoff, Methyl, Ethyl, *n*-Propyl, *n*-Butyl, *t*-Butyl, 1-Methylethyl, 1,1-Dimethylethyl, 1-Methylpropyl, 1,1-Dimethylpropyl, 2-Methylpropyl, 2-Methylbutyl, 3-Methylbutyl, 2-Hydroxyethyl, Cyanomethyl, 2-Cyanoethyl, 3-Cyanopropyl, 1,1-Difluormethyl, Trifluormethyl, 2-Fluorethyl, 2,2,2-Trifluorethyl, Chlormethyl, 1-Chlorethyl, 2-Chlorethyl, 3-Chlorpropyl, 2,2-Difluorpropyl, Cyclopropyl, 1-Methylcyclopropyl, 1-Cyanocyclopropyl, 2-Methylcyclopropyl, Cyclopropylmethyl, 2,2-Dichlorcyclopropyl, 2,2-Dichlor-1-methylcyclopropyl, 2,2-Difluorcyclopropyl, 2-Fluorcyclopropyl, 1,1'-Bi(cyclopropyl)-1-yl, (2,2-Difluorcyclopropyl)methyl, Cyclobutyl, 3-Ethyloxetan-3-yl, Cyclopentyl, Cyclopentylmethyl, 1-(Cyclopent-1-en-1-yl)methyl, (2-Methyl-1,3-dioxolan-2-yl)methyl, Tetrahydrofuran-2-ylmethyl, Cyclohexyl, 2-Trifluormethylcyclohexyl, 3-Trifluormethylcyclohexyl, 4-Trifluormethylcyclohexyl, 2-Chlorcyclohexyl, 3-Chlorcyclohexyl, 4-Chlorcyclohexyl, 5-Methyl-1,3-dioxan-5-yl, 1-Acetylpiperidin-4-yl, 1-Methylpiperidin-4-yl, Prop-1-en-1-yl, 1-Methylprop-1-en-1-yl, 2-Methylprop-1-en-1-yl, Prop-2-enyl, But-2-en-1-yl, 3-Methylbut-1-en-1-yl, Prop-1-in-1-yl, (4-Methyl-1,2,5-oxadiazol-3-yl)methyl, (3,5-Dimethyl-1,2-oxazol-4-yl)methyl, 1*H*-Tetrazol-5-ylmethyl, (5-Methyl-2-thienyl)methyl, 2-Furylmethyl, (3-Methyl-1,2-oxazol-5-yl)methyl, 3-Thienylmethyl, Benzyl, 4-Chlorbenzyl, 3-Chlorbenzyl, 2-Chlorbenzyl, 1-(4-Chlorphenyl)ethyl, 1-(4-Methylphenyl)ethyl, 1-(3-Chlorphenyl)ethyl, 1-(2-Chlorphenyl)ethyl, 1-(4-Chlorphenyl)cyclopropyl, Pyrimidin-2-ylmethyl, Methoxy, (Methylsulfanyl)methyl, (Methylsulfinyl)methyl, (Methylsulfonyl)methyl, Phenyl, 2-Chlorophenyl, 3-Chlorophenyl, 4-Chlorophenyl, 2-Trifluormethylphenyl, 3-Trifluormethylphenyl, 4-Trifluormethylphenyl, 2-Methylphenyl, 3-Methylphenyl, 4-Methylphenyl, Pyridin-2-yl, Pyridin-3-yl, Pyridin-4-yl, 3-Chlorpyridin-4-yl, 2-Chlorpyridin-4-yl, 2-Methylpyridin-4-yl, 6-Methylpyridin-3-yl, 5-Chlorpyridin-3-yl, 4-Chlropyridin-3-yl, Pyrimidin-5-yl, (6-Chlorpyridin-3-yl)methyl, Methoxycarbonyl, Ethoxycarbonyl, *N-*Methylcarboxamid, *N*-Ethylcarboxamid, *N*-Cyclopropylcarboxamid, *N-*Cyclopropylmethylcarboxamid, 3-Methoxy-3-oxopropanoyl, 3-(Methylamino)-3-oxopropanoyl, 3-(Cyclopropylamino)-3-oxopropanoyl, 3-(Cyclopropylmethylamino)-3-oxopropanoyl steht;
- Z¹: für 1-Chlorcyclopropyl, Trifluormethyl oder Pentafluorethyl, und
- Z²: für Chlor oder Trifluormethyl steht;
- Z⁵: für Methyl und Ethyl.

Des Weiteren werden Verbindungen der Formel (III) beansprucht, die zur Darstellung der erfindungsgemäßen Verbindungen verwendet werden können: 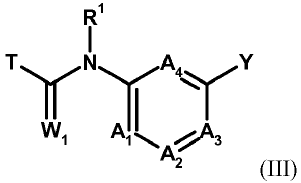
- R¹: für Wasserstoff, gegebenenfalls substituiertes C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Cyano-C₁-C₂-alkyl, Aryl(C₁-C₃)-alkyl oder Heteroaryl(C₁-C₃)-alkyl steht,
die chemische Gruppierung
- A¹: für CR²,
- A₂: für CR³,
- A₃: für CR⁴ oder Stickstoff, und
- A₄: für CR⁵ stehen,
- R², R³, R⁴ und R⁵: unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N,N*-Di-C₂-C₆-alkylamino, *N*-C₂-C₇-Alkylaminocarbonyl, *N*-C₂-C₇-Cycloalkylaminocarbonyl, C₂-C₄-Alkoxycarbonyl, Aryl, Heteroaryl oder N-Heteroaryl stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 5- oder 6-gliedrigen Ring bilden, der 0, 1 oder 2 Stickstoffatome enthält;
- T: für 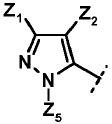 (T-6) steht,
wobei
- Z¹: für Wasserstoff, Halogen, Cyano, Nitro, gegebenenfalls substituiertes C₁-C₆-Alkyl, C₂-C₄-Alkenyl, C₂-C₄-Alkinyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N,N*-Di-(C₁-C₆)alkylamino, -C(=W)NR¹¹R¹⁰, -C(=W)OR¹², -S(O)₂NR¹³R¹⁴, -S(O)ₙR¹⁵, - S(O)(=NR¹⁶)R¹⁷ oder gegebenenfalls substituiertes Phenyl und Pyridinyl steht;
- Z²: für C₁-C₆-Halogenalkyl steht
- Z⁵: für Wasserstoff, gegebenenfalls substituiertes C₁-C₆-Alkyl, C₂-C₄-Alkenyl, C₂-C₄-Alkinyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfmyl, C₁-C₆-Halogenalkylsulfinyl, C₁-C₆-Alkylsulfonyl, C₁-C₆-Halogenalkylsulfonyl, *N,N*-Di-(C₁-C₆)alkylamino, -C(=W)NR¹¹R¹⁰ -C(=W)OR¹², -S(O)₂NR¹³R¹⁴, -S(O)ₙR¹⁵, -S(O)(=NR¹⁶)R¹⁷ oder gegebenenfalls substituiertes Phenyl und Pyridinyl steht;
- R¹⁰: ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, C₂-C₇-Alkylcarbonyl und C₂-C₇-Alkoxycarbonyl;
- R¹¹: ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, C₂-C₇-Alkylcarbonyl und C₂-C₇-Alkoxycarbonyl, Aryl oder Heteroaryl;
- R¹²: ausgewählt ist aus Wasserstoff oder den gegebenenfalls substituierten Gruppierung C₁-C₆-Alkyl, C₁-C₆-Halogenalkyl, C₂-C₆-Alkenyl, C₂-C₆-Halogenalkenyl, C₂-C₆-Alkinyl, C₃-C₆-Cycloalkyl, C₃-C₆-Halogencycloalkyl, C₄-C₇-Alkylcycloalkyl und C₄-C₇-Cycloalkylalkyl, Aryl oder Heteroaryl;
- R¹³: ausgewählt ist aus Wasserstoff oder den gegebenenfalls substituierten Gruppierungen C₁-C₆-Alkyl, C₁-C₆-Halogenalkyl, C₂-C₆-Alkenyl, C₂-C₆-Alkinyl, C₃-C₆-Cycloalkyl, C₄-C₇-Alkylcycloalkyl, C₄-C₇-Cycloalkylalkyl, C₂-C₇-Alkylcarbonyl und C₂-C₇-Alkoxycarbonyl, Aryl oder Heteroaryl;
- R¹⁴: ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
- R¹⁵: ausgewählt ist aus den gegebenenfalls substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl, C₁-C₄-Haloalkyl, Aryl oder Heteroaryl;
- R¹⁶: ausgewählt ist aus Wasserstoff oder den gegebenenfalls substituierten Gruppierungen C₁-C₆-Alkyl, C₁-C₆-Halogenalkyl, C₂-C₆-Alkenyl, C₂-C₆-Alkinyl, C₃-C₆-Cycloalkyl, C₄₋C₇-Alkylcycloalkyl, C₄-C₇-Cycloalkylalkyl, C₂-C₇-Alkylcarbonyl, C₂-C₇-Alkoxycarbonyl, Cyano oder Nitro;
- R¹⁷: ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl, C₄-C₇-Cycloalkyl, Aryl oder Heteroaryl;
- n: die Werte 0, 1 oder 2 annehmen kann;
- Y: für CN oder CH₂NH₂ steht.

Bevorzugt sind Verbindungen (III), in denen 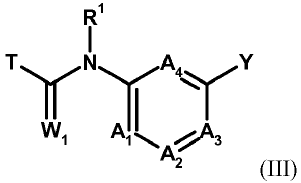
- R¹: für Wasserstoff oder den gegebenenfalls substituierten Gruppierungen C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Cyano-C₁-C₂-alkyl, Aryl(C₁-C₃)-alkyl, Heteroaryl(C₁-C₃)-alkyl steht;
die chemische Gruppierung
- A₁: für CR²,
- A₂: für CR³,
- A₃: für CR⁴, und
- A₄: für CR⁵ stehen,
- R², R³, und R⁵: unabhängig voneinander für Wasserstoff, Fluor, Chlor, Brom, CN, NO₂, Methyl, Ethyl, Fluormethyl, Difluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Methylsulfonyl, Methylsulfinyl, Trifluormethylsulfonyl, Trifluormethylsulfmyl und *N-*Cyclopropylaminocarbonyl stehen;
- R⁴: für Wasserstoff, Fluor, Chlor, Brom, CN, NO₂, Methyl, Ethyl, Fluormethyl, Difluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Methylsulfonyl, Methylsulfinyl, Trifluormethylsulfonyl, Trifluormethylsulfinyl, *N*-Cyclopropylaminocarbonyl stehen und *N*-Triazolyl;
- R³ und R⁴: können gemeinsam mit dem Kohlenstoff, an den sie gebunden sind, einen 6-gliedrigen Ring bilden, der 0, 1 oder 2 Stickstoffatome und/oder 0 oder 1 Sauerstoffatom und/oder 0 oder 1 Schwefelatom enthält, oder
- R² und R³: können gemeinsam mit dem Kohlenstoff, an den sie gebunden sind, einen 6-gliedrigen Ring bilden, der 0, 1 oder 2 Stickstoffatome enthält und/oder 0 oder 1 Sauerstoffatom und/oder 0 oder 1 Schwefelatom enthält;
- T: für 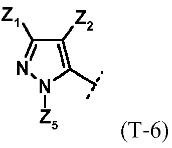 steht,
wobei
- Z¹: für Wasserstoff, Chlor, Brom, Iod, Cyano, Nitro oder die gegebenenfalls substituierten Gruppierungen C₁-C₄-Alkyl, C₁-C₄-Alkenyl, C₁-C₄-Halogenalkyl, C₃-C₆-Halogencycloalkyl, C₁-C₄-Alkylthio, C₁-C₄-Alkylsulfinyl, C₁-C₄-Alkylsulfonyl steht.
- Z²: für C₁-C₄-Halogenalkyl steht,
- Z⁵: für Wasserstoff oder die gegebenenfalls substituierten Gruppierungen C₁-C₄-Alkyl, C₂-C₄-Alkenyl, C₂-C₄-Alkinyl, C₁-C₄-Halogenalkyl, C₃-C₄-Cycloalkyl oder C₁-C₃-Alkoxy steht;
- n: die Werte 0, 1 oder 2 annehmen kann;
- Y: für CN oder CH₂NH₂ steht.

Ganz besonders bevorzugt sind Verbindungen (III), in denen 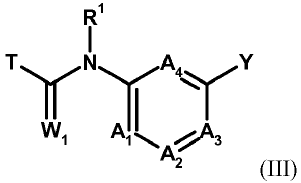
- R¹: für Wasserstoff, Methyl, Ethyl, n-Propyl, Isopropyl, n-Butyl, 2-Butinyl, Isobutyl, sec-Butyl, Methoxymethyl, Ethoxymethyl, Methylcarbonyl, Ethylcarbonyl, Allyl, Propargyl, Isopropylcarbonyl, Methoxycarbonyl, Ethoxycarbonyl, Isopropoxycarbonyl, Cyanomethyl, 2-Cyanoethyl steht;
die chemische Gruppierung
- A₁: für CR²,
- A₂: für CR³,
- A₃: für CR⁴, und
- A₄: für CR⁵ stehen,
- R²: für Wasserstoff oder Chlor steht,
- R³ und R⁴ und R⁵: unabhängig voneinander für Wasserstoff, Fluor, Chlor, Brom, Methyl oder Ethyl stehen,
- T: für 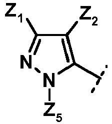 (T-6) steht,
wobei
- Z¹: für Trifluormethyl oder Pentafluorethyl, und
- Z²: für Trifluormethyl steht;
- Z⁵: für Methyl und Ethyl,
- Y: für CN oder CH₂NH₂ steht.

Erfindungsgemäß steht "Alkyl" - in Alleinstellung oder als Bestandteil einer chemischen Gruppe - für geradkettige oder verzweigte Kohlenstoffwasserstoffe, vorzugsweise mit 1 bis 6 Kohlenstoffatomen, wie beispielsweise Methyl, Ethyl, n-Propyl, Isopropyl, n-Butyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylpropyl, 1,3-Dimethylbutyl, 1,4-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl. Ferner bevorzugt für Alkyle mit 1 bis 4 Kohlenstoffatomen, wie unter anderem Methyl, Ethyl, Ethyl, n-Propyl, Isopropyl, n-Butyl, Isobutyl, sec-Butyl oder tert-Butyl. Die erfindungsgemäßen Alkyle können mit einem oder mehreren, gleichen oder verschiedenen Resten substituiert sein.

Erfindungsgemäß steht "Alkenyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Doppelbindung, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-dimethyl-3-butenyl, 1,2-Dimethyl-2-butenyl, 1,2-Dimethyl-3-butenyl, 1,3-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl. Ferner bevorzugt für Alkenyle mit 2 bis 4 Kohlenstoffatomen, wie unter anderem 2-Propenyl, 2-Butenyl oder 1-Methyl-2-propenyl. Die erfindungsgemäßen Alkenyle können mit einem oder mehreren, gleichen oder verschiedenen Resten substituiert sein.

Erfindungsgemäß steht "Alkinyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Dreifachbindung wie beispielsweise 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-3-butinyl, 1,2-Dimethyl-3-butinyl, 2,2-Dimethyl-3-butinyl, 1-Ethyl-3-butinyl, 2-Ethyl-3-butinyl, 1-Ethyl-1-methyl-2-propinyl und 2,5-Hexadiynyl. Ferner bevorzugt für Alkinyle mit 2 bis 4 Kohlenstoffatomen wie unter anderem Ethinyl, 2-Propinyl oder 2-Butinyl-2-propenyl. Die erfindungsgemäßen Alkinyle können mit einem oder mehreren, gleichen oder verschiedenen Resten substituiert sein.

Erfindungsgemäß steht "Cycloalkyl" - in Alleinstellung oder als Bestandteil einer chemischen Gruppe - für mono-, bi- oder tricyclische Kohlenwasserstoffe, vorzugsweise mit 3 bis 10 Kohlenstoffen wie beispielsweise Cyclopropyl, Cyclobutyl, Cyclopentyl, Cyclohexyl, Cycloheptyl, Cyclooctyl, Bicyclo[2.2.1]heptyl, Bicyclo[2.2.2]octyl oder Adamantyl. Ferner bevorzugt für Cycloalkyle mit 3, 4, 5, 6 oder 7 Kohlenstoffatomen, wie unter anderem Cyclopropyl oder Cyclobutyl. Die erfindungsgemäßen Cycloalkyle können mit einem oder mehreren, gleichen oder verschiedenen Resten substituiert sein.

Erfindungsgemäß steht "Alkylcycloalkyl" für mono-, bi- oder tricyclisches Alkylcycloalkyl, vorzugsweise mit 4 bis 10 oder 4 bis 7 Kohlenstoffatomen, wie beispielsweise Ethylcyclopropyl, Isopropylcyclobutyl, 3-Methylcyclopentyl und 4-Methyl-cyclohexyl. Ferner bevorzugt für Alkylcycloalkyle mit 4, 5 oder 7 Kohlenstoffatomen wie unter anderen Ethylcyclopropyl oder 4-Methyl-cyclohexyl. Die erfindungsgemäßen Alkylcycloalkyle können mit einem oder mehreren, gleichen oder verschiedenen Resten substituiert sein.

Erfindungsgemäß steht "Cycloalkylalkyl" für mono, bi- oder tricyclisches Cycloalkylalkyl, vorzugsweise mit 4 bis 10 oder 4 bis 7 Kohlenstoffatomen, wie beispielsweise Cyclopropylmethyl, Cyclobutylmethyl, Cyclopentylmethyl, Cyclohexylmethyl und Cyclopentylethyl. Ferner bevorzugt für Cycloalkylalkyle mit 4, 5 oder 7 Kohlenstoffatomen wie unter anderen Cyclopropylmethyl oder Cyclobutylmethyl. Die erfindungsgemäßen Cycloalkylalkyle können mit einem oder mehreren, gleichen oder verschiedenen Resten substituiert sein.

Erfindungsgemäß steht "Halogen" für Fluor, Chlor, Brom oder Iod, insbesondere für Fluor, Chlor oder Brom.

Die erfindungsgemäßen mit Halogen substituierten chemischen Gruppen, wie beispielsweise Halogenalkyl, Halogencycloalkyl, Halogenalkyloxy, Halogenalkylthio, Halogenalkylsunfinyl oder Halogenalkylsulfonyl sind einfach oder mehrfach bis zur maximal möglichen Substituentenzahl mit Halogen substituiert. Bei mehrfacher Substitution mit Halogen, können die Halogenatome gleich oder verschieden sein und können alle an eines oder an mehrere Kohlenstoffatome gebunden sein. Dabei steht Halogen insbesondere für Fluor, Chlor, Brom oder Iod, vorzugsweise für Fluor, Chlor oder Brom und besonders bevorzugt für Fluor.

Erfindungsgemäß steht "Halogencycloalkyl" für mono-, bi- oder tricyclisches Halogencycloalkyl, vorzugsweise mit 3 bis 10 Kohlenstoffatomen, wie unter anderen 1-Fluor-cyclopropyl, 2-Fluor-cyclopropyl oder 1-Fluor-cyclobutyl. Ferner bevorzugt für Halogencycloalkyl mit 3, 5 oder 7 Kohlenstoffatomen. Die erfindungsgemäßen Halogencycloalkyle können mit einem oder mehreren, gleichen oder verschiedenen Resten substituiert sein.

Erfindungsgemäß steht "Halogenalkyl" "Halogenalkenyl" oder "Halogenalkinyl" für mit Halogen substituierte Alkyle, Alkenyle oder Alkinyle mit vorzugsweise 1 bis 9 gleichen oder verschiedenen Halogenatomen, wie beispielsweise Monohaloalkyl (= Monohalogenalkyl) wie CH₂CH₂Cl, CH₂CH₂F, CHCICH₃, CHFCH₃, CH₂Cl, CH₂F; Perhaloalkyl wie CCl₃ oder CF₃ oder CF₂CF₃; Polyhaloalkyl wie CHF₂, CH₂F, CH₂CHFCl, CHCl₂, CF₂CF₂H, CH₂CF₃. Entsprechendes gilt für Haloalkenyl und andere durch Halogen substituierten Reste. Haloalkoxy ist z.B. OCF₃, OCHF₂, OCH₂F, OCF₂CF₃, OCH₂CF₃ und OCH₂CH₂Cl;

Weitere Beispiele für Halogenalkyle sind Trichlormethyl, Chlordifluormethyl, Dichlorfluormethyl, Chlormethyl, Brommethyl, 1-Fluorethyl, 2-Fluorethyl, 2,2-Difluorethyl, 2,2,2-Trifluorethyl, 2,2,2-Trichlorethyl, 2-Chlor-2,2-difluorethyl, Pentafluorethyl und Pentafluor-tert-butyl. Bevorzugt sind Halogenalkyle mit 1 bis 4 Kohlenstoffatomen und 1 bis 9, vorzugsweise 1 bis 5 gleichen oder verschiedenen Halogenatomen, die ausgewählt sind unter Fluor, Chlor oder Brom. Besonders bevorzugt sind Halogenalkyle mit 1 oder 2 Kohlenstoffatomen und mit 1 bis 5 gleichen oder verschiedenen Halogenatomen, die ausgewählt sind unter Fluor oder Chlor, wie unter anderen Difluormethyl, Trifluormethyl oder 2,2-Difluorethyl.

Erfindungsgemäß steht "Hydroxyalkyl" für geradkettigen oder verzweigten Alkohol, vorzugsweise mit 1 bis 6 Kohlenstoffatomen, wie beispielsweise Methanol, Ethanol, n-Propanol, Isopropanol, n-Butanol, Isobutanol, sec-Butanol und tert-Butanol. Ferner bevorzugt für Hydroxyalkylgruppen mit 1 bis 4 Kohlenstoffatomen. Die erfindungsgemäßen Hydroxyalkylgruppen können mit einem oder mehreren, gleichen oder verschiedenen Resten substituiert sein

Erfindungsgemäß steht "Alkoxy" für geradkettiges oder verzweigtes O-Alkyl, vorzugsweise mit 1 bis 6 Kohlenstoffatomen, wie beispielsweise Methoxy, Ethoxy, n-Propoxy, Isopropoxy, n-Butoxy, Isobutoxy, sec-Butoxy und tert-Butoxy. Ferner bevorzugt für Alkoxygruppen mit 1 bis 4 Kohlenstoffatomen. Die erfindungsgemäßen Alkoxygruppen können mit einem oder mehreren, gleichen oder verschiedenen Resten substituiert sein.

Erfindungsgemäß steht "Halogenalkoxy" für mit Halogen substituiertes geradkettiges oder verzweigtes O-Alkyl, vorzugsweise mit 1 bis 6 Kohlenstoffatomen , wie unter anderem Difluormethoxy, Trifluormethoxy, 2,2-Difluorethoxy, 1,1,2,2-Tetrafluorethoxy, 2,2,2-Trifluorethoxy und 2-Chlor-1,1,2-trifluorethoxy. Ferner bevorzugt für Halogenalkoxygruppen mit 1 bis 4 Kohlenstoffatomen. Die erfindungsgemäßen Halogenalkoxygruppen können mit einem oder mehreren, gleichen oder verschiedenen Resten substituiert sein.

Erfindungsgemäß steht "Alkylthio" für geradkettiges oder verzweigtes S-Alkyl, vorzugsweise mit 1 bis 6 Kohlenstoffatomen, wie beispielsweise Methylthio, Ethylthio, n-Propylthio, Isopropylthio, n-Butylthio, Isobutylthio, sec-Butylthio und tert-Butylthio. Ferner bevorzugt für Alkylthiogruppen mit 1 bis 4 Kohlenstoffatomen. Die erfindungsgemäßen Alkylthiogruppen können mit einem oder mehreren, gleichen oder verschiedenen Resten substituiert sein.

Beispiele für Halogenalkylthioalkyle, d.h. mit Halogen substituierte Alkylthiogruppen, sind unter anderem Difluormethylthio, Trifluormethylthio, Trichlormethylthio, Chlordifluormethylthio, 1-Fluorethylthio, 2-Fluorethylthio, 2,2-Difluorethylthio, 1,1,2,2-Tetrafluorethylthio, 2,2,2-Trifluorethylthio oder 2-Chlor-1,1,2-trifluorethylthio.

Erfindungsgemäß steht "Alkylsulfinyl" für geradkettiges oder verzweigtes Alkylsulfinyl, vorzugsweise mit 1 bis 6 Kohlenstoffatomen wie beispielsweise Methylsulfinyl, Ethylsulfinyl, n-Propylsulfinyl, Isopropylsulfinyl, n-Butylsulfinyl, Isobutylsulfinyl, sec-Butylsulfmyl und tert-Butylsulfinyl. Ferner bevorzugt für Alkylsulfinylgruppen mit 1 bis 4 Kohlenstoffatomen. Die erfindungsgemäßen Alkylsulfinylgruppen können mit einem oder mehreren, gleichen oder verschiedenen Resten substituiert sein.

Beispiele für Halogenalkylsulfinylgrupen, d.h. mit Halogen substituierte Alkylsulfinylgruppen, sind unter anderem Difluormethylsulfinyl, Trifluormethylsulfinyl, Trichlormethylsulfinyl, Chlordifluormethylsulfinyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1,2-trifluorethylsulfinyl.

Erfindungsgemäß steht "Alkylsulfonyl" für geradkettiges oder verzweigtes Alkylsulfonyl, vorzugsweise mit 1 bis 6 Kohlenstoffatomen wie beispielsweise Methylsulfonyl, Ethylsulfonyl, n-Propylsulfonyl, Isopropylsulfonyl, n-Butylsulfonyl, Isobutylsulfonyl, sec-Butylsulfonyl und tert-Butylsulfonyl. Ferner bevorzugt für Alkylsulfonylgruppen mit 1 bis 4 Kohlenstoffatomen. Die erfindungsgemäßen Alkylsulfonylgruppen können mit einem oder mehreren, gleichen oder verschiedenen Resten substituiert sein.

Beispiele für Halogenalkylsulfonylgrupen, d.h. mit Halogen substituierte Alkylsulfonylgruppen sind unter anderem Difluormethylsulfonyl, Trifluormethylsulfonyl, Trichlormethylsulfonyl, Chlordifluormethylsulfonyl, 1-Fluorethylsulfonyl, 2-Fluorethylsulfonyl, 2,2-Difluorethylsulfonyl, 1,1,2,2-Tetrafluorethylsulfonyl, 2,2,2-Trifluorethylsulfonyl und 2-Chlor-1,1,2-trifluorethylsulfonyl.

Erfindungsgemäß steht "Alkylcarbonyl" für geradkettiges oder verzweigtes Alkyl-C(=O), vorzugsweise mit 2 bis 7 Kohlenstoffatomen, wie Methylcarbonyl, Ethylcarbonyl, n-Propylcarbonyl, Isopropylcarbonyl, sec-Butylcarbonyl und tert-Butylcarbonyl. Ferner bevorzugt für Alkylcarbonyle mit 1 bis 4 Kohlenstoffatomen. Die erfindungsgemäßen Alkylcarbonyle können mit einem oder mehreren, gleichen oder verschiedenen Resten substituiert sein.

Erfindungsgemäß steht "Cycloalkylcarbonyl" für geradkettiges oder verzweigtes Cycloalkylcarbonyl, vorzugsweise mit 3 bis 10 Kohlenstoffatomen im Cycloalkylteil, wie beispielsweise Cyclopropylcarbonyl, Cyclobutylcarbonyl, Cyclopentylcarbonyl, Cyclohexyl-carbonyl, Cycloheptylcarbonyl, Cyclooctylcarbonyl, Bicyclo[2.2.1]heptyl, Bycyclo[2.2.2]octylcarbonyl und Adamantylcarbonyl. Ferner bevorzugt für Cycloalkylcarbonyl mit 3, 5 oder 7 Kohlenstoffatomen im Cycloalkylteil. Die erfindungsgemäßen Cycloalkylcarbonylgruppen können mit einem oder mehreren, gleichen oder verschiedenen Resten substituiert sein.

Erfindungsgemäß steht "Alkoxycarbonyl" - in Alleinstellung oder als Bestandteil einer chemischen Gruppe - für geradkettiges oder verzweigtes Alkoxycarbonyl, vorzugsweise mit 1 bis 6 Kohlenstoffatomen oder 1 bis 4 Kohlenstoffatomen im Alkoxyteil, wie beispielsweise Methoxycarbonyl, Ethoxycarbonyl, n-Propoxycarbonyl, Isopropoxycarbonyl, sec-Butoxycarbonyl und tert-Butoxycarbonyl. Die erfindungsgemäßen Alkoxycarbonylgruppen können mit einem oder mehreren, gleichen oder verschiedenen Resten substituiert sein.

Erfindungsgemäß steht "Alkylaminocarbonyl" für geradkettiges oder verzweigtes Alkylaminocarbonyl mit vorzugsweise 1 bis 6 Kohlenstoffatomen oder 1 bis 4 Kohlenstoffatomen im Alkylteil, wie beispielsweise Methylaminocarbonyl, Ethylaminocarbonyl, n-Proylaminocarbonyl, Isopropylaminocarbonyl, sec-Butylaminocarbonyl und tert-Butylaminocarbonyl. Die erfindungsgemäßen Alkylaminocarbonylgruppen können mit einem oder mehreren, gleichen oder verschiedenen Resten substituiert sein.

Erfindungsgemäß steht "*N,N*-Dialkylamino-carbonyl" für geradkettiges oder verzweigtes *N,N-*Dialkylaminocarbonyl mit vorzugsweise 1 bis 6 Kohlenstoffatomen oder 1 bis 4 Kohlenstoffatomen im Alkylteil, wie beispielsweise *N,N*-Dimethylamino-carbonyl, *N.N*-Diethylamino-carbonyl, *N,N*-Di(n-propylamino)-carbonyl, *N,N*-Di-(isopropylamino)-carbonyl und *N,N*-Di-(sec-butylamino)-carbonyl. Die erfindungsgemäßen *N,N*-Dialkylamino-carbonylgruppen können mit einem oder mehreren, gleichen oder verschiedenen Resten substituiert sein.

Erfindungsgemäß steht "Aryl" für ein mono-, bi- oder polycyclisches aromatisches System mit vorzugsweise 6 bis 14, insbesondere 6 bis 10 Ring-Kohlenstoffatomen, wie beispielsweise Phenyl, Naphthyl, Anthryl, Phenanthrenyl, vorzugsweise Phenyl. Ferner steht Aryl auch für mehrcyclische Systeme, wie Tetrahydronaphtyl, Indenyl, Indanyl, Fluorenyl, Biphenyl, wobei die Bindungsstelle am aromatischen System ist. Die erfindungsgemäßen Arylgruppen können mit einem oder mehreren, gleichen oder verschiedenen Resten substituiert sein.

Beispiele substitutierter Aryle stellen die Arylalkyle dar, die gleichfalls mit einem oder mehreren, gleichen oder verschiedenen Resten im Alkyl- und/oder Arylteil substituiert sein können. Beispiele solcher Arylalkyle sind unter anderem Benzyl und 1-Phenylethyl.

Erfindungsgemäß steht "Heterocyclus", "heterocyclischer Ring" oder "heterocyclisches Ringsystem" für ein carbocyclisches Ringsystem mit mindestens einem Ring, in dem mindestens ein Kohlenstoffatom durch ein Heteroatom ersetzt ist, vorzugsweise durch ein Heteroatom aus der Gruppe N, O, S, P, B, Si, Se und der gesättigt, ungesättigt oder heteroaromatisch ist und dabei unsubstituiert oder mit einem Substituenten Z substituiert sein kann, wobei die Bindungsstelle an einem Ringatom lokalisiert ist. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen. Die heterocyclischen Ringe enthalten gewöhnlicherweise nicht mehr als 4 Stickstoffatome, und/oder nicht mehr als 2 Sauerstoffatome und/oder nicht mehr als 2 Schwefelatome. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden erfindungsgemäß auch mehrcyclische Systeme umfaßt, wie beispielsweise 8-Aza-bicyclo[3.2.1]octanyl oder 1-Aza-bicyclo[2.2.1]heptyl. Im Falle von gegebenenfalls substituiertem Heterocyclyl werden erfindungsgemäß auch spirocyclische Systeme umfasst, wie beispielsweise 1-Oxa-5-aza-spiro[2.3]hexyl.

Erfindungsgemäße Heterocyclylgruppen sind beispielsweise Piperidinyl, Piperazinyl, Morpholinyl, Thiomorpholinyl, Dihydropyranyl, Tetrahydropyranyl, Dioxanyl, Pyrrolinyl, Pyrrolidinyl, Imidazolinyl, Imidazolidinyl, Thiazolidinyl, Oxazolidinyl, Dioxolanyl, Dioxolyl, Pyrazolidinyl, Tetrahydrofuranyl, Dihydrofuranyl, Oxetanyl, Oxiranyl, Azetidinyl, Aziridinyl, Oxazetidinyl, Oxaziridinyl, Oxazepanyl, Oxazinanyl, Azepanyl, Oxopyrrolidinyl, Dioxopyrrolidinyl, Oxomorpholinyl, Oxopiperazinyl und Oxepanyl.

Eine besondere Bedeutung kommt Heteroarylen, also heteroaromatischen Systemen zu. Erfindungsgemäß steht der Ausdruck Heteroaryl für heteroaromatische Verbindungen, das heißt vollständig ungesättigte aromatische heterocyclische Verbindungen, die unter die vorstehende Definiton von Heterocyclen fallen. Vorzugsweise für 5- bis 7-gliedrige Ringe mit 1 bis 3, vorzugsweise 1 oder 2 gleichen oder verschiedenen Heteroatomen aus der oben genannten Gruppe. Erfindungsgemäße Heteroaryle sind beispielsweise Furyl, Thienyl, Pyrazolyl, Imidazolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl, 1,2,4-Triazolonyl und 1,2,4-Diazepinyl. Die erfindungsgemäßen Heteroarylgruppen können ferner mit einem oder mehreren, gleichen oder verschiedenen Resten substituiert sein.

Substituierte Gruppen, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy oder eine der Carboxygruppe äquivalente Gruppe, Cyano, Isocyano, Azido, Alkoxycarbonyl, Alkylcarbonyl, Formyl, Carbamoyl, Mono- und *N,N*-Dialkylamino-carbonyl, substituiertes Amino, wie Acylamino, Mono- und *N,N*-Dialkylamino, Trialkylsilyl und gegebenenfalls substituiertes Cycloalkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfonylgruppe umfasst sind, Alkylsulfonyl, Alkylphosphinyl, Alkylphosphonyl und, im Falle cyclischer Reste (= "cyclischer Grundkörper"), auch Alkyl, Haloalkyl, Alkylthio-alkyl, Alkoxy-alkyl, gegebenfalls substituiertes Mono- und *N,N*-Dialkyl-aminoalkyl und Hydroxyalkyl bedeutet.

Im Begriff "substituierte Gruppen" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Alkenylthio, Alkinylthio, Alkenyloxycarbonyl, Alkinyloxycarbonyl, Alkenylcarbonyl, Alkinylcarbonyl, Mono- und *N,N*-Dialkenylamino-carbonyl, Mono- und Dialkinylaminocarbonyl, Mono- und *N,N-*Dialkenylamino, Mono- und *N,N*-Dialkinylamino, Trialkenylsilyl, Trialkinylsilyl, gegebenenfalls substituiertes Cycloalkenyl, gegebenenfalls substituiertes Cycloalkinyl, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden oder einer Oxogruppe, Iminogruppe sowie einer substituierten Iminogruppe.

Wenn zwei oder mehrere Reste einen oder mehrere Ringe bilden, so können diese carbocyclisch, heterocyclisch, gesättigt, teilgesättigt, ungesättigt, beispielsweise auch aromatisch und weiter substituiert sein.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

### Bevorzugte Substituenten für die Substituentenebenen sind beispielsweise

Amino, Hydroxy, Halogen, Nitro, Cyano, Isocyano, Mercapto, Isothiocyanato, Carboxy, Carbonamid, SF₅, Aminosulfonyl, Alkyl, Cycloalkyl, Alkenyl, Cycloalkenyl, Alkinyl, *N*-Mono-alkyl-amino, *N,N-*Dialkylamino, *N*-Alkanoylamino, Alkoxy, Alkenyloxy, Alkinyloxy, Cycloalkoxy, Cycloalkenyloxy, Alkoxycarbonyl, Alkenyloxycarbonyl, Alkinyloxycarbonyl, Aryloxycarbonyl, Alkanoyl, Alkenylcarbonyl, Alkinylcarbonyl, Arylcarbonyl, Alkylthio, Cycloalkylthio, Alkenylthio, Cycloalkenylthio, Alkinylthio, Alkylsulfenyl und Alkylsulfinyl, wobei beide Enantiomere der Alkylsulfmylgruppe umfasst sind, Alkylsulfonyl, *N*-Mono-alkyl-aminosulfonyl, *N,N*-Dialkyl-aminosulfonyl, Alkylphosphinyl, Alkylphosphonyl, wobei für Alkylphosphinyl bzw. Alkylphosphonyl beide Enantiomere umfasst sind, *N*-Alkyl-aminocarbonyl, *N,N*-Dialkyl-amino-carbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl und Trialkylsilyl.

Substituenten, die aus mehreren Substituentenebenen zusammengesetzt sind, sind bevorzugt Alkoxyalkyl, Alkylthioalkyl, Alkylthioalkoxy, Alkoxyalkoxy, Phenethyl, Benzyloxy, Halogenalkyl, Halogencycloalkyl, Halogenalkoxy, Halogenalkylthio, Halogenalkylsulfinyl, Halogenalkylsulfonyl, Halogenalkanoyl, Halogenalkylcarbonyl, Halogenalkoxycarbonyl, Halogenalkoxyalkoxy, Halogenalkoxyalkylthio, Halogenalkoxyalkanoyl, Halogenalkoxyalkyl.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl *N*-substituiert sind; vorzugsweise *N*-Mono- und *N,N*-Dialkylamino, (z.B. Methylamino, Ethylamino, *N,N*-Dimethylamino, *N,N-*Diethylamino, *N,N*-Di-n-propylamino, *N,N*-Diisopropylamino oder *N,N*-Dibutylamino), *N*-Mono- oder *N,N*-Dialkoxyalkylaminogruppen (z.B. *N*-Methoxymethylamino, *N*-Methoxyethylamino, *N,N*-Di-(methoxymethyl)-amino oder *N,N*-Di-(methoxyethyl)-amino), *N*-Mono- und *N,N*-Diarylamino, wie gegebenenfalls substituierte Aniline, Acylamino, *N,N*-diacylamino, *N*-Alkyl-*N*-arylamino, *N*-Alkyl-*N-*acylamino sowie gesättigte *N*-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Erfindungsgemäß umfasst der Begriff "cyclische Aminogruppen" heteroaromatische oder aliphatische Ringsysteme mit einem oder mehreren Stickstoffatomen. Die Heterocyclen sind gesättigt oder ungesättigt, bestehen aus einem oder mehreren, gegebenenfalls kondensierten Ringsystemen und beinhalten gegebenenfalls weitere Heteroatome, wie beispielsweise ein oder zwei Stickstoff-, Sauerstoff- und/oder Schwefelatome. Ferner umfasst der Begriff auch solche Gruppen, die einen Spiroring oder verbrücktes Ringsystem aufweisen. Die Anzahl der Atome, die die cyclische Aminogruppe bilden, ist beliebig und kann z.B. im Falle eines Einringsystems aus 3 bis 8 Ringatomen und im Falle eines Zweiringsystems aus 7 bis 11 Atomen.

Beispielhaft für cyclische Aminogruppen mit gesättigten und ungesättigten monocyclischen Gruppen mit einem Stickstoffatom als Heteroatom seien 1-Azetidinyl, Pyrrolidino, 2-Pyrrolidin-1-yl, 1-Pyrrolyl, Piperidino, 1,4-Dihydropyrazin-1-yl, 1,2,5,6-Tetrahydropyrazin-1-yl, 1,4-Dihydropyridin-1-yl, 1,2,5,6-Tetrahydropyridin-1-yl, Homopiperidinyl genannt; beispielhaft für cyclische Aminogruppen mit gesättigten und ungesättigten monocyclischen Gruppen mit zwei oder mehreren Stickstoffatomen als Heteroatome seien 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-piperazin-1-yl, 1,2-Dihydro-pyrimidin-1-yl, Perhydropyrimidin-1-yl, 1,4-Diazacycloheptan-1-yl, genannt; beispielhaft für cyclische Aminogruppen mit gesättigten und ungesättigten monocyclischen Gruppen mit einem oder zwei Sauerstoffatomen und einem bis drei Stickstoffatomen als Heteroatome, wie beispielsweise Oxazolidin-3-yl, 2,3-Dihydroisoxazol-2-yl, Isoxazol-2-yl, 1,2,3-Oxadiazin-2-yl, Morpholino, beispielhaft für cyclische Aminogruppen mir gesättigten und ungesättigten monocyclischen Gruppen mit einem bis drei Stickstoffatomen und einem bis zwei Schwefelatomen als Heteroatome seien Thiazolidin-3-yl, Isothiazolin-2-yl, Thiomorpholino, oder Dioxothiomorpholino genannt; beispielhaft für cyclische Aminogruppen mit gesättigten und ungesättigten kondensierten cyclischen Gruppen seien Indol-1-yl, 1,2-Dihydrobenzimidazol-1-yl, Perhydropyrrolo[1,2-a]pyrazin-2-yl genannt; beispielhaft für cyclische Aminogruppen mit spirocyclischen Gruppen sei das 2-Azaspiro[4,5]decan-2-yl genannt; beispielhaft für cyclische Aminogruppen mit verbrückten heterocyclischen Gruppen sei das 2-Azabicyclo[2,2,1]heptan-7-yl genannt.

Substituiertes Amino schließt auch quartäre Ammoniumverbindungen (Salze) mit vier organischen Substituenten am Stickstoffatom ein.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, (C₁-C₄)Alkylthio, (C₁-C₄)Halogenalkylthio, Cyano, Isocyano und Nitro substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl.

Gegebenenfalls substituiertes Cycloalkyl ist vorzugsweise Cycloalkyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl und (C₁-C₄)Halogenalkoxy substituiert ist, insbesondere durch einen oder zwei (C₁-C₄)Alkylreste substituiert ist,

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkylreste substituiert ist.

Beispiele für Alkyl substituierte Heteroaryle sind Furylmethyl, Thienylmethyl, Pyrazolylmethyl, Imidazolylmethyl, 1,2,3- und 1,2,4-Triazolylmethyl, Isoxazolylmethyl, Thiazolylmethyl, Isothiazolylmethyl, 1,2,3-, 1,3,4-, 1,2,4- und 1,2,5-Oxadiazolylmethyl, Azepinylmethyl, Pyrrolylmethyl, Pyridylmethyl" Pyridazinylmethyl, Pyrimidinylmethyl, Pyrazinylmethyl, 1,3,5-, 1,2,4- und 1,2,3-Triazinylmethyl, 1,2,4-, 1,3,2-, 1,3,6- und 1,2,6-Oxazinylmethyl, Oxepinylmethyl, Thiepinylmethyl und 1,2,4-Diazepinylmethyl.

Erfindungsgemäß geeignete Salze der erfindungsgemäßen Verbindungen, beispielsweise Salze mit Basen oder Säureadditionssalze, sind alle üblichen nicht toxischen Salze, vorzugsweise landwirtschaftlich und/oder physiologisch annehmbare Salze. Beispielsweise Salze mit Basen oder Säureadditionssalze. Bevorzugt werden Salze mit anorganischen Basen, wie beispielsweise Alkalimetallsalze (z.B. Natrium-, Kalium- oder Cäsiumsalze), Erdalkalimetallsalze (z.B. Calzium- oder Magnesiumsalze), Ammoniumsalze oder Salze mit organischen Basen, insbesondere mit organischen Aminen, wie beispielsweise Triethylammonium-, Dicyclohexylammonium-, *N,N*'-Dibenzylethylen-diammonium-, Pyridinium-, Picolinium- oder Ethanolammoniumsalze, Salze mit anorganischen Säuren (z.B. Hydrochloride, Hydrobromide, Dihydrosulfate, Trihydrosulfate, oder Phosphate), Salze mit organischen Carbonsäuren oder organischen Sulfosäuren (z.b. Formiate, Acetate, Trifluoracetate, Maleate, Tartrate, Methansulfonate, Benzolsulfonate oder 4-Toluolsulfonate). Bekannterweise können tert- Amine, wie beispielsweise manche der erfindungsgemäßen Verbindungen, *N*-Oxide bilden, welche ebenfalls erfindungsgemäße Salze darstellen.

Die erfindungsgemäßen Verbindungen können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

Die erfindungsgemäßen Verbindungen können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Verbindungen der allgemeinen Formel (I) können mit anderen insektiziden, nematiziden akariziden oder antimikrobiellen Wirkstoffen gemischt oder gemeinsam angewendet werden. In diesen Mischungen oder gemeinsamen Anwendungen treten synergistische Wirkungen auf, d.h. die beobachtete Wirkung dieser Mischungen oder gemeinsamen Anwendungen ist höher als die Summe der Wirkungen der Einzelwirkstoffe innerhalb dieser Anwendungen. Beispiele für solche Misch- bzw. Kombinationspartner sind:
(1) Acetylcholinesterase (AChE) Inhibitoren, wie beispielsweise
   Carbamate, z.B. Alanycarb (II-1-1), Aldicarb (II-1-2), Bendiocarb (II-1-3), Benfuracarb (II-1-4), Butocarboxim (II-1-5), Butoxycarboxim (II-1-6), Carbaryl (II-1-7), Carbofuran (II-1-8), Carbosulfan (II-1-9), Ethiofencarb (II-1-10), Fenobucarb (II-1-11), Formetanate (II-1-12), Furathiocarb (II-1-13), Isoprocarb (II-1-14), Methiocarb (II-1-15), Methomyl (II-1-16), Metolcarb (II-1-17), Oxamyl (II-1-18), Pirimicarb (II-1-19), Propoxur (II-1-20), Thiodicarb (II-1-21), Thiofanox (II-1-22), Triazamate (II-1-23), Trimethacarb (II-1-24), XMC (II-1-25) und Xylylcarb (II-1-26); oder
   Organophosphate, z.B. Acephate (II-1-27), Azamethiphos (II-1-28), Azinphos-ethyl (II-1-29), Azinphosmethyl (II-1-30), Cadusafos (II-1-31), Chlorethoxyfos (II-1-32), Chlorfenvinphos (II-1-33), Chlormephos (II-1-34), Chlorpyrifos (II-1-35), Chlorpyrifos-methyl (II-1-36), Coumaphos (II-1-37), Cyanophos (II-1-38), Demeton-S-methyl (II-1-39), Diazinon (II-1-40), Dichlorvos/DDVP (II-1-41), Dicrotophos (II-1-42), Dimethoate (II-1-43), Dimethylvinphos (II-1-44), Disulfoton (II-1-45), EPN (II-1-46), Ethion (II-1-47), Ethoprophos (II-1-48), Famphur (II-1-49), Fenamiphos (II-1-50), Fenitrothion (II-1-51), Fenthion (II-1-52), Fosthiazate (II-1-53), Heptenophos (II-1-54), Imicyafos (II-1-55), Isofenphos (II-1-56), Isopropyl O-(methoxyaminothio-phosphoryl) salicylat (II-1-57), Isoxathion (II-1-58), Malathion (II-1-59), Mecarbam (II-1-60), Methamidophos (II-1-61), Methidathion (II-1-62), Mevinphos (II-1-63), Monocrotophos (II-1-64), Naled (II-1-65), Omethoate (II-1-66), Oxydemeton-methyl (II-1-67), Parathion (II-1-68), Parathionmethyl (II-1-69), Phenthoate (II-1-70), Phorate (II-1-71), Phosalone (II-1-72), Phosmet (II-1-73), Phosphamidon (II-1-74), Phoxim (II-1-75), Pirimiphos-methyl (II-1-76), Profenofos (II-1-77), Propetamphos (II-1-78), Prothiofos (II-1-79), Pyraclofos (II-1-80), Pyridaphenthion (II-1-81), Quinalphos (II-1-82), Sulfotep (II-1-83), Tebupirimfos (II-1-84), Temephos (II-1-85), Terbufos (II-1-86), Tetrachlorvinphos (II-1-87), Thiometon (II-1-88), Triazophos (II-1-89), Triclorfon (II-1-90) und Vamidothion (II-1-91).
(2) GABA-gesteuerte Chlorid-Kanal-Antagonisten, wie beispielsweise
   Cyclodien-organochlorine, z.B. Chlordane (II-2-1) und Endosulfan (II-2-2); oder
   Phenylpyrazole (Fiprole), z.B. Ethiprole (II-2-3) und Fipronil (II-2-4).
(3) Natrium-Kanal-Modulatoren / Spannungsabhängige Natrium-Kanal-Blocker, wie beispielsweise
   Pyrethroide, z.B. Acrinathrin (II-3-1), Allethrin (II-3-2), d-cis-trans Allethrin (II-3-3), d-trans Allethrin (II-3-4), Bifenthrin (II-3-5), Bioallethrin (II-3-6), Bioallethrin S-cyclopentenyl Isomer (II-3-7), Bioresmethrin (II-3-8), Cycloprothrin (II-3-9), Cyfluthrin (II-3-10), beta-Cyfluthrin (II-3-11), Cyhalothrin (II-3-12), lambda-Cyhalothrin (II-3-13), gamma-Cyhalothrin (II-3-14), Cypermethrin (II-3-15), alpha-Cypermethrin (II-3-16), beta-Cypermethrin (II-3-17), theta-Cypermethrin (II-3-18), zeta-Cypermethrin (II-3-19), Cyphenothrin [(1R)-trans-Isomere] (II-3-20), Deltamethrin (II-3-21), Empenthrin [(EZ)-(1R)-Isomere) (II-3-22), Esfenvalerate (II-3-23), Etofenprox (II-3-24), Fenpropathrin (II-3-25), Fenvalerate (II-3-26), Flucythrinate (II-3-27), Flumethrin (II-3-28), tau-Fluvalinate (II-3-29), Halfenprox (II-3-30), Imiprothrin (II-3-31), Kadethrin (II-3-32), Permethrin (II-3-33), Phenothrin [(1R)-trans-Isomer) (II-3-34), Prallethrin (II-3-35), Pyrethrine (pyrethrum) (II-3-36), Resmethrin (II-3-37), Silafluofen (II-3-38), Tefluthrin (II-3-39), Tetramethrin (II-3-40), Tetramethrin [(1R)- Isomere)] (II-3-41), Tralomethrin (II-3-42) und Transfluthrin (II-3-43); oder
   DDT (II-3-44); oder Methoxychlor (II-3-45).
(4) Nikotinerge Acetylcholin-Rezeptor (nAChR) Agonisten, wie beispielsweise
   Neonikotinoide, z.B. Acetamiprid (II-4-1), Clothianidin (II-4-2), Dinotefuran (II-4-3), Imidacloprid (II-4-4), Nitenpyram (II-4-5), Thiacloprid (II-4-6) und Thiamethoxam (II-4-7); oder
   Nikotin (II-4-8).
(5) Nikotinerge Acetylcholin-Rezeptor (nAChR) allosterische Aktivatoren, wie beispielsweise Spinosine, z.B. Spinetoram (II-5-1) und Spinosad (II-5-2).
(6) Chlorid-Kanal-Aktivatoren, wie beispielsweise
   Avermectine/Milbemycine, z.B. Abamectin (II-6-1), Emamectin-benzoat (II-6-2), Lepimectin (II-6-3) und Milbemectin (II-6-4).
(7) Juvenilhormon-Imitatoren, wie beispielsweise
   Juvenilhormon-Analoge, z.B. Hydroprene (II-7-1), Kinoprene (II-7-2) und Methoprene (II-7-3); oder Fenoxycarb (II-7-4); oder Pyriproxyfen (II-7-5).
(8) Wirkstoffe mit unbekannten oder nicht spezifischen Wirkmechanismen, wie beispielsweise Alkylhalide, z.B. Methylbromid (II-8-1) und andere Alkylhalide; oder
   Chloropicrin (II-8-2); oder Sulfurylfluorid (II-8-3); oder Borax (II-8-4); oder Brechweinstein (II-8-5).
(9) Selektive Fraßhemmer, z.B. Pymetrozine (II-9-1); oder Flonicamid (II-9-2).
(10) Milbenwachstumsinhibitoren, z.B. Clofentezine (II-10-1), Hexythiazox (II-10-2) und Diflovidazin (II-10-3); oder
   Etoxazole (II-10-4).
(11) Mikrobielle Disruptoren der Insektendarmmembran, z.B. *Bacillus thuringiensis* Subspezies israelensis (II-11-1), *Bacillus sphaericus* (II-11-2), *Bacillus thuringiensis* Subspezies aizawai (II-11-3), *Bacillus thuringiensis* Subspezies kurstaki (II-11-4), *Bacillus thuringiensis* Subspezies tenebrionis (II-11-5) und BT Pflanzenproteine: Cry1Ab, Cry1Ac, Cry1Fa, Cry2Ab, mCry3A, Cry3Ab, Cry3Bb, Cry34/35Ab1 (II-11-6).
(12) Inhibitoren der oxidativen Phosphorylierung, ATP-Disruptoren, wie beispielsweise Diafenthiuron (II-12-1); oder
   Organozinnverbindungen, z.B. Azocyclotin (II-12-2), Cyhexatin (II-12-3) und Fenbutatin-oxid (II-12-4); oder
   Propargite (II-12-5); oder Tetradifon (II-12-6).
(13) Entkoppler der oxidativen Phoshorylierung durch Unterbrechung des H-Protongradienten, wie beispielsweise Chlorfenapyr (II-13-1), DNOC (II-13-2) und Sulfluramid (II-13-3).
(14) Nikotinerge Acetylcholin-Rezeptor-Antagonisten, wie beispielsweise Bensultap (II-14-1), Cartaphydrochlorid (II-14-2), Thiocyclam (II-14-3) und Thiosultap-Natrium (II-14-4).
(15) Inhibitoren der Chitinbiosynthese, Typ 0, wie beispielsweise Bistrifluron (II-15-1), Chlorfluazuron (II-15-2), Diflubenzuron (II-15-3), Flucycloxuron (II-15-4), Flufenoxuron (II-15-5), Hexaflumuron (11-15-6), Lufenuron (II-15-7), Novaluron (II-15-8), Noviflumuron (II-15-9), Teflubenzuron (11-15-10) und Triflumuron (II-15-11).
(16) Inhibitoren der Chitinbiosynthese, Typ 1, wie beispielsweise Buprofezin (II-16-1).
(17) Häutungsstörende Wirkstoffe, Dipteran, wie beispielsweise Cyromazine (II-17-1).
(18) Ecdyson-Rezeptor Agonisten, wie beispielsweise Chromafenozide (II-18-1), Halofenozide (II-18-2), Methoxyfenozide (II-18-3) und Tebufenozide (II-18-4).
(19) Oktopaminerge Agonisten, wie beispielsweise Amitraz (II-19-1).
(20) Komplex-III-Elektronentransportinhibitoren, wie beispielsweise Hydramethylnon (II-20-1); oder Acequinocyl (II-20-2); oder Fluacrypyrim (II-20-3).
(21) Komplex-I-Elektronentransportinhibitoren, beispielsweise
   METI-Akarizide, z.B. Fenazaquin (II-21-1), Fenpyroximate (II-21-2), Pyrimidifen (II-21-3), Pyridaben (II-21-4), Tebufenpyrad (II-21-5) und Tolfenpyrad (II-21-6); oder
   Rotenone (Derris) (II-21-7).
(22) Spannungsabhängige Natriumkanal-Blocker, z.B. Indoxacarb (II-22-1); oder Metaflumizone (II-22-2).
(23) Inhibitoren der Acetyl-CoA-Carboxylase, wie beispielsweise
   Tetron- und Tetramsäurederivate, z.B. Spirodiclofen (II-23-1), Spiromesifen (II-23-2) und Spirotetramat (II-23-3).
(24) Komplex-IV-Elektronentransportinhibitoren, wie beispielsweise
   Phosphine, z.B. Aluminiumphosphid (II-24-1), Calciumphosphid (II-24-2), Phosphin (II-24-3) und Zinkphosphid (II-24-4); oder
   Cyanid (II-24-5).
(25) Komplex-II-Elektronentransportinhibitoren, wie beispielsweise Cyenopyrafen (II-25-1).
(28) Ryanodinrezeptor-Effektoren, wie beispielsweise
   Diamide, z.B. Chlorantraniliprole (II-28-1) und Flubendiamide (II-28-2).

Weitere Wirkstoffe mit unbekanntem Wirkmechanismus, wie beispielsweise Amidoflumet (II-29-1), Azadirachtin (II-29-2), Benclothiaz (II-29-3), Benzoximate (II-29-4), Bifenazate (II-29-5), Bromopropylate (II-29-6), Chinomethionat (II-29-7), Cryolite (II-29-8), Cyantraniliprole (Cyazypyr) (II-29-9), Cyflumetofen (II-29-10), Dicofol (II-29-11), Diflovidazin (II-29-12), Fluensulfone (II-29-13), Flufenerim (II-29-14), Flufiprole (II-29-15), Fluopyram (II-29-16), Fufenozide (II-29-17), Imidaclothiz (II-29-18), Iprodione (II-29-19), Pyridalyl (II-29-20), Pyrifluquinazon (II-29-21) und Iodmethan (II-29-22); desweiteren Präparate auf Basis von Bacillus firmus (I-1582, BioNeem, Votivo) (II-29-23) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid (II-29-24) (bekannt aus WO2005/077934)*,* 4-{[(6-Brompyrid-3-yl)methyl](2-fluorethyl)amino}furan-2(5H)-on (II-29-25) (bekannt aus WO2007/115644), 4-{[(6-Fluorpyrid-3-yl)methyl](2,2-difluorethyl)amino}furan-2(5H)-on (II-29-26) (bekannt aus WO2007/115644), 4-{[(2-Chlor-1,3-thiazol-5-yl)methyl](2-f[uorethyl)amino}furan-2(5H)-on (II-29-27) (bekannt aus WO2007/115644), 4-{[(6-Chlorpyrid-3-yl)methyl](2-fluorethyl)amino}furan-2(5H)-on (II-29-28) (bekannt aus WO2007/115644), 4-{[(6-Chlorpyrid-3-yl)methyl](2,2-difluorethyl)amino}furan-2(5H)-on (II-29-29) (bekannt aus WO2007/115644), 4-{[(6-Chlor-5-fluorpyrid-3-yl)methyl](methyl)amino}furan-2(5H)-on (II-29-30) (bekannt aus WO2007/115643), 4-{[(5,6-Dichlorpyrid-3-yl)methyl](2-fluorethyl)amino}furan-2(5H)-on (II-29-31) (bekannt aus WO2007/115646), 4-{[(6-Chlor-5-fluorpyrid-3-yl)methyl](cyclopropyl)amino}furan-2(5H)-on (II-29-32) (bekannt aus WO2007/115643), 4-{[(6-Chlorpyrid-3-yl)methyl](cyclopropyl)amino}furan-2(5H)-on (II-29-33) (bekannt aus EP-A-0 539 588), 4-{[(6-Chlorpyrid-3-yl)methyl](methyl)amino}furan-2(5H)-on (II-29-34) (bekannt aus EP-A-0 539 588), {[1-(6-Chlorpyridin-3-yl)ethyl](methyl)oxido-λ⁴-sulfanyliden}cyanamid (II-29-35) (bekannt aus WO2007/149134) und seine Diastereomere {[(1R)-1-(6-Chlorpyridin-3-yl)ethyl](methyl)oxido-λ⁴-sulfanyliden}cyanamid (A) (II-29-36) und {[(1S)-1-(6-Chlorpyridin-3-yl)ethyl](methyl)oxido-λ⁴-sulfanyliden}cyanamid (B) (II-29-37) (ebenfalls bekannt aus WO2007/149134) sowie Sulfoxaflor (II-29-38) (ebenfalls bekannt aus WO2007/149134) und seine Diastereomere [(R)-Methyl(oxido){(1R)-1-[6-(trifluormethyl)pyridin-3-yl]ethyl}-λ⁴-sulfanyliden]cyanamid (A1) (II-29-39) und [(S)-Methyl(oxido) {(1S)-1-[6-(trifluormethyl)pyridin-3-yl]ethyl} -λ⁴-sulfanyliden]cyanamid (A2) (II-29-40), bezeichnet als Diastereomerengruppe A (bekannt aus WO 2010/074747, WO 2010/074751), [(R)-Methyl(oxido) {(1S)-1-[6-(trifluormethyl)pyridin-3-yl]ethyl}-λ⁴-sulfanyliden]cyanamid (B1) (II-29-41) und [(S)-Methyl(oxido){(1R)-1-[6-(trifluormethyl)pyridin-3-yl]ethyl}-λ⁴-sulfanyliden]cyanamid (B2) (II-29-42), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (II-29-43) (bekannt aus WO2006/089633), 3-(4'-Fluor-2,4-dimethylbiphenyl-3-yl)-4-hydroxy-8-oxa-1-azaspiro[4.5]dec-3-en-2-on (II-29-44) (bekannt aus WO2008/067911) 1-{2-Fluor-4-methyl-5-[(2,2,2-trifluorethyl)sulfinyl]phenyl}-3-(trifluormethyl)-1H-1,2,4-triazol-5-amin (II-29-45) (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (II-29-46) (bekannt aus WO2008/066153), 2-Cyan-3-(difluormethoxy)-N,N-dimethylbenzolsulfonamid (II-29-47) (bekannt aus WO2006/056433), 2-Cyan-3-(difluormethoxy)-N-methylbenzolsulfonamid (II-29-48) (bekannt aus WO2006/100288), 2-Cyan-3-(difluormethoxy)-N-ethylbenzolsulfonamid (II-29-49) (bekannt aus WO2005/035486), 4-(Difluormethoxy)-N-ethyl-N-methyl-1,2-benzothiazol-3-amin-1,1-dioxid (II-29-50) (bekannt aus WO2007/057407), N-[1-(2,3-Dimethylphenyl)-2-(3,5-dimethylphenyl)ethyl]-4,5-dihydro-1,3-thiazol-2-amin (II-29-51) (bekannt aus WO2008/104503), {1'-[(2E)-3-(4-Chlorphenyl)prop-2-en-1-yl]-5-fluorspiro[indol-3,4'-piperidin]-l(2H)-yl}(2-chlorpyridin-4-yl)methanon (II-29-52) (bekannt aus WO2003/106457), 3-(2,5-Dimethylphenyl)-4-hydroxy-8-methoxy-1,8-diazaspiro[4.5]dec-3-en-2-on (II-29-53) (bekannt aus WO2009/049851 3-(2,5-Dimethylphenyl)-8-methoxy-2-oxo-1,8-diazaspiro[4.5]dec-3-en-4-yl-ethylcarbonat (II-29-54) (bekannt aus WO2009/049851), 4-(But-2-in-1-yloxy)-6-(3,5-dimethylpiperidin-1-yl)-5-fluorpyrimidin (II-29-55) (bekannt aus WO2004/099160), (2,2,3,3,4,4,5,5-Octafluorpentyl)(3,3,3-trifluorpropyl)malononitril (II-29-56) (bekannt aus WO2005/063094), (2,2,3,3,4,4,5,5-Octafluorpentyl)(3,3,4,4,4-pentafluorbutyl)malononitril (II-29-57) (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (II-29-58) (bekannt aus WO2007/040280), 2-Ethyl-7-methoxy-3-methyl-6-[(2,2,3,3-tetrafluor-2,3-dihydro-1,4-benzodioxin-6-yl)oxy]chinolin-4-yl-methylcarbonat (II-29-59) (bekannt aus JP2008/110953), 2-Ethyl-7-methoxy-3-methyl-6-[(2,2,3,3-tetrafluor-2,3-dihydro-1,4-benzodioxin-6-yl)oxy]chinolin-4-ylacetat (II-29-60) (bekannt aus JP2008/110953), PF1364 (CAS-Reg.Nr. 1204776-60-2) (II-29-61) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (II-29-62) (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (II-29-63) (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (II-29-64) (bekannt aus WO2005/085216), 4-{[(6-Chlorpyndin-3-yl)methyl](cyclopropyl)amino}-1,3-oxazol-2(5H)-on (II-29-65), 4-{[(6-Chlorpyndin-3-yl)methyl](2,2-difluorethyl)amino}-1,3-oxazol-2(5H)-on (II-29-66), 4-{[(6-Chlorpyridin-3-yl)methyl](ethyl)amino}-1,3-oxazol-2(5H)-on (II-29-67), 4-{[(6-Chlorpyridin-3-yl)methyl](methyl)amino}-1,3-oxazol-2(5H)-on (II-29-68) (alle bekannt aus WO2010/005692), NNI-0711 (II-29-69) (bekannt aus WO2002/096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (II-29-70) (bekannt aus WO2002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (II-29-71) (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (II-29-72) (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (II-29-73) (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (II-29-74) (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (II-29-75) (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (II-29-76) (bekannt aus WO2007/101369), 2-{6-[2-(5-Fluorpyridin-3-yl)-1,3-thiazol-5-yl]pyridin-2-yl}pyrimidin (II-29-77) (bekannt aus WO2010/006713), 2-{6-[2-(Pyridin-3-yl)-1,3-thiazol-5-yl]pyridin-2-yl}pyrimidin (II-29-78) (bekannt aus WO20101006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (II-29-79) (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (II-29-80) (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (II-29-81) (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (II-29-82) (bekannt aus WO2010/069502) und (1E)-N-[(6-Chlorpyridin-3-yl)methyl]-N'-cyan-N-(2,2-difluorethyl)ethanimidamid (II-29-83) (bekannt aus WO2008/009360).

Antimikrobiell wirkende Verbindungen:
(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des syn-epimeren Razemates 1RS,4SR,9RS und des anti-empimeren Razemates 1RS,4SR,9SR, Isopyrazam (anti-epimeres Razemat), Isopyrazam (anti-epimeres Enantiomer 1R,4S,9S), Isopyrazam (anti-epimeres Enantiomer 1S,4R,9R), Isopyrazam (syn-epimeres Razemat 1RS,4SR,9RS), Isopyrazam (syn-epimeres Enantiomer 1R,4S,9R), Isopyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid und N-[1-(2,4-Dichlorphenyl)-1-methoxypropan-2-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Dimoxystrobin, Enestroburin, Famoxadon, Fenamidon, Fluoxastrobin, Kresoxim-Methyl, Metominostrobin, Orysastrobin, Picoxystrobin, Pyraclostrobin, Pyrametostrobin, Pyraoxystrobin, Pyribencarb, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(E)-1-[3-(trifluormethyl)phenyl]ethyliden}amino)oxy]methyl}phenyl)ethanamid, (2E)-2-(Methoxyimino)-N-methyl,-2-{2-[(E)-({1-[3 -(trifluormethyl)phenyl]ethoxy}imino)methyl]phenyl}ethanamid, (2E)-2-{2-[([(1E)-1-(3-{[(E)-1-Fluor-2-phenylethenyl]oxy}phenyl)ethyliden]amino}oxy)methyl]phenyl}-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2- {[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethylphenoxy)methyl]phenyl}-2-methoxy-N-methylacetamid und (2R)-2-{2-[(2,5-Dimethylphenoxy)methyl]phenyl} -2-methoxy-N-methylacetamid.
(4) Inhibitoren der Mitose und Zellteilung, wie beispielsweise Benomyl, Carbendazim, Chlorfenazol, Diethofencarb, Ethaboxam, Fluopicolid, Fuberidazol, Pencycuron, Thiabendazol, Thiophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim und Pyrimethanil.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flumorph, Iprovalicarb, Mandipropamid, Polyoxins, Polyoxorim, Validamycin A und Valifenalat.
(10) Inhibitoren der Lipid- und Membran-Synthese, wie beispielsweise Biphenyl, Chloroneb, Dicloran, Edifenphos, Etridiazol, Iodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon und Tricyclazol.
(12) Inhibitoren der Nukleinsäuresynthese, wie beispielsweise Benalaxyl, Benalaxyl-M (Kiralaxyl), Bupirimat, Clozylacon, Dimethirimol, Ethirimol, Furalaxyl, Hymexazol, Metalaxyl, Metalaxyl-M (Mefenoxam), Ofurace, Oxadixyl, Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Chlazafenon, Cufraneb, Cyflufenamid, Cymoxanil, Cyprosulfamide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrrolnitrin, Tebufloquin, Tecloftalam, Tolnifanid, Triazoxid, Trichlamid, Zarilamid, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(SS)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Methyl-6-octyl[1,2,4]triazolo[1,5-a]pyrimidin-7-amin, Ethyl-(2Z)-3-amino-2-cyan-3-phenylprop-2-enoat, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl} -2-phenylacetamid, N- {(Z)-[(Cyclopropylmethoxy)imino][6-(difluormethoxy)-2,3-difluorphenyl]methyl}-2-phenylacetamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid, N-Methyl-2-(1-{[5-methyl-3-(triftuormethyl)-1H-pyrazol-1-yl]acetyl}piperidin-4-yl)-N-[(1S)-1,2,3,4-tetrahydronaphthalen-1-yl]-1,3-thiazol-4-carboxamid, Pentyl-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol und Chinolin-8-olsulfat(2:1).

Alle genannten Mischpartner der Klassen (1) bis (15) können, wenn sie auf Grund ihrer funktionellen Gruppen dazu imstande sind, gegebenenfalls mit geeigneten Basen oder Säuren Salze bilden.
(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dimethyl-1H-pyrazol-4-carboxamid, 2-Chlor-N- [4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N- [4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon und N-[2-(4-{[3-(4-Chlorphenyl)prop-2-in-1-yl]oxy}-3-methoxyphenyl)ethyl]-N2-(methylsulfonyl)valinamid.
Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Schließlich wurde gefunden, dass die neuen Verbindungen der Formel (I) sich bei guter Pflanzenverträglichkeit, günstiger Warmblütertoxizität und guter Umweltverträglichkeit vor allem zur Bekämpfung von tierischen Schädlingen, insbesondere von Arthropoden, Insekten, Spinnentieren, Helminthen, Nematoden und Mollusken, die in der Landwirtschaft, in den Forsten, im Vorrats- und Materialschutz sowie auf dem Hygienesektor oder im Bereich der Tiergesundheit vorkommen, eignen. Gleichfalls können die erfindungsgemäßen Verbindungen im Bereich der Tiergesundheit verwendet werden, beispielsweise zur Bekämpfung von Endo- und/oder Ectoparasiten.

Die erfindungsgemäßen Verbindungen können als Mittel zur Bekämpfung tierischer Schädlinge, vorzugsweise als Pflanzenschutzmittel eingesetzt werden. Sie sind gegen normal sensible und resistente Arten sowie gegen alle oder einzelne Entwicklungsstadien wirksam.

Die erfindungsgemäßen Verbindungen können in allgemein bekannte Formulierungen überführt werden. Solche Formulierungen enthalten im Allgemeinen von 0,01 bis 98 Gew.-% Wirkstoff, vorzugsweise von 0,5 bis 90 Gew.-%.

Die erfindungsgemäßen Verbindungen können in ihren handelsüblichen Formulierungen sowie in den aus diesen Formulierungen bereiteten Anwendungsformen in Mischung mit anderen Wirkstoffen oder Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise von 0,00001 bis 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen).

Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder Endoparasiten. Der Begriff Endoparasiten schließt insbesondere Helminthen wie Cestoden, Nematoden oder Trematoden, und Protozoen wie Coccidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen.

Außerdem wurde gefunden, daß die erfindungsgemäßen Verbindungen eine hohe insektizide Wirkung gegen Insekten zeigen, die technische Materialien zerstören. Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam.

Unter Pflanzen werden alle Pflanzenarten, Pflanzensorten und Pflanzenpopulationen, wie erwünschte und unerwünschte Wildpflanzen oder Kulturpflanzen verstanden. Erfindungsgemäß zu behandelnde Kulturpflanzen sind Pflanzen, die natürlich vorkommen, oder solche, die durch konventionelle Züchtungs- und Optimierungsmethoden oder durch biotechnologische und gentechnologische Methoden oder durch Kombinationen der vorgenannten Methoden erhalten wurden. Der Begriff Kulturpflanze umfasst selbstverständlich auch transgene Pflanzen.

Unter Pflanzensorten versteht man Pflanzen mit neuen Eigenschaften, sogenannten Traits, die sowohl durch konventionelle Züchtung, durch Mutagenese oder durch rekombinante DNA-Techniken oder einer Kombination hieraus gezüchtet worden sind. Dies können Sorten, Rassen, Bio- und Genotypen sein.

Unter Pflanzenteilen werden alle oberirdischen und unterirdischen Teile und Organe der Pflanzen, wie Spross, Blatt, Blüte und Wurzel verstanden, insbesondere Blätter, Nadeln, Stängel, Stämme, Blüten, Fruchtkörper, Früchte, Samen, Wurzeln, Knollen und Rhizome. Der Begriff Pflanzenteilen umfasst weiterhin Erntegut sowie vegetatives und generatives Vermehrungsmaterial, wie z.B. Stecklinge, Knollen, Rhizome, Ableger und Samen bzw. Saatgut.

In einer erfindungsgemäßen Ausführungsform werden natürlich vorkommende oder durch konventionelle Züchtungs- und Optimierungsmethoden (z.B. Kreuzung oder Protoplastenfusion) erhaltene Pflanzenarten und Pflanzensorten sowie deren Pflanzenteile behandelt.

In einer weiteren erfindungsgemäßen Ausführungsform werden transgene Pflanzen, die durch gentechnologische Methoden gegebenenfalls in Kombination mit konventionellen Methoden erhalten wurden und deren Teile behandelt.

Das erfindungsgemäße Behandlungsverfahren wird vorzugsweise auf genetisch modifizierten Organismen, wie beispielsweise Pflanzen oder Pflanzenteile, verwendet.

Genetisch modifizierte Pflanzen, sogenannte transgene Pflanzen, sind Pflanzen, bei denen ein heterologes Gen stabil in das Genom integriert worden ist.

Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im Allgemeinen von 1 bis 10 Tagen, vorzugsweise 1 bis 7 Tagen, nach der Behandlung der Pflanzen mit den Wirkstoffen.

Pflanzen, die weiterhin vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Neben den vorgenannten Pflanzen und Pflanzensorten, können auch solche erfindungsgemäß behandelt werden, die gegen einen oder mehrere abiotische Streßfaktoren widerstandsfähig sind.

Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosateacetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier-DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insecticidal proteins, VIP),
   die unter http://www.lifesci.sussex.ac.uk/Home/Neil_Crickmore/Bt/vip.html
   angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier-DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die erfindungsgemäße Behandlung der Pflanzen und Pflanzenteile mit den Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Saatgut, weiterhin durch ein- oder mehrschichtiges Umhüllen.

Insbesondere eignen sich die erfindungsgemäßen Mischungen zur Behandlung von Saatgut. Bevorzugt sind dabei die vorstehend als bevorzugt oder besonders bevorzugt genannten erfindungsgemäßen Kombinationen zu nennen. So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt wird, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und auch der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ein weiterer Vorteil besteht in der synergistischen Erhöhung der insektiziden Wirksamkeit der erfindungsgemäßen Mittel gegenüber dem insektiziden Einzelwirkstoff, die über die zu erwartende Wirksamkeit der beiden einzeln angewendeten Wirkstoffe hinausgeht. Vorteilhaft ist auch die synergistische Erhöhung der fungiziden Wirksamkeit der erfindungsgemäßen Mittel gegenüber dem fungiziden Einzelwirkstoff, die über die zu erwartende Wirksamkeit des einzeln angewendeten Wirkstoffs hinausgeht. Damit wird eine Optimierung der Menge der eingesetzten Wirkstoffe ermöglicht.

Ebenso ist es als vorteilhaft anzusehen, dass die erfindungsgemäßen Mischungen insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den erfindungsgemäßen Mitteln können bestimmte Schädlinge bereits durch die Expression des z.B. insektiziden Proteins kontrolliert werden, und zusätzlich durch die erfindungsgemäßen Mittel vor Schäden bewahrt werden.

Die erfindungsgemäßen Mittel eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs). Die erfindungsgemäßen Mittel eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit einem erfindungsgemäßen Mittel eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie *Bacillus, Rhizobium, Pseudomonas, Serratia, Trichoderma, Clavibacter, Glomus* oder *Gliocladium* stammen. Die vorliegende Erfindung eignet sich besonders für die Behandlung von transgenem Saatgut, das zumindest ein heterologes Gen enthält, das aus *Bacillus sp.* stammt und dessen Genprodukt Wirksamkeit gegen Maiszünsler und/oder Maiswurzel-Bohrer zeigt. Besonders bevorzugt handelt es sich dabei um ein heterologes Gen, das aus *Bacillus thuringiensis* stammt.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Darüber hinaus können die erfindungsgemäßen Verbindungen zur Bekämpfung einer Vielzahl verschiedener Schädlinge einschließlich beispielsweise schädlicher saugender Insekten, beißender Insekten und anderen an Pflanzen parasitierenden Schädlingen, Vorratsschädlingen, Schädlingen, die industrielle Materialien zerstören und Hygieneschädlingen einschließlich Parasiten im Bereich Tiergesundheit verwendet und zu ihrer Bekämpfung wie zum Beispiel ihrer Auslöschung und Ausmerzung eingesetzt werden. Die vorliegende Erfindung schließt somit auch ein Verfahren zur Bekämpfung von Schädlingen ein.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder Endoparasiten. Der Begriff Endoparasiten schließt insbesondere Helminthen wie Cestoden, Nematoden oder Trematoden, und Protozoen wie Kozzidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen.

Zu diesen Parasiten gehören:
Aus der Ordnung der Anoplurida z.B. Haematopinus spp., Linognathus spp., Pediculus spp., Phtirus spp., Solenopotes spp.; spezielle Beispiele sind: Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;
Aus der Ordnung der Mallophagida und den Unterordnungen Amblycerina und Ischnocerina z.B. Trimenopon spp., Menopon spp., Trinoton spp., Bovicola spp., Werneckiella spp., Lepikentron spp., Damalina spp., Trichodectes spp., Felicola spp.; spezielle Beispiele sind: Bovicola bovis, Bovicola ovis, Bovicola limbata, Damalina bovis, Trichodectes canis, Felicola subrostratus, Bovicola caprae, Lepikentron ovis, Werneckiella equi;
Aus der Ordnung der Diptera und den Unterordnungen Nematocerina und Brachycerina z.B. Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; spezielle Beispiele sind: Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca;
Aus der Ordnung der Siphonapterida z.B. Pulex spp., Ctenocephalides spp., Tunga spp., Xenopsylla spp., Ceratophyllus spp.; spezielle Beispiele sind: Ctenocephalides canis, Ctenocephalides felis, Pulex irritans, Tunga penetrans, Xenopsylla cheopis;
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp. (z.B. Suppella longipalpa);
Aus der Unterklasse der Acari (Acarina) und den Ordnungen der Meta- und Mesostigmata z.B. Argas spp., Ornithodorus spp., Otobius spp., Ixodes spp., Amblyomma spp., Rhipicephalus (Boophilus) spp., Dermacentor spp., Haemophysalis spp., Hyalomma spp., Dermanyssus spp., Rhipicephalus spp. (der ursprünglichen Gattung der Mehrwirtszecken), Ornithonyssus spp., Pneumonyssus spp., Raillietia spp., Pneumonyssus spp., Sternostoma spp., Varroa spp., Acarapis spp.; spezielle Beispiele sind: Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Ornithonyssus bursa, Ornithonyssus sylviarum, Varroa jacobsoni;
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.; spezielle Beispiele sind: Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic mange, Pneumonyssoides caninum, Acarapis woodi.

Die erfindungsgemäßen Wirkstoffe eignen sich auch zur Bekämpfung von Arthropoden, Helminthen und Protozoen, die Tiere befallen. Zu den Tieren zählen landwirtschaftliche Nutztiere wie z.B. Rinder, Schafe, Ziegen, Pferde, Schweine, Esel, Kamele, Büffel, Kaninchen, Hühner, Puten, Enten, Gänse, Zuchtfische, Honigbienen. Zu den Tieren zählen außerdem Haustiere - die auch als Heimtiere bezeichnet werden - wie z.B. Hunde, Katzen, Stubenvögel, Aquarienfische sowie sogenannte Versuchstiere wie z.B. Hamster, Meerschweinchen, Ratten und Mäuse.

Durch die Bekämpfung dieser Arthropoden, Helminthen und/oder Protozoen sollen Todesfälle vermindert und die Leistung (bei Fleisch, Milch, Wolle, Häuten, Eiern, Honig usw.) und die Gesundheit des Wirtstieres verbessert werden, so dass durch den Einsatz der erfindungsgemäßen Wirkstoffe eine wirtschaftlichere und einfachere Tierhaltung möglich ist.

So ist es beispielsweise wünschenswert, die Aufnahme von Blut des Wirts durch die Parasiten (falls zutreffend) zu verhindern oder zu unterbrechen. Eine Bekämpfung der Parasiten kann außerdem dazu beitragen, die Übertragung infektiöser Substanzen zu verhindern.

Der Begriff "Bekämpfung", so wie er hier bezogen auf den Bereich Tiergesundheit verwendet wird, bedeutet, dass die Wirkstoffe wirken, indem sie das Vorkommen des betreffenden Parasiten in einem mit solchen Parasiten befallenen Tier auf unschädliche Niveaus reduzieren. Genauer gesagt bedeutet "Bekämpfung", wie hier verwendet, dass der Wirkstoff den betreffenden Parasiten tötet, sein Wachstum hemmt oder seine Proliferation inhibiert.

Im allgemeinen können die erfindungsgemäßen Wirkstoffe, wenn sie für die Behandlung von Tieren eingesetzt werden, direkt angewendet werden. Vorzugsweise werden sie als pharmazeutische Zusammensetzungen angewendet, die im Stand der Technik bekannte pharmazeutisch unbedenkliche Exzipienten und/oder Hilfsstoffe enthalten können.

Die Anwendung (= Verabreichung) der Wirkstoffe im Bereich Tiergesundheit und in der Tierhaltung erfolgt in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subkutan, intravenös, intraperitoneal u.a.), Implantate, durch nasale Applikation, durch dermale Applikation in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw. Die Wirkstoffe können als Shampoo oder als geeignete, in Aerosolen oder drucklosen Sprays, z.B. Pumpsprays und Zerstäubersprays, anwendbare, Formulierungen formuliert werden.

Bei der Anwendung für Nutztiere, Geflügel, Haustiere etc. kann man die erfindungsgemäßen Wirkstoffe als Formulierungen (beispielsweise Pulver, Spritzpulver [wettable powders, "WP"], Emulsionen, Emulsionskonzentrate [emulsifiable concentrates ,"EC"], fließfähige Mittel, homogene Lösungen und Suspensionskonzentrate [suspension concentrates, "SC"]), die die Wirkstoffe in einer Menge von 1 bis 80 Gew.-% enthalten, direkt oder nach Verdünnung (z.B. 100- bis 10 000facher Verdünnung) anwenden oder sie als chemisches Bad verwenden.

Beim Einsatz im Bereich Tiergesundheit können die erfindungsgemäßen Wirkstoffe in Kombination mit geeigneten Synergisten oder anderen Wirkstoffen wie beispielsweise Akariziden, Insektiziden, Anthelmintika, Mittel gegen Protozoen, verwendet werden.

Die erfindungsgemäßen Verbindungen können nach üblichen, dem Fachmann bekannten Methoden hergestellt werden.

### Darstellungsverfahren

Die im Folgenden aufgeführten Darstellungsverfahren gelten insbesondere für die Verbindungen der allgemeinen Formel (I). Analog gelten sie aber auch für die Verbindungen der allgemeinen Formel (II).
1) Verbindungen des Typs **(1-1),** mit W¹ und W² gleich Sauerstoff und A¹-A⁴, T und Q gemäß den oben beschriebenen Bedeutungen, können gegebenenfalls nach dem in Reaktionsschema 1 abgebildeten allgemeinem Darstellungsverfahren A hergestellt werden.

### Reaktionsschema 1: Darstellungsverfahren A

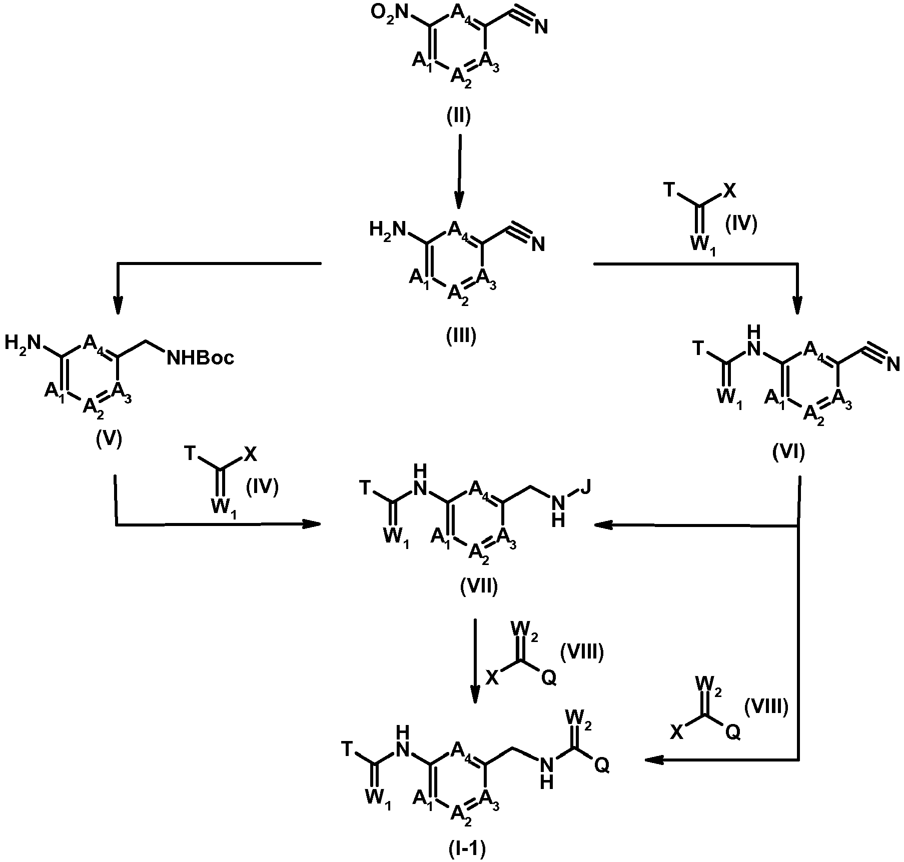

Erfindungsgemäße Verbindungen des Typs **(1-1)** können durch die Umsetzung von Aminen der allgemeinen Struktur **(VII),** mit J gleich Wasserstoff, mit aktivierten Carbonsäurederivaten der allgemeinen Struktur **(VIII)** dargestellt werden. Die Reaktion kann mit oder ohne Lösungsmittel durchgeführt werden. In diesem Schritt kann ebenfalls eine geeignete Base zum Einsatz kommen.

Verdünnungsmittel werden vorteilhaft in einer solchen Menge eingesetzt, dass das Reaktionsgemisch während des ganzen Verfahrens gut rührbar bleibt.

Als Lösungsmittel kann jedes Lösungsmittel verwendet werden, das die Reaktion nicht beeinträchtigt, wie zum Beispiel Wasser. In Frage kommen aromatische Kohlenwasserstoffe wie Benzol oder Toluol; halogenierte Kohlenwasserstoffe wie Dichlormethan, Chloroform oder Tetrachlorkohlenwasserstoff, offenkettige oder zyklische Ether wie Diethylether, Dioxan, Tetrahydrofuran oder 1,2-Dimethoxyethan; Ester wie Ethylacetat und Butylacetat; Ketone wie zum Beispiel Aceton, Methyl-isobutylketon und Cyclohexanon; Amide wie Dimethylformamid und Dimethylacetamid; Nitrile wie Acetonitril; und andere inerte Lösungsmittel wie 1,3-Dimethyl-2-imidazolidinon; die Lösungsmittel können allein oder in Kombination von zwei oder mehr eingesetzt werden.

Als Base kann eine organische Base wie Triethylamin, Ethyl-diisopropylamin, Tri-n-butylamin, Pyridin und 4-Dimethylamino-pyridin verwendet werden; Des Weiteren können beispielsweise folgende Basen eingesetzt werden: Alkalimetallhydroxide wie z.B Natriumhydroxid und Kaliumhydroxid; Carbonate wie Natriumhydrogencarbonat und Kaliumcarbonat; Phosphate wie Dikalium-hydrogenphosphat und Tri-natriumphosphat; Alkalimetallhydride wie Natriumhydrid; Alkalimetallalkoholate wie Natriummethanolat und Natriumethanolat. Diese Basen können in Verhältnissen von 0.01 bis 5.0 Moläquivalenten in Bezug auf **(VII)** und **(VIII)** eingesetzt werden.

Die geeignete Reaktionstemperatur liegt im Bereich von -20°C bis zum Siedepunkt des jeweiligen Lösungsmittels und die Reaktionsdauer liegt je nach Wahl der Reaktanden, Lösungsmittel und Reaktionstemperatur zwischen wenigen Minuten bis 96 Stunden.

Als aktivierte Carbonsäurederivate **(VIII)** mit W² gleich Sauerstoff und Q gemäß den oben beschriebenen Bedeutungen können Carbonsäurehalogenide eingesetzt werden, d.h. **(VIII)** mit X gleich Fluor, Chlor oder Brom. Diese Carbonsäurehalogenide können aus der entsprechenden Carbonsäure **(VIII)** mit X gleich Hydroxy mittels Halogenierungsreagenzien wie Thionylchlorid, Thionylbromid, Phosphorylchlorid, Oxalylchlorid, Phosphortrichlorid, etc. hergestellt werden. [Houben-Weyl, 1952, Bd. VIII, S.463 ff.].

Als aktivierte Carbonsäurederivate **(VIII)** können des Weiteren auch gemischte Anhydride eingesetzt werden [J. Am. Chem. Soc. 1967, 5012]. Bei diesem Verfahren können verschiedene Chlorameisensäureester zum Einsatz kommen, wie z.B. Chlorameisensäureisobutylester, Chlorameisensäureisopropylester. Ebenfalls können dafür Diethylacetylchlorid, Trimethylacetylchlorid und ähnliche verwendet werden.

Die Darstellung von Carboxamiden repräsentiert durch die Formel **(1-1)** kann aber auch durch Reaktion von Aminen **(VII)** (J gleich Wasserstoff) mit Carbonsäuren **(VIII)** (X gleich Hydroxy) unter der Verwendung von Kupplungsreagenzien wie Dicyclohexylcarbodiimid und Additiven wie 1-Hydroxybenzotriazol durchgeführt werden (Chem. Ber. 1970, 788). Verwendbar sind ferner Kupplungsreagenzien wie 1-Ethyl-3-(3-dimethylaminopropyl)carbodiimid, 1,1'-Carbonyl-1H-imidazol und ähnliche Verbindungen.

Als Kupplungsreagenzien zur Durchführung des Dartellungsverfahrens finden alle, die zur Herstellung einer Ester- oder Amidbindung geeignet sind [vgl. z.B. Bodansky et al., Peptide Synthesis, 2nd ed., Wiley & Sons, New York, 1976; Gross, Meienhofer, The Peptide: Analysis, Synthesis, Biology (Academic Press, New York, 1979); Tetrahedron 2005, 61, 10827-10852; Chem. Soc. Rev. 2009, 38, 606-631], Verwendung.

Verbindungen des Typs **(VII),** beziehungsweise die entsprechenden Ammoniumsalze, mit W¹ gleich Sauerstoff, J gleich Wasserstoff und A¹-A⁴ und T gemäß den oben beschriebenen Bedeutungen können gegebenenfalls durch Reduktion des Nitrils (**VI**) mittels in der Literatur beschriebener Methoden hergestellt werden, wie z.B. durch Behandlung mit Boran oder Edelmetall-katalysierter Hydrierung. Alternativ kann die Boc-geschützte Verbindung **(VII)** mit J gleich Boc ausgehend von (**VI**) analog Literaturvorschrift hergestellt werden (Tetrahedron Lett. 2000, 41, 3513-3515; Tetrahedron 2003, 59, 5417-5423). Die freien Amine der Verbindungen des Typs **(VII)** (mit J gleich Wasserstoff), beziehungsweise deren Ammoniumsalze, können ausgehend von der Boc-geschützten Verbindung **(VII)** (mit J gleich Boc) mittels in der Literatur bekannter Methoden hergestellt werden, z. B. durch Behandlung mit Salzsäure oder Trifluoressigsäure.

Verbindungen der allgemeinen Struktur **(VI)** mit W¹ gleich Sauerstoff und A¹-A⁴ und T gemäß den oben beschriebenen Bedeutungen können durch die Umsetzung eines Anilins der allgemeinen Struktur **(III)** mit aktivierten Carbonsäurederivaten der allgemeinen Struktur **(IV)** dargestellt werden. Dabei gelten dieselben Bedingungen für die Wahl des Lösungsmittels, der Reaktionsbedingungen, der Reaktionsdauer und der Reagenzien wie bei der oben beschriebenen Darstellung von **(1-1).**

Als aktivierte Carbonsäurederivate der allgemeinen Struktur **(IV)** mit W¹ gleich Sauerstoff und T gemäß den oben beschriebenen Bedeutungen können Carbonsäurehalogenide eingesetzt werden, d.h. **(IV)** mit X gleich Fluor, Chlor oder Brom. Diese Carbonsäurehalogenide können aus der entsprechenden Carbonsäure **(IV)** mit X gleich Hydroxy mittels Halogenierungsreagenzien wie Thionylchlorid, Thionylbromid, Phosphorylchlorid, Oxalylchlorid, Phosphortrichlorid, etc. hergestellt werden. [Houben-Weyl, 1952, Bd. VIII, S.463 ff.].

Als aktivierte Carbonsäurederivate **(IV)** können des Weiteren auch gemischte Anhydride eingesetzt werden [J. Am. Chem. Soc. **1967,** 5012]. Bei diesem Verfahren können verschiedene Chlorameisensäureester zum Einsatz kommen, wie z.B. Chlorameisensäureisobutylester, Chlorameisensäureisopropylester. Ebenfalls können dafür Diethylacetylchlorid, Trimethylacetylchlorid und ähnliche verwendet werden.

Die Darstellung von Carboxamiden repräsentiert durch die Formel (**VI**) kann aber auch durch Reaktion von Anilinen (**III**) mit Carbonsäuren **(IV)** (X gleich Hydroxy) unter der Verwendung von Kupplungsreagenzien wie Dicyclohexylcarbodiimid und Additiven wie 1-Hydroxybenzotriazol durchgeführt werden.[Chem. Ber. 1970, 788] Verwendbar sind ferner Kupplungsreagenzien wie 1-Ethyl-3-(3-dimethylaminopropyl)carbodiimid, 1,1'-Carbonyl-1*H*-imidazol und ähnliche Verbindungen.

Als Kupplungsreagenzien zur Durchführung des Dartellungsverfahrens finden alle, die zur Herstellung einer Ester- oder Amidbindung geeignet sind (vgl. z. B. Bodansky et al., Peptide Synthesis, 2nd ed., Wiley & Sons, New York, 1976; Gross, Meienhofer, The Peptide: Analysis, Synthesis, Biology (Academic Press, New York, 1979); Tetrahedron 2005, 61, 10827-10852; Chem. Soc. Rev. 2009, 38, 606-631), Verwendung.

Heterocyklische Carbonsäuren **(IV)** mit W¹ gleich Sauerstoff, X gleich Hydroxy und T gemäß den oben beschriebenen Bedeutungen können mittels literaturbekannter Methoden hergestellt werden, vgl. z. B. WO2010/051926.

Alternativ können Boc-geschützte Verbindungen des Typs **(VII),** mit J gleich Boc, durch die Umsetztung eines Anilins der allgemeinen Struktur **(V)** mit aktivierten Carbonsäurederivaten der allgemeinen Struktur **(IV)** dargestellt werden. Dabei gelten dieselben Bedingungen für die Wahl des Lösungsmittels, der Reaktionsbedingungen, der Reaktionsdauer und der Reagenzien wie bei der oben beschriebenen Darstellung von (**VI**).

Verbindungen der allgemeinen Struktur **(V)** mit A¹-A⁴ gemäß den oben beschriebenen Bedeutungen können aus Nitrilen des Typs (**III**) durch Reduktion und Boc-Schützung gemäß literaturbekannter Vorschriften hergestellt werden (Tetrahedron Lett. 2000, 41, 3513-3515; Tetrahedron 2003, 59, 5417-5423).

Verbindungen der allgemeinen Struktur (**III**) mit A¹-A⁴ gemäß den oben beschriebenen Bedeutungen können durch Reduktion der Nitroverbindung mit der allgemeinen Struktur (**II**) hergestellt werden. In der Literatur existieren eine Vielzahl von Methoden für diese Transformation, beispielsweise die Verwendung von Zinnchlorid unter sauren Bedingungen oder die Edelmetall-katalysierte Hydrierung.

Erfindungsgemäße Verbindungen des Typs **(1-1)** können zudem durch Umsetzung von Nitrilen der allgemeinen Struktur (**VI**) mit Reduktionsmittels wie Natriumborhydrid in Anwesenheit aktivierter Carbonsäurederivate **(VIII),** insbesondere Carbonsäureanhydride, hergestellt werden (Tetrahedron Lett. 2000, 41, 3513-3515; Tetrahedron 2003, 59, 5417-5423).
2) Verbindungen des Typs **(1-2),** mit W¹ und W² gleich Sauerstoff und A¹-A⁴, T, Q und R¹ gemäß den oben beschriebenen Bedeutungen, können gegebenenfalls nach dem in Reaktionsschema 2 abgebildeten allgemeinem Darstellungsverfahren B hergestellt werden.

### Reaktionsschema 2: Darstellungsverfahren B

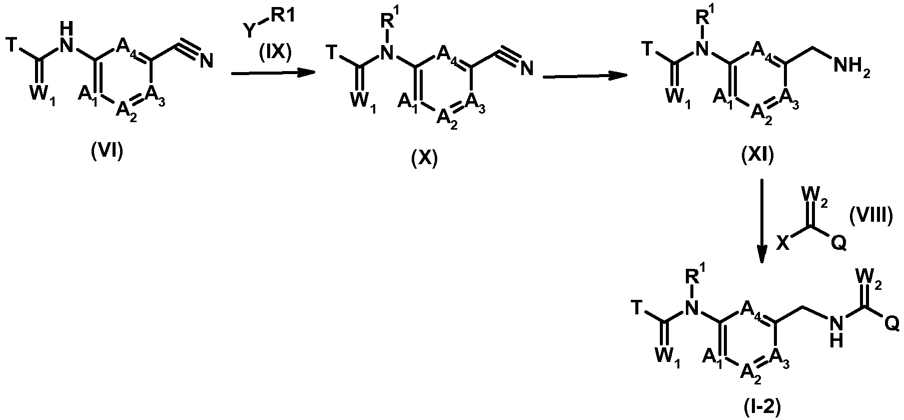

Verbindungen der allgemeinen Struktur **(1-2)** mit W¹ und W² gleich Sauerstoff und A¹-A⁴, R¹ und T gemäß den oben beschriebenen Bedeutungen können durch die Umsetzung eines Amins der allgemeinen Struktur **(XI)** mit aktivierten Carbonsäurederivaten der allgemeinen Struktur (VIII) dargestellt werden. Dabei gelten dieselben Bedingungen für die Wahl des Lösungsmittels, der Reaktionsbedingungen, der Reaktionsdauer und der Reagenzien wie bei der oben beschriebenen Darstellung von **(1-1).**

Verbindungen der allgemeinen Struktur **(XI)** mit W¹ gleich Sauerstoff und A¹-A⁴, R¹ und T gemäß den oben beschriebenen Bedeutungen können aus Nitrilen der allgemeinen Struktur **(X)** hergestellt werden. Dabei gelten dieselben Bedingungen wie bei der oben beschriebenen Darstellung von **(VII)** (J gleich Wasserstoff) aus dem Nitril **(VI)**.

Verbindungen der allgemeinen Struktur **(X)** mit W¹ gleich Sauerstoff und A¹-A⁴, R¹ und T gemäß den oben beschriebenen Bedeutungen können durch Reaktion von Verbindungen des Typs (**VI**) mit Verbindungen des Typs **(IX)** erhalten werden. In Verbindungen der allgemeinen Struktur **(IX)** kommt R¹ die oben beschriebene Bedeutung zu, Y steht für eine gute Abgabgsgruppe, beispielsweise Chlor, Brom, Iod, C₁-C₄-Alkylcarbonyloxy (z.B. Pivaloyl), C₁-C₄-Alkylsulfonat (z.B. Methansulfonyloxy), C₁-C₄-Haloalkylsulfonat (z.B. Trifluormethansulfonyloxy), Arylsulfonat (z.B. p-Toluolsulfonyloxy) oder Azolyl (z.B. Imidazol-1-yl). Gegebenenfalls ist es vorteilhaft, die Reaktion in Gegenwart einer geeigneten Base analog obiger Definition durchzuführen.
3) Verbindungen des Typs **(1-3),** mit W¹ und W² gleich Sauerstoff und A¹-A⁴, T, Q, M¹ gemäß den oben beschriebenen Bedeutungen, können gegebenenfalls nach dem in Reaktionsschema 3 abgebildeten allgemeinem Darstellungsverfahren C hergestellt werden.

### Reaktionsschema 3: Darstellungsverfahren C

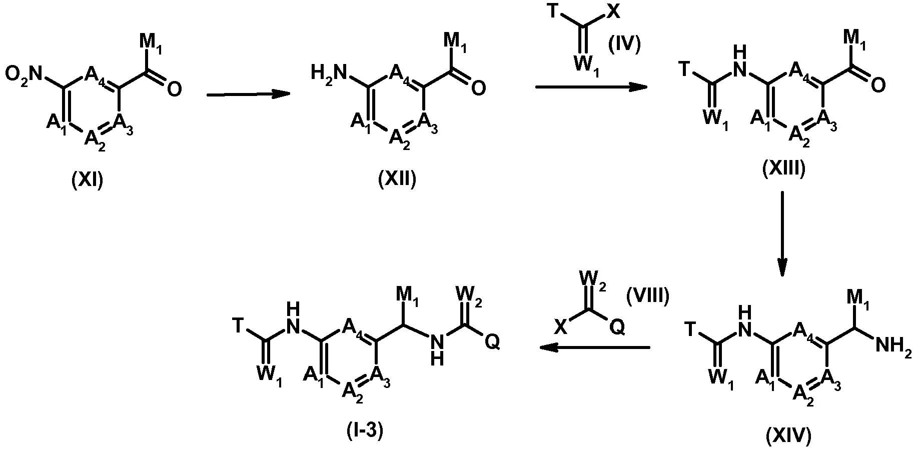

Erfindungsgemäße Verbindungen der allgemeinen Struktur **(1-3)** mit W¹ und W² gleich Sauerstoff und A¹-A⁴, M¹ und T gemäß den oben beschriebenen Bedeutungen können durch die Umsetzung eines Amins der allgemeinen Struktur **(XIV)** mit aktivierten Carbonsäurederivaten der allgemeinen Struktur **(VIII)** dargestellt werden. Dabei gelten dieselben Bedingungen für die Wahl des Lösungsmittels, der Reaktionsbedingungen, der Reaktionsdauer und der Reagenzien wie bei der oben beschriebenen Darstellung von **(1-1).**

Verbindungen der allgemeinen Struktur **(XIV)** mit W¹ gleich Sauerstoff und A¹-A⁴, M¹ und T gemäß den oben beschriebenen Bedeutungen können durch reduktive Aminierung von Verbindungen der allgemeinen Struktur **(XIII)** hergestellt werden. In der Literatur existieren eine Vielzahl von Methoden für diese Transformation, beispielsweise die Verwendung von Natriumcyanoborhydrid und Ammoniumacetat in Methanol (Org. React. 2002, 59, 1-714, Org. Process Res. Dev. 2006, 10, 971-1031).

Verbindungen der allgemeinen Struktur **(XIII)** mit W¹ gleich Sauerstoff und A¹-A⁴, M₁ und T gemäß den oben beschriebenen Bedeutungen können durch die Umsetzung eines Anilins der allgemeinen Struktur **(XII)** mit aktivierten Carbonsäurederivaten der allgemeinen Struktur **(IV)** dargestellt werden. Dabei gelten dieselben Bedingungen für die Wahl des Lösungsmittels, der Reaktionsbedingungen, der Reaktionsdauer und der Reagenzien wie bei der oben beschriebenen Darstellung von **(VI)**.

Aniline der allgemeinen Struktur **(XII)** mit A¹-A⁴ und M₁ gemäß den oben beschriebenen Bedeutungen können durch Reduktion der Nitroverbindung mit der allgemeinen Struktur **(XI)** hergestellt werden. In der Literatur existieren eine Vielzahl von Methoden für diese Transformation, beispielsweise die Verwendung von Zinnchlorid unter sauren Bedingungen oder die Metall-katalysierte Hydrierung.

Hydrierungen können in einem geeigneten Lösungsmittel in Anwesenheit eines Katalysators unter Wasserstoffatmosphäre (Normaldruck oder Hochdruck). Als Katalysatoren können Palladiumkatalysatoren wie z.B. Palladium auf Kohle, Nickelkatalysatoren wie Raney-Nickel, Kobaltkatalysatoren, Rutheniumkatalysatoren, Rhodiumkatalysatoren, Platinkatalysatoren und diesen ähnliche Verbindungen verwendet werden. Geeignete Lösungsmittel sind Wasser, Alkohole wie Methanol und Ethanol, aromatische Kohlenwasserstoffe wie Benzol und Toluol, offenkettige oder zyklische Ether wie Diethylether, Dioxan und Tetrahydrofuran sowie Ester wie Essigsäureethylester. Die Reduktionen können in einem Druckbereich von 1 bar bis 100 bar durchgeführt werden, wobei die Temperatur zwischen -20°C und dem Siedepunkt des eingesetzten Lösungsmittels variieren kann. Je nach Reaktionsbedingungen liegen die Reaktionszeiten zwischen wenigen Minuten und 96 Stunden.

Die Metall-vermittelten Reduktionen wie z.B. mit Zinn(II)chlorid können nach einem in Organic Syntheses Coll. Vol. (III), 453 beschriebenen Verfahren durchgeführt werden.

Zur Deblockierung/Abspaltung der Schutzgruppe SG können alle bekannten geeigneten sauren oder basischen Reaktionshilfsmittel nach den in der Literatur beschriebenen Verfahrensweises verwendet werden. Bei Verwendung von Schutzgruppen für Aminogruppen des Carbamat-Typs werden bevorzugt saure Reaktionshilfsmittel verwendet. Bei Verwendung der tert-Butylcarbamat-Schutzgruppe (BOC-Gruppe) werden beispielsweise Mischungen von Mineralsäuren wie Salzsäure, Bromwasserstoffsäure, Salpetersäure, Schwefelsäure, Phosphorsäure oder organischen Säuren wie Benzoesäure, Ameisensäure, Essigsäure, Trifluoressigsäure, Methansulfonsäure, Benzolsulfonsäure oder Toluolsulfonsäure und einem geeigneten Verdünnungsmittel wie Wasser und/oder einem organischen Lösungsmittel wie Tetrahydrofuran, Dioxan, Dichlormethan, Chloroform, Essigester, Ethanol oder Methanol verwendet. Bevorzugt sind Mischungen von Salzsäure oder Essigsäure mit Wasser und/oder einem organischen Lösungsmittel wie Essigester.

Es ist bekannt, dass manche Reaktionen und Herstellungsverfahren besonders gut in Gegenwart von Verdünnungs- bzw. Lösungsmittel und basischer oder saurer Reaktionshilfsmitteln durchführbar sind. Mischungungen der Verdünnungs- bzw. Lösungsmittel sind ebenfalls einsetzbar. Die Verdünnungs- bzw. Lösungsmittel werden vorteilhafterweise in einer solchen Menge eingesetzt, dass das Reaktionsgemisch während des ganzen Verfahrens gut rührbar ist.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-tert-butylether, n-Butylether, Anisol, Phenetol, Cyclohexylmethylether, Dimethylether, Diethylether, Diproplether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril), Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäuretriamid, Formamid, N-Methyl-formamid, *N,N-*Dimethyl-formamid, *N,N-*Dipropyl-formamid, *N,N-*Dibutyl-formamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, *N,N'*-1,4-Diformyl-piperazin) und Ketone (z.B. Aceton, Acetophenon, Methylethylketon, Methylbutylketon).

Als basische Reaktionshilfsmittel zur Durchführung der erfindungsgemäßen Verfahren können alle geeigneten Säurebindemittel eingesetzt werden. Als Beispiele sind zu nennen: Erdalkali- oder Alkalimetallverbindungen (z.B. Hydroxide, Hydride, Oxide und Carbonate des Lithiums, Na-triums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo(5.4.0)undecen (DBU), Cyclohexyltetrabutyl-guanidin (CyTBG), Cyclohexyltetramethylguanidin (CyTMG), *N,N,N,N-*Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, *N*,*N-*Dimethylanilin, *N,N-*Dimethyl-toluidin, *N,N-*Dimethyl-p-aminopyridin, N-Methyl-pyrrolidin, N-Methylpiperidin, N-Methyl-imidazol, N-Methyl-pyrazol, N-Methyl-morpholin, N-Methylhexamethylendiamin, Pyridin, 4-Pyrrolidinopyridin, 4-Dimethylamino-pyridin, Chinolin, α-Picolin, β-Picolin, Isochinolin, Pyrimidin, Acridin, *N,N,*N',N'-Tetramethylendiamin, *N,N,*N',N'-Tetraethylendiamin, Chinoxalin, N-Propyl-diisopropylamin, N-Ethyl-diisopropylamin, *N,N'*-Dimethylcyclohexylamin, 2,6-Lutidin, 2,4-Lutidin oder Triethyldiamin).

Als saure Reaktionshilfsmittel zur Durchführung der erfindungsgemäßen Verfahren können alle Mineralsäuren (z.B. Halogenwasserstoff-säuren wie Fluorwasserstoffsäure, Chlorwasserstoffsäure, Bromwasserstoffsäure oder Iodwasserstoffsäure sowie Schwefelsäure, Phosphorsäure, Phosphorige Säure, Salpetersäure), Lewis Säuren (z.B. Aluminium(III)-chlorid, Bortrifluorid oder sein Etherat, Titan-(V)chlorid, Zinn(V)-chlorid, und organische Säuren (z.B. Ameisensäure, Essigsäure, Propionsäure, Malonsäure, Milchsäure, Oxalsäure, Fumarsäure, Adipinsäure, Stearinsäure, Weinsäure, Ölsäure, Methansulfonsäure, Benzoesäure, Benzolsulfonsäure oder para-Toluolsulfonsäure eingesetzt werden.

Sofern in den Reaktionsschemata Schutzgruppen vorgesehen sind, können alle allgemein bekannten Schutzgruppen verwendet werden. Insbesondere solche, die von Greene T. W., Wuts P. G. W. in Protective Groups in Organic Synthesis; John Wiley & Sons, Inc. 1999, "Protection for the hydroxyl group including 1,2- and 1,3-diols" beschrieben sind.

Weiterhin eignen sich auch Schutzgruppen
vom Typ eines substituierten Methylethers (z.B. Methoxymethylether (MOM), Methylthiomethylether (MTM), (Phenyl-dimethylsilyl)methoxymethylether (SNOM-OR), Benzyloxymethylether (BOM-OR) para-Methoxybenzyloxymethylether (PMBM-OR), para-Nitrobenzyloxymethyl-ether, ortho-Nitrobenzyloxymethylether (NBOM-OR), (4-Methoxyphenoxy)-methylether (p-AOM-OR), Guaiacolmethylether (GUM-OR), tert-Butoxymethylether, 4-Pentyloxy-methylether (POM-OR), Silyloxymethylether, 2-Methoxy-ethoxy-methylether (MEM-OR), 2,2,2-Trichlorethoxymethylether, Bis(2-chlorethoxy)-methylether, 2-(Trimethyl-silyl)ethoxymethylether (SEM-OR), Methoxymethylether (MM-OR));
vom Typ eines substituierten Ethylethers (z.B. 1-Ethoxyethylether (EE-OR), 1-(2-Chlorethoxy)ethylether (CEE-OR), 1-[2-(Trimethylsilyl)ethoxy]ethylether (SEE-OR), 1-Methyl-1-methoxyethylether (MIP-OR), 1-Methyl-1-benzyloxyethylether (MBE-OR), 1-Methyl-1-benzyloxy-2-fluor-ethylether (MIP-OR), 1-Methyl-1-phenoxyethylether, 2,2,-Trichlorethylether, 1,1-Dianisyl-2,2,2-trichlorethylether (DATE-OR), 1,1,1,3,3,3-Hexafluor-2-phenylisopropylether (HIP-OR), 2-Trimethylsilylethylether, 2-(Benzylthio)ethylether, 2-(Phenylselenyl)ethylether), eines Ethers (z.B. Tetrahydropyranylether (THP-OR), 3-Brom-tetrahydropyranylether (3-BrTHP-OR), Tetrahydrothiopyranylether, 1-Methoxy-cyclohexylether, 2- und 4-Picolylether, 3-Methyl-2-picolyl-N-oxido-ether, 2-Quinolinylmethylether (Qm-OR), 1-Pyrenylmethylether, Dipenylmethylether (DPM-OR), para, para'-Dinitrobenzhydrylether (DNB-OR), 5-Dibenzosuberylether, Triphenylmethylether (Tr-OR), alpha-Naphthyldiphenylmethylether, para-Methoxy-phenyldiphenylmethylether (MMTrOR), Di(para-methoxy-phenyl)phenylmethylether (DMTr-OR), Tri(para-methoxy-phenyl)phenylmethylether (TMTr-OR), 4-(4'-Brom-phenacyloxy) phenyldiphenylmethylether, 4,4',4"-Tris(4,5-dichlorphthalimido-phenyl)methylether (CPTr-OR), 4,4',4"-Tris(benzoyloxyphenyl)-methylether (TBTr-OR), 4,4'-Dimethoxy-3"-[N-(imidazolylmethyl)]-tritylether (IDTr-OR), 4,4'-Dimethoxy-3"-[N-(imidazolylethyl)carbamoyl]tritylether (IETr-OR), 1,1-Bis(4-methoxy-phenyl)-1'-pyrenyl-methylether (Bmpm-OR), 9-Anthrylether, 9-(9-Phenyl)xanthenylether (Pixyl-OR), 9-(9-Phenyl-10-oxo)anthryl (Tritylon-Ether), 4-Methoxy-tetrahydropyranylether (MTHP-OR), 4-Methoxy-tetrahydrothiopyranylether, 4-Methoxy-tetrahydrothiopyranyl-S,S-dioxid, 1-[(2-Chlor-4-methyl)phenyl]-4-methoxypiperidin-4-yl-ether (CTMP-OR), 1-(2-Fluorphenyl)-4-methoxy-piperidin-4-yl-ether (Fpmp-OR), 1,4-Dioxan-2-yl-ether, Tetrahydrofuranylether, Tetrahydrothiofuranylether, 2,3,3a,4,5,6,7,7a-Octahydro-7,8,8-trimethyl-4,7-methanbenzofuran-2-yl-ether (MBF-OR), tert-Butylether, Allylether, Propargylether, para-Chlorphenylether, para-Methoxy-phenylether, para-Nitro-phenylether, para-2,4-Dinitro-phenylether (DNP-OR), 2,3,5,6-Tetrafluor-4-(trifluormethyl)phenylether, Benzylether (Bn-OR));
vom Typ eines sustituierten Benzylethers (z.B. para-Methoxy-benzylether (MPM-OR), 3,4-Dimethoxybenzylether (DMPM-OR), ortho-Nitro-benzylether, para-Nitro-benzylether, para-Halo-benzylether, 2,6-Dichlor-benzylether, para-Aminoacyl-benzylether (PAB-OR), para-Azido-benzylether (Azb-OR), 4-Azido-3-chlor-benzylether, 2-Trifluormethyl-benzylether, para-(Methylsulfinyl)benzylether (Msib-OR));
vom Typ eines Silylethers (z.B. Trimethylsilylether (TMS-OR), Triethylsilylether (TES-OR), Triisopropylsilylether (TIPS-OR), Dimethylisopropylsilylether (IPDMS-OR), Diethylisopropylsilylether (DEIPS-OR), Dimethylhexylsilylether (TDS-OR), tert-Butyldimethylsilylether (TBDMS-OR), tert-Butyldiphenylsilylether(TBDPS-OR), Tribenzylsilylether, Tri-para-xylylsilylether, Triphenylsilylether (TPS-OR), Diphenylmethylsilylether (DPMS-OR), Di-tert-butylmethylsilylether (DTBMS-OR), Tris(trimethylsilyl)silylether (Sisylether), Di-tert-butylmethylsilylether (DTBMS-OR), Tris(trimethylsilyl)silylether (Sisylether), (2-Hydroxystyryl)-dimethylsilylether (HSDMS-OR), (2-Hydroxystyryl)diisopropylsilylether (HSDIS-OR), tert-Butylmethoxyphenyl-silylether (TBMPS-OR), tert-Butoxydiphenylsilylether (DPTBOS-OR));
vom Typ eines Esters (z.B. Formiatester, Benzoylformiatester, Acetatester (Ac-OR), Chloracetatester, Dichloracetatester, Trichloracetatester, Trifluoracetatester, (TFA-OR), Methoxyacetatester, Triphenylmethoxyacetatester, Phenoxyacetatester, para-Chlor- phenoxyacetatester, Phenylacetatester, Diphenylacetatester (DPA-OR), Nicotinatester, 3-Phenyl-propionatester, 4-Pentoatester, 4-Oxopentoatester (Levulinate) (Lev-OR) 4,4-(Ethylendithio)-pentanoatester (LevS-OR), 5-[3-Bis(4-methoxyphenyl)hydroxy-methoxyphenoxy]-levulinatester, Pivaloatester (Pv-OR), 1-Adamantanoatester, Crotonatester, 4-Methoxy-crotonatester, Benzoatester (Bz-OR), para-Phenyl-benzoatester, 2,4,6-Trimethyl-benzoatester (Mesitoate), 4-(Methylthiomethoxy)-butyratester (MTMB-OR), 2-(Methylthiomethoxymethyl)-benzoatester (MTMT-OR),
vom Typ eines Esters (z.B. Methylcarbonat, Methoxymethylcarbonat, 9-Fluorenylmethylcarbonat (Fmoc-OR), Ethylcarbonat, 2,2,2-Trichlorethylcarbonat (Troc-OR), 1,1-Dimethyl-2,2,2-trichlorethylcarbonat (TCBOC-OR), 2-(Trimethylsilyl)ethylcarbonat (TMSEC-OR), 2-(Phenylsulfonyl)-ethylcarbonat (Psec-OR), 2-(Triphenylphosphonio)-ethylcarbonat (Peoc-OR), tert-Butylcarbonat (Boc-OR), Isobutylcarbonat, Vinylcarbonat, Allylcarbonat (Alloc-OR), para-Nitro-phenylcarbonat, Benzylcarbonat (Z-OR), para-Methoxy-benzylcarbonat, 3,4-Dimethoxy-benzylcarbonat, ortho-Nitro-benzylcarbonat, para-Nitro-benzylcarbonat, 2-Dansylethylcarbonat (Dnseoc-OR), 2-(4-Nitrophenyl)ethylcarbonat (Npeoc-OR), 2-(2,4-Dinitrophenyl)ethylcarbonat (Dnpeoc)), und
vom Typ eines Sulfats (z.B. Allylsulfonat (Als-OR), Methansulfonat (Ms-OR), Benzylsulfonat, Tosylat (Ts-OR), 2-[(4-Nitrophenyl)ethyl]sulfonat (Npes-OR)).

Als Katalysatoren zur Durchführung einer katalytischen Hydrierung im erfindungsgemäßen Verfahren sind alle üblichen Hydrierkatalysatoren, wie beispielsweise Platin-Katalysatoren (z.B. Platin-Platte, Platin-Schwamm, Platin-Schwarz, kolloidales Platin, Platinoxid, Platindraht), Palladium-Katalysatoren (z.B. Palladium-Schwamm, Palladium-Schwarz, Palladiumoxid, Palladium-Kohle, kolloidales Palladium, Palladium-Bariumsulfat, Palladium-Bariumcarbonat, Palladium-Hydroxid , NickelKatalysatoren (z.B. reduziertes Nickel, Nickeloxid, Raney-Nickel), Ruthenium-Katalysatoren, Cobalt-Katalysatoren (z.B. reduziertes Cobalt, Raney-Cobalt), Kupfer-Katalysatoren (z.B. reduziertes Kupfer, Raney-Kupfer, Ullmann-Kupfer) geeignet. Bevorzugt werden Edelmetalllkatalysatoren (z.B. Platin- und Palladium- oder Ruthenium-Katalysatoren) verwendet, die gegebenenfalls auf einem geeigneten Träger (z.B. Kohlenstoff oder Silizium) aufgebraucht sind, Rhodium-Katalysatoren (z.B. Tris(triphenylphosphin)rhodium(I)-chlorid in Gegenwart von Triphenylphosphin). Ferner können "chiralen Hydrierkatalysatoren" (z. B. solche die chirale Diphosphinliganden enhalten wie (2S,3S)-(-)-2,3-Bis(diphenylphosphino)-butan [(S,S)-Chiraphos] oder (R)-(+)-2,2'- bzw. (S)-(-)-2,2'-Bis(diphenylphosphino)-1,1'-binaphthalin [R(+)-BINAP bzw. S(-)-BINAP]) verwendet werden, wodurch der Anteil eines Isomers im Isomerengemisch erhöht wird bzw. das Entstehen eines anderen Isomers nahezu vollständig unterdrückt wird.

Die Herstellung von Salzen der erfindungsgemäßen Verbindungen erfolgt nach Standardverfahren. Repräsentative Säureadditionssalze sind beispielsweise solche die durch Reaktion mit anorganischen Säuren, wie beispielsweise Schwefelsäure, Salzsäure, Bromwasserstoffsäure, Phosphorsäure oder organischen Carbonsäuren wie Essigsäure, Trifluoressigsäure, Zitronensäure, Bernsteinsäure, Buttersäure, Milchsäure, Ameisensäure, Fumarsäure, Maleinsäure, Malonsäure, Camphersäure, Oxalsäure, Phthalsäure, Propionsäure, Glycolsäure, Glutarsäure, Stearinsäure, Salicylsäure, Sorbinsäure, Weinsäure, Zimtsäure, Valeriansäure, Pikrinsäure, Benzoesäure oder organischen Sulfonsäuren wie Methansulfonsäure und 4-Toluolsulfonsäure gebildet werden.

Repräsentativ sind auch Salze von erfindungsgemäßen Verbindungen, die aus organischen Basen, wie beispielsweise Pyridin oder Triethylamine gebildet werden oder solche, die aus anorganischen Basen, wie beispielsweise Hydride, Hydroxide oder Karbonate des Natriums, Lithiums, Calciums, Magnesiums oder Bariums, gebildet werden, wenn die Verbindungen der allgemeinen Formel (I) ein zu dieser Salzbildung geeignetes Strukturelement aufweist.

### Experimenteller Teil - Beispiele

Die im Folgenden aufgeführten Beispiele dienen zur exemplarischen Darlegung der Darstellungsverfahren und nicht zur Einschränkung der erfindungsgemäßen Ansprüche.

### Darstellungsverfahren A

### Beispiel 1: N-{3-[(Butyrylamino)methyl]-4-chlorphenyl}-1-methyl-3-(pentafluorethyl)-4-(trifluormethyl)-1H-pyrazol-5-carboxamid (Am-41)

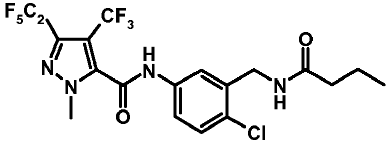

Zu einer Lösung aus [2-Chlor-5-({[1-methyl-3-(pentafluorethyl)-4-(trifluormethyl)-1*H-*pyrazol-5-yl]carbonyl}amino)phenyl]methanaminiumchlorid (100 mg, 0,21 mmol) und Pyridin (34 mg, 0,43 mmol, 2,1 Äq.) in THF (3 ml) gab man Butanoylchlorid (24 mg, 0,23 mmol, 1,1 Äq.) in THF (1,5 ml). Nach 20 h bei Raumtemperatur wurde im Vakuum eingeengt und per Säulenchromatographie an Kieselgel (Hexan/Ethylacetat) zu *N-*{3-[(Butyrylamino)methyl]-4-chlorphenyl}-1-methyl-3-(pentafluorethyl)-4-(trifluormethyl)-1*H-*pyrazol-5-carboxamid (80 mg, 75%) gereinigt.
¹H-NMR (300 MHz, CD₃OD): δ = 0,97 (t, 3H), 1,62-1,74 (m, 2H), 2,26 (t, 2H), 4,00 (s, 3H), 4,84 (s, 2H), 7,38-7,42 (m, 1H), 7,53-7,57 (m, 1H), 7,73 (m, 1H) ppm.
HPLC-MS^{a)}: logP = 3,70, Masse (m/z) = 521 [M+H]⁺.

### Beispiel 2: [2-Chlor-5-({[1-methyl-3-(pentafluorethyl)-4-(trifluormethyl)-1H-pyrazol-5-yl]carbonyl}amino)phenyl]methanaminiumchlorid (Vorstufe)

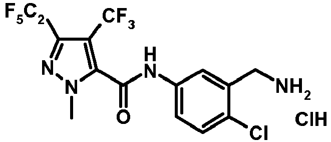

Zu *tert*-Butyl-[2-chlor-5-({[1-methyl-3-(pentafluorethyl)-4-(trifluormethyl)-1*H*-pyrazol-5-yl]carbonyl}amino)benzyl]carbamat (924 mg, 1,68 mmol) in 1,4-Dioxan (2 ml) gab man HCl (16,7 ml einer 4N Lösung in 1,4-Dioxan) und beließ 5 h bei Raumtemperatur. Es wurde im Vakuum eingeengt, mit Ethylacetat gewaschen und man erhielt [2-Chlor-5-({[1-methyl-3-(pentafluorethyl)-4-(trifluormethyl)-1*H*-pyrazol-5-yl]carbonyl}amino)phenyl]methanaminiumchlorid (820 mg, >95%) als weißen Feststoff.
¹H-NMR (300 MHz, d₆-DMSO): δ = 4,03 (s, 3H), 4,12 (s, 2H), 7,59 (d, 1H), 7,74 (d, 1H), 7,98 (s, 1H), 8,61 (br. s, 3H), 11,79 (s, 1H) ppm.

### Beispiel 3: tert-Butyl[2-chlor-5-({[1-methyl-3-(pentafluorethyl)-4-(trifluormethyl)-1H-pyrazol-5-yl]carbonyl}amino)benzyl]carbamat(Am-74)

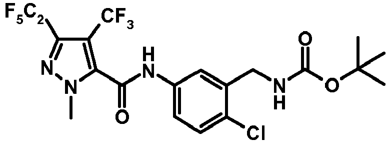

Zu 1-Methyl-3-(pentafluorethyl)-4-(trifluormethyl)-1*H-*pyrazol-5-carbonsäure (1,28 g, 4,1 mmol; Darstellung gemäß WO2010/051926) und *tert*-Butyl-(5-amino-2-chlorbenzyl)carbamat (1,05 g, 4,1 mmol, 1,0 Äq.) in THF (15 ml) gab man 4-(4,6-Dimethoxy-l,3,5-triazin-2-yl)-4-methylmorpholin-4-iumchlorid (=DMTMM, siehe Tetrahedron 1999, 55, 13159-13170) (1,25 g, 4,51 mmol, 1,1 Äq.) und beließ 18 h bei Raumtemperatur. Es wurde mit Wasser (30 ml) versetzt, mit Ethylacetat (3x30 ml) extrahiert und über MgSO4 getrocknete. Nach Einengen im Vakuum reinigte man per Säulenchromatographie an Kieselgel (Hexan/Ethylacetat) und erhielt *tert*-Butyl-[2-chlor-5-({[1-methyl-3-(pentafluorethyl)-4-(trifluormethyl)-1*H*-pyrazol-5-yl]carbonyl}amino)benzyl]carbamat (924 mg, 41%).
¹H-NMR (300 MHz, d₆-DMSO): δ = 1,41 (s, 9H), 4,00 (s, 3H), 4,19 (d, 2H), 7,45-7,48 (m, 2H), 7,58-7,66 (m, 2H), 11,32 (s, 1H) ppm.

### Beispiel 4: tert-Butyl-amino-2-chlorbenzyl)carbamat

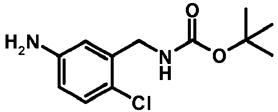

Zu einer Lösung von 5-Amino-2-chlorbenzonitril (1,05 g, 6,88 mmol) und NiCl₂·6H₂O (250 mg, 1,05 mmol, 0,15 Äq.) in Methanol (25 ml) gab man bei 0°C Di-*tert*-butyldicarbonat (1,8 g, 8,3 mmol, 1,2 Äq.). Innerhalb 10 min wurde portionsweise Natriumborhydrid (1,82 g, 48,2 mmol, 7,0 Äq.) zugegeben und anschl. auf Raumtemp. erwärmt. Nach 3 h wurde Diethylentriamin (3,7 ml, 3,5 g, 34 mmol, 5,0 Äq.) zugegeben und 30 min belassen. Es wurde im Vakuum eingeengt, mit ges. wäßr. Natriumhydrogencarbonatlösung versetzt, mit Ethylacetat extrahiert, die vereinigten organischen Phasen mit ges. wäßr. Natriumhydrogencarbonatlösung gewaschen und über MgSO₄ getrocknet. Nach Einengen im Vakuum wurde per Säulenchromatographie an Kieselgel (Hexan/Ethylacetat) zu *tert*-Butyl-(5-amino-2-chlorbenzyl)carbamat (1,06 g, 60%) gereinigt.
¹H-NMR (300 MHz, CDCl₃): δ = 1,45 (s, 9H), 3,68 (br. s, 2H), 4,29 (br. d, 2H), 4,98 (br. s, 1H), 6,52 (dm, 1H), 6,69 (m, 1H), 7,09 (d, 1H) ppm.

### Beispiel 5: 5-Amino-2-chlorbenzonitril

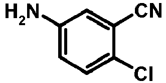

Zu 2-Chlor-5-nitrobenzonitril (3,38 g, 18,5 mmol) und SnCl₂·2H₂O (18,9 g, 83,9 mmol, 4,5 Äq.) in *iso*Propanol (35 ml) gab man tropfenweise konz. HCl (15,6 ml). Es wurde 2 h bei 120°C erhitzt, anschl. auf Raumtemperatur gekühlt und mit wässr. NaOH basisch gestellt. Man extrahierte mit Dichlormethan, trocknete über Na₂SO₄ und filtrierte durch Kieselgel. Nach Einengen im Vakuum erhielt man 5-Amino-2-chlorbenzonitril (2,58 g, 91%).
¹H-NMR (300 MHz, CDCl₃): δ = 3,90 (br. s, 2H), 6,79 (dm, 1H), 6,91 (m, 1H), 7,22-7,26 (m, 1H) ppm.

### Darstellungsverfahren B

### Beispiel 6: N-[3-(Acetamidomethyl)-2,4-difluorphenyl]-N,1-dimethyl-3-pentafluorethyl)-4-(trifluormethyl)-1H-pyrazol-5-carboxamid (Am-7)

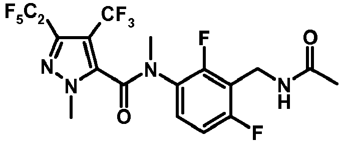

Zu einer Lösung aus [2,6-Difluor-3-(methyl{[1-methyl-3-(pentafluorethyl)-4-(trifluormethyl)-1H pyrazol-5-yl]carbonyl}amino)phenyl]methanaminiumchlorid (130 mg, 0,26 mmol) und Pyridin (43 mg, 0,54 mmol, 2,1 Äq.) in THF (2,5 ml) gab man Essigsäureanhydrid (34 mg, 0,34 mmol, 1,3 Äq) in THF (1 ml) und beließ 20 h bei Raumtemperatur. Nach Einengen im Vakuum reinigte man per Säulen-Chromatographie (Hexan/Ethylacetat) zu *N*-[3-(Acetamidomethyl)-2,4-difluorphenyl]-*N*,1-dimethyl-3-(pentafluorethyl)-4-(trifluormethyl)-1*H-*pyrazol-5-carboxamid (131 mg, 99%).
¹H-NMR (300 MHz, CD₃OD): δ = aufgrund von Rotameren nicht interpretierbar
HPLC-MS^{a)}: logP = 2,85, Masse (m/z) = 509 [M+H]⁺.

### Beispiel 7: [2,6-Difluor-3-(methyl{[1-methyl-3-(pentafluorethyl)-4-(trifluormethyl)-1H-pyrazol-5-yl]carbonyl}amino)phenyl]methanaminiumchlorid

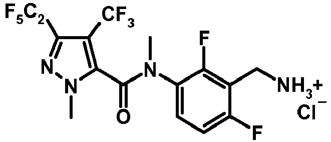

Eine Lösung aus *tert*-Butyl-[2,6-difluor-3-(methyl{[1-methyl-3-(pentafluorethyl)-4-(trifluormethyl)-1*H-*pyrazol-5-yl]carbonyl}amino)benzyl]carbamat (1,2 g, 2,1 mmol) in HCl (21 ml einer 4N Lösung in 1,4-Dioxan, 40 Äq.) wurde 6,5 h bei Raumtemperatur gerührt und anschl. im Vakuum eingeent. Man erhielt [2,6-Difluor-3-(methyl{[1-methyl-3-(pentafluorethyl)-4-(trifluormethyl)-1*H*-pyrazol-5-yl]carbonyl}amino)phenyl]methanaminiumchlorid (954 mg, 89%), welches ohne weitere Aufreinigung weiter umgesetzt wurde.
HPLC-MS^{a)}: Masse (m/z) = 467 [M-Cl]⁺.

### Beispiel 8: tert-Butyl-[2,6-difluor-3-(methyl{[1-methyl-3-(pentafluorethyl)-4-(trifluormethyl)-1H pyrazol-5-yl]carbonyl}amino)benzyl]carbamat(Am-108)

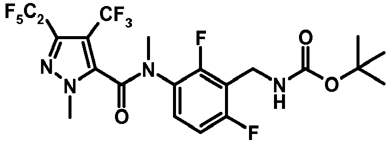

Zu einer Lösung aus *N*-(3-Cyan-2,4-difluorphenyl)-*N*,1-dimethyl-3-(pentafluorethyl)-4-(trifluormethyl)-1*H-*pyrazol-5-carboxamid (1,30g, 2,81 mmol) und NiCl₂·6H₂O (102 mg, 0,43 mmol, 0,15 Äq.) in Methanol (25 ml) gab man bei 0°C Di-*tert*-butyldicarbonat (737 mg, 3,38 mmol, 1,2 Äq.). Innerhalb 5 min wurde portionsweise Natriumborhydrid (0,74 g, 19,7 mmol, 7,0 Äq.) zugegeben und anschl. auf Raumtemp. erwärmt. Nach 6 h wurde Diethylentriamin (1,5 ml, 1,5 g, 14 mmol, 5,0 Äq.) zugegeben und 10 min belassen. Es wurde im Vakuum eingeengt, mit ges. wäßr. Natriumhydrogencarbonatlösung versetzt, mit Ethylacetat extrahiert, die vereinigten organischen Phasen mit ges. wäßr. Natriumhydrogencarbonatlösung gewaschen und über MgSO₄ getrocknet. Es wurde über Kieselgel filtriert und man erhielt *tert*-Butyl-[2,6-difluor-3-(methyl{[1-methyl-3-(pentafluorethyl)-4-(trifluormethyl)-1*H*-pyrazol-5-yl]carbonyl}amino)benzyl]carbamat (1,2 g, 76%), welches ohne weitere Aufreinigung umgesetzt wurde.
¹H-NMR (300 MHz, CDCl₃): δ = 1,42 (s, 9H), 1,44 (s, 3H), 3,46 (s, 3H), 3,93 (br. 2, 2H), 4,38 (br. s, 1H), 7,25-7,26 (m, 2H) ppm.
HPLC-MS^{a)}: Masse (m/z) = 511 [M-*t*Bu]⁺.

### Beispiel 9: N-(3-Cyan-2,4-difluorphenyl)-N,1-dimethyl-4-pentafluorethyl)-4-(trifluormethyl)-1H-pyrazol-5-carboxamid

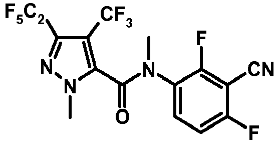

Zu einer Lösung aus *N*-(3-Cyan-2,4-difluorphenyl)-1-methyl-3-(pentafluorethyl)-4-(trifluormethyl)-1*H-*pyrazol-5-carboxamid (1,25 g, 2,79 mmol) in DMF (4 ml) gab man Kaliumcarbonat (1,28 g, 5,58 mmol, 2,0 Äq.) und Iodmethan (0,5 ml, 1,2 g, 8,4 mmol, 3,0 Äq.). Nach 5h wurde mit Wasser versetzt, mit Ethylacetat extrahiert, mit ges. wäßr. Kochsalzlösung gewaschen und über MgSO₄ getrocknete. Nach Filtration über Kieselgel erhielt man *N*-(3-Cyan-2,4-difluorphenyl)-*N*,1-dimethyl-3-(pentafluorethyl)-4-(trifluormethyl)-1*H*-pyrazol-5-carboxamid (1,27 g, 98%) als Gemisch von Rotameren, welches ohne weitere Aufreinigung weiter umgesetzt wurde.
¹H-NMR (300 MHz, CDCl₃): δ = 3,25 (s, 3H), 3,49 (s, 3H'), 3,99 (s, 3H), 4,02 (s, 3H'), 7,18 (m, 1H), 7,61 (m, 1H) ppm.

### Beispiel 10: N-(3-Cyan-2,4-difluorphenyl)-1-methyl-3-(pentafluorethyl)-4-(trifluormethyl)-1H-pyrazol-5-carboxamid

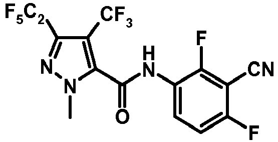

Zu 1-Methyl-3-(pentafluorethyl)-4-(trifluormethyl)-1*H*-pyrazol-5-carbonsäure (2,03 g, 6,50 mmol) in Dichlormethan (15 ml) gab man Oxalylchlorid (0,85 ml, 1,2 g, 9,8 mmol) gefolgt von DMF (3 Tropfen) und erhitze auf 50°C. Nach 5 h bei 50°C wurde im Vakuum eingeengt, mit THF (15 ml) und Pyridin (1,1 ml, 1,0 g, 13 mmol, 2 Äq.) versetzt, gefolgt von 3-Amino-2,6-difluorbenzonitril (1,0 g, 6,5 mmol, 1,0 Äq.) in THF (3 ml).Man beließ 24 h bei Raumtemperatur, versetzte mit HCl (1N wässr. Lösung), extrahierte mit Ethylacetat und wusch mit HCl (1N wässr. Lösung) und NaOH (2N wässr. Lösung). Es wurde über MgSO₄ getrocknet und durch Kieselgel filtriert. Nach Einengen im Vakuum reinigte man per Säulenchromatographie an Kieselgel (Hexan/Ethylacetat) und erhielt *N*-(3-Cyan-2,4-difluorphenyl)-1-methyl-3-(pentafluorethyl)-4-(trifluormethyl)-1*H*-pyrazol-5-carboxamid (1,29 g, 44%).
¹H-NMR (300 MHz, d₆-DMSO): δ = 4,06 (s, 3H), 7,53 (tm, 1H), 8,30 (qm, 1H), 11,46 (s, 1H) ppm.
HPLC-MS^{a)}: Masse (m/z) = 447 [M-H]⁺.

### Beispiel 11: 1-Methyl-N-{4-methyl-3-[(propionylamino)methyl]phenyl}-3-(pentafluorethyl)-4-(trifluormethyl)-1H-pyrazol-5-carboxamid (Am-68)

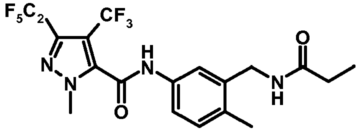

Zu einer Lösung aus *N*-(3-Cyan-4-methylphenyl)-1-methyl-3-(pentafluorethyl)-4-(trifluormethyl)-1*H-*pyrazol-5-carboxamid (221 mg, 0,52 mmol) und NiCl₂·6H₂O (16 mg, 0,07 mmol, 0,13 Äq.) in Methanol (5 ml) gab man bei 0°C Propionäureanhydrid (101 mg, 0,778 mmol, 1,5 Äq.). Es wurde portionsweise Natriumborhydrid (137 mg, 3,63 mmol, 7,0 Äq.) zugegeben und anschl. auf Raumtemp. erwärmt. Nach 3,5 h wurde Diethylentriamin (0,3 ml, 0,3 g, 2,6 mmol, 5,0 Äq.) zugegeben und 10 min belassen. Es wurde im Vakuum eingeengt, mit ges. wäßr. Natriumhydrogencarbonatlösung versetzt, mit Ethylacetat extrahiert, die vereinigten organischen Phasen mit NaOH (1N wässr. Lösung) und HCl (2N wässr. Lösung) gewaschen und über MgSO₄ getrocknet. Nach Einengen im Vakuum reinigte man per Säulen-Chromatographie an Kieselgel (Hexan/Ethylacetat) zu 1-Methyl-*N*-{4-methyl-3-[(propionylamino)methyl]phenyl}-3-(pentafluorethyl)-4-(trifluormethyl)-1*H*-pyrazol-5-carboxamid (75 mg, 30%).
¹H-NMR (300 MHz, CDCl₃): δ = 1,20 (t, 3H), 2,23 (q, 2H), 2,31 (s, 3H), 4,00 (s, 3H), 4,40 (d, 2H), 6,58 (br. s, 1H), 7,16 (d, 1H), 7,50 (dm, 1H), 7,67 (s, 1H), 10,47 (s, 1H) ppm.
HPLC-MS^{a)}: Masse (m/z) = 487 [M+H]⁺.

### Darstellungsverfahren C

### Beispiel 12: N-{4-Chlor-3-[1-(propionylamino)ethyl]phenyl}-1-methyl-3-(pentafluorethyl)-4-(trifluormethyl)-1H-pyrazol-5-carboxamid (Am-28)

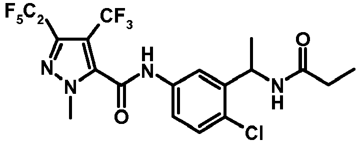

Zu einer Lösung aus *N*-[3-(1-Aminoethyl)-4-chlorphenyl]-1-methyl-3-(pentafluorethyl)-4-(trifluormethyl)-1*H-*pyrazol-5-carboxamid (80 mg, 0,17 mmol) und Pyridin (18 mg, 0,22 mmol, 1,3 Äq.) in THF (3 ml) gab man Propionsäurechlorid (21 mg, 0,22 mmol, 1,3 Äq.) in THF (1 ml). Nach 28 h bei Raumtemperatur wurde im Vakuum eingeengt und per Säulen-Chromatographie (Hexan/Ethylacetat) zu N-{4-Chlor-3-[1-(propionylamino)ethyl]phenyl}-1-methyl-3-(pentafluorethyl)-4-(trifluormethyl)-1*H-*pyrazol-5-carboxamid (80 mg, 89%) gereinigt.
¹H-NMR (300 MHz, d₆-DMSO): δ = 0,98 (t, 3H), 1,32 (d, 3H), 2,18 (q, 2H), 4,01 (s, 3H), 5,09 (quint, 1H), 7,44 (d, 1H), 7,53 (dm, 1H), 7,71 (m, 1H), 8,45 (br. s, 1H), 11,30 (s, 1H) ppm.
HPLC-MS^{a)}: logP = 3,55; Masse (m/z) = 521 [M+H]⁺.

### Beispiel 13: N-[3-(1-Aminoethyl)-4-chlorphenyl]-1-methyl-3-pentafluorethyl)-4-(trifluormethyl)-1H-pyrazol-5-carboxamid

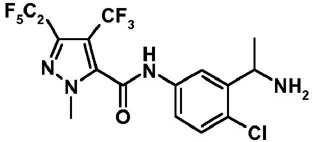

Zu einer Lösung aus *N*-(3-Acetyl-4-chlorphenyl)-1-methyl-3-(pentafluorethyl)-4-(trifluormethyl)-1*H-*pyrazol-5-carboxamid (400 mg, 0,863 mmol) und Ammoniumacetat (1,33 g, 17,3 mmol, 20 Äq.) in Methanol (15 ml) gab man Natriumcyanoborhydrid (163 mg, 2,59 mmol, 3,0 Äq.). Es wurde 4 h bei Raumtemperatur gerührt, anschl. auf 80°C erhitzt und 3 h bei dieser Temperatur belassen. Man kühlte auf Raumtemp. ab und beließ über Nacht. Es wurde mit ges. wässr. Kochsalzlösung versetzt, mit Ethylacetat extrahiert, mit ges. wässr. Kochsalzlösung gewaschen und über MgSO₄ getrocknet. Nach Einengen im Vakuum reinigte man per Säulen-Chromatographie (Hexan/Ethylacetat) zu *N*-[3-(1-Aminoethyl)-4-chlorphenyl]-1-methyl-3-(pentafluorethyl)-4-(trifluormethyl)-1*H-* pyrazol-5-carboxamid (186 mg, 46%).
¹H-NMR (300 MHz, CDCl₃): δ = 1,39 (d, 3H), 1,61 (br. s, 2H), 4,10 (s, 3H), 4,55 (q, 1H), 7,36 (d, 1H), 7,51 (dm, 1H), 7,73 (m, 1H), 7,92 (br. s, 1H) ppm.
HPLC-MS^{a)}: Masse (m/z) = 463 [M-H]⁺.

### Beispiel 14: N-(3-Acetyl-4-chlorphenyl)-1-methyl-3-(pentafluorethyl)-4-(trifluormethyl)-1H-pyrazol-5-carboxamid

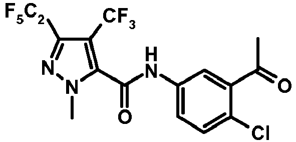

Zu 1-Methyl-3-(pentafluorethyl)-4-(trifluormethyl)-1*H*-pyrazol-5-carbonsäure (1,56 g, 5,00 mmol) in Dichlormethan (20 ml) gab man Oxalylchlorid (0,65 ml, 0,95 g, 7,5 mmol, 1,5 Äq.) gefolgt von DMF (3 Tropfen) und erhitze auf 40°C. Nach 9 h bei 40°C wurde im Vakuum eingeengt, mit THF (20 ml) und Pyridin (0,8 ml, 0,8 g, 2,0 Äq.) versetzt und 1-(5-Amino-2-chlorphenyl)ethanon (0,85 g, 5,00 mmol, 1,0 Äq.) in THF (5 ml) zugegeben. Es wurde 2 d bei Raumtemperatur belassen. Man versetzte mit HCl (1N wässr. Lösung), extrahierte mit Ethylacetat, wusch mit HCl (1N wässr. Lösung), NaOH (1N wässr. Lösung) und ges. wässr. Kochsalz-Lösung. Es wurde über MgSO₄ getrocknete und nach Einengen im Vakuum per Säulenchromatographie an Kieselgel (Hexan/Ethylacetat) zu *N*-(3-Acetyl-4-chlorphenyl)-1-methyl-3-(pentafluorethyl)-4-(trifluormethyl)-1*H*-pyrazol-5-carboxamid (1,85 g, 80%) gereinigt.
¹H-NMR (300 MHz, d₆-DMSO): δ = 2,63 (s, 3H), 4,07 (s, 3H), 7,46 (d, 1H), 7,74 (m, 1H), 7,84 (dm, 1H), 8,50 (br. s, 1H) ppm.
HPLC-MS^{a)}: Masse (m/z) = 462 [M-H]⁺.
a) Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18).Agilent 1100 LC-System; 50*4,6 Zorbax Eclipse Plus C18 1,8 microm; Eluent A: Acetonitril (0,1 % Ameisensäure); Eluent B: Wasser (0,09 % Ameisensäure); linearer Gradient von 10 % Acetonitril bis 95 % Acetonitril in 4,25 min, dann 95% Acetonitril für weitere 1,25 min; Ofentemperatur 55 °C; Fluß:2,0 mL/min. Die Massendetektion erfolgt über ein Agilent MSD-System.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).Die lambdamaX-Werte wurden an Hand der UV-Spektren von 200 nm bis 400 nm in den Maxima der chromatographischen Signale ermittelt.

**Tabelle 1**

| Die folgenden | | | | | | | | | | | | | |
|---|---|---|---|---|---|---|---|---|---|---|---|---|---|
| 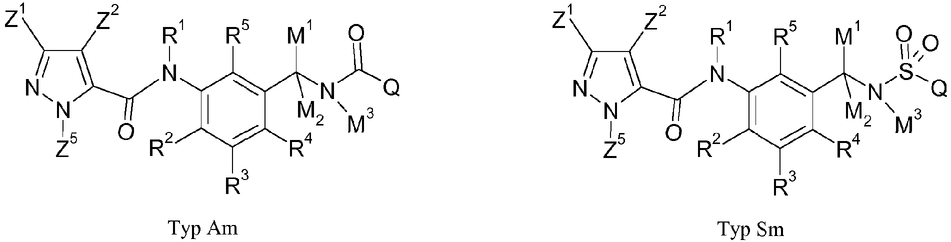 | | | | | | | | | | | | | |

| **Nr.** | **Typ** | **M¹** | **M²** | **M³** | **Q** | **R¹** | **R²** | **R³** | **R⁴** | **R⁵** | **Z¹** | **Z²** | **Z⁵** |
|---|---|---|---|---|---|---|---|---|---|---|---|---|---|
| 1 | Am | H | H | H | Ethyl | CH₃ | H | H | F | H | CF₃CF₂ | CF₃ | CH₃ |
| 2 | Am | H | H | H | Cyclopropyl | CH₃ | H | H | F | H | CF₃CF₂ | CF₃ | CH₃ |
| 3 | Am | H | H | H | Ethyl | Ethyl | H | H | F | H | CF₃CF₂ | CF₃ | CH₃ |
| 4 | Am | H | H | H | Ethyl | H | H | H | F | H | CF₃CF₂ | CF₃ | CH₃ |
| 5 | Am | H | H | H | 2-Chlorethyl | CH₃ | H | H | F | H | CF₃CF₂ | CF₃ | CH₃ |
| 6 | Am | H | H | H | Ethyl | H | H | H | F | F | CF₃CF₂ | CF₃ | CH₃ |
| 7 | Am | H | H | H | CH₃ | CH₃ | H | H | F | F | CF₃CF₂ | CF₃ | CH₃ |
| 8 | Am | H | H | H | CH₃ | CH₃ | H | H | F | H | CF₃CF₂ | CF₃ | CH₃ |
| 9 | Am | H | H | H | CH₃ | Ethyl | H | H | F | H | CF₃CF₂ | CF₃ | CH₃ |
| 10 | Am | H | H | H | Cyclopropyl | CH₃ | H | H | H | H | CF₃CF₂ | CF₃ | CH₃ |
| 11 | Am | H | H | H | CH₃ | H | H | H | F | F | CF₃CF₂ | CF₃ | CH₃ |
| 12 | Am | H | H | H | Cyclopropyl | Cyan-methyl | H | H | F | H | CF₃CF₂ | CF₃ | CH₃ |
| 13 | Am | H | H | H | CH₃ | H | H | H | F | H | CF₃CF₂ | CF₃ | CH₃ |
| 14 | Am | H | H | H | Cyclopropyl | Ethyl | H | H | F | H | CF₃CF₂ | CF₃ | CH₃ |
| 15 | Am | H | H | H | Prop-2-en-1-yl | H | H | H | F | H | CF₃CF₂ | CF₃ | CH₃ |
| 16 | Am | H | H | H | Ethyl | CH₃ | H | H | F | F | CF₃CF₂ | CF₃ | CH₃ |
| 17 | Am | H | H | H | Ethyl | CH₃ | H | H | H | H | CF₃CF₂ | CF₃ | CH₃ |
| 18 | Am | H | H | H | Cyclopropylmethyl | CH₃ | H | H | F | H | CF₃CF₂ | CF₃ | CH₃ |
| 19 | Am | H | H | H | CH₃ | Cyan-methyl | H | H | F | H | CF₃CF₂ | CF₃ | CH₃ |
| 20 | Am | H | H | H | Cyclopropyl | H | H | H | F | H | CF₃CF₂ | CF₃ | CH₃ |
| 21 | Am | H | H | H | Cyclopropylmethyl | H | H | H | F | H | CF₃CF₂ | CF₃ | CH₃ |
| 22 | Am | H | H | H | 1-Fluorethyl | H | H | H | F | H | CF₃CF₂ | CF₃ | CH₃ |
| 23 | Am | H | H | H | Ethyl | H | H | H | H | H | CF₃CF₂ | CF₃ | CH₃ |
| 24 | Am | H | H | H | (Methylsulfanyl)methyl | H | H | H | F | H | CF₃CF₂ | CF₃ | CH₃ |
| 25 | Am | CH₃ | H | H | Cyclopropylmethyl | H | H | H | Cl | H | CF₃CF₂ | CF₃ | CH₃ |
| 26 | Am | H | H | H | 2-Chlorethyl | H | H | H | F | H | CF₃CF₂ | CF₃ | CH₃ |
| 27 | Am | H | H | H | CH₃ | CH₃ | H | H | H | H | CF₃CF₂ | CF₃ | CH₃ |
| 28 | Am | CH₃ | H | H | Ethyl | H | H | H | Cl | H | CF₃CF₂ | CF₃ | CH₃ |
| 29 | Am | H | H | H | Propyl | Ethyl | H | H | F | H | CF₃CF₂ | CF₃ | CH₃ |
| 30 | Am | H | H | H | Cyclopropyl | H | H | H | F | F | CF₃CF₂ | CF₃ | CH₃ |
| 31 | Am | H | H | H | Cyclopropylmethyl | H | H | H | F | F | CF₃CF₂ | CF₃ | CH₃ |
| 32 | Am | H | H | H | Ethyl | Cyan-methyl | H | H | F | H | CF₃CF₂ | CF₃ | CH₃ |
| 33 | Am | H | H | H | 3-Chlorpropyl | H | H | H | F | H | CF₃CF₂ | CF₃ | CH₃ |
| 34 | Am | H | H | H | Cyclopropylmethyl | CH₃ | H | H | F | F | CF₃CF₂ | CF₃ | CH₃ |
| 35 | Am | H | H | H | Cyclopropylmethyl | H | H | H | Cl | H | CF₃CF₂ | CF₃ | CH₃ |
| 36 | Am | H | H | H | Prop-2-en-1-yl | CH₃ | H | H | F | H | CF₃CF₂ | CF₃ | CH₃ |
| 37 | Am | H | H | H | (2E)-But-2-en-1-yl | H | H | H | F | H | CF₃CF₂ | CF₃ | CH₃ |
| 38 | Am | H | H | H | Cyclopropylmethyl | Ethyl | H | H | F | H | CF₃CF₂ | CF₃ | CH₃ |
| 39 | Am | H | H | H | Ethyl | H | H | H | Cl | H | CF₃CF₂ | CF₃ | CH₃ |
| 40 | Am | CH₃ | H | H | CH₃ | H | H | H | Cl | H | CF₃CF₂ | CF₃ | CH₃ |
| 41 | Am | H | H | H | Propyl | H | H | H | Cl | H | CF₃CF₂ | CF₃ | CH₃ |
| 42 | Am | H | H | H | Cyclopropyl | H | H | H | H | H | CF₃CF₂ | CF₃ | CH₃ |
| 43 | Am | H | H | H | Chlormethyl | H | H | H | F | H | CF₃CF₂ | CF₃ | CH₃ |
| 44 | Am | H | H | H | Cyclopropylmethyl | H | H | H | H | H | CF₃CF₂ | CF₃ | CH₃ |
| 45 | Am | H | H | H | CH₃ | H | H | H | H | H | CF₃CF₂ | CF₃ | CH₃ |
| 46 | Am | H | H | H | Difluormethyl | H | H | H | F | H | CF₃CF₂ | CF₃ | CH₃ |
| 47 | Am | H | H | H | Prop-2-en-1-yl | Ethyl | H | H | F | H | CF₃CF₂ | CF₃ | CH₃ |
| 48 | Am | H | H | H | Cyclopropylmethyl | Cyan-methyl | H | H | F | H | CF₃CF₂ | CF₃ | CH₃ |
| 49 | Am | H | H | H | Chlormethyl | CH₃ | H | H | F | H | CF₃CF₂ | CF₃ | CH₃ |
| 50 | Am | H | H | H | 2-Methylpropyl | H | H | H | F | H | CF₃CF₂ | CF₃ | CH₃ |
| 51 | Am | H | H | H | 2,2-Difluorpropyl | H | H | H | F | H | CF₃CF₂ | CF₃ | CH₃ |
| 52 | Am | H | H | H | Propyl | H | H | H | F | H | CF₃CF₂ | CF₃ | CH₃ |
| 53 | Am | H | H | H | Methoxy | H | H | H | H | H | CF₃CF₂ | CF₃ | CH₃ |
| 54 | Am | H | H | H | CH₃ | H | H | H | Cl | H | CF₃CF₂ | CF₃ | CH₃ |
| 55 | Am | H | H | H | Ethoxy | H | H | H | F | H | CF₃CF₂ | CF₃ | CH₃ |
| 56 | Am | H | H | H | Cyclobutyl | H | H | H | H | H | CF₃CF₂ | CF₃ | CH₃ |
| 57 | Am | H | H | H | 2,2,2-Trifluorethyl | H | H | H | H | H | CF₃CF₂ | CF₃ | CH₃ |
| 58 | Am | H | H | H | 1-Chlorethyl | H | H | H | H | H | CF₃CF₂ | CF₃ | CH₃ |
| 59 | Am | H | H | H | (Methylsulfanyl)methyl | H | H | H | F | F | CF₃CF₂ | CF₃ | CH₃ |
| 60 | Am | H | H | H | Cyclopropyl | H | H | H | Cl | H | CF₃CF₂ | CF₃ | CH₃ |
| 61 | Am | H | H | H | 1-Chlorethyl | H | H | H | F | H | CF₃CF₂ | CF₃ | CH₃ |
| 62 | Am | H | H | H | Butyl | H | H | H | F | H | CF₃CF₂ | CF₃ | CH₃ |
| 63 | Am | H | H | H | tert-Butoxy | CH₃ | H | H | F | H | CF₃CF₂ | CF₃ | CH₃ |
| 64 | Am | H | H | H | CH₃ | H | H | H | Br | H | CF₃CF₂ | CF₃ | CH₃ |
| 65 | Am | H | H | H | 2-Methylprop-1-en-1-yl | H | H | H | H | H | CF₃CF₂ | CF₃ | CH₃ |
| 66 | Am | H | H | H | 2-Methylcyclopropyl | H | H | H | F | H | CF₃CF₂ | CF₃ | CH₃ |
| 67 | Am | H | H | H | 2-Methylpropyl | H | H | H | H | H | CF₃CF₂ | CF₃ | CH₃ |
| 68 | Am | H | H | H | Ethyl | H | H | H | CH₃ | H | CF₃CF₂ | CF₃ | CH₃ |
| 69 | Am | H | H | H | Prop-2-en-1-yl | H | H | F | H | H | CF₃CF₂ | CF₃ | CH₃ |
| 70 | Am | H | H | H | (1E)-Prop-1-en-1-yl | H | H | H | H | H | CF₃CF₂ | CF₃ | CH₃ |
| 71 | Am | H | H | H | Ethyl | H | H | H | F | H | 1-Chlorcyclopropyl | Cl | CH₃ |
| 72 | Am | H | H | H | 2-Cyanethyl | H | H | H | F | H | CF₃CF₂ | CF₃ | CH₃ |
| 73 | Am | H | H | H | 4-Fluorphenyl | H | H | H | H | H | CF₃CF₂ | CF₃ | CH₃ |
| 74 | Am | H | H | H | tert-Butoxy | H | H | H | Cl | H | CF₃CF₂ | CF₃ | CH₃ |
| 75 | Sm | H | H | H | Ethyl | H | H | H | Cl | H | CF₃CF₂ | CF₃ | CH₃ |
| 76 | Am | H | H | H | Ethyl | H | H | F | H | H | CF₃CF₂ | CF₃ | CH₃ |
| 77 | Am | H | H | H | (Methylsulfinyl)methyl | H | H | H | F | F | CF₃CF₂ | CF₃ | CH₃ |
| 78 | Am | H | H | H | 6-Chlorpyridin-3-yl | H | H | H | F | H | CF₃CF₂ | CF₃ | CH₃ |
| 79 | Am | H | H | H | Cyclopropylmethyl | H | H | H | F | H | 1-Chlorcyclopropyl | Cl | CH₃ |
| 80 | Am | H | H | H | Methylsulfanyl | H | H | H | F | H | CF₃CF₂ | CF₃ | CH₃ |
| 81 | Am | H | H | H | Cyclopropyl | H | H | F | H | H | CF₃CF₂ | CF₃ | CH₃ |
| 82 | Am | H | H | H | Prop-1-yn-1-yl | H | H | H | F | H | CF₃CF₂ | CF₃ | CH₃ |
| 83 | Am | H | H | H | Ethyl | H | Cl | H | H | H | CF₃CF₂ | CF₃ | CH₃ |
| 84 | Am | H | H | H | Ethyl | H | H | H | Cl | Cl | CF₃CF₂ | CF₃ | CH₃ |
| 85 | Am | H | H | H | Cyanmethyl | H | H | H | F | H | CF₃CF₂ | CF₃ | CH₃ |
| 86 | Am | H | H | H | Pyridin-3-yl | H | H | H | H | H | CF₃CF₂ | CF₃ | CH₃ |
| 87 | Am | H | H | H | Butan-2-yl | H | H | H | H | H | CF₃CF₂ | CF₃ | CH₃ |
| 88 | Am | H | H | H | 1-Cyancyclopropyl | H | H | H | Cl | H | CF₃CF₂ | CF₃ | CH₃ |
| 89 | Am | H | H | H | 3-Cyanopropyl | H | H | H | F | H | CF₃CF₂ | CF₃ | CH₃ |
| 90 | Am | H | H | H | Cyclopropylmethyl | H | H | H | Cl | Cl | CF₃CF₂ | CF₃ | CH₃ |
| 91 | Am | H | H | H | 1-Methylcyclopropyl | H | H | H | F | H | CF₃CF₂ | CF₃ | CH₃ |
| 92 | Am | H | H | H | CH₃ | H | H | H | Cl | Cl | CF₃CF₂ | CF₃ | CH₃ |
| 93 | Am | H | H | H | 2-Methylpropyl | H | H | H | Cl | H | CF₃CF₂ | CF₃ | CH₃ |
| 94 | Am | H | H | H | Aminomethyl | H | H | H | F | H | CF₃CF₂ | CF₃ | CH₃ |
| 95 | Am | H | H | H | Aminomethyl | H | H | H | F | H | CF₃CF₂ | CF₃ | CH₃ |
| 96 | Sm | H | H | H | CH₃ | H | H | H | H | H | CF₃CF₂ | CF₃ | CH₃ |
| 97 | Am | H | H | H | Cyclopropylcarbamoyl | H | H | H | F | H | CF₃CF₂ | CF₃ | CH₃ |
| 98 | Am | H | H | H | CH₃ | H | H | H | F | H | 1-Chlorcyclopropyl | Cl | CH₃ |
| 99 | Am | H | H | H | CH₃ | H | H | F | H | H | CF₃CF₂ | CF₃ | CH₃ |
| 100 | Am | H | H | H | Cyclopentyl | H | H | H | H | H | CF₃CF₂ | CF₃ | CH₃ |
| 101 | Am | H | H | H | Propan-2-yl | H | H | H | Cl | H | CF₃CF₂ | CF₃ | CH₃ |
| 102 | Am | H | H | H | 2-Methylprop-1-en-1-yl | H | H | H | F | H | CF₃CF₂ | CF₃ | CH₃ |
| 103 | Am | H | H | H | 4-(Methylsulfonyl)phenyl | H | H | H | F | H | CF₃CF₂ | CF₃ | CH₃ |
| 104 | Am | H | H | H | Ethylcarbamoyl | H | H | H | F | H | CF₃CF₂ | CF₃ | CH₃ |
| 105 | Am | H | H | H | 2-Acetamidoethyl | H | H | H | F | H | CF₃CF₂ | CF₃ | CH₃ |
| 106 | Am | H | H | H | 2,2-Dimethylpropyl | H | H | H | H | H | CF₃CF₂ | CF₃ | CH₃ |
| 107 | Am | H | H | Propan-1,3-diyl | | H | H | H | F | H | CF₃CF₂ | CF₃ | CH₃ |
| 108 | Am | H | H | H | tert-Butoxy | H | H | H | F | F | CF₃CF₂ | CF₃ | CH₃ |
| 109 | Am | H | H | H | 4-Chlorphenyl | H | H | H | Cl | H | CF₃CF₂ | CF₃ | CH₃ |
| 110 | Am | H | H | H | (Cyclopropylmethyl)carbamoyl | H | H | H | F | H | CF₃CF₂ | CF₃ | CH₃ |
| 111 | Am | H | H | H | Phenyl | H | H | H | Cl | H | CF₃CF₂ | CF₃ | CH₃ |
| 112 | Am | H | H | H | Cyclopent-1-en-1-ylmethyl | H | H | H | F | H | CF₃CF₂ | CF₃ | CH₃ |
| 113 | Am | H | H | H | Benzyl | H | H | H | Cl | H | CF₃CF₂ | CF₃ | CH₃ |
| 114 | Am | H | H | H | Ethyl | H | H | H | 1H-1,2,4-Triazol-1-yl | H | CF₃CF₂ | CF₃ | CH₃ |
| 115 | Am | H | H | H | (1E)-3-Methylbut-1-en-1-yl | H | H | H | F | H | CF₃CF₂ | CF₃ | CH₃ |
| 116 | Am | H | H | H | 2,2-Dimethylpropyl | H | H | H | Cl | H | CF₃CF₂ | CF₃ | CH₃ |
| 117 | Am | H | H | H | Pyridin-2-yl | H | H | H | H | H | CF₃CF₂ | CF₃ | CH₃ |
| 118 | Am | H | H | H | (Methylsulfonyl)methyl | H | H | H | F | F | CF₃CF₂ | CF₃ | CH₃ |
| 119 | Am | H | H | H | tert-Butoxy | H | H | H | H | H | CF₃CF₂ | CF₃ | CH₃ |
| 120 | Am | H | H | H | 5-Oxo-2,5-dihydrofuran-3-yl | H | H | H | H | H | CF₃CF₂ | CF₃ | CH₃ |
| 121 | Am | H | H | H | Methoxymethyl | H | H | H | H | H | CF₃CF₂ | CF₃ | CH₃ |
| 122 | Am | H | H | H | (2E)-But-2-en-2-yl | H | H | H | H | H | CF₃CF₂ | CF₃ | CH₃ |
| 123 | Am | H | H | H | (6-Chlorpyridin-3-yl)methyl | H | H | H | F | H | CF₃CF₂ | CF₃ | CH₃ |
| 124 | Am | H | H | H | CH₃ | H | H | H | 1H-1,2,4-Triazol- | H | CF₃CF₂ | CF₃ | CH₃ |
| | | | | | | | | | 1-yl | | | | |
| 125 | Am | H | H | H | 2-[(Cyclopropylmethyl)amino]-2-oxoethyl | H | H | H | F | H | CF₃CF₂ | CF₃ | CH₃ |
| 126 | Am | H | H | H | 3-Methylbutyl | H | H | H | F | H | CF₃CF₂ | CF₃ | CH₃ |
| 127 | Am | H | H | H | Acetamidomethyl | H | H | H | F | H | CF₃CF₂ | CF₃ | CH₃ |
| 128 | Am | H | H | H | Propan-2-ylamino | H | H | H | Cl | H | CF₃CF₂ | CF₃ | CH₃ |
| 129 | Am | H | H | H | Phenylamino | H | H | H | Cl | H | CF₃CF₂ | CF₃ | CH₃ |
| 130 | Am | H | H | H | Benzylamino | H | H | H | Cl | H | CF₃CF₂ | CF₃ | CH₃ |
| 131 | Am | H | H | H | 2-Methoxy-2-oxoethyl | H | H | H | F | H | CF₃CF₂ | CF₃ | CH₃ |
| 132 | Am | H | H | H | Carboxymethyl | H | H | H | F | H | CF₃CF₂ | CF₃ | CH₃ |
| 133 | Am | H | H | H | 2-(Methylamino)-2-oxoethyl | H | H | H | F | H | CF₃CF₂ | CF₃ | CH₃ |
| 134 | Am | H | H | H | 2-Chlorphenyl | H | H | H | F | H | CF₃CF₂ | CF₃ | CH₃ |
| 135 | Am | H | H | H | 4-Methylphenyl | H | H | H | F | H | CF₃CF₂ | CF₃ | CH₃ |
| 136 | Am | H | H | H | 3-Chlorphenyl | H | H | H | F | H | CF₃CF₂ | CF₃ | CH₃ |
| 137 | Am | H | H | H | Cyclopropyl | H | H | H | 1H-1,2,4-Triazol-1-yl | H | CF₃CF₂ | CF₃ | CH₃ |
| 138 | Am | H | H | H | H | H | H | H | F | H | CF₃CF₂ | CF₃ | CH₃ |
| 139 | Am | H | H | H | Pyrimidin-5-ylmethyl | H | H | H | F | H | CF₃CF₂ | CF₃ | CH₃ |
| 140 | Am | H | H | H | Ethoxycarbonyl | H | H | H | F | H | CF₃CF₂ | CF₃ | CH₃ |
| 141 | Am | H | H | H | Methylcarbamoyl | H | H | H | F | H | CF₃CF₂ | CF₃ | CH₃ |
| 142 | Am | H | H | H | 2-Aminoethyl | H | H | H | F | H | CF₃CF₂ | CF₃ | CH₃ |
| 143 | Am | H | H | H | Pyrimidin-5-yl | H | H | H | F | H | CF₃CF₂ | CF₃ | CH₃ |
| 144 | Am | H | H | H | 5-Chlorpyridin-3-yl | H | H | H | F | H | CF₃CF₂ | CF₃ | CH₃ |
| 145 | Am | H | H | H | 6-Methylpyridin-3-yl | H | H | H | F | H | CF₃CF₂ | CF₃ | CH₃ |
| 146 | Am | H | H | H | 2-Methylpyridin-4-yl | H | H | H | F | H | CF₃CF₂ | CF₃ | CH₃ |
| 147 | Am | H | H | H | 3-Chlorpyridin-4-yl | H | H | H | F | H | CF₃CF₂ | CF₃ | CH₃ |
| 148 | Am | H | H | H | Pyridin-4-yl | H | H | H | F | H | CF₃CF₂ | CF₃ | CH₃ |
| 149 | Am | H | H | H | 1-(4-Chlorphenyl)cyclopropyl | H | H | H | F | H | CF₃CF₂ | CF₃ | CH₃ |
| 150 | Am | H | H | H | 1-(2-Chlorphenyl)ethyl | H | H | H | F | H | CF₃CF₂ | CF₃ | CH₃ |
| 151 | Am | H | H | H | 1-(3-Chlorphenyl)ethyl | H | H | H | F | H | CF₃CF₂ | CF₃ | CH₃ |
| 152 | Am | H | H | H | 1-(4-Methylphenyl)ethyl | H | H | H | F | H | CF₃CF₂ | CF₃ | CH₃ |
| 153 | Am | H | H | H | 1-(4-Chlorphenyl)ethyl | H | H | H | F | H | CF₃CF₂ | CF₃ | CH₃ |
| 154 | Am | H | H | H | Cyclopentylmethyl | H | H | H | F | H | CF₃CF₂ | CF₃ | CH₃ |
| 155 | Am | H | H | H | Thiophen-3-ylmethyl | H | H | H | F | H | CF₃CF₂ | CF₃ | CH₃ |
| 156 | Am | H | H | H | (3-Methyl-1,2-oxazol-5-yl)methyl | H | H | H | F | H | CF₃CF₂ | CF₃ | CH₃ |
| 157 | Am | H | H | H | 2-Furylmethyl | H | H | H | F | H | CF₃CF₂ | CF₃ | CH₃ |
| 158 | Am | H | H | H | (5-Methyl-2-thienyl)methyl | H | H | H | F | H | CF₃CF₂ | CF₃ | CH₃ |
| 159 | Am | H | H | H | 1H-Tetrazol-5-ylmethyl | H | H | H | F | H | CF₃CF₂ | CF₃ | CH₃ |
| 160 | Am | H | H | H | (3,5-Dimethyl-1,2-oxazol-4-yl)methyl | H | H | H | F | H | CF₃CF₂ | CF₃ | CH₃ |
| 161 | Am | H | H | H | (4-Methyl-1,2,5-oxadiazol-3-yl)methyl | H | H | H | F | H | CF₃CF₂ | CF₃ | CH₃ |
| 162 | Am | H | H | H | Tetrahydrofuran-2-ylmethyl | H | H | H | F | H | CF₃CF₂ | CF₃ | CH₃ |
| 163 | Am | H | H | H | 2-Chlorbenzyl | H | H | H | F | H | CF₃CF₂ | CF₃ | CH₃ |
| 164 | Am | H | H | H | 3-Chlorbenzyl | H | H | H | F | H | CF₃CF₂ | CF₃ | CH₃ |
| 165 | Am | H | H | H | 3-Ethyloxetan-3-yl | H | H | H | F | H | CF₃CF₂ | CF₃ | CH₃ |
| 166 | Am | H | H | H | 2-Fluorcyclopropyl | H | H | H | F | H | CF₃CF₂ | CF₃ | CH₃ |
| 167 | Am | H | H | H | 2,2-Dichlor-1-methylcyclopropyl | H | H | H | F | H | CF₃CF₂ | CF₃ | CH₃ |
| 168 | Am | H | H | H | 1,1'-Bi(cyclopropyl)-1-yl | H | H | H | F | H | CF₃CF₂ | CF₃ | CH₃ |
| 169 | Am | H | H | H | (2,2-Difluorcyclopropyl)methyl | H | H | H | F | H | CF₃CF₂ | CF₃ | CH₃ |
| 170 | Am | H | H | H | 2-Methylbutyl | H | H | H | F | H | CF₃CF₂ | CF₃ | CH₃ |
| 171 | Am | H | H | H | tert-Butyl | H | H | H | F | H | CF₃CF₂ | CF₃ | CH₃ |
| 172 | Am | H | H | H | 2,2-Dichlorcyclopropyl | H | H | H | F | H | CF₃CF₂ | CF₃ | CH₃ |
| 173 | Am | H | H | H | Ethyl | CH₃ | H | H | F | H | CF₃ | CF₃ | CH₃ |
| 174 | Am | H | H | H | Ethyl | CH₃ | H | H | Cl | H | CF₃CF₂ | CF₃ | CH₃ |
| 175 | Am | H | H | H | Ethyl | CH₃ | H | H | F | H | CF₃CF₂ | CF₃ | Ethyl |
| 176 | Am | H | H | H | Ethyl | CH₃ | H | H | F | Cl | CF₃CF₂ | CF₃ | CH₃ |
| 177 | Am | H | H | H | Ethyl | CH₃ | H | H | Br | H | CF₃CF₂ | CF₃ | CH₃ |
| 178 | Am | H | H | H | Ethyl | CH₃ | H | F | F | H | CF₃CF₂ | CF₃ | CH₃ |
| 179 | Am | H | H | H | Ethyl | CH₃ | H | Cl | F | H | CF₃CF₂ | CF₃ | CH₃ |
| 180 | Am | H | H | H | Propyl | CH₃ | H | H | F | H | CF₃CF₂ | CF₃ | CH₃ |
| 181 | Am | H | H | H | Cyclopropyl | CH₃ | H | H | F | F | CF₃CF₂ | CF₃ | CH₃ |
| 182 | Am | CH₃ | H | H | Cyclopropyl | CH₃ | H | H | F | H | CF₃CF₂ | CF₃ | CH₃ |
| 183 | Am | H | H | H | Cyclopropyl | CH₃ | H | H | F | H | CF₃ | CF₃ | CH₃ |
| 184 | Am | H | H | H | Cyclopropyl | CH₃ | H | H | Cl | H | CF₃CF₂ | CF₃ | CH₃ |
| 185 | Am | H | H | H | Cyclopropyl | CH₃ | H | H | F | H | CF₃CF₂ | CF₃ | Ethyl |
| 186 | Am | H | H | H | Cyclopropyl | CH₃ | H | H | F | Cl | CF₃CF₂ | CF₃ | CH₃ |
| 187 | Am | H | H | H | Cyclopropyl | CH₃ | H | H | Br | H | CF₃CF₂ | CF₃ | CH₃ |
| 188 | Am | H | H | H | Cyclopropyl | CH₃ | H | F | F | H | CF₃CF₂ | CF₃ | CH₃ |
| 189 | Am | H | H | H | Cyclopropyl | CH₃ | H | Cl | F | H | CF₃CF₂ | CF₃ | CH₃ |
| 190 | Am | H | H | H | Ethyl | Ethyl | H | H | F | F | CF₃CF₂ | CF₃ | CH₃ |
| 191 | Am | CH₃ | H | H | Ethyl | Ethyl | H | H | F | H | CF₃CF₂ | CF₃ | CH₃ |
| 192 | Am | H | H | H | Ethyl | Ethyl | H | H | F | H | CF₃ | CF₃ | CH₃ |
| 193 | Am | H | H | H | Ethyl | Ethyl | H | H | Cl | H | CF₃CF₂ | CF₃ | CH₃ |
| 194 | Am | H | H | H | Ethyl | Ethyl | H | H | H | H | CF₃CF₂ | CF₃ | CH₃ |
| 195 | Am | H | H | H | Ethyl | Ethyl | H | H | F | Cl | CF₃CF₂ | CF₃ | CH₃ |
| 196 | Am | H | H | H | Ethyl | Ethyl | H | H | Br | H | CF₃CF₂ | CF₃ | CH₃ |
| 197 | Am | H | H | H | Ethyl | Ethyl | H | H | F | H | CF₃CF₂ | CF₃ | Ethyl |
| 198 | Am | H | H | H | Ethyl | Ethyl | H | F | F | H | CF₃CF₂ | CF₃ | CH₃ |
| 199 | Am | H | H | H | Ethyl | Ethyl | H | Cl | F | H | CF₃CF₂ | CF₃ | CH₃ |
| 200 | Am | CH₃ | H | H | Ethyl | H | H | H | F | H | CF₃CF₂ | CF₃ | CH₃ |
| 201 | Am | H | H | H | Ethyl | H | H | H | F | H | CF₃ | CF₃ | CH₃ |
| 202 | Am | H | H | H | Ethyl | H | H | H | F | H | CF₃CF₂ | CF₃ | Ethyl |
| 203 | Am | H | H | H | Ethyl | H | H | H | F | Cl | CF₃CF₂ | CF₃ | CH₃ |
| 204 | Am | H | H | H | Ethyl | H | H | H | Br | H | CF₃CF₂ | CF₃ | CH₃ |
| 205 | Am | H | H | H | Ethyl | H | H | F | F | H | CF₃CF₂ | CF₃ | CH₃ |
| 206 | Am | H | H | H | Ethyl | H | H | Cl | F | H | CF₃CF₂ | CF₃ | CH₃ |
| 207 | Am | H | H | H | Propyl | H | H | H | F | F | CF₃CF₂ | CF₃ | CH₃ |
| 208 | Am | CH₃ | H | H | Ethyl | H | H | H | F | F | CF₃CF₂ | CF₃ | CH₃ |
| 209 | Am | H | H | H | Ethyl | H | H | H | F | F | CF₃ | CF₃ | CH₃ |
| 210 | Am | H | H | H | Ethyl | H | H | H | Cl | F | CF₃CF₂ | CF₃ | CH₃ |
| 211 | Am | H | H | H | Ethyl | H | H | H | H | F | CF₃CF₂ | CF₃ | CH₃ |
| 212 | Am | H | H | H | Ethyl | H | H | F | F | F | CF₃CF₂ | CF₃ | CH₃ |
| 213 | Am | H | H | H | Ethyl | H | H | Cl | F | F | CF₃CF₂ | CF₃ | CH₃ |
| 214 | Am | H | H | H | Ethyl | H | H | H | F | F | CF₃CF₂ | CF₃ | Ethyl |
| 215 | Am | H | H | H | Ethyl | H | H | H | Br | F | CF₃CF₂ | CF₃ | CH₃ |
| 216 | Am | H | H | H | CH₃ | CH₃ | H | H | F | F | CF₃CF₂ | CF₃ | Ethyl |
| 217 | Am | H | H | H | CH₃ | CH₃ | H | H | F | F | CF₃ | CF₃ | CH₃ |
| 218 | Am | H | H | H | CH₃ | CH₃ | H | H | F | Cl | CF₃CF₂ | CF₃ | CH₃ |
| 219 | Am | H | H | H | CH₃ | CH₃ | H | H | H | F | CF₃CF₂ | CF₃ | CH₃ |
| 220 | Am | H | H | H | CH₃ | CH₃ | H | H | Cl | F | CF₃CF₂ | CF₃ | CH₃ |
| 221 | Am | H | H | H | CH₃ | CH₃ | H | H | Br | F | CF₃CF₂ | CF₃ | CH₃ |
| 222 | Am | H | H | H | CH₃ | CH₃ | H | F | F | F | CF₃CF₂ | CF₃ | CH₃ |
| 223 | Am | H | H | H | CH₃ | CH₃ | H | Cl | F | F | CF₃CF₂ | CF₃ | CH₃ |
| 224 | Am | H | H | H | Propyl | CH₃ | H | H | F | F | CF₃CF₂ | CF₃ | CH₃ |
| 225 | Am | CH₃ | H | H | CH₃ | CH₃ | H | H | F | F | CF₃CF₂ | CF₃ | CH₃ |
| 226 | Am | H | H | H | CH₃ | Ethyl | H | H | F | F | CF₃CF₂ | CF₃ | CH₃ |
| 227 | Am | CH₃ | H | H | CH₃ | CH₃ | H | H | F | H | CF₃CF₂ | CF₃ | CH₃ |
| 228 | Am | H | H | H | CH₃ | CH₃ | H | Cl | F | H | CF₃CF₂ | CF₃ | CH₃ |
| 229 | Am | H | H | H | CH₃ | CH₃ | H | H | F | H | CF₃CF₂ | CF₃ | Ethyl |
| 230 | Am | H | H | H | CH₃ | CH₃ | H | H | F | H | CF₃ | CF₃ | CH₃ |
| 231 | Am | H | H | H | CH₃ | CH₃ | H | H | Cl | H | CF₃CF₂ | CF₃ | CH₃ |
| 232 | Am | H | H | H | CH₃ | CH₃ | H | H | Br | H | CF₃CF₂ | CF₃ | CH₃ |
| 233 | Am | H | H | H | CH₃ | CH₃ | H | F | F | H | CF₃CF₂ | CF₃ | CH₃ |
| 234 | Am | CH₃ | H | H | CH₃ | Ethyl | H | H | F | H | CF₃CF₂ | CF₃ | CH₃ |
| 235 | Am | H | H | H | CH₃ | Ethyl | H | H | F | H | CF₃ | CF₃ | CH₃ |
| 236 | Am | H | H | H | CH₃ | Ethyl | H | H | Cl | H | CF₃CF₂ | CF₃ | CH₃ |
| 237 | Am | H | H | H | CH₃ | Ethyl | H | H | H | H | CF₃CF₂ | CF₃ | CH₃ |
| 238 | Am | H | H | H | CH₃ | Ethyl | H | H | F | H | CF₃CF₂ | CF₃ | Ethyl |
| 239 | Am | H | H | H | CH₃ | Ethyl | H | H | F | Cl | CF₃CF₂ | CF₃ | CH₃ |
| 240 | Am | H | H | H | CH₃ | Ethyl | H | H | Br | H | CF₃CF₂ | CF₃ | CH₃ |
| 241 | Am | H | H | H | CH₃ | Ethyl | H | F | F | H | CF₃CF₂ | CF₃ | CH₃ |
| 242 | Am | H | H | H | CH₃ | Ethyl | H | Cl | F | H | CF₃CF₂ | CF₃ | CH₃ |
| 243 | Am | H | H | H | Cyclopropyl | CH₃ | H | H | H | F | CF₃CF₂ | CF₃ | CH₃ |
| 244 | Am | CH₃ | H | H | Cyclopropyl | CH₃ | H | H | H | H | CF₃CF₂ | CF₃ | CH₃ |
| 245 | Am | H | H | H | Cyclopropyl | CH₃ | H | H | H | H | CF₃ | CF₃ | CH₃ |
| 246 | Am | H | H | H | Cyclopropyl | Ethyl | H | H | H | H | CF₃CF₂ | CF₃ | CH₃ |
| 247 | Am | H | H | H | Cyclopropylmethyl | CH₃ | H | H | H | H | CF₃CF₂ | CF₃ | CH₃ |
| 248 | Am | H | H | H | Cyclopropyl | CH₃ | H | H | H | H | CF₃CF₂ | CF₃ | Ethyl |
| 249 | Am | H | H | H | Cyclopropyl | CH₃ | H | H | H | Cl | CF₃CF₂ | CF₃ | CH₃ |
| 250 | Am | H | H | H | Cyclopropyl | CH₃ | H | F | H | H | CF₃CF₂ | CF₃ | CH₃ |
| 251 | Am | H | H | H | Cyclopropyl | CH₃ | H | Cl | H | H | CF₃CF₂ | CF₃ | CH₃ |
| 252 | Am | H | H | H | Propyl | CH₃ | H | H | H | H | CF₃CF₂ | CF₃ | CH₃ |
| 253 | Am | CH₃ | H | H | CH₃ | H | H | H | F | F | CF₃CF₂ | CF₃ | CH₃ |
| 254 | Am | H | H | H | CH₃ | H | H | H | F | F | CF₃ | CF₃ | CH₃ |
| 255 | Am | H | H | H | CH₃ | H | H | H | Cl | F | CF₃CF₂ | CF₃ | CH₃ |
| 256 | Am | H | H | H | CH₃ | H | H | H | H | F | CF₃CF₂ | CF₃ | CH₃ |
| 257 | Am | H | H | H | CH₃ | H | H | H | F | F | CF₃CF₂ | CF₃ | Ethyl |
| 258 | Am | H | H | H | CH₃ | H | H | H | F | Cl | CF₃CF₂ | CF₃ | CH₃ |
| 259 | Am | H | H | H | CH₃ | H | H | H | Br | F | CF₃CF₂ | CF₃ | CH₃ |
| 260 | Am | H | H | H | CH₃ | H | H | F | F | F | CF₃CF₂ | CF₃ | CH₃ |
| 261 | Am | H | H | H | CH₃ | H | H | Cl | F | F | CF₃CF₂ | CF₃ | CH₃ |
| 262 | Am | CH₃ | H | H | CH₃ | H | H | H | F | H | CF₃CF₂ | CF₃ | CH₃ |
| 263 | Am | H | H | H | CH₃ | H | H | H | F | H | CF₃ | CF₃ | CH₃ |
| 264 | Am | H | H | H | CH₃ | H | H | H | F | H | CF₃CF₂ | CF₃ | Ethyl |
| 265 | Am | H | H | H | CH₃ | H | H | F | F | H | CF₃CF₂ | CF₃ | CH₃ |
| 266 | Am | H | H | H | CH₃ | H | H | Cl | F | H | CF₃CF₂ | CF₃ | CH₃ |
| 267 | Am | H | H | H | Cyclopropyl | Ethyl | H | H | F | F | CF₃CF₂ | CF₃ | CH₃ |
| 268 | Am | CH₃ | H | H | Cyclopropyl | Ethyl | H | H | F | H | CF₃CF₂ | CF₃ | CH₃ |
| 269 | Am | H | H | H | Cyclopropyl | Ethyl | H | H | F | H | CF₃ | CF₃ | CH₃ |
| 270 | Am | H | H | H | Cyclopropyl | Ethyl | H | H | F | H | CF₃CF₂ | CF₃ | Ethyl |
| 271 | Am | H | H | H | Cyclopropyl | Ethyl | H | H | F | Cl | CF₃CF₂ | CF₃ | CH₃ |
| 272 | Am | H | H | H | Cyclopropyl | Ethyl | H | F | F | H | CF₃CF₂ | CF₃ | CH₃ |
| 273 | Am | H | H | H | Cyclopropyl | Ethyl | H | Cl | F | H | CF₃CF₂ | CF₃ | CH₃ |
| 274 | Am | H | H | H | Cyclopropyl | Ethyl | H | H | Br | H | CF₃CF₂ | CF₃ | CH₃ |
| 275 | Am | CH₃ | H | H | Ethyl | CH₃ | H | H | F | F | CF₃CF₂ | CF₃ | CH₃ |
| 276 | Am | H | H | H | Ethyl | CH₃ | H | H | F | F | CF₃ | CF₃ | CH₃ |
| 277 | Am | H | H | H | Ethyl | CH₃ | H | H | Cl | F | CF₃CF₂ | CF₃ | CH₃ |
| 278 | Am | H | H | H | Ethyl | CH₃ | H | H | H | F | CF₃CF₂ | CF₃ | CH₃ |
| 279 | Am | H | H | H | Ethyl | CH₃ | H | F | F | F | CF₃CF₂ | CF₃ | CH₃ |
| 280 | Am | H | H | H | Ethyl | CH₃ | H | Cl | F | F | CF₃CF₂ | CF₃ | CH₃ |
| 281 | Am | H | H | H | Ethyl | CH₃ | H | H | F | F | CF₃CF₂ | CF₃ | Ethyl |
| 282 | Am | H | H | H | Ethyl | CH₃ | H | H | Br | F | CF₃CF₂ | CF₃ | CH₃ |
| 283 | Am | CH₃ | H | H | Ethyl | CH₃ | H | H | H | H | CF₃CF₂ | CF₃ | CH₃ |
| 284 | Am | H | H | H | Ethyl | CH₃ | H | H | H | H | CF₃ | CF₃ | CH₃ |
| 285 | Am | H | H | H | Ethyl | CH₃ | H | F | H | H | CF₃CF₂ | CF₃ | CH₃ |
| 286 | Am | H | H | H | Ethyl | CH₃ | H | Cl | H | H | CF₃CF₂ | CF₃ | CH₃ |
| 287 | Am | H | H | H | Ethyl | CH₃ | H | H | H | Cl | CF₃CF₂ | CF₃ | CH₃ |
| 288 | Am | H | H | H | Ethyl | CH₃ | H | H | H | H | CF₃CF₂ | CF₃ | Ethyl |
| 289 | Am | CH₃ | H | H | Cyclopropylmethyl | CH₃ | H | H | F | H | CF₃CF₂ | CF₃ | CH₃ |
| 290 | Am | H | H | H | Cyclopropylmethyl | CH₃ | H | H | Cl | H | CF₃CF₂ | CF₃ | CH₃ |
| 291 | Am | H | H | H | Cyclopropylmethyl | CH₃ | H | F | F | H | CF₃CF₂ | CF₃ | CH₃ |
| 292 | Am | H | H | H | Cyclopropylmethyl | CH₃ | H | Cl | F | H | CF₃CF₂ | CF₃ | CH₃ |
| 293 | Am | H | H | H | Cyclopropylmethyl | CH₃ | H | H | F | Cl | CF₃CF₂ | CF₃ | CH₃ |
| 294 | Am | H | H | H | Cyclopropylmethyl | CH₃ | H | H | Br | H | CF₃CF₂ | CF₃ | CH₃ |
| 295 | Am | H | H | H | Cyclopropylmethyl | CH₃ | H | H | F | H | CF₃ | CF₃ | CH₃ |
| 296 | Am | H | H | H | Cyclopropylmethyl | CH₃ | H | H | F | H | CF₃CF₂ | CF₃ | Ethyl |
| 297 | Am | CH₃ | H | H | Cyclopropyl | H | H | H | F | H | CF₃CF₂ | CF₃ | CH₃ |
| 298 | Am | H | H | H | Cyclopropyl | H | H | H | F | H | CF₃ | CF₃ | CH₃ |
| 299 | Am | H | H | H | Cyclopropyl | H | H | F | F | H | CF₃CF₂ | CF₃ | CH₃ |
| 300 | Am | H | H | H | Cyclopropyl | H | H | Cl | F | H | CF₃CF₂ | CF₃ | CH₃ |
| 301 | Am | H | H | H | Cyclopropyl | H | H | H | F | Cl | CF₃CF₂ | CF₃ | CH₃ |
| 302 | Am | H | H | H | Cyclopropyl | H | H | H | F | H | CF₃CF₂ | CF₃ | Ethyl |
| 303 | Am | H | H | H | Cyclopropyl | H | H | H | Br | H | CF₃CF₂ | CF₃ | CH₃ |
| 304 | Am | CH₃ | H | H | Cyclopropylmethyl | H | H | H | F | H | CF₃CF₂ | CF₃ | CH₃ |
| 305 | Am | H | H | H | Cyclopropylmethyl | H | H | H | F | H | CF₃ | CF₃ | CH₃ |
| 306 | Am | H | H | H | Cyclopropylmethyl | H | H | F | F | H | CF₃CF₂ | CF₃ | CH₃ |
| 307 | Am | H | H | H | Cyclopropylmethyl | H | H | Cl | F | H | CF₃CF₂ | CF₃ | CH₃ |
| 308 | Am | H | H | H | Cyclopropylmethyl | H | H | H | F | Cl | CF₃CF₂ | CF₃ | CH₃ |
| 309 | Am | H | H | H | Cyclopropylmethyl | H | H | H | F | H | CF₃CF₂ | CF₃ | Ethyl |
| 310 | Am | H | H | H | Cyclopropylmethyl | H | H | H | Br | H | CF₃CF₂ | CF₃ | CH₃ |
| 311 | Am | CH₃ | H | H | Ethyl | H | H | H | H | H | CF₃CF₂ | CF₃ | CH₃ |
| 312 | Am | H | H | H | Ethyl | H | H | H | H | H | CF₃ | CF₃ | CH₃ |
| 313 | Am | H | H | H | Ethyl | H | H | Cl | H | H | CF₃CF₂ | CF₃ | CH₃ |
| 314 | Am | H | H | H | Ethyl | H | H | H | H | Cl | CF₃CF₂ | CF₃ | CH₃ |
| 315 | Am | H | H | H | Ethyl | H | H | H | H | H | CF₃CF₂ | CF₃ | Ethyl |
| 316 | Am | H | H | H | Propyl | H | H | H | H | H | CF₃CF₂ | CF₃ | CH₃ |
| 317 | Am | CH₃ | H | H | Cyclopropyl | H | H | H | Cl | H | CF₃CF₂ | CF₃ | CH₃ |
| 318 | Am | CH₃ | H | H | Cyclopropylmethyl | H | H | H | Cl | F | CF₃CF₂ | CF₃ | CH₃ |
| 319 | Am | CH₃ | H | H | Cyclopropylmethyl | H | H | H | Cl | H | CF₃ | CF₃ | CH₃ |
| 320 | Am | CH₃ | H | H | Cyclopropylmethyl | CH₃ | H | H | Cl | H | CF₃CF₂ | CF₃ | CH₃ |
| 321 | Am | CH₃ | H | H | Cyclopropylmethyl | Ethyl | H | H | Cl | H | CF₃CF₂ | CF₃ | CH₃ |
| 322 | Am | CH₃ | H | H | Cyclopropylmethyl | H | H | H | H | H | CF₃CF₂ | CF₃ | CH₃ |
| 323 | Am | CH₃ | H | H | Propyl | H | H | H | Cl | H | CF₃CF₂ | CF₃ | CH₃ |
| 324 | Am | CH₃ | H | H | Cyclopropylmethyl | H | H | F | Cl | H | CF₃CF₂ | CF₃ | CH₃ |
| 325 | Am | CH₃ | H | H | Cyclopropylmethyl | H | H | Cl | Cl | H | CF₃CF₂ | CF₃ | CH₃ |
| 326 | Am | CH₃ | H | H | Cyclopropylmethyl | H | H | H | Cl | Cl | CF₃CF₂ | CF₃ | CH₃ |
| 327 | Am | CH₃ | H | H | Cyclopropylmethyl | H | H | H | Cl | H | CF₃CF₂ | CF₃ | Ethyl |
| 328 | Am | CH₃ | H | H | Cyclopropylmethyl | H | H | H | Br | H | CF₃CF₂ | CF₃ | CH₃ |
| 329 | Am | CH₃ | H | H | CH₃ | CH₃ | H | H | H | H | CF₃CF₂ | CF₃ | CH₃ |
| 330 | Am | H | H | H | CH₃ | CH₃ | H | H | H | H | CF₃ | CF₃ | CH₃ |
| 331 | Am | H | H | H | CH₃ | CH₃ | H | F | H | H | CF₃CF₂ | CF₃ | CH₃ |
| 332 | Am | H | H | H | CH₃ | CH₃ | H | Cl | H | H | CF₃CF₂ | CF₃ | CH₃ |
| 333 | Am | H | H | H | CH₃ | CH₃ | H | H | H | Cl | CF₃CF₂ | CF₃ | CH₃ |
| 334 | Am | H | H | H | CH₃ | CH₃ | H | H | H | H | CF₃CF₂ | CF₃ | Ethyl |
| 335 | Am | CH₃ | H | H | CH₃ | H | H | H | F | F | CF₃ | CF₃ | CH₃ |
| 336 | Am | CH₃ | H | H | CH₃ | H | H | H | F | H | CF₃ | CF₃ | CH₃ |
| 337 | Am | CH₃ | H | H | CH₃ | CH₃ | H | H | F | F | CF₃ | CF₃ | CH₃ |
| 338 | Am | CH₃ | H | H | CH₃ | CH₃ | H | H | F | H | CF₃ | CF₃ | CH₃ |
| 339 | Am | CH₃ | H | H | CH₃ | Ethyl | H | H | F | F | CF₃CF₂ | CF₃ | CH₃ |
| 340 | Am | H | H | H | CH₃ | Ethyl | H | H | F | F | CF₃ | CF₃ | CH₃ |
| 341 | Am | CH₃ | H | H | CH₃ | Ethyl | H | H | F | F | CF₃ | CF₃ | CH₃ |
| 342 | Am | CH₃ | H | H | CH₃ | Ethyl | H | H | F | H | CF₃ | CF₃ | CH₃ |
| 343 | Am | CH₃ | H | H | CH₃ | H | H | H | Cl | F | CF₃CF₂ | CF₃ | CH₃ |
| 344 | Am | H | H | H | CH₃ | H | H | H | Cl | F | CF₃ | CF₃ | CH₃ |
| 345 | Am | CH₃ | H | H | CH₃ | H | H | H | Cl | F | CF₃ | CF₃ | CH₃ |
| 346 | Am | H | H | H | CH₃ | H | H | H | Cl | H | CF₃ | CF₃ | CH₃ |
| 347 | Am | CH₃ | H | H | CH₃ | H | H | H | Cl | H | CF₃ | CF₃ | CH₃ |
| 348 | Am | CH₃ | H | H | CH₃ | CH₃ | H | H | Cl | F | CF₃CF₂ | CF₃ | CH₃ |
| 349 | Am | H | H | H | CH₃ | CH₃ | H | H | Cl | F | CF₃ | CF₃ | CH₃ |
| 350 | Am | CH₃ | H | H | CH₃ | CH₃ | H | H | Cl | F | CF₃ | CF₃ | CH₃ |
| 351 | Am | CH₃ | H | H | CH₃ | CH₃ | H | H | Cl | H | CF₃CF₂ | CF₃ | CH₃ |
| 352 | Am | H | H | H | CH₃ | CH₃ | H | H | Cl | H | CF₃ | CF₃ | CH₃ |
| 353 | Am | CH₃ | H | H | CH₃ | CH₃ | H | H | Cl | H | CF₃ | CF₃ | CH₃ |
| 354 | Am | H | H | H | CH₃ | Ethyl | H | H | Cl | F | CF₃CF₂ | CF₃ | CH₃ |
| 355 | Am | CH₃ | H | H | CH₃ | Ethyl | H | H | Cl | F | CF₃CF₂ | CF₃ | CH₃ |
| 356 | Am | H | H | H | CH₃ | Ethyl | H | H | Cl | F | CF₃ | CF₃ | CH₃ |
| 357 | Am | CH₃ | H | H | CH₃ | Ethyl | H | H | Cl | F | CF₃ | CF₃ | CH₃ |
| 358 | Am | CH₃ | H | H | CH₃ | Ethyl | H | H | Cl | H | CF₃CF₂ | CF₃ | CH₃ |
| 359 | Am | H | H | H | CH₃ | Ethyl | H | H | Cl | H | CF₃ | CF₃ | CH₃ |
| 360 | Am | CH₃ | H | H | CH₃ | Ethyl | H | H | Cl | H | CF₃ | CF₃ | CH₃ |
| 361 | Am | CH₃ | H | H | CH₃ | H | H | H | H | F | CF₃CF₂ | CF₃ | CH₃ |
| 362 | Am | H | H | H | CH₃ | H | H | H | H | F | CF₃ | CF₃ | CH₃ |
| 363 | Am | CH₃ | H | H | CH₃ | H | H | H | H | F | CF₃ | CF₃ | CH₃ |
| 364 | Am | CH₃ | H | H | CH₃ | H | H | H | H | H | CF₃CF₂ | CF₃ | CH₃ |
| 365 | Am | H | H | H | CH₃ | H | H | H | H | H | CF₃ | CF₃ | CH₃ |
| 366 | Am | CH₃ | H | H | CH₃ | H | H | H | H | H | CF₃ | CF₃ | CH₃ |
| 367 | Am | CH₃ | H | H | CH₃ | CH₃ | H | H | H | F | CF₃CF₂ | CF₃ | CH₃ |
| 368 | Am | H | H | H | CH₃ | CH₃ | H | H | H | F | CF₃ | CF₃ | CH₃ |
| 369 | Am | CH₃ | H | H | CH₃ | CH₃ | H | H | H | F | CF₃ | CF₃ | CH₃ |
| 370 | Am | CH₃ | H | H | CH₃ | CH₃ | H | H | H | H | CF₃ | CF₃ | CH₃ |
| 371 | Am | H | H | H | CH₃ | Ethyl | H | H | H | F | CF₃CF₂ | CF₃ | CH₃ |
| 372 | Am | CH₃ | H | H | CH₃ | Ethyl | H | H | H | F | CF₃CF₂ | CF₃ | CH₃ |
| 373 | Am | H | H | H | CH₃ | Ethyl | H | H | H | F | CF₃ | CF₃ | CH₃ |
| 374 | Am | CH₃ | H | H | CH₃ | Ethyl | H | H | H | F | CF₃ | CF₃ | CH₃ |
| 375 | Am | CH₃ | H | H | CH₃ | Ethyl | H | H | H | H | CF₃CF₂ | CF₃ | CH₃ |
| 376 | Am | H | H | H | CH₃ | Ethyl | H | H | H | H | CF₃ | CF₃ | CH₃ |
| 377 | Am | CH₃ | H | H | CH₃ | Ethyl | H | H | H | H | CF₃ | CF₃ | CH₃ |
| 378 | Am | CH₃ | H | H | Ethyl | H | H | H | F | F | CF₃ | CF₃ | CH₃ |
| 379 | Am | CH₃ | H | H | Ethyl | H | H | H | F | H | CF₃ | CF₃ | CH₃ |
| 380 | Am | CH₃ | H | H | Ethyl | CH₃ | H | H | F | F | CF₃ | CF₃ | CH₃ |
| 381 | Am | CH₃ | H | H | Ethyl | CH₃ | H | H | F | H | CF₃CF₂ | CF₃ | CH₃ |
| 382 | Am | CH₃ | H | H | Ethyl | CH₃ | H | H | F | H | CF₃ | CF₃ | CH₃ |
| 383 | Am | CH₃ | H | H | Ethyl | Ethyl | H | H | F | F | CF₃CF₂ | CF₃ | CH₃ |
| 384 | Am | H | H | H | Ethyl | Ethyl | H | H | F | F | CF₃ | CF₃ | CH₃ |
| 385 | Am | CH₃ | H | H | Ethyl | Ethyl | H | H | F | F | CF₃ | CF₃ | CH₃ |
| 386 | Am | CH₃ | H | H | Ethyl | Ethyl | H | H | F | H | CF₃ | CF₃ | CH₃ |
| 387 | Am | CH₃ | H | H | Ethyl | H | H | H | Cl | F | CF₃CF₂ | CF₃ | CH₃ |
| 388 | Am | H | H | H | Ethyl | H | H | H | Cl | F | CF₃ | CF₃ | CH₃ |
| 389 | Am | CH₃ | H | H | Ethyl | H | H | H | Cl | F | CF₃ | CF₃ | CH₃ |
| 390 | Am | H | H | H | Ethyl | H | H | H | Cl | H | CF₃ | CF₃ | CH₃ |
| 391 | Am | CH₃ | H | H | Ethyl | H | H | H | Cl | H | CF₃ | CF₃ | CH₃ |
| 392 | Am | CH₃ | H | H | Ethyl | CH₃ | H | H | Cl | F | CF₃CF₂ | CF₃ | CH₃ |
| 393 | Am | H | H | H | Ethyl | CH₃ | H | H | Cl | F | CF₃ | CF₃ | CH₃ |
| 394 | Am | CH₃ | H | H | Ethyl | CH₃ | H | H | Cl | F | CF₃ | CF₃ | CH₃ |
| 395 | Am | CH₃ | H | H | Ethyl | CH₃ | H | H | Cl | H | CF₃CF₂ | CF₃ | CH₃ |
| 396 | Am | H | H | H | Ethyl | CH₃ | H | H | Cl | H | CF₃ | CF₃ | CH₃ |
| 397 | Am | CH₃ | H | H | Ethyl | CH₃ | H | H | Cl | H | CF₃ | CF₃ | CH₃ |
| 398 | Am | H | H | H | Ethyl | Ethyl | H | H | Cl | F | CF₃CF₂ | CF₃ | CH₃ |
| 399 | Am | CH₃ | H | H | Ethyl | Ethyl | H | H | Cl | F | CF₃CF₂ | CF₃ | CH₃ |
| 400 | Am | H | H | H | Ethyl | Ethyl | H | H | Cl | F | CF₃ | CF₃ | CH₃ |
| 401 | Am | CH₃ | H | H | Ethyl | Ethyl | H | H | Cl | F | CF₃ | CF₃ | CH₃ |
| 402 | Am | CH₃ | H | H | Ethyl | Ethyl | H | H | Cl | H | CF₃CF₂ | CF₃ | CH₃ |
| 403 | Am | H | H | H | Ethyl | Ethyl | H | H | Cl | H | CF₃ | CF₃ | CH₃ |
| 404 | Am | CH₃ | H | H | Ethyl | Ethyl | H | H | Cl | H | CF₃ | CF₃ | CH₃ |
| 405 | Am | CH₃ | H | H | Ethyl | H | H | H | H | F | CF₃CF₂ | CF₃ | CH₃ |
| 406 | Am | H | H | H | Ethyl | H | H | H | H | F | CF₃ | CF₃ | CH₃ |
| 407 | Am | CH₃ | H | H | Ethyl | H | H | H | H | F | CF₃ | CF₃ | CH₃ |
| 408 | Am | CH₃ | H | H | Ethyl | H | H | H | H | H | CF₃ | CF₃ | CH₃ |
| 409 | Am | CH₃ | H | H | Ethyl | CH₃ | H | H | H | F | CF₃CF₂ | CF₃ | CH₃ |
| 410 | Am | H | H | H | Ethyl | CH₃ | H | H | H | F | CF₃ | CF₃ | CH₃ |
| 411 | Am | CH₃ | H | H | Ethyl | CH₃ | H | H | H | F | CF₃ | CF₃ | CH₃ |
| 412 | Am | CH₃ | H | H | Ethyl | CH₃ | H | H | H | H | CF₃ | CF₃ | CH₃ |
| 413 | Am | H | H | H | Ethyl | Ethyl | H | H | H | F | CF₃CF₂ | CF₃ | CH₃ |
| 414 | Am | CH₃ | H | H | Ethyl | Ethyl | H | H | H | F | CF₃CF₂ | CF₃ | CH₃ |
| 415 | Am | H | H | H | Ethyl | Ethyl | H | H | H | F | CF₃ | CF₃ | CH₃ |
| 416 | Am | CH₃ | H | H | Ethyl | Ethyl | H | H | H | F | CF₃ | CF₃ | CH₃ |
| 417 | Am | CH₃ | H | H | Ethyl | Ethyl | H | H | H | H | CF₃CF₂ | CF₃ | CH₃ |
| 418 | Am | H | H | H | Ethyl | Ethyl | H | H | H | H | CF₃ | CF₃ | CH₃ |
| 419 | Am | CH₃ | H | H | Ethyl | Ethyl | H | H | H | H | CF₃ | CF₃ | CH₃ |
| 420 | Am | CH₃ | H | H | Cyclopropyl | H | H | H | F | F | CF₃CF₂ | CF₃ | CH₃ |
| 421 | Am | H | H | H | Cyclopropyl | H | H | H | F | F | CF₃ | CF₃ | CH₃ |
| 422 | Am | CH₃ | H | H | Cyclopropyl | H | H | H | F | F | CF₃ | CF₃ | CH₃ |
| 423 | Am | CH₃ | H | H | Cyclopropyl | H | H | H | F | H | CF₃ | CF₃ | CH₃ |
| 424 | Am | CH₃ | H | H | Cyclopropyl | CH₃ | H | H | F | F | CF₃CF₂ | CF₃ | CH₃ |
| 425 | Am | H | H | H | Cyclopropyl | CH₃ | H | H | F | F | CF₃ | CF₃ | CH₃ |
| 426 | Am | CH₃ | H | H | Cyclopropyl | CH₃ | H | H | F | F | CF₃ | CF₃ | CH₃ |
| 427 | Am | CH₃ | H | H | Cyclopropyl | CH₃ | H | H | F | H | CF₃ | CF₃ | CH₃ |
| 428 | Am | CH₃ | H | H | Cyclopropyl | Ethyl | H | H | F | F | CF₃CF₂ | CF₃ | CH₃ |
| 429 | Am | H | H | H | Cyclopropyl | Ethyl | H | H | F | F | CF₃ | CF₃ | CH₃ |
| 430 | Am | CH₃ | H | H | Cyclopropyl | Ethyl | H | H | F | F | CF₃ | CF₃ | CH₃ |
| 431 | Am | CH₃ | H | H | Cyclopropyl | Ethyl | H | H | F | H | CF₃ | CF₃ | CH₃ |
| 432 | Am | H | H | H | Cyclopropyl | H | H | H | Cl | F | CF₃CF₂ | CF₃ | CH₃ |
| 433 | Am | CH₃ | H | H | Cyclopropyl | H | H | H | Cl | F | CF₃CF₂ | CF₃ | CH₃ |
| 434 | Am | H | H | H | Cyclopropyl | H | H | H | Cl | F | CF₃ | CF₃ | CH₃ |
| 435 | Am | CH₃ | H | H | Cyclopropyl | H | H | H | Cl | F | CF₃ | CF₃ | CH₃ |
| 436 | Am | H | H | H | Cyclopropyl | H | H | H | Cl | H | CF₃ | CF₃ | CH₃ |
| 437 | Am | CH₃ | H | H | Cyclopropyl | H | H | H | Cl | H | CF₃ | CF₃ | CH₃ |
| 438 | Am | H | H | H | Cyclopropyl | CH₃ | H | H | Cl | F | CF₃CF₂ | CF₃ | CH₃ |
| 439 | Am | CH₃ | H | H | Cyclopropyl | CH₃ | H | H | Cl | F | CF₃CF₂ | CF₃ | CH₃ |
| 440 | Am | H | H | H | Cyclopropyl | CH₃ | H | H | Cl | F | CF₃ | CF₃ | CH₃ |
| 441 | Am | CH₃ | H | H | Cyclopropyl | CH₃ | H | H | Cl | F | CF₃ | CF₃ | CH₃ |
| 442 | Am | CH₃ | H | H | Cyclopropyl | CH₃ | H | H | Cl | H | CF₃CF₂ | CF₃ | CH₃ |
| 443 | Am | H | H | H | Cyclopropyl | CH₃ | H | H | Cl | H | CF₃ | CF₃ | CH₃ |
| 444 | Am | CH₃ | H | H | Cyclopropyl | CH₃ | H | H | Cl | H | CF₃ | CF₃ | CH₃ |
| 445 | Am | H | H | H | Cyclopropyl | Ethyl | H | H | Cl | F | CF₃CF₂ | CF₃ | CH₃ |
| 446 | Am | CH₃ | H | H | Cyclopropyl | Ethyl | H | H | Cl | F | CF₃CF₂ | CF₃ | CH₃ |
| 447 | Am | H | H | H | Cyclopropyl | Ethyl | H | H | Cl | F | CF₃ | CF₃ | CH₃ |
| 448 | Am | CH₃ | H | H | Cyclopropyl | Ethyl | H | H | Cl | F | CF₃ | CF₃ | CH₃ |
| 449 | Am | H | H | H | Cyclopropyl | Ethyl | H | H | Cl | H | CF₃CF₂ | CF₃ | CH₃ |
| 450 | Am | CH₃ | H | H | Cyclopropyl | Ethyl | H | H | Cl | H | CF₃CF₂ | CF₃ | CH₃ |
| 451 | Am | H | H | H | Cyclopropyl | Ethyl | H | H | Cl | H | CF₃ | CF₃ | CH₃ |
| 452 | Am | CH₃ | H | H | Cyclopropyl | Ethyl | H | H | Cl | H | CF₃ | CF₃ | CH₃ |
| 453 | Am | H | H | H | Cyclopropyl | H | H | H | H | F | CF₃CF₂ | CF₃ | CH₃ |
| 454 | Am | CH₃ | H | H | Cyclopropyl | H | H | H | H | F | CF₃CF₂ | CF₃ | CH₃ |
| 455 | Am | H | H | H | Cyclopropyl | H | H | H | H | F | CF₃ | CF₃ | CH₃ |
| 456 | Am | CH₃ | H | H | Cyclopropyl | H | H | H | H | F | CF₃ | CF₃ | CH₃ |
| 457 | Am | CH₃ | H | H | Cyclopropyl | H | H | H | H | H | CF₃CF₂ | CF₃ | CH₃ |
| 458 | Am | H | H | H | Cyclopropyl | H | H | H | H | H | CF₃ | CF₃ | CH₃ |
| 459 | Am | CH₃ | H | H | Cyclopropyl | H | H | H | H | H | CF₃ | CF₃ | CH₃ |
| 460 | Am | CH₃ | H | H | Cyclopropyl | CH₃ | H | H | H | F | CF₃CF₂ | CF₃ | CH₃ |
| 461 | Am | H | H | H | Cyclopropyl | CH₃ | H | H | H | F | CF₃ | CF₃ | CH₃ |
| 462 | Am | CH₃ | H | H | Cyclopropyl | CH₃ | H | H | H | F | CF₃ | CF₃ | CH₃ |
| 463 | Am | CH₃ | H | H | Cyclopropyl | CH₃ | H | H | H | H | CF₃ | CF₃ | CH₃ |
| 464 | Am | H | H | H | Cyclopropyl | Ethyl | H | H | H | F | CF₃CF₂ | CF₃ | CH₃ |
| 465 | Am | CH₃ | H | H | Cyclopropyl | Ethyl | H | H | H | F | CF₃CF₂ | CF₃ | CH₃ |
| 466 | Am | H | H | H | Cyclopropyl | Ethyl | H | H | H | F | CF₃ | CF₃ | CH₃ |
| 467 | Am | CH₃ | H | H | Cyclopropyl | Ethyl | H | H | H | F | CF₃ | CF₃ | CH₃ |
| 468 | Am | CH₃ | H | H | Cyclopropyl | Ethyl | H | H | H | H | CF₃CF₂ | CF₃ | CH₃ |
| 469 | Am | H | H | H | Cyclopropyl | Ethyl | H | H | H | H | CF₃ | CF₃ | CH₃ |
| 470 | Am | CH₃ | H | H | Cyclopropyl | Ethyl | H | H | H | H | CF₃ | CF₃ | CH₃ |
| 471 | Am | CH₃ | H | H | Cyclopropylmethyl | H | H | H | F | F | CF₃CF₂ | CF₃ | CH₃ |
| 472 | Am | H | H | H | Cyclopropylmethyl | H | H | H | F | F | CF₃ | CF₃ | CH₃ |
| 473 | Am | CH₃ | H | H | Cyclopropylmethyl | H | H | H | F | F | CF₃ | CF₃ | CH₃ |
| 474 | Am | CH₃ | H | H | Cyclopropylmethyl | H | H | H | F | H | CF₃ | CF₃ | CH₃ |
| 475 | Am | CH₃ | H | H | Cyclopropylmethyl | CH₃ | H | H | F | F | CF₃CF₂ | CF₃ | CH₃ |
| 476 | Am | H | H | H | Cyclopropylmethyl | CH₃ | H | H | F | F | CF₃ | CF₃ | CH₃ |
| 477 | Am | CH₃ | H | H | Cyclopropylmethyl | CH₃ | H | H | F | F | CF₃ | CF₃ | CH₃ |
| 478 | Am | CH₃ | H | H | Cyclopropylmethyl | CH₃ | H | H | F | H | CF₃ | CF₃ | CH₃ |
| 479 | Am | H | H | H | Cyclopropylmethyl | Ethyl | H | H | F | F | CF₃CF₂ | CF₃ | CH₃ |
| 480 | Am | CH₃ | H | H | Cyclopropylmethyl | Ethyl | H | H | F | F | CF₃CF₂ | CF₃ | CH₃ |
| 481 | Am | H | H | H | Cyclopropylmethyl | Ethyl | H | H | F | F | CF₃ | CF₃ | CH₃ |
| 482 | Am | CH₃ | H | H | Cyclopropylmethyl | Ethyl | H | H | F | F | CF₃ | CF₃ | CH₃ |
| 483 | Am | CH₃ | H | H | Cyclopropylmethyl | Ethyl | H | H | F | H | CF₃CF₂ | CF₃ | CH₃ |
| 484 | Am | H | H | H | Cyclopropylmethyl | Ethyl | H | H | F | H | CF₃ | CF₃ | CH₃ |
| 485 | Am | CH₃ | H | H | Cyclopropylmethyl | Ethyl | H | H | F | H | CF₃ | CF₃ | CH₃ |
| 486 | Am | H | H | H | Cyclopropylmethyl | H | H | H | Cl | F | CF₃CF₂ | CF₃ | CH₃ |
| 487 | Am | H | H | H | Cyclopropylmethyl | H | H | H | Cl | F | CF₃ | CF₃ | CH₃ |
| 488 | Am | CH₃ | H | H | Cyclopropylmethyl | H | H | H | Cl | F | CF₃ | CF₃ | CH₃ |
| 489 | Am | H | H | H | Cyclopropylmethyl | H | H | H | Cl | H | CF₃ | CF₃ | CH₃ |
| 490 | Am | H | H | H | Cyclopropylmethyl | CH₃ | H | H | Cl | F | CF₃CF₂ | CF₃ | CH₃ |
| 491 | Am | CH₃ | H | H | Cyclopropylmethyl | CH₃ | H | H | Cl | F | CF₃CF₂ | CF₃ | CH₃ |
| 492 | Am | H | H | H | Cyclopropylmethyl | CH₃ | H | H | Cl | F | CF₃ | CF₃ | CH₃ |
| 493 | Am | CH₃ | H | H | Cyclopropylmethyl | CH₃ | H | H | Cl | F | CF₃ | CF₃ | CH₃ |
| 494 | Am | H | H | H | Cyclopropylmethyl | CH₃ | H | H | Cl | H | CF₃ | CF₃ | CH₃ |
| 495 | Am | CH₃ | H | H | Cyclopropylmethyl | CH₃ | H | H | Cl | H | CF₃ | CF₃ | CH₃ |
| 496 | Am | H | H | H | Cyclopropylmethyl | Ethyl | H | H | Cl | F | CF₃CF₂ | CF₃ | CH₃ |
| 497 | Am | CH₃ | H | H | Cyclopropylmethyl | Ethyl | H | H | Cl | F | CF₃CF₂ | CF₃ | CH₃ |
| 498 | Am | H | H | H | Cyclopropylmethyl | Ethyl | H | H | Cl | F | CF₃ | CF₃ | CH₃ |
| 499 | Am | CH₃ | H | H | Cyclopropylmethyl | Ethyl | H | H | Cl | F | CF₃ | CF₃ | CH₃ |
| 500 | Am | H | H | H | Cyclopropylmethyl | Ethyl | H | H | Cl | H | CF₃CF₂ | CF₃ | CH₃ |
| 501 | Am | H | H | H | Cyclopropylmethyl | Ethyl | H | H | Cl | H | CF₃ | CF₃ | CH₃ |
| 502 | Am | CH₃ | H | H | Cyclopropylmethyl | Ethyl | H | H | Cl | H | CF₃ | CF₃ | CH₃ |
| 503 | Am | H | H | H | Cyclopropylmethyl | H | H | H | H | F | CF₃CF₂ | CF₃ | CH₃ |
| 504 | Am | CH₃ | H | H | Cyclopropylmethyl | H | H | H | H | F | CF₃CF₂ | CF₃ | CH₃ |
| 505 | Am | H | H | H | Cyclopropylmethyl | H | H | H | H | F | CF₃ | CF₃ | CH₃ |
| 506 | Am | CH₃ | H | H | Cyclopropylmethyl | H | H | H | H | F | CF₃ | CF₃ | CH₃ |
| 507 | Am | H | H | H | Cyclopropylmethyl | H | H | H | H | H | CF₃ | CF₃ | CH₃ |
| 508 | Am | CH₃ | H | H | Cyclopropylmethyl | H | H | H | H | H | CF₃ | CF₃ | CH₃ |
| 509 | Am | H | H | H | Cyclopropylmethyl | CH₃ | H | H | H | F | CF₃CF₂ | CF₃ | CH₃ |
| 510 | Am | CH₃ | H | H | Cyclopropylmethyl | CH₃ | H | H | H | F | CF₃CF₂ | CF₃ | CH₃ |
| 511 | Am | H | H | H | Cyclopropylmethyl | CH₃ | H | H | H | F | CF₃ | CF₃ | CH₃ |
| 512 | Am | CH₃ | H | H | Cyclopropylmethyl | CH₃ | H | H | H | F | CF₃ | CF₃ | CH₃ |
| 513 | Am | CH₃ | H | H | Cyclopropylmethyl | CH₃ | H | H | H | H | CF₃CF₂ | CF₃ | CH₃ |
| 514 | Am | H | H | H | Cyclopropylmethyl | CH₃ | H | H | H | H | CF₃ | CF₃ | CH₃ |
| 515 | Am | CH₃ | H | H | Cyclopropylmethyl | CH₃ | H | H | H | H | CF₃ | CF₃ | CH₃ |
| 516 | Am | H | H | H | Cyclopropylmethyl | Ethyl | H | H | H | F | CF₃CF₂ | CF₃ | CH₃ |
| 517 | Am | CH₃ | H | H | Cyclopropylmethyl | Ethyl | H | H | H | F | CF₃CF₂ | CF₃ | CH₃ |
| 518 | Am | H | H | H | Cyclopropylmethyl | Ethyl | H | H | H | F | CF₃ | CF₃ | CH₃ |
| 519 | Am | CH₃ | H | H | Cyclopropylmethyl | Ethyl | H | H | H | F | CF₃ | CF₃ | CH₃ |
| 520 | Am | H | H | H | Cyclopropylmethyl | Ethyl | H | H | H | H | CF₃CF₂ | CF₃ | CH₃ |
| 521 | Am | CH₃ | H | H | Cyclopropylmethyl | Ethyl | H | H | H | H | CF₃CF₂ | CF₃ | CH₃ |
| 522 | Am | H | H | H | Cyclopropylmethyl | Ethyl | H | H | H | H | CF₃ | CF₃ | CH₃ |
| 523 | Am | CH₃ | H | H | Cyclopropylmethyl | Ethyl | H | H | H | H | CF₃ | CF₃ | CH₃ |
| 524 | Sm | H | H | H | Ethyl | CH₃ | H | H | F | H | CF₃CF₂ | CF₃ | CH₃ |
| 525 | Sm | H | H | H | Ethyl | CH₃ | H | H | F | H | CF₃ | CF₃ | CH₃ |
| 526 | Sm | H | H | H | Ethyl | CH₃ | H | H | Cl | H | CF₃CF₂ | CF₃ | CH₃ |
| 527 | Sm | H | H | H | Ethyl | CH₃ | H | H | F | H | CF₃CF₂ | CF₃ | Ethyl |
| 528 | Sm | H | H | H | Ethyl | CH₃ | H | H | F | Cl | CF₃CF₂ | CF₃ | CH₃ |
| 529 | Sm | H | H | H | Ethyl | CH₃ | H | H | Br | H | CF₃CF₂ | CF₃ | CH₃ |
| 530 | Sm | H | H | H | Ethyl | CH₃ | H | F | F | H | CF₃CF₂ | CF₃ | CH₃ |
| 531 | Sm | H | H | H | Ethyl | CH₃ | H | Cl | F | H | CF₃CF₂ | CF₃ | CH₃ |
| 532 | Sm | H | H | H | Propyl | CH₃ | H | H | F | H | CF₃CF₂ | CF₃ | CH₃ |
| 533 | Sm | H | H | H | Cyclopropyl | CH₃ | H | H | F | H | CF₃CF₂ | CF₃ | CH₃ |
| 534 | Sm | H | H | H | Cyclopropyl | CH₃ | H | H | F | F | CF₃CF₂ | CF₃ | CH₃ |
| 535 | Sm | CH₃ | H | H | Cyclopropyl | CH₃ | H | H | F | H | CF₃CF₂ | CF₃ | CH₃ |
| 536 | Sm | H | H | H | Cyclopropyl | CH₃ | H | H | F | H | CF₃ | CF₃ | CH₃ |
| 537 | Sm | H | H | H | Cyclopropyl | CH₃ | H | H | Cl | H | CF₃CF₂ | CF₃ | CH₃ |
| 538 | Sm | H | H | H | Cyclopropyl | CH₃ | H | H | F | H | CF₃CF₂ | CF₃ | Ethyl |
| 539 | Sm | H | H | H | Cyclopropyl | CH₃ | H | H | F | Cl | CF₃CF₂ | CF₃ | CH₃ |
| 540 | Sm | H | H | H | Cyclopropyl | CH₃ | H | H | Br | H | CF₃CF₂ | CF₃ | CH₃ |
| 541 | Sm | H | H | H | Cyclopropyl | CH₃ | H | F | F | H | CF₃CF₂ | CF₃ | CH₃ |
| 542 | Sm | H | H | H | Cyclopropyl | CH₃ | H | Cl | F | H | CF₃CF₂ | CF₃ | CH₃ |
| 543 | Sm | H | H | H | Ethyl | Ethyl | H | H | F | H | CF₃CF₂ | CF₃ | CH₃ |
| 544 | Sm | H | H | H | Ethyl | Ethyl | H | H | F | F | CF₃CF₂ | CF₃ | CH₃ |
| 545 | Sm | CH₃ | H | H | Ethyl | Ethyl | H | H | F | H | CF₃CF₂ | CF₃ | CH₃ |
| 546 | Sm | H | H | H | Ethyl | Ethyl | H | H | F | H | CF₃ | CF₃ | CH₃ |
| 547 | Sm | H | H | H | Ethyl | Ethyl | H | H | Cl | H | CF₃CF₂ | CF₃ | CH₃ |
| 548 | Sm | H | H | H | Ethyl | Ethyl | H | H | H | H | CF₃CF₂ | CF₃ | CH₃ |
| 549 | Sm | H | H | H | Cyclopropylmethyl | Ethyl | H | H | F | H | CF₃CF₂ | CF₃ | CH₃ |
| 550 | Sm | H | H | H | Ethyl | Ethyl | H | H | F | H | CF₃CF₂ | CF₃ | Ethyl |
| 551 | Sm | H | H | H | Ethyl | Ethyl | H | H | F | Cl | CF₃CF₂ | CF₃ | CH₃ |
| 552 | Sm | H | H | H | Ethyl | Ethyl | H | H | Br | H | CF₃CF₂ | CF₃ | CH₃ |
| 553 | Sm | H | H | H | Propyl | Ethyl | H | H | F | H | CF₃CF₂ | CF₃ | CH₃ |
| 554 | Sm | H | H | H | Ethyl | Ethyl | H | F | F | H | CF₃CF₂ | CF₃ | CH₃ |
| 555 | Sm | H | H | H | Ethyl | Ethyl | H | Cl | F | H | CF₃CF₂ | CF₃ | CH₃ |
| 556 | Sm | H | H | H | Ethyl | H | H | H | F | H | CF₃CF₂ | CF₃ | CH₃ |
| 557 | Sm | CH₃ | H | H | Ethyl | H | H | H | F | H | CF₃CF₂ | CF₃ | CH₃ |
| 558 | Sm | H | H | H | Ethyl | H | H | H | F | H | CF₃ | CF₃ | CH₃ |
| 559 | Sm | H | H | H | Ethyl | H | H | H | F | H | CF₃CF₂ | CF₃ | Ethyl |
| 560 | Sm | H | H | H | Ethyl | H | H | H | F | Cl | CF₃CF₂ | CF₃ | CH₃ |
| 561 | Sm | H | H | H | Ethyl | H | H | H | Br | H | CF₃CF₂ | CF₃ | CH₃ |
| 562 | Sm | H | H | H | Ethyl | H | H | F | F | H | CF₃CF₂ | CF₃ | CH₃ |
| 563 | Sm | H | H | H | Ethyl | H | H | Cl | F | H | CF₃CF₂ | CF₃ | CH₃ |
| 564 | Sm | H | H | H | Propyl | H | H | H | F | H | CF₃CF₂ | CF₃ | CH₃ |
| 565 | Sm | H | H | H | Ethyl | H | H | H | F | F | CF₃CF₂ | CF₃ | CH₃ |
| 566 | Sm | CH₃ | H | H | Ethyl | H | H | H | F | F | CF₃CF₂ | CF₃ | CH₃ |
| 567 | Sm | H | H | H | Ethyl | H | H | H | F | F | CF₃ | CF₃ | CH₃ |
| 568 | Sm | H | H | H | Ethyl | H | H | H | Cl | F | CF₃CF₂ | CF₃ | CH₃ |
| 569 | Sm | H | H | H | Ethyl | H | H | H | H | F | CF₃CF₂ | CF₃ | CH₃ |
| 570 | Sm | H | H | H | Cyclopropyl | H | H | H | F | F | CF₃CF₂ | CF₃ | CH₃ |
| 571 | Sm | H | H | H | Cyclopropylmethyl | H | H | H | F | F | CF₃CF₂ | CF₃ | CH₃ |
| 572 | Sm | H | H | H | Propyl | H | H | H | F | F | CF₃CF₂ | CF₃ | CH₃ |
| 573 | Sm | H | H | H | Ethyl | H | H | F | F | F | CF₃CF₂ | CF₃ | CH₃ |
| 574 | Sm | H | H | H | Ethyl | H | H | Cl | F | F | CF₃CF₂ | CF₃ | CH₃ |
| 575 | Sm | H | H | H | Ethyl | H | H | H | F | F | CF₃CF₂ | CF₃ | Ethyl |
| 576 | Sm | H | H | H | Ethyl | H | H | H | Br | F | CF₃CF₂ | CF₃ | CH₃ |
| 577 | Sm | H | H | H | CH₃ | CH₃ | H | H | F | F | CF₃CF₂ | CF₃ | CH₃ |
| 578 | Sm | CH₃ | H | H | CH₃ | CH₃ | H | H | F | F | CF₃CF₂ | CF₃ | CH₃ |
| 579 | Sm | H | H | H | CH₃ | CH₃ | H | H | F | F | CF₃ | CF₃ | CH₃ |
| 580 | Sm | H | H | H | CH₃ | Ethyl | H | H | F | F | CF₃CF₂ | CF₃ | CH₃ |
| 581 | Sm | H | H | H | CH₃ | CH₃ | H | H | Cl | F | CF₃CF₂ | CF₃ | CH₃ |
| 582 | Sm | H | H | H | CH₃ | CH₃ | H | H | H | F | CF₃CF₂ | CF₃ | CH₃ |
| 583 | Sm | H | H | H | Cyclopropylmethyl | CH₃ | H | H | F | F | CF₃CF₂ | CF₃ | CH₃ |
| 584 | Sm | H | H | H | Propyl | CH₃ | H | H | F | F | CF₃CF₂ | CF₃ | CH₃ |
| 585 | Sm | H | H | H | CH₃ | CH₃ | H | H | F | Cl | CF₃CF₂ | CF₃ | CH₃ |
| 586 | Sm | H | H | H | CH₃ | CH₃ | H | H | F | F | CF₃CF₂ | CF₃ | Ethyl |
| 587 | Sm | H | H | H | CH₃ | CH₃ | H | H | Br | F | CF₃CF₂ | CF₃ | CH₃ |
| 588 | Sm | H | H | H | CH₃ | CH₃ | H | F | F | F | CF₃CF₂ | CF₃ | CH₃ |
| 589 | Sm | H | H | H | CH₃ | CH₃ | H | Cl | F | F | CF₃CF₂ | CF₃ | CH₃ |
| 590 | Sm | H | H | H | CH₃ | CH₃ | H | H | F | H | CF₃CF₂ | CF₃ | CH₃ |
| 591 | Sm | CH₃ | H | H | CH₃ | CH₃ | H | H | F | H | CF₃CF₂ | CF₃ | CH₃ |
| 592 | Sm | H | H | H | CH₃ | CH₃ | H | H | F | H | CF₃ | CF₃ | CH₃ |
| 593 | Sm | H | H | H | CH₃ | CH₃ | H | H | Cl | H | CF₃CF₂ | CF₃ | CH₃ |
| 594 | Sm | H | H | H | CH₃ | CH₃ | H | H | Br | H | CF₃CF₂ | CF₃ | CH₃ |
| 595 | Sm | H | H | H | CH₃ | CH₃ | H | Cl | F | H | CF₃CF₂ | CF₃ | CH₃ |
| 596 | Sm | H | H | H | CH₃ | CH₃ | H | H | F | H | CF₃CF₂ | CF₃ | Ethyl |
| 597 | Sm | H | H | H | CH₃ | CH₃ | H | F | F | H | CF₃CF₂ | CF₃ | CH₃ |
| 598 | Sm | H | H | H | CH₃ | Ethyl | H | H | F | H | CF₃CF₂ | CF₃ | CH₃ |
| 599 | Sm | CH₃ | H | H | CH₃ | Ethyl | H | H | F | H | CF₃CF₂ | CF₃ | CH₃ |
| 600 | Sm | H | H | H | CH₃ | Ethyl | H | H | F | H | CF₃ | CF₃ | CH₃ |
| 601 | Sm | H | H | H | CH₃ | Ethyl | H | H | Cl | H | CF₃CF₂ | CF₃ | CH₃ |
| 602 | Sm | H | H | H | CH₃ | Ethyl | H | H | H | H | CF₃CF₂ | CF₃ | CH₃ |
| 603 | Sm | H | H | H | CH₃ | Ethyl | H | H | F | H | CF₃CF₂ | CF₃ | Ethyl |
| 604 | Sm | H | H | H | CH₃ | Ethyl | H | H | F | Cl | CF₃CF₂ | CF₃ | CH₃ |
| 605 | Sm | H | H | H | CH₃ | Ethyl | H | H | Br | H | CF₃CF₂ | CF₃ | CH₃ |
| 606 | Sm | H | H | H | CH₃ | Ethyl | H | F | F | H | CF₃CF₂ | CF₃ | CH₃ |
| 607 | Sm | H | H | H | CH₃ | Ethyl | H | Cl | F | H | CF₃CF₂ | CF₃ | CH₃ |
| 608 | Sm | H | H | H | Cyclopropyl | CH₃ | H | H | H | H | CF₃CF₂ | CF₃ | CH₃ |
| 609 | Sm | H | H | H | Cyclopropyl | H | H | H | H | H | CF₃CF₂ | CF₃ | CH₃ |
| 610 | Sm | H | H | H | Cyclopropyl | CH₃ | H | H | H | F | CF₃CF₂ | CF₃ | CH₃ |
| 611 | Sm | CH₃ | H | H | Cyclopropyl | CH₃ | H | H | H | H | CF₃CF₂ | CF₃ | CH₃ |
| 612 | Sm | H | H | H | Cyclopropyl | CH₃ | H | H | H | H | CF₃ | CF₃ | CH₃ |
| 613 | Sm | H | H | H | Cyclopropyl | Ethyl | H | H | H | H | CF₃CF₂ | CF₃ | CH₃ |
| 614 | Sm | H | H | H | Cyclopropylmethyl | CH₃ | H | H | H | H | CF₃CF₂ | CF₃ | CH₃ |
| 615 | Sm | H | H | H | Cyclopropyl | CH₃ | H | H | H | H | CF₃CF₂ | CF₃ | Ethyl |
| 616 | Sm | H | H | H | Cyclopropyl | CH₃ | H | H | H | Cl | CF₃CF₂ | CF₃ | CH₃ |
| 617 | Sm | H | H | H | Cyclopropyl | CH₃ | H | F | H | H | CF₃CF₂ | CF₃ | CH₃ |
| 618 | Sm | H | H | H | Cyclopropyl | CH₃ | H | Cl | H | H | CF₃CF₂ | CF₃ | CH₃ |
| 619 | Sm | H | H | H | Propyl | CH₃ | H | H | H | H | CF₃CF₂ | CF₃ | CH₃ |
| 620 | Sm | H | H | H | CH₃ | H | H | H | F | F | CF₃CF₂ | CF₃ | CH₃ |
| 621 | Sm | CH₃ | H | H | CH₃ | H | H | H | F | F | CF₃CF₂ | CF₃ | CH₃ |
| 622 | Sm | H | H | H | CH₃ | H | H | H | F | F | CF₃ | CF₃ | CH₃ |
| 623 | Sm | H | H | H | CH₃ | H | H | H | Cl | F | CF₃CF₂ | CF₃ | CH₃ |
| 624 | Sm | H | H | H | CH₃ | H | H | H | H | F | CF₃CF₂ | CF₃ | CH₃ |
| 625 | Sm | H | H | H | CH₃ | H | H | H | F | F | CF₃CF₂ | CF₃ | Ethyl |
| 626 | Sm | H | H | H | CH₃ | H | H | H | F | Cl | CF₃CF₂ | CF₃ | CH₃ |
| 627 | Sm | H | H | H | CH₃ | H | H | H | Br | F | CF₃CF₂ | CF₃ | CH₃ |
| 628 | Sm | H | H | H | CH₃ | H | H | F | F | F | CF₃CF₂ | CF₃ | CH₃ |
| 629 | Sm | H | H | H | CH₃ | H | H | Cl | F | F | CF₃CF₂ | CF₃ | CH₃ |
| 630 | Sm | H | H | H | CH₃ | H | H | H | F | H | CF₃CF₂ | CF₃ | CH₃ |
| 631 | Sm | CH₃ | H | H | CH₃ | H | H | H | F | H | CF₃CF₂ | CF₃ | CH₃ |
| 632 | Sm | H | H | H | CH₃ | H | H | H | F | H | CF₃ | CF₃ | CH₃ |
| 633 | Sm | H | H | H | CH₃ | H | H | H | Cl | H | CF₃CF₂ | CF₃ | CH₃ |
| 634 | Sm | H | H | H | CH₃ | H | H | H | F | H | CF₃CF₂ | CF₃ | Ethyl |
| 635 | Sm | H | H | H | CH₃ | H | H | H | Br | H | CF₃CF₂ | CF₃ | CH₃ |
| 636 | Sm | H | H | H | CH₃ | H | H | F | F | H | CF₃CF₂ | CF₃ | CH₃ |
| 637 | Sm | H | H | H | CH₃ | H | H | Cl | F | H | CF₃CF₂ | CF₃ | CH₃ |
| 638 | Sm | H | H | H | Cyclopropyl | Ethyl | H | H | F | H | CF₃CF₂ | CF₃ | CH₃ |
| 639 | Sm | H | H | H | Cyclopropyl | Ethyl | H | H | F | F | CF₃CF₂ | CF₃ | CH₃ |
| 640 | Sm | CH₃ | H | H | Cyclopropyl | Ethyl | H | H | F | H | CF₃CF₂ | CF₃ | CH₃ |
| 641 | Sm | H | H | H | Cyclopropyl | Ethyl | H | H | F | H | CF₃ | CF₃ | CH₃ |
| 642 | Sm | H | H | H | Cyclopropyl | Ethyl | H | H | Cl | H | CF₃CF₂ | CF₃ | CH₃ |
| 643 | Sm | H | H | H | Cyclopropyl | Ethyl | H | H | F | H | CF₃CF₂ | CF₃ | Ethyl |
| 644 | Sm | H | H | H | Cyclopropyl | Ethyl | H | H | F | Cl | CF₃CF₂ | CF₃ | CH₃ |
| 645 | Sm | H | H | H | Cyclopropyl | Ethyl | H | F | F | H | CF₃CF₂ | CF₃ | CH₃ |
| 646 | Sm | H | H | H | Cyclopropyl | Ethyl | H | Cl | F | H | CF₃CF₂ | CF₃ | CH₃ |
| 647 | Sm | H | H | H | Cyclopropyl | Ethyl | H | H | Br | H | CF₃CF₂ | CF₃ | CH₃ |
| 648 | Sm | H | H | H | Ethyl | CH₃ | H | H | F | F | CF₃CF₂ | CF₃ | CH₃ |
| 649 | Sm | CH₃ | H | H | Ethyl | CH₃ | H | H | F | F | CF₃CF₂ | CF₃ | CH₃ |
| 650 | Sm | H | H | H | Ethyl | CH₃ | H | H | F | F | CF₃ | CF₃ | CH₃ |
| 651 | Sm | H | H | H | Ethyl | CH₃ | H | H | Cl | F | CF₃CF₂ | CF₃ | CH₃ |
| 652 | Sm | H | H | H | Ethyl | CH₃ | H | H | H | F | CF₃CF₂ | CF₃ | CH₃ |
| 653 | Sm | H | H | H | Ethyl | CH₃ | H | F | F | F | CF₃CF₂ | CF₃ | CH₃ |
| 654 | Sm | H | H | H | Ethyl | CH₃ | H | Cl | F | F | CF₃CF₂ | CF₃ | CH₃ |
| 655 | Sm | H | H | H | Ethyl | CH₃ | H | H | F | F | CF₃CF₂ | CF₃ | Ethyl |
| 656 | Sm | H | H | H | Ethyl | CH₃ | H | H | Br | F | CF₃CF₂ | CF₃ | CH₃ |
| 657 | Sm | H | H | H | Ethyl | CH₃ | H | H | H | H | CF₃CF₂ | CF₃ | CH₃ |
| 658 | Sm | CH₃ | H | H | Ethyl | CH₃ | H | H | H | H | CF₃CF₂ | CF₃ | CH₃ |
| 659 | Sm | H | H | H | Ethyl | CH₃ | H | H | H | H | CF₃ | CF₃ | CH₃ |
| 660 | Sm | H | H | H | Ethyl | CH₃ | H | F | H | H | CF₃CF₂ | CF₃ | CH₃ |
| 661 | Sm | H | H | H | Ethyl | CH₃ | H | Cl | H | H | CF₃CF₂ | CF₃ | CH₃ |
| 662 | Sm | H | H | H | Ethyl | CH₃ | H | H | H | Cl | CF₃CF₂ | CF₃ | CH₃ |
| 663 | Sm | H | H | H | Ethyl | CH₃ | H | H | H | H | CF₃CF₂ | CF₃ | Ethyl |
| 664 | Sm | H | H | H | Cyclopropylmethyl | CH₃ | H | H | F | H | CF₃CF₂ | CF₃ | CH₃ |
| 665 | Sm | CH₃ | H | H | Cyclopropylmethyl | CH₃ | H | H | F | H | CF₃CF₂ | CF₃ | CH₃ |
| 666 | Sm | H | H | H | Cyclopropylmethyl | CH₃ | H | H | F | H | CF₃ | CF₃ | CH₃ |
| 667 | Sm | H | H | H | Cyclopropylmethyl | CH₃ | H | H | Cl | H | CF₃CF₂ | CF₃ | CH₃ |
| 668 | Sm | H | H | H | Cyclopropylmethyl | CH₃ | H | F | F | H | CF₃CF₂ | CF₃ | CH₃ |
| 669 | Sm | H | H | H | Cyclopropylmethyl | CH₃ | H | Cl | F | H | CF₃CF₂ | CF₃ | CH₃ |
| 670 | Sm | H | H | H | Cyclopropylmethyl | CH₃ | H | H | F | Cl | CF₃CF₂ | CF₃ | CH₃ |
| 671 | Sm | H | H | H | Cyclopropylmethyl | CH₃ | H | H | Br | H | CF₃CF₂ | CF₃ | CH₃ |
| 672 | Sm | H | H | H | Cyclopropylmethyl | CH₃ | H | H | F | H | CF₃CF₂ | CF₃ | Ethyl |
| 673 | Sm | H | H | H | Cyclopropyl | H | H | H | F | H | CF₃CF₂ | CF₃ | CH₃ |
| 674 | Sm | CH₃ | H | H | Cyclopropyl | H | H | H | F | H | CF₃CF₂ | CF₃ | CH₃ |
| 675 | Sm | H | H | H | Cyclopropyl | H | H | H | F | H | CF₃ | CF₃ | CH₃ |
| 676 | Sm | H | H | H | Cyclopropyl | H | H | H | Cl | H | CF₃CF₂ | CF₃ | CH₃ |
| 677 | Sm | H | H | H | Cyclopropyl | H | H | F | F | H | CF₃CF₂ | CF₃ | CH₃ |
| 678 | Sm | H | H | H | Cyclopropyl | H | H | Cl | F | H | CF₃CF₂ | CF₃ | CH₃ |
| 679 | Sm | H | H | H | Cyclopropyl | H | H | H | F | Cl | CF₃CF₂ | CF₃ | CH₃ |
| 680 | Sm | H | H | H | Cyclopropyl | H | H | H | F | H | CF₃CF₂ | CF₃ | Ethyl |
| 681 | Sm | H | H | H | Cyclopropyl | H | H | H | Br | H | CF₃CF₂ | CF₃ | CH₃ |
| 682 | Sm | H | H | H | Cyclopropylmethyl | H | H | H | F | H | CF₃CF₂ | CF₃ | CH₃ |
| 683 | Sm | CH₃ | H | H | Cyclopropylmethyl | H | H | H | F | H | CF₃CF₂ | CF₃ | CH₃ |
| 684 | Sm | H | H | H | Cyclopropylmethyl | H | H | H | F | H | CF₃ | CF₃ | CH₃ |
| 685 | Sm | H | H | H | Cyclopropylmethyl | H | H | H | Cl | H | CF₃CF₂ | CF₃ | CH₃ |
| 686 | Sm | H | H | H | Cyclopropylmethyl | H | H | H | H | H | CF₃CF₂ | CF₃ | CH₃ |
| 687 | Sm | H | H | H | Cyclopropylmethyl | H | H | F | F | H | CF₃CF₂ | CF₃ | CH₃ |
| 688 | Sm | H | H | H | Cyclopropylmethyl | H | H | Cl | F | H | CF₃CF₂ | CF₃ | CH₃ |
| 689 | Sm | H | H | H | Cyclopropylmethyl | H | H | H | F | Cl | CF₃CF₂ | CF₃ | CH₃ |
| 690 | Sm | H | H | H | Cyclopropylmethyl | H | H | H | F | H | CF₃CF₂ | CF₃ | Ethyl |
| 691 | Sm | H | H | H | Cyclopropylmethyl | H | H | H | Br | H | CF₃CF₂ | CF₃ | CH₃ |
| 692 | Sm | H | H | H | Ethyl | H | H | H | H | H | CF₃CF₂ | CF₃ | CH₃ |
| 693 | Sm | CH₃ | H | H | Ethyl | H | H | H | H | H | CF₃CF₂ | CF₃ | CH₃ |
| 694 | Sm | H | H | H | Ethyl | H | H | H | H | H | CF₃ | CF₃ | CH₃ |
| 695 | Sm | H | H | H | Ethyl | H | H | F | H | H | CF₃CF₂ | CF₃ | CH₃ |
| 696 | Sm | H | H | H | Ethyl | H | H | Cl | H | H | CF₃CF₂ | CF₃ | CH₃ |
| 697 | Sm | H | H | H | Ethyl | H | H | H | H | Cl | CF₃CF₂ | CF₃ | CH₃ |
| 698 | Sm | H | H | H | Ethyl | H | H | H | H | H | CF₃CF₂ | CF₃ | Ethyl |
| 699 | Sm | H | H | H | Propyl | H | H | H | H | H | CF₃CF₂ | CF₃ | CH₃ |
| 700 | Sm | CH₃ | H | H | Cyclopropylmethyl | H | H | H | Cl | H | CF₃CF₂ | CF₃ | CH₃ |
| 701 | Sm | CH₃ | H | H | CH₃ | H | H | H | Cl | H | CF₃CF₂ | CF₃ | CH₃ |
| 702 | Sm | CH₃ | H | H | Ethyl | H | H | H | Cl | H | CF₃CF₂ | CF₃ | CH₃ |
| 703 | Sm | CH₃ | H | H | Cyclopropyl | H | H | H | Cl | H | CF₃CF₂ | CF₃ | CH₃ |
| 704 | Sm | CH₃ | H | H | Cyclopropylmethyl | H | H | H | Cl | F | CF₃CF₂ | CF₃ | CH₃ |
| 705 | Sm | CH₃ | H | H | Cyclopropylmethyl | H | H | H | Cl | H | CF₃ | CF₃ | CH₃ |
| 706 | Sm | CH₃ | H | H | Cyclopropylmethyl | CH₃ | H | H | Cl | H | CF₃CF₂ | CF₃ | CH₃ |
| 707 | Sm | CH₃ | H | H | Cyclopropylmethyl | Ethyl | H | H | Cl | H | CF₃CF₂ | CF₃ | CH₃ |
| 708 | Sm | CH₃ | H | H | Cyclopropylmethyl | H | H | H | H | H | CF₃CF₂ | CF₃ | CH₃ |
| 709 | Sm | CH₃ | H | H | Propyl | H | H | H | Cl | H | CF₃CF₂ | CF₃ | CH₃ |
| 710 | Sm | CH₃ | H | H | Cyclopropylmethyl | H | H | F | Cl | H | CF₃CF₂ | CF₃ | CH₃ |
| 711 | Sm | CH₃ | H | H | Cyclopropylmethyl | H | H | Cl | Cl | H | CF₃CF₂ | CF₃ | CH₃ |
| 712 | Sm | CH₃ | H | H | Cyclopropylmethyl | H | H | H | Cl | Cl | CF₃CF₂ | CF₃ | CH₃ |
| 713 | Sm | CH₃ | H | H | Cyclopropylmethyl | H | H | H | Cl | H | CF₃CF₂ | CF₃ | Ethyl |
| 714 | Sm | CH₃ | H | H | Cyclopropylmethyl | H | H | H | Br | H | CF₃CF₂ | CF₃ | CH₃ |
| 715 | Sm | H | H | H | CH₃ | CH₃ | H | H | H | H | CF₃CF₂ | CF₃ | CH₃ |
| 716 | Sm | CH₃ | H | H | CH₃ | CH₃ | H | H | H | H | CF₃CF₂ | CF₃ | CH₃ |
| 717 | Sm | H | H | H | CH₃ | CH₃ | H | H | H | H | CF₃ | CF₃ | CH₃ |
| 718 | Sm | H | H | H | CH₃ | CH₃ | H | F | H | H | CF₃CF₂ | CF₃ | CH₃ |
| 719 | Sm | H | H | H | CH₃ | CH₃ | H | Cl | H | H | CF₃CF₂ | CF₃ | CH₃ |
| 720 | Sm | H | H | H | CH₃ | CH₃ | H | H | H | Cl | CF₃CF₂ | CF₃ | CH₃ |
| 721 | Sm | H | H | H | CH₃ | CH₃ | H | H | H | H | CF₃CF₂ | CF₃ | Ethyl |
| 722 | Sm | CH₃ | H | H | CH₃ | H | H | H | F | F | CF₃ | CF₃ | CH₃ |
| 723 | Sm | CH₃ | H | H | CH₃ | H | H | H | F | H | CF₃ | CF₃ | CH₃ |
| 724 | Sm | CH₃ | H | H | CH₃ | CH₃ | H | H | F | F | CF₃ | CF₃ | CH₃ |
| 725 | Sm | CH₃ | H | H | CH₃ | CH₃ | H | H | F | H | CF₃ | CF₃ | CH₃ |
| 726 | Sm | CH₃ | H | H | CH₃ | Ethyl | H | H | F | F | CF₃CF₂ | CF₃ | CH₃ |
| 727 | Sm | H | H | H | CH₃ | Ethyl | H | H | F | F | CF₃ | CF₃ | CH₃ |
| 728 | Sm | CH₃ | H | H | CH₃ | Ethyl | H | H | F | F | CF₃ | CF₃ | CH₃ |
| 729 | Sm | CH₃ | H | H | CH₃ | Ethyl | H | H | F | H | CF₃ | CF₃ | CH₃ |
| 730 | Sm | CH₃ | H | H | CH₃ | H | H | H | Cl | F | CF₃CF₂ | CF₃ | CH₃ |
| 731 | Sm | H | H | H | CH₃ | H | H | H | Cl | F | CF₃ | CF₃ | CH₃ |
| 732 | Sm | CH₃ | H | H | CH₃ | H | H | H | Cl | F | CF₃ | CF₃ | CH₃ |
| 733 | Sm | H | H | H | CH₃ | H | H | H | Cl | H | CF₃ | CF₃ | CH₃ |
| 734 | Sm | CH₃ | H | H | CH₃ | H | H | H | Cl | H | CF₃ | CF₃ | CH₃ |
| 735 | Sm | CH₃ | H | H | CH₃ | CH₃ | H | H | Cl | F | CF₃CF₂ | CF₃ | CH₃ |
| 736 | Sm | H | H | H | CH₃ | CH₃ | H | H | Cl | F | CF₃ | CF₃ | CH₃ |
| 737 | Sm | CH₃ | H | H | CH₃ | CH₃ | H | H | Cl | F | CF₃ | CF₃ | CH₃ |
| 738 | Sm | CH₃ | H | H | CH₃ | CH₃ | H | H | Cl | H | CF₃CF₂ | CF₃ | CH₃ |
| 739 | Sm | H | H | H | CH₃ | CH₃ | H | H | Cl | H | CF₃ | CF₃ | CH₃ |
| 740 | Sm | CH₃ | H | H | CH₃ | CH₃ | H | H | Cl | H | CF₃ | CF₃ | CH₃ |
| 741 | Sm | H | H | H | CH₃ | Ethyl | H | H | Cl | F | CF₃CF₂ | CF₃ | CH₃ |
| 742 | Sm | CH₃ | H | H | CH₃ | Ethyl | H | H | Cl | F | CF₃CF₂ | CF₃ | CH₃ |
| 743 | Sm | H | H | H | CH₃ | Ethyl | H | H | Cl | F | CF₃ | CF₃ | CH₃ |
| 744 | Sm | CH₃ | H | H | CH₃ | Ethyl | H | H | Cl | F | CF₃ | CF₃ | CH₃ |
| 745 | Sm | CH₃ | H | H | CH₃ | Ethyl | H | H | Cl | H | CF₃CF₂ | CF₃ | CH₃ |
| 746 | Sm | H | H | H | CH₃ | Ethyl | H | H | Cl | H | CF₃ | CF₃ | CH₃ |
| 747 | Sm | CH₃ | H | H | CH₃ | Ethyl | H | H | Cl | H | CF₃ | CF₃ | CH₃ |
| 748 | Sm | CH₃ | H | H | CH₃ | H | H | H | H | F | CF₃CF₂ | CF₃ | CH₃ |
| 749 | Sm | H | H | H | CH₃ | H | H | H | H | F | CF₃ | CF₃ | CH₃ |
| 750 | Sm | CH₃ | H | H | CH₃ | H | H | H | H | F | CF₃ | CF₃ | CH₃ |
| 751 | Sm | CH₃ | H | H | CH₃ | H | H | H | H | H | CF₃CF₂ | CF₃ | CH₃ |
| 752 | Sm | H | H | H | CH₃ | H | H | H | H | H | CF₃ | CF₃ | CH₃ |
| 753 | Sm | CH₃ | H | H | CH₃ | H | H | H | H | H | CF₃ | CF₃ | CH₃ |
| 754 | Sm | CH₃ | H | H | CH₃ | CH₃ | H | H | H | F | CF₃CF₂ | CF₃ | CH₃ |
| 755 | Sm | H | H | H | CH₃ | CH₃ | H | H | H | F | CF₃ | CF₃ | CH₃ |
| 756 | Sm | CH₃ | H | H | CH₃ | CH₃ | H | H | H | F | CF₃ | CF₃ | CH₃ |
| 757 | Sm | CH₃ | H | H | CH₃ | CH₃ | H | H | H | H | CF₃ | CF₃ | CH₃ |
| 758 | Sm | H | H | H | CH₃ | Ethyl | H | H | H | F | CF₃CF₂ | CF₃ | CH₃ |
| 759 | Sm | CH₃ | H | H | CH₃ | Ethyl | H | H | H | F | CF₃CF₂ | CF₃ | CH₃ |
| 760 | Sm | H | H | H | CH₃ | Ethyl | H | H | H | F | CF₃ | CF₃ | CH₃ |
| 761 | Sm | CH₃ | H | H | CH₃ | Ethyl | H | H | H | F | CF₃ | CF₃ | CH₃ |
| 762 | Sm | CH₃ | H | H | CH₃ | Ethyl | H | H | H | H | CF₃CF₂ | CF₃ | CH₃ |
| 763 | Sm | H | H | H | CH₃ | Ethyl | H | H | H | H | CF₃ | CF₃ | CH₃ |
| 764 | Sm | CH₃ | H | H | CH₃ | Ethyl | H | H | H | H | CF₃ | CF₃ | CH₃ |
| 765 | Sm | CH₃ | H | H | Ethyl | H | H | H | F | F | CF₃ | CF₃ | CH₃ |
| 766 | Sm | CH₃ | H | H | Ethyl | H | H | H | F | H | CF₃ | CF₃ | CH₃ |
| 767 | Sm | CH₃ | H | H | Ethyl | CH₃ | H | H | F | F | CF₃ | CF₃ | CH₃ |
| 768 | Sm | CH₃ | H | H | Ethyl | CH₃ | H | H | F | H | CF₃CF₂ | CF₃ | CH₃ |
| 769 | Sm | CH₃ | H | H | Ethyl | CH₃ | H | H | F | H | CF₃ | CF₃ | CH₃ |
| 770 | Sm | CH₃ | H | H | Ethyl | Ethyl | H | H | F | F | CF₃CF₂ | CF₃ | CH₃ |
| 771 | Sm | H | H | H | Ethyl | Ethyl | H | H | F | F | CF₃ | CF₃ | CH₃ |
| 772 | Sm | CH₃ | H | H | Ethyl | Ethyl | H | H | F | F | CF₃ | CF₃ | CH₃ |
| 773 | Sm | CH₃ | H | H | Ethyl | Ethyl | H | H | F | H | CF₃ | CF₃ | CH₃ |
| 774 | Sm | CH₃ | H | H | Ethyl | H | H | H | Cl | F | CF₃CF₂ | CF₃ | CH₃ |
| 775 | Sm | H | H | H | Ethyl | H | H | H | Cl | F | CF₃ | CF₃ | CH₃ |
| 776 | Sm | CH₃ | H | H | Ethyl | H | H | H | Cl | F | CF₃ | CF₃ | CH₃ |
| 777 | Sm | H | H | H | Ethyl | H | H | H | Cl | H | CF₃ | CF₃ | CH₃ |
| 778 | Sm | CH₃ | H | H | Ethyl | H | H | H | Cl | H | CF₃ | CF₃ | CH₃ |
| 779 | Sm | CH₃ | H | H | Ethyl | CH₃ | H | H | Cl | F | CF₃CF₂ | CF₃ | CH₃ |
| 780 | Sm | H | H | H | Ethyl | CH₃ | H | H | Cl | F | CF₃ | CF₃ | CH₃ |
| 781 | Sm | CH₃ | H | H | Ethyl | CH₃ | H | H | Cl | F | CF₃ | CF₃ | CH₃ |
| 782 | Sm | CH₃ | H | H | Ethyl | CH₃ | H | H | Cl | H | CF₃CF₂ | CF₃ | CH₃ |
| 783 | Sm | H | H | H | Ethyl | CH₃ | H | H | Cl | H | CF₃ | CF₃ | CH₃ |
| 784 | Sm | CH₃ | H | H | Ethyl | CH₃ | H | H | Cl | H | CF₃ | CF₃ | CH₃ |
| 785 | Sm | H | H | H | Ethyl | Ethyl | H | H | Cl | F | CF₃CF₂ | CF₃ | CH₃ |
| 786 | Sm | CH₃ | H | H | Ethyl | Ethyl | H | H | Cl | F | CF₃CF₂ | CF₃ | CH₃ |
| 787 | Sm | H | H | H | Ethyl | Ethyl | H | H | Cl | F | CF₃ | CF₃ | CH₃ |
| 788 | Sm | CH₃ | H | H | Ethyl | Ethyl | H | H | Cl | F | CF₃ | CF₃ | CH₃ |
| 789 | Sm | CH₃ | H | H | Ethyl | Ethyl | H | H | Cl | H | CF₃CF₂ | CF₃ | CH₃ |
| 790 | Sm | H | H | H | Ethyl | Ethyl | H | H | Cl | H | CF₃ | CF₃ | CH₃ |
| 791 | Sm | CH₃ | H | H | Ethyl | Ethyl | H | H | Cl | H | CF₃ | CF₃ | CH₃ |
| 792 | Sm | CH₃ | H | H | Ethyl | H | H | H | H | F | CF₃CF₂ | CF₃ | CH₃ |
| 793 | Sm | H | H | H | Ethyl | H | H | H | H | F | CF₃ | CF₃ | CH₃ |
| 794 | Sm | CH₃ | H | H | Ethyl | H | H | H | H | F | CF₃ | CF₃ | CH₃ |
| 795 | Sm | CH₃ | H | H | Ethyl | H | H | H | H | H | CF₃ | CF₃ | CH₃ |
| 796 | Sm | CH₃ | H | H | Ethyl | CH₃ | H | H | H | F | CF₃CF₂ | CF₃ | CH₃ |
| 797 | Sm | H | H | H | Ethyl | CH₃ | H | H | H | F | CF₃ | CF₃ | CH₃ |
| 798 | Sm | CH₃ | H | H | Ethyl | CH₃ | H | H | H | F | CF₃ | CF₃ | CH₃ |
| 799 | Sm | CH₃ | H | H | Ethyl | CH₃ | H | H | H | H | CF₃ | CF₃ | CH₃ |
| 800 | Sm | H | H | H | Ethyl | Ethyl | H | H | H | F | CF₃CF₂ | CF₃ | CH₃ |
| 801 | Sm | CH₃ | H | H | Ethyl | Ethyl | H | H | H | F | CF₃CF₂ | CF₃ | CH₃ |
| 802 | Sm | H | H | H | Ethyl | Ethyl | H | H | H | F | CF₃ | CF₃ | CH₃ |
| 803 | Sm | CH₃ | H | H | Ethyl | Ethyl | H | H | H | F | CF₃ | CF₃ | CH₃ |
| 804 | Sm | CH₃ | H | H | Ethyl | Ethyl | H | H | H | H | CF₃CF₂ | CF₃ | CH₃ |
| 805 | Sm | H | H | H | Ethyl | Ethyl | H | H | H | H | CF₃ | CF₃ | CH₃ |
| 806 | Sm | CH₃ | H | H | Ethyl | Ethyl | H | H | H | H | CF₃ | CF₃ | CH₃ |
| 807 | Sm | CH₃ | H | H | Cyclopropyl | H | H | H | F | F | CF₃CF₂ | CF₃ | CH₃ |
| 808 | Sm | H | H | H | Cyclopropyl | H | H | H | F | F | CF₃ | CF₃ | CH₃ |
| 809 | Sm | CH₃ | H | H | Cyclopropyl | H | H | H | F | F | CF₃ | CF₃ | CH₃ |
| 810 | Sm | CH₃ | H | H | Cyclopropyl | H | H | H | F | H | CF₃ | CF₃ | CH₃ |
| 811 | Sm | CH₃ | H | H | Cyclopropyl | CH₃ | H | H | F | F | CF₃CF₂ | CF₃ | CH₃ |
| 812 | Sm | H | H | H | Cyclopropyl | CH₃ | H | H | F | F | CF₃ | CF₃ | CH₃ |
| 813 | Sm | CH₃ | H | H | Cyclopropyl | CH₃ | H | H | F | F | CF₃ | CF₃ | CH₃ |
| 814 | Sm | CH₃ | H | H | Cyclopropyl | CH₃ | H | H | F | H | CF₃ | CF₃ | CH₃ |
| 815 | Sm | CH₃ | H | H | Cyclopropyl | Ethyl | H | H | F | F | CF₃CF₂ | CF₃ | CH₃ |
| 816 | Sm | H | H | H | Cyclopropyl | Ethyl | H | H | F | F | CF₃ | CF₃ | CH₃ |
| 817 | Sm | CH₃ | H | H | Cyclopropyl | Ethyl | H | H | F | F | CF₃ | CF₃ | CH₃ |
| 818 | Sm | CH₃ | H | H | Cyclopropyl | Ethyl | H | H | F | H | CF₃ | CF₃ | CH₃ |
| 819 | Sm | H | H | H | Cyclopropyl | H | H | H | Cl | F | CF₃CF₂ | CF₃ | CH₃ |
| 820 | Sm | CH₃ | H | H | Cyclopropyl | H | H | H | Cl | F | CF₃CF₂ | CF₃ | CH₃ |
| 821 | Sm | H | H | H | Cyclopropyl | H | H | H | Cl | F | CF₃ | CF₃ | CH₃ |
| 822 | Sm | CH₃ | H | H | Cyclopropyl | H | H | H | Cl | F | CF₃ | CF₃ | CH₃ |
| 823 | Sm | H | H | H | Cyclopropyl | H | H | H | Cl | H | CF₃ | CF₃ | CH₃ |
| 824 | Sm | CH₃ | H | H | Cyclopropyl | H | H | H | Cl | H | CF₃ | CF₃ | CH₃ |
| 825 | Sm | H | H | H | Cyclopropyl | CH₃ | H | H | Cl | F | CF₃CF₂ | CF₃ | CH₃ |
| 826 | Sm | CH₃ | H | H | Cyclopropyl | CH₃ | H | H | Cl | F | CF₃CF₂ | CF₃ | CH₃ |
| 827 | Sm | H | H | H | Cyclopropyl | CH₃ | H | H | Cl | F | CF₃ | CF₃ | CH₃ |
| 828 | Sm | CH₃ | H | H | Cyclopropyl | CH₃ | H | H | Cl | F | CF₃ | CF₃ | CH₃ |
| 829 | Sm | CH₃ | H | H | Cyclopropyl | CH₃ | H | H | Cl | H | CF₃CF₂ | CF₃ | CH₃ |
| 830 | Sm | H | H | H | Cyclopropyl | CH₃ | H | H | Cl | H | CF₃ | CF₃ | CH₃ |
| 831 | Sm | CH₃ | H | H | Cyclopropyl | CH₃ | H | H | Cl | H | CF₃ | CF₃ | CH₃ |
| 832 | Sm | H | H | H | Cyclopropyl | Ethyl | H | H | Cl | F | CF₃CF₂ | CF₃ | CH₃ |
| 833 | Sm | CH₃ | H | H | Cyclopropyl | Ethyl | H | H | Cl | F | CF₃CF₂ | CF₃ | CH₃ |
| 834 | Sm | H | H | H | Cyclopropyl | Ethyl | H | H | Cl | F | CF₃ | CF₃ | CH₃ |
| 835 | Sm | CH₃ | H | H | Cyclopropyl | Ethyl | H | H | Cl | F | CF₃ | CF₃ | CH₃ |
| 836 | Sm | CH₃ | H | H | Cyclopropyl | Ethyl | H | H | Cl | H | CF₃CF₂ | CF₃ | CH₃ |
| 837 | Sm | H | H | H | Cyclopropyl | Ethyl | H | H | Cl | H | CF₃ | CF₃ | CH₃ |
| 838 | Sm | CH₃ | H | H | Cyclopropyl | Ethyl | H | H | Cl | H | CF₃ | CF₃ | CH₃ |
| 839 | Sm | H | H | H | Cyclopropyl | H | H | H | H | F | CF₃CF₂ | CF₃ | CH₃ |
| 840 | Sm | CH₃ | H | H | Cyclopropyl | H | H | H | H | F | CF₃CF₂ | CF₃ | CH₃ |
| 841 | Sm | H | H | H | Cyclopropyl | H | H | H | H | F | CF₃ | CF₃ | CH₃ |
| 842 | Sm | CH₃ | H | H | Cyclopropyl | H | H | H | H | F | CF₃ | CF₃ | CH₃ |
| 843 | Sm | CH₃ | H | H | Cyclopropyl | H | H | H | H | H | CF₃CF₂ | CF₃ | CH₃ |
| 844 | Sm | H | H | H | Cyclopropyl | H | H | H | H | H | CF₃ | CF₃ | CH₃ |
| 845 | Sm | CH₃ | H | H | Cyclopropyl | H | H | H | H | H | CF₃ | CF₃ | CH₃ |
| 846 | Sm | CH₃ | H | H | Cyclopropyl | CH₃ | H | H | H | F | CF₃CF₂ | CF₃ | CH₃ |
| 847 | Sm | H | H | H | Cyclopropyl | CH₃ | H | H | H | F | CF₃ | CF₃ | CH₃ |
| 848 | Sm | CH₃ | H | H | Cyclopropyl | CH₃ | H | H | H | F | CF₃ | CF₃ | CH₃ |
| 849 | Sm | CH₃ | H | H | Cyclopropyl | CH₃ | H | H | H | H | CF₃ | CF₃ | CH₃ |
| 850 | Sm | H | H | H | Cyclopropyl | Ethyl | H | H | H | F | CF₃CF₂ | CF₃ | CH₃ |
| 851 | Sm | CH₃ | H | H | Cyclopropyl | Ethyl | H | H | H | F | CF₃CF₂ | CF₃ | CH₃ |
| 852 | Sm | H | H | H | Cyclopropyl | Ethyl | H | H | H | F | CF₃ | CF₃ | CH₃ |
| 853 | Sm | CH₃ | H | H | Cyclopropyl | Ethyl | H | H | H | F | CF₃ | CF₃ | CH₃ |
| 854 | Sm | CH₃ | H | H | Cyclopropyl | Ethyl | H | H | H | H | CF₃CF₂ | CF₃ | CH₃ |
| 855 | Sm | H | H | H | Cyclopropyl | Ethyl | H | H | H | H | CF₃ | CF₃ | CH₃ |
| 856 | Sm | CH₃ | H | H | Cyclopropyl | Ethyl | H | H | H | H | CF₃ | CF₃ | CH₃ |
| 857 | Sm | CH₃ | H | H | Cyclopropylmethyl | H | H | H | F | F | CF₃CF₂ | CF₃ | CH₃ |
| 858 | Sm | H | H | H | Cyclopropylmethyl | H | H | H | F | F | CF₃ | CF₃ | CH₃ |
| 859 | Sm | CH₃ | H | H | Cyclopropylmethyl | H | H | H | F | F | CF₃ | CF₃ | CH₃ |
| 860 | Sm | CH₃ | H | H | Cyclopropylmethyl | H | H | H | F | H | CF₃ | CF₃ | CH₃ |
| 861 | Sm | CH₃ | H | H | Cyclopropylmethyl | CH₃ | H | H | F | F | CF₃CF₂ | CF₃ | CH₃ |
| 862 | Sm | H | H | H | Cyclopropylmethyl | CH₃ | H | H | F | F | CF₃ | CF₃ | CH₃ |
| 863 | Sm | CH₃ | H | H | Cyclopropylmethyl | CH₃ | H | H | F | F | CF₃ | CF₃ | CH₃ |
| 864 | Sm | CH₃ | H | H | Cyclopropylmethyl | CH₃ | H | H | F | H | CF₃ | CF₃ | CH₃ |
| 865 | Sm | H | H | H | Cyclopropylmethyl | Ethyl | H | H | F | F | CF₃CF₂ | CF₃ | CH₃ |
| 866 | Sm | CH₃ | H | H | Cyclopropylmethyl | Ethyl | H | H | F | F | CF₃CF₂ | CF₃ | CH₃ |
| 867 | Sm | H | H | H | Cyclopropylmethyl | Ethyl | H | H | F | F | CF₃ | CF₃ | CH₃ |
| 868 | Sm | CH₃ | H | H | Cyclopropylmethyl | Ethyl | H | H | F | F | CF₃ | CF₃ | CH₃ |
| 869 | Sm | CH₃ | H | H | Cyclopropylmethyl | Ethyl | H | H | F | H | CF₃CF₂ | CF₃ | CH₃ |
| 870 | Sm | H | H | H | Cyclopropylmethyl | Ethyl | H | H | F | H | CF₃ | CF₃ | CH₃ |
| 871 | Sm | CH₃ | H | H | Cyclopropylmethyl | Ethyl | H | H | F | H | CF₃ | CF₃ | CH₃ |
| 872 | Sm | H | H | H | Cyclopropylmethyl | H | H | H | Cl | F | CF₃CF₂ | CF₃ | CH₃ |
| 873 | Sm | H | H | H | Cyclopropylmethyl | H | H | H | Cl | F | CF₃ | CF₃ | CH₃ |
| 874 | Sm | CH₃ | H | H | Cyclopropylmethyl | H | H | H | Cl | F | CF₃ | CF₃ | CH₃ |
| 875 | Sm | H | H | H | Cyclopropylmethyl | H | H | H | Cl | H | CF₃ | CF₃ | CH₃ |
| 876 | Sm | H | H | H | Cyclopropylmethyl | CH₃ | H | H | Cl | F | CF₃CF₂ | CF₃ | CH₃ |
| 877 | Sm | CH₃ | H | H | Cyclopropylmethyl | CH₃ | H | H | Cl | F | CF₃CF₂ | CF₃ | CH₃ |
| 878 | Sm | H | H | H | Cyclopropylmethyl | CH₃ | H | H | Cl | F | CF₃ | CF₃ | CH₃ |
| 879 | Sm | CH₃ | H | H | Cyclopropylmethyl | CH₃ | H | H | Cl | F | CF₃ | CF₃ | CH₃ |
| 880 | Sm | H | H | H | Cyclopropylmethyl | CH₃ | H | H | Cl | H | CF₃ | CF₃ | CH₃ |
| 881 | Sm | CH₃ | H | H | Cyclopropylmethyl | CH₃ | H | H | Cl | H | CF₃ | CF₃ | CH₃ |
| 882 | Sm | H | H | H | Cyclopropylmethyl | Ethyl | H | H | Cl | F | CF₃CF₂ | CF₃ | CH₃ |
| 883 | Sm | CH₃ | H | H | Cyclopropylmethyl | Ethyl | H | H | Cl | F | CF₃CF₂ | CF₃ | CH₃ |
| 884 | Sm | H | H | H | Cyclopropylmethyl | Ethyl | H | H | Cl | F | CF₃ | CF₃ | CH₃ |
| 885 | Sm | CH₃ | H | H | Cyclopropylmethyl | Ethyl | H | H | Cl | F | CF₃ | CF₃ | CH₃ |
| 886 | Sm | H | H | H | Cyclopropylmethyl | Ethyl | H | H | Cl | H | CF₃CF₂ | CF₃ | CH₃ |
| 887 | Sm | H | H | H | Cyclopropylmethyl | Ethyl | H | H | Cl | H | CF₃ | CF₃ | CH₃ |
| 888 | Sm | CH₃ | H | H | Cyclopropylmethyl | Ethyl | H | H | Cl | H | CF₃ | CF₃ | CH₃ |
| 889 | Sm | H | H | H | Cyclopropylmethyl | H | H | H | H | F | CF₃CF₂ | CF₃ | CH₃ |
| 890 | Sm | CH₃ | H | H | Cyclopropylmethyl | H | H | H | H | F | CF₃CF₂ | CF₃ | CH₃ |
| 891 | Sm | H | H | H | Cyclopropylmethyl | H | H | H | H | F | CF₃ | CF₃ | CH₃ |
| 892 | Sm | CH₃ | H | H | Cyclopropylmethyl | H | H | H | H | F | CF₃ | CF₃ | CH₃ |
| 893 | Sm | H | H | H | Cyclopropylmethyl | H | H | H | H | H | CF₃ | CF₃ | CH₃ |
| 894 | Sm | CH₃ | H | H | Cyclopropylmethyl | H | H | H | H | H | CF₃ | CF₃ | CH₃ |
| 895 | Sm | H | H | H | Cyclopropylmethyl | CH₃ | H | H | H | F | CF₃CF₂ | CF₃ | CH₃ |
| 896 | Sm | CH₃ | H | H | Cyclopropylmethyl | CH₃ | H | H | H | F | CF₃CF₂ | CF₃ | CH₃ |
| 897 | Sm | H | H | H | Cyclopropylmethyl | CH₃ | H | H | H | F | CF₃ | CF₃ | CH₃ |
| 898 | Sm | CH₃ | H | H | Cyclopropylmethyl | CH₃ | H | H | H | F | CF₃ | CF₃ | CH₃ |
| 899 | Sm | CH₃ | H | H | Cyclopropylmethyl | CH₃ | H | H | H | H | CF₃CF₂ | CF₃ | CH₃ |
| 900 | Sm | H | H | H | Cyclopropylmethyl | CH₃ | H | H | H | H | CF₃ | CF₃ | CH₃ |
| 901 | Sm | CH₃ | H | H | Cyclopropylmethyl | CH₃ | H | H | H | H | CF₃ | CF₃ | CH₃ |
| 902 | Sm | H | H | H | Cyclopropylmethyl | Ethyl | H | H | H | F | CF₃CF₂ | CF₃ | CH₃ |
| 903 | Sm | CH₃ | H | H | Cyclopropylmethyl | Ethyl | H | H | H | F | CF₃CF₂ | CF₃ | CH₃ |
| 904 | Sm | H | H | H | Cyclopropylmethyl | Ethyl | H | H | H | F | CF₃ | CF₃ | CH₃ |
| 905 | Sm | CH₃ | H | H | Cyclopropylmethyl | Ethyl | H | H | H | F | CF₃ | CF₃ | CH₃ |
| 906 | Sm | H | H | H | Cyclopropylmethyl | Ethyl | H | H | H | H | CF₃CF₂ | CF₃ | CH₃ |
| 907 | Sm | CH₃ | H | H | Cyclopropylmethyl | Ethyl | H | H | H | H | CF₃CF₂ | CF₃ | CH₃ |
| 908 | Sm | H | H | H | Cyclopropylmethyl | Ethyl | H | H | H | H | CF₃ | CF₃ | CH₃ |
| 909 | Sm | CH₃ | H | H | Cyclopropylmethyl | Ethyl | H | H | H | H | CF₃ | CF₃ | CH₃ |
| 910 | Am | H | H | H | Methyl | H | H | H | F | H | CF₃ | Br | CH₃ |
| 911 | Am | H | H | H | Cyclopropyl | H | H | H | F | H | CF₃ | Br | CH₃ |
| 912 | Am | H | H | H | Ethyl | H | H | H | F | H | CF₃ | Br | CH₃ |
| 913 | Am | H | H | H | Ethyl | H | H | H | Cl | H | CF₃ | Br | CH₃ |
| 914 | Am | H | H | H | Allyl | H | H | H | H | H | CF₃ | Br | CH₃ |
| 915 | Am | H | H | H | Cyclopropylmethyl | H | H | H | H | H | CF₃ | Br | CH₃ |
| 916 | Am | H | H | H | Allyl | H | H | H | Cl | H | CF₃ | Br | CH₃ |
| 917 | Am | H | H | H | Cyclopropylmethyl | H | H | H | F | H | CF₃ | Br | CH₃ |
| 918 | Am | H | H | H | Cyclopropyl | H | H | H | H | H | CF₃CF₂ | NO₂ | CH₃ |
| 919 | Am | H | H | H | Methyl | H | H | H | F | H | CF₃CF₂ | NO₂ | CH₃ |
| 920 | Am | H | H | H | Ethyl | H | H | H | F | H | CF₃CF₂ | NO₂ | CH₃ |
| 921 | Am | H | H | H | Cyclopropyl | H | H | H | F | H | CF₃CF₂ | NO₂ | CH₃ |
| 922 | Am | H | H | H | Cyclopropyl | H | H | H | H | H | CF₃ | Cl | CH₃ |
| 923 | Am | H | H | H | Ethyl | H | H | H | F | H | CF₃ | Cl | CH₃ |
| 924 | Am | H | H | H | Cyclopropyl | H | H | H | F | H | CF₃ | Cl | CH₃ |
| 925 | Am | H | H | H | Methyl | H | H | H | H | H | CF₃ | Cl | CH₃ |
| 926 | Am | H | H | H | Methyl | H | H | H | F | H | CF₃CF₂ | Br | CH₃ |
| 927 | Am | H | H | H | Methyl | H | H | H | H | H | CF₃CF₂ | NO₂ | CH₃ |
| 928 | Am | H | H | H | Ethyl | H | H | H | H | H | CF₃CF₂ | NO₂ | CH₃ |
| 929 | Am | H | H | H | Ethyl | H | H | H | H | H | CF₃CF₂ | Br | CH₃ |
| 930 | Am | H | H | H | Cyclopropyl | H | H | H | H | H | CF₃CF₂ | Br | CH₃ |
| 931 | Am | H | H | H | Methyl | H | H | H | H | H | CF₃CF₂ | Br | CH₃ |
| 932 | Am | H | H | H | Ethyl | H | H | H | F | H | CF₃CF₂ | Br | CH₃ |
| 933 | Am | H | H | H | Cyclopropyl | H | H | H | F | H | CF₃CF₂ | Br | CH₃ |
| 934 | Am | H | H | H | Cyclopropyl | H | H | H | F | H | CF₃CF₂ | SMe | CH₃ |
| 935 | Am | H | H | H | Methyl | H | H | H | F | H | CF₃CF₂ | SMe | CH₃ |
| 936 | Am | H | H | H | Methyl | H | H | H | H | H | CF₃CF₂ | SMe | CH₃ |
| 937 | Am | H | H | H | Methyl | H | H | H | F | H | CF₃ | Cl | CH₃ |
| 938 | Am | H | H | H | Ethyl | H | H | H | H | H | CF₃ | Cl | CH₃ |

**Tabelle 2**

| Die folgenden | | | | | | | | | | | | | |
|---|---|---|---|---|---|---|---|---|---|---|---|---|---|
| 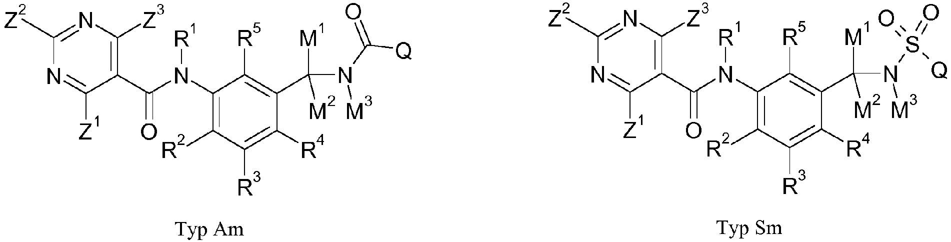 | | | | | | | | | | | | | |

| **Nr.** | **Typ** | **M¹** | **M²** | **M³** | **Q** | **R¹** | **R²** | **R³** | **R⁴** | **R⁵** | **Z¹** | **Z²** | **Z³** |
|---|---|---|---|---|---|---|---|---|---|---|---|---|---|
| 939 | Am | H | H | H | Ethyl | H | H | H | H | H | CF₃ | CF₃ | H |
| 940 | Am | H | H | H | Cyclopropyl | H | H | H | F | H | CF₃ | CF₃ | H |
| 941 | Am | H | H | H | Methyl | H | H | H | F | H | CF₃ | CF₃ | H |
| 942 | Am | H | H | H | Ethyl | H | H | H | F | H | CF₃ | CF₃ | H |
| 943 | Am | H | H | H | Cyclopropyl | H | H | H | H | H | CF₃ | CF₃ | H |
| 944 | Am | H | H | H | Methyl | H | H | H | H | H | CF₃ | CF₃ | H |

### NMR-Peak-Listenverfahren

Die IH-NMR-Daten ausgewählter Beispiele werden in Form von IH-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:

**δ₁ (Intensität₁⁾ ; δ₂ (Intensität₂);........; δᵢ (Intensitätᵢ⁾;......; δₙ (Intensitätₙ)**

| **Peaklisten** |
|---|
| Beispiel Nr. 1, Lösungsmittel: DMSO-d6, NMR-Spektrometer: 399.95 MHz |
| 8.3418 (0.33); 8.2674 (0.88); 8.2529 (1.66); 8.2384 (0.86); 7.3560 (0.35); 7.3481 (0.59); 7.3400 (0.37); 7.3331 (0.33); 7.3245 (0.51); 7.2378 (1.44); 7.2318 (1.67); 7.2213 (1.56); 7.2156 (1.67); 7.1881 (2.74); 7.1647 (3.16); 7.1558 (1.40); 7.1510 (1.57); 7.1441 (1.33); 7.1343 (0.55); 7.1232 (0.34); 5.7571 (3.71); 4.3313 (0.67); 4.3172 (0.66); 4.2192 (0.33); 4.2040 (0.34); 4.1806 (2.01); 4.1668 (3.70); 4.1533 (2.05); 4.1297 (0.42); 4.0547 (3.01); 3.9531 (16.00); 3.4079 (19.42); 3.3200 (21.07); 3.1944 (3.52); 2.5062 (38.57); 2.5021 (47.55); 2.4985 (35.38); 2.1563 (1.00); 2.1457 (1.38); 2.1374 (1.23); 2.1267 (4.01); 2.1077 (4.32); 2.0887 (1.61); 1.3981 (0.39); 1.2987 (0.54); 1.2589 (0.71); 1.2362 (0.49); 1.0430 (1.03); 1.0220 (6.36); 1.0026 (11.44); 0.9836 (5.19); -0.0002 (16.65) |
| Beispiel Nr. 2, Lösungsmittel: DMSO-d6, NMR-Spektrometer: 601.6 MHz |
| 8.5604 (0.46); 7.2449 (0.95); 7.2405 (1.04); 7.2054 (0.79); 7.1910 (1.54); 7.1640 (0.76); 7.1595 (0.68); 7.1495 (0.39); 7.1450 (0.36); 4.1932 (0.81); 4.1894 (0.85); 4.1834 (0.87); 4.1798 (0.83); 4.0599 (1.29); 3.9498 (6.53); 3.4090 (7.07); 3.3411 (10.18); 3.1977 (1.32); 2.5091 (5.34); 2.5063 (11.46); 2.5033 (16.00); 2.5004 (12.06); 2.4977 (5.87); 1.5643 (0.48); 0.6982 (0.41); 0.6906 (1.31); 0.6873 (0.99); 0.6825 (0.96); 0.6806 (0.90); 0.6774 (0.62); 0.6692 (0.57); 0.6633 (0.69); 0.6585 (0.53); 0.6560 (0.52); 0.6545 (0.53); 0.6526 (0.53); 0.6506 (0.53); -0.0002 (6.86) |
| Beispiel Nr. 3, Lösungsmittel: DMSO-d6, NMR-Spektrometer: 601.6 MHz |
| 8.3150 (0.77); 8.3052 (1.51); 8.2954 (0.78); 7.3626 (0.40); 7.2241 (1.15); 7.2196 (1.40); 7.2148 (1.96); 7.2091 (1.31); 7.2003 (2.25); 7.1850 (1.62); 7.1439 (0.80); 7.1369 (1.04); 7.1316 (0.89); 7.1242 (0.69); 7.1178 (0.46); 4.3304 (0.32); 4.1891 (1.57); 4.1785 (2.97); 4.1684 (1.62); 4.1525 (0.33); 4.0562 (1.97); 3.9870 (0.36); 3.9751 (0.99); 3.9633 (1.28); 3.9523 (1.63); 3.9405 (16.00); 3.9287 (0.52); 3.8087 (0.34); 3.7968 (1.18); 3.7850 (1.42); 3.7740 (1.23); 3.7622 (0.99); 3.3521 (112.39); 3.3373 (0.36); 3.0762 (1.63); 2.5253 (0.44); 2.5223 (0.58); 2.5191 (0.69); 2.5102 (13.68); 2.5073 (28.27); 2.5043 (37.98); 2.5013 (27.59); 2.4984 (12.87); 2.1514 (0.68); 2.1376 (1.17); 2.1324 (0.86); 2.1247 (2.55); 2.1198 (2.50); 2.1120 (2.54); 2.1072 (2.55); 2.0993 (0.89); 2.0948 (1.02); 2.0791 (0.56); 1.1529 (4.41); 1.1410 (9.38); 1.1290 (4.29); 1.1059 (5.12); 1.0344 (0.80); 1.0218 (1.66); 1.0137 (5.94); 1.0092 (1.10); 1.0011 (12.16); 0.9884 (5.68); 0.9369 (0.53); 0.9251 (1.12); 0.9133 (0.52); -0.0002 (2.07) |
| Beispiel Nr. 4, Lösungsmittel: DMSO-d6, NMR-Spektrometer: 399.95 MHz |
| 11.2369 (3.39); 8.3685 (0.92); 8.3535 (1.75); 8.3392 (0.89); 7.6365 (0.77); 7.6294 (1.04); 7.6251 (0.95); 7.6180 (1.12); 7.6074 (1.22); 7.5962 (1.04); 7.5801 (1.64); 7.5735 (1.34); 7.5634 (1.63); 7.5570 (1.23); 7.2521 (1.74); 7.2289 (2.57); 7.2058 (1.54); 4.2978 (3.87); 4.2831 (3.81); 4.0078 (16.00); 3.3263 (40.12); 3.3039 (0.41); 2.5064 (22.13); 2.5024 (28.80); 2.4984 (20.58); 2.4771 (0.74); 2.4584 (0.59); 2.1826 (1.82); 2.1635 (5.49); 2.1445 (5.62); 2.1255 (1.88); 1.0677 (0.37); 1.0530 (6.44); 1.0340 (13.09); 1.0150 (5.96); 1.0047 (0.85); 0.9861 (1.44); 0.9676 (0.83); 0.0079 (0.55); -0.0002 (12.30); -0.0085 (0.49) |
| Beispiel Nr. 5, Lösungsmittel: DMSO-d6, NMR-Spektrometer: 399.95 MHz |
| 8.6232 (0.37); 8.5796 (0.84); 8.5648 (1.65); 8.5502 (0.81); 7.3816 (0.35); 7.3651 (0.44); 7.3594 (0.69); 7.3361 (0.55); 7.2591 (1.34); 7.2525 (1.53); 7.2427 (1.42); 7.2363 (1.42); 7.2135 (1.23); 7.1912 (2.69); 7.1680 (2.13); 7.1380 (1.12); 7.1303 (1.15); 7.1268 (1.28); 7.1194 (1.12); 7.1093 (0.68); 7.0981 (0.46); 4.3908 (0.41); 4.3772 (0.67); 4.3643 (0.39); 4.2236 (3.77); 4.2090 (3.73); 4.0960 (0.33); 4.0479 (3.34); 3.9470 (16.00); 3.9041 (15.20); 3.8397 (0.89); 3.8338 (3.07); 3.8249 (1.83); 3.8178 (6.72); 3.8100 (1.26); 3.8020 (3.22); 3.4345 (0.70); 3.3973 (21.68); 3.3816 (0.87); 3.3331 (210.20); 3.2978 (0.35); 3.1837 (4.34); 3.1745 (1.41); 3.1614 (1.18); 2.6759 (0.91); 2.6711 (1.76); 2.6550 (1.39); 2.6472 (2.00); 2.6439 (2.14); 2.6318 (3.54); 2.6279 (3.67); 2.6155 (1.88); 2.6124 (1.83); 2.5416 (1.00); 2.5246 (3.92); 2.5113 (70.99); 2.5071 (136.78); 2.5026 (175.40); 2.4981 (129.28); 2.4939 (66.57); 2.3383 (0.40); 2.3337 (0.79); 2.3292 (1.04); 2.3248 (0.77); 1.2583 (0.40); 1.2352 (1.24); 0.0080 (1.75); -0.0002 (46.49); -0.0084 (2.14) |
| Beispiel Nr. 6, Lösungsmittel: DMSO-d6, NMR-Spektrometer: 399.95 MHz |
| 11.1219 (3.55); 8.2437 (0.80); 8.2311 (1.51); 8.2182 (0.78); 7.8589 (0.66); 7.8438 (0.82); 7.8367 (1.32); 7.8220 (1.33); 7.8149 (0.80); 7.7998 (0.66); 7.1985 (0.91); 7.1951 (0.98); 7.1756 (1.76); 7.1725 (1.84); 7.1528 (0.85); 7.1498 (0.87); 4.3376 (3.41); 4.3248 (3.43); 4.0304 (16.00); 3.9041 (12.95); 3.3308 (121.58); 3.1738 (0.78); 3.1609 (0.75); 2.6754 (0.62); 2.6708 (0.85); 2.6664 (0.63); 2.5106 (58.00); 2.5064 (110.46); 2.5019 (141.45); 2.4974 (104.14); 2.4931 (53.17); 2.3333 (0.66); 2.3286 (0.88); 2.3242 (0.67); 2.0978 (1.64); 2.0788 (5.27); 2.0599 (5.45); 2.0409 (1.82); 0.9871 (6.18); 0.9682 (12.71); 0.9492 (5.84); 0.0078 (1.48); -0.0002 (35.16); -0.0085 (1.56) |
| Beispiel Nr. 7, Lösungsmittel: DMSO-d6, NMR-Spektrometer: 399.95 MHz |
| 8.3484 (0.43); 8.3364 (0.79); 8.3233 (0.45); 8.2597 (0.63); 7.6376 (0.33); 7.6225 (0.40); 7.6156 (0.65); 7.6011 (0.67); 7.5941 (0.43); 7.5793 (0.35); 7.3818 (0.40); 7.3603 (0.89); 7.3452 (0.90); 7.3239 (0.44); 7.2826 (0.54); 7.2634 (0.95); 7.2604 (0.98); 7.2380 (0.48); 7.1775 (0.34); 7.0469 (0.35); 4.3474 (1.72); 4.3346 (1.73); 4.2478 (0.66); 4.1607 (0.43); 4.1098 (0.55); 4.0967 (0.56); 4.0598 (8.65); 4.0193 (1.75); 3.9043 (16.00); 3.3935 (4.35); 3.3344 (282.02); 3.2920 (0.36); 3.1926 (10.83); 3.1740 (1.09); 3.1619 (0.98); 2.8911 (0.40); 2.7315 (0.35); 2.6762 (0.80); 2.6717 (1.08); 2.6672 (0.79); 2.5416 (0.77); 2.5246 (4.50); 2.5115 (72.62); 2.5071 (139.58); 2.5027 (178.47); 2.4981 (129.63); 2.4939 (65.07); 2.3338 (0.82); 2.3294 (1.10); 2.3250 (0.80); 1.8131 (11.07); 1.7448 (4.94); 1.2362 (0.45); 1.1399 (0.40); 0.0080 (2.10); -0.0002 (53.20); - 0.0084 (2.17) |
| Beispiel Nr. 8, Lösungsmittel: DMSO-d6, NMR-Spektrometer: 399.95 MHz |
| 8.3429 (0.45); 8.3279 (0.89); 8.3131 (0.46); 7.2217 (0.73); 7.2152 (1.06); 7.1981 (2.52); 7.1895 (1.13); 7.1830 (0.83); 7.1757 (2.66); 7.1701 (0.95); 5.7539 (2.29); 4.3238 (0.38); 4.3102 (0.38); 4.1686 (1.06); 4.1529 (2.12); 4.1381 (1.13); 4.0593 (1.79); 4.0395 (0.90); 4.0217 (0.90); 3.9549 (10.31); 3.4135 (15.38); 3.3485 (155.08); 3.3254 (0.82); 3.1995 (2.40); 2.5088 (12.34); 2.5047 (16.47); 1.9894 (3.90); 1.8722 (2.60); 1.8388 (16.00); 1.1934 (1.03); 1.1756 (2.03); 1.1579 (1.00); -0.0002 (0.84) |
| Beispiel Nr. 9, Lösungsmittel: DMSO-d6, NMR-Spektrometer: 399.95 MHz |
| 8.3652 (0.60); 8.3505 (1.15); 8.3358 (0.59); 7.3572 (0.39); 7.3387 (0.34); 7.2260 (0.85); 7.2172 (1.07); 7.2109 (1.38); 7.2037 (2.73); 7.1953 (1.29); 7.1809 (1.70); 7.1685 (0.90); 7.1617 (0.85); 7.1572 (1.06); 7.1501 (0.84); 7.1398 (0.43); 7.1357 (0.41); 4.1774 (1.45); 4.1647 (2.27); 4.1520 (1.51); 4.1134 (0.39); 4.0581 (1.46); 3.9867 (0.75); 3.9693 (1.05); 3.9522 (1.46); 3.9370 (12.66); 3.9164 (0.48); 3.9041 (10.14); 3.8156 (1.01); 3.7979 (1.20); 3.7812 (0.96); 3.7635 (0.73); 3.4064 (0.45); 3.3427 (342.34); 3.3012 (0.41); 3.1736 (0.61); 3.1615 (0.60); 2.6758 (0.59); 2.6717 (0.81); 2.6675 (0.62); 2.5418 (0.73); 2.5070 (97.11); 2.5028 (125.11); 2.4984 (93.15); 2.3337 (0.53); 2.3294 (0.72); 2.3253 (0.55); 1.8689 (1.91); 1.8354 (16.00); 1.1614 (3.39); 1.1435 (7.31); 1.1256 (3.28); 0.9488 (0.38); 0.9311 (0.82); 0.9131 (0.37); 0.0073 (1.20); -0.0002 (28.34); -0.0083 (1.39) |
| Beispiel Nr. 10, Lösungsmittel: DMSO-d6, NMR-Spektrometer: 399.95 MHz |
| 11.2095 (0.41); 8.5455 (0.82); 8.5310 (1.57); 8.5165 (0.83); 7.4646 (0.36); 7.3380 (0.41); 7.3177 (2.03); 7.2979 (3.32); 7.2784 (2.52); 7.2580 (0.35); 7.1889 (2.29); 7.1697 (1.69); 7.1369 (3.23); 7.0731 (1.83); 7.0527 (1.50); 4.3405 (0.74); 4.3258 (0.74); 4.1913 (2.45); 4.1834 (2.78); 4.1766 (2.79); 4.1689 (2.47); 4.0612 (2.61); 4.0379 (1.60); 4.0201 (1.62); 4.0023 (0.57); 3.9274 (16.00); 3.4351 (20.96); 3.3195 (54.85); 3.2098 (3.18); 2.6747 (0.41); 2.6703 (0.56); 2.6659 (0.41); 2.5405 (0.44); 2.5058 (60.23); 2.5014 (77.74); 2.4969 (58.01); 2.3324 (0.40); 2.3282 (0.54); 2.3237 (0.41); 1.9886 (6.72); 1.6385 (0.34); 1.6317 (0.32); 1.6199 (0.43); 1.5934 (0.46); 1.5812 (0.89); 1.5742 (1.00); 1.5682 (0.85); 1.5624 (1.73); 1.5501 (1.06); 1.5431 (1.02); 1.5310 (0.51); 1.3978 (9.38); 1.2981 (0.72); 1.2585 (1.04); 1.2354 (0.58); 1.1923 (1.93); 1.1745 (3.75); 1.1567 (1.93); 0.7087 (1.17); 0.6915 (5.07); 0.6798 (4.72); 0.6705 (4.85); 0.6622 (2.06); 0.6511 (4.11); 0.6428 (1.60); 0.6323 (0.61); 0.0077 (1.45); -0.0002 (31.06); -0.0082 |
| (1.58) |
| Beispiel Nr. 11, Lösungsmittel: DMSO-d6, NMR-Spektrometer: 399.95 MHz |
| 11.1270 (2.60); 8.3242 (0.58); 8.3121 (1.06); 8.2998 (0.58); 7.8625 (0.50); 7.8476 (0.61); 7.8404 (0.99); 7.8255 (0.99); 7.8184 (0.61); 7.8032 (0.49); 7.1996 (0.73); 7.1770 (1.37); 7.1579 (0.64); 7.1548 (0.65); 4.3289 (2.53); 4.3159 (2.51); 4.0314 (11.89); 3.9041 (6.94); 3.3311 (100.05); 2.6757 (0.49); 2.6711 (0.66); 2.6668 (0.48); 2.5107 (42.95); 2.5065 (81.75); 2.5021 (104.22); 2.4976 (76.24); 2.4934 (38.74); 2.3332 (0.46); 2.3287 (0.62); 2.3243 (0.46); 1.8005 (16.00); 0.0078 (1.11); -0.0002 (25.28); - 0.0083 (1.05) |
| Beispiel Nr. 12, Lösungsmittel: DMSO-d6, NMR-Spektrometer: 399.95 MHz |
| 8.6107 (0.92); 8.5961 (1.81); 8.5812 (0.89); 7.3323 (1.37); 7.3256 (1.58); 7.3164 (1.47); 7.3096 (1.53); 7.3012 (1.41); 7.2787 (2.82); 7.2556 (2.17); 7.2163 (1.00); 7.2056 (1.27); 7.1979 (1.10); 7.1871 (0.73); 7.1765 (0.53); 5.1965 (3.27); 5.1523 (4.32); 4.8965 (4.13); 4.8524 (3.21); 4.2103 (3.41); 4.1958 (3.49); 4.0468 (0.44); 3.9816 (16.00); 3.3248 (190.37); 3.3015 (2.14); 2.6709 (0.42); 2.6663 (0.33); 2.5243 (0.82); 2.5196 (1.21); 2.5109 (20.82); 2.5063 (45.24); 2.5017 (64.11); 2.4972 (47.72); 2.4926 (22.94); 2.3286 (0.41); 2.0739 (1.63); 1.5992 (0.37); 1.5872 (0.79); 1.5795 (0.88); 1.5686 (1.49); 1.5560 (0.97); 1.5486 (0.92); 1.5366 (0.44); 1.2982 (0.60); 1.2586 (0.86); 1.2360 (0.36); 0.7184 (0.42); 0.7079 (1.31); 0.6999 (3.85); 0.6883 (3.58); 0.6804 (1.11); 0.6720 (2.28); 0.6645 (1.77); 0.6518 (2.51); 0.6450 (1.68); 0.6363 (0.79); 0.6247 (0.50); 0.0081 (0.80); -0.0002 (30.31); -0.0085 (1.08) |
| Beispiel Nr. 13, Lösungsmittel: DMSO-d6, NMR-Spektrometer: 601.6 MHz |
| 11.2623 (1.20); 8.4425 (0.44); 8.4328 (0.89); 8.4233 (0.46); 7.6698 (0.38); 7.6652 (0.48); 7.6628 (0.48); 7.6574 (0.50); 7.6504 (0.53); 7.6433 (0.41); 7.5519 (0.69); 7.5478 (0.70); 7.5411 (0.73); 7.5367 (0.64); 7.2476 (0.51); 7.2322 (0.90); 7.2168 (0.48); 4.2824 (2.30); 4.2727 (2.31); 4.0084 (9.61); 3.3424 (264.00); 3.3279 (0.40); 3.3187 (1.99); 2.6175 (0.34); 2.6144 (0.47); 2.6114 (0.34); 2.5237 (0.64); 2.5207 (0.83); 2.5176 (0.84); 2.5087 (24.61); 2.5057 (54.48); 2.5027 (74.89); 2.4996 (53.26); 2.4966 (23.66); 2.3899 (0.34); 2.3869 (0.47); 2.3839 (0.34); 2.0769 (0.35); 1.9899 (0.43); 1.8760 (16.00); 0.0052 (0.79); -0.0002 (25.85); -0.0058 (0.74) |
| Beispiel Nr. 14, Lösungsmittel: DMSO-d6, NMR-Spektrometer: 399.95 MHz |
| 8.5805 (0.83); 8.5660 (1.68); 8.5514 (0.83); 7.3675 (0.52); 7.3440 (0.41); 7.2433 (1.32); 7.2366 (1.54); 7.2268 (1.49); 7.2214 (2.55); 7.1995 (2.80); 7.1763 (2.31); 7.1490 (1.04); 7.1418 (1.10); 7.1378 (1.28); 7.1305 (1.11); 7.1201 (0.64); 7.1163 (0.62); 7.1091 (0.48); 4.3504 (0.34); 4.2065 (3.65); 4.1918 (3.68); 4.0568 (2.20); 3.9742 (1.04); 3.9564 (1.41); 3.9396 (2.40); 3.9322 (16.00); 3.9222 (1.96); 3.9043 (0.46); 3.8286 (0.39); 3.8107 (1.40); 3.7928 (1.65); 3.7764 (1.28); 3.7586 (1.02); 3.3506 (0.63); 3.3272 (210.14); 3.3071 (0.89); 2.5247 (0.63); 2.5200 (0.92); 2.5113 (14.80); 2.5068 (31.80); 2.5021 (44.81); 2.4976 (33.28); 2.4931 (15.93); 2.0741 (1.66); 1.9888 (0.56); 1.6032 (0.38); 1.5907 (0.75); 1.5843 (0.86); 1.5780 (0.65); 1.5725 (1.62); 1.5642 (0.68); 1.5580 (0.86); 1.5408 (0.44); 1.3978 (0.36); 1.2586 (0.43); 1.1749 (0.41); 1.1633 (4.66); 1.1454 (10.48); 1.1275 (4.53); 0.9471 (0.58); 0.9295 (1.28); 0.9117 (0.57); 0.7037 (0.39); 0.6914 (1.84); 0.6841 (5.34); 0.6736 (6.10); 0.6605 (2.20); 0.6552 (2.36); 0.6495 |
| (1.91); 0.6399 (0.80); 0.6340 (0.46); 0.0080 (0.37); -0.0002 (12.47); -0.0085 (0.42) |
| Beispiel Nr. 15, Lösungsmittel: DMSO-d6, NMR-Spektrometer: 399.95 MHz |
| 11.2463 (3.76); 8.4634 (0.92); 8.4487 (1.74); 8.4344 (0.86); 7.6368 (0.71); 7.6301 (1.04); 7.6260 (0.98); 7.6186 (1.11); 7.6080 (1.25); 7.5969 (1.07); 7.5859 (1.72); 7.5795 (1.35); 7.5693 (1.70); 7.5632 (1.27); 7.2587 (1.70); 7.2355 (2.71); 7.2124 (1.53); 5.9493 (0.43); 5.9317 (0.85); 5.9240 (0.48); 5.9145 (0.47); 5.9064 (1.37); 5.8890 (1.37); 5.8811 (0.54); 5.8716 (0.51); 5.8637 (0.98); 5.8464 (0.48); 5.1397 (1.72); 5.1356 (1.82); 5.0966 (1.59); 5.0926 (1.80); 5.0882 (1.62); 5.0851 (1.93); 5.0814 (1.69); 5.0596 (1.66); 5.0561 (1.55); 4.3679 (0.41); 4.3519 (0.41); 4.3001 (3.92); 4.2856 (3.89); 4.0085 (16.00); 3.9042 (8.21); 3.3942 (0.44); 3.3866 (0.53); 3.3813 (0.62); 3.3361 (200.51); 3.1743 (0.59); 3.1616 (0.56); 2.9655 (4.28); 2.9482 (4.21); 2.6759 (0.56); 2.6716 (0.70); 2.6670 (0.53); 2.6206 (0.34); 2.5422 (0.75); 2.5068 (91.78); 2.5025 (115.94); 2.4981 (85.71); 2.3333 (0.50); 2.3292 (0.68); 2.3249 (0.50); 1.8079 (0.56); 1.8044 (0.58); 1.7906 (0.58); 1.7870 (0.58); 1.4976 (0.48); 1.4812 (0.47); 0.0076 (1.24); -0.0002 (28.57); -0.0080 (1.44) |
| Beispiel Nr. 16, Lösungsmittel: DMSO-d6, NMR-Spektrometer: 399.95 MHz |
| 8.2694 (0.69); 8.2565 (1.26); 8.2433 (0.76); 8.2019 (0.77); 8.1682 (0.92); 7.6323 (0.46); 7.6178 (0.58); 7.6105 (0.98); 7.5959 (0.98); 7.5889 (0.64); 7.5742 (0.50); 7.3369 (1.25); 7.3227 (1.26); 7.2772 (0.85); 7.2549 (1.49); 7.2321 (0.76); 7.1723 (0.56); 7.0518 (0.53); 7.0325 (0.71); 7.0099 (0.37); 4.3560 (2.65); 4.3430 (2.73); 4.3032 (1.02); 4.2874 (1.15); 4.1692 (0.67); 4.1402 (0.52); 4.1230 (0.54); 4.1090 (0.82); 4.0956 (0.92); 4.0829 (0.66); 4.0590 (12.84); 4.0298 (2.98); 3.9755 (0.46); 3.9532 (0.60); 3.9044 (16.00); 3.5088 (0.40); 3.3904 (6.83); 3.3331 (185.46); 3.1917 (15.03); 3.1748 (1.63); 3.1617 (1.49); 3.0782 (0.36); 2.6761 (0.76); 2.6718 (1.05); 2.6675 (0.78); 2.5413 (0.78); 2.5071 (137.56); 2.5028 (176.48); 2.4984 (133.00); 2.3726 (0.35); 2.3538 (0.37); 2.3338 (0.91); 2.3295 (1.14); 2.3251 (0.87); 2.1107 (1.24); 2.0917 (3.88); 2.0726 (4.16); 2.0533 (2.09); 2.0155 (2.54); 1.3511 (1.15); 1.3361 (0.59); 1.2980 (0.39); 1.2585 (0.66); 1.2489 (0.93); 1.2343 (1.76); 1.0864 (0.44); 1.0678 (0.81); 1.0489 (0.45); 0.9946 (4.40); 0.9842 (1.26); 0.9757 (8.93); 0.9653 (2.40); 0.9564 (7.69); 0.9468 (2.96); 0.9356 (8.19); 0.9169 (4.12); 0.8537 (0.42); 0.0075 (1.82); -0.0002 (43.81); -0.0080 (2.70) |
| Beispiel Nr. 17, Lösungsmittel: DMSO-d6, NMR-Spektrometer: 399.95 MHz |
| 8.2632 (0.75); 8.2484 (1.38); 8.2337 (0.74); 7.4564 (0.42); 7.3307 (0.33); 7.3071 (2.01); 7.2876 (3.46); 7.2682 (2.62); 7.1804 (2.19); 7.1611 (1.64); 7.1313 (3.06); 7.0685 (1.68); 7.0492 (1.38); 6.8704 (0.35); 5.7595 (2.38); 4.3179 (0.67); 4.3031 (0.67); 4.2144 (0.55); 4.1991 (0.54); 4.1762 (1.92); 4.1608 (1.97); 4.1502 (1.99); 4.1352 (1.93); 4.1120 (0.63); 4.0969 (0.54); 4.0592 (2.41); 3.9349 (16.00); 3.4333 (23.32); 3.3282 (28.51); 3.3045 (0.50); 3.2079 (3.23); 2.6712 (0.43); 2.6667 (0.32); 2.5415 (0.33); 2.5386 (0.33); 2.5246 (1.73); 2.5198 (2.55); 2.5110 (24.27); 2.5067 (48.42); 2.5022 (62.90); 2.4976 (45.40); 2.4932 (21.91); 2.3334 (0.32); 2.3289 (0.44); 2.3244 (0.34); 2.1834 (0.66); 2.1675 (0.87); 2.1466 (2.17); 2.1273 (5.88); 2.1083 (6.09); 2.0894 (2.08); 1.3552 (4.24); 1.2982 (0.81); 1.2584 (1.16); 1.2347 (0.43); 1.0553 (0.97); 1.0363 (2.13); 1.0276 (6.99); 1.0172 (1.30); 1.0087 (14.40); 0.9896 (6.48); 0.0080 (1.33); -0.0002 (37.65); -0.0085 (1.31) |
| Beispiel Nr. 18, Lösungsmittel: DMSO-d6, NMR-Spektrometer: 399.95 MHz |
| 8.3358 (0.39); 8.2653 (0.90); 8.2512 (1.70); 8.2366 (0.87); 7.4288 (0.33); 7.4096 (0.50); 7.3550 (0.73); 7.3316 (0.83); 7.2542 (1.41); 7.2480 (1.62); 7.2381 (1.53); 7.2320 (1.50); 7.2058 (1.15); 7.1834 (2.68); 7.1602 (2.12); 7.1350 (1.14); 7.1240 (1.39); 7.1163 (1.22); 7.1060 (0.71); 7.0955 (0.47); 4.3493 (0.56); 4.3411 (0.56); 4.1947 (2.96); 4.1807 (3.06); 4.0536 (3.35); 3.9513 (16.00); 3.9046 (5.90); 3.4348 (0.71); 3.4072 (20.23); 3.3820 (0.53); 3.3319 (56.83); 3.1944 (4.05); 3.1747 (0.80); 3.1617 (0.75); 2.6719 (0.56); 2.5069 (73.66); 2.5027 (91.67); 2.4985 (68.47); 2.3295 (0.54); 2.0603 (1.28); 2.0423 (1.71); 2.0355 (3.78); 2.0326 (3.86); 2.0177 (3.93); 1.3513 (0.69); 1.2984 (0.47); 1.2585 (0.71); 1.2492 (0.47); 1.2342 (1.15); 1.0032 (0.41); 0.9965 (0.51); 0.9842 (0.97); 0.9779 (0.82); 0.9657 (1.31); 0.9536 (0.81); 0.9469 (0.83); 0.9342 (0.45); 0.4553 (1.28); 0.4447 (3.72); 0.4408 (3.91); 0.4356 (1.92); 0.4307 (1.96); 0.4246 (3.75); 0.4207 (3.70); 0.4107 (1.40); 0.1425 (1.55); 0.1299 (4.02); 0.1175 (3.74); 0.1061 (1.00); 0.0078 (1.03); -0.0002 (22.34) |
| Beispiel Nr. 19, Lösungsmittel: DMSO-d6, NMR-Spektrometer: 601.6 MHz |
| 8.4265 (0.37); 8.4166 (0.74); 8.4067 (0.38); 7.9545 (2.05); 7.3034 (0.65); 7.3013 (0.65); 7.2964 (0.69); 7.2890 (1.52); 7.2736 (0.76); 7.2343 (0.38); 7.2276 (0.51); 7.2224 (0.43); 7.2141 (0.33); 5.2101 (1.53); 5.1807 (1.83); 4.8945 (1.77); 4.8651 (1.54); 4.4422 (3.58); 4.1852 (0.67); 4.1750 (0.69); 4.1652 (0.71); 4.1556 (0.72); 3.9957 (7.04); 3.9478 (0.60); 3.3606 (27.24); 2.8921 (16.00); 2.7324 (13.12); 2.7319 (12.45); 2.5507 (0.63); 2.5112 (4.14); 2.5082 (8.93); 2.5052 (12.19); 2.5022 (8.88); 2.4993 (4.19); 1.8786 (0.40); 1.8387 (10.20); -0.0002 (1.64) |
| Beispiel Nr. 20, Lösungsmittel: DMSO-d6, NMR-Spektrometer: 601.6 MHz |
| 11.2816 (2.52); 8.6563 (1.03); 8.6467 (2.02); 8.6371 (1.02); 7.6632 (0.76); 7.6570 (1.07); 7.6498 (1.09); 7.6429 (1.13); 7.6372 (0.82); 7.5658 (1.46); 7.5620 (1.49); 7.5551 (1.54); 7.5513 (1.34); 7.2563 (1.26); 7.2408 (2.25); 7.2256 (1.16); 4.3199 (4.24); 4.3102 (4.21); 4.0102 (16.00); 3.9976 (0.76); 3.3564 (206.84); 3.3328 (0.90); 2.8907 (1.74); 2.7306 (1.57); 2.6156 (0.41); 2.5038 (60.80); 2.3880 (0.37); 1.6404 (0.49); 1.6324 (0.89); 1.6275 (0.94); 1.6199 (1.62); 1.6120 (1.04); 1.6068 (0.87); 1.5988 (0.42); 1.2334 (0.47); 0.7127 (0.52); 0.7043 (2.04); 0.6992 (3.91); 0.6921 (3.21); 0.6807 (3.82); 0.6758 (1.95); 0.6704 (2.53); 0.6675 (3.26); 0.6628 (1.66); 0.6546 (0.55); -0.0002 (4.68) |
| Beispiel Nr. 21, Lösungsmittel: DMSO-d6, NMR-Spektrometer: 600,13 MHz |
| 7,400 (0,39); 7,390 (0, 68); 7,380 (0,36); 7,322 (0,33); 7,291 (0, 62); 7,286 (0,7); 7,280 (0,7); 7,275 (0, 66); 7,173 (0,59); 7,158 (1,24); 7,143 (0, 96); 7,113 (0,36); 7,107 (0,49); 7,100 (0,58); 7,094 (0,51); 7,087 (0,39); 5,765 (0,55); 4,078 (1,13); 4,069 (1,34); 4,056 (1, 92); 4,048 (0,89); 4,036 (2,37); 4,024 (2,37); 4,012 (0,8); 3,960 (6,74); 3,441 (1, 94); 3,425 (0,72); 3,411 (7, 68); 3,353 (6, 04); 3,214 (0,33); 3,193 (1,46); 2,512 (4,48); 2,509 (9, 94); 2,506 (13,84); 2,503 (10,07); 2,500 (4, 66); 1,992 (10,21); 1,410 (0,47); 1,397 (4,1); 1,389 (16); 1,373 (1,27); 1,368 (2,45); 1,296 (0,5); 1,188 (2,7); 1,176 (5,45); 1,164 (2, 66); 0,000 (1,18) |
| Beispiel Nr. 22, Lösungsmittel: DMSO-d6, NMR-Spektrometer: 399.95 MHz |
| 11.2563 (3.44); 8.7984 (0.75); 8.7841 (1.44); 8.7694 (0.75); 7.6096 (1.36); 7.6040 (2.01); 7.5979 (2.68); 7.5812 (3.03); 7.2634 (1.40); 7.2512 (0.51); 7.2393 (2.50); 7.2221 (0.42); 7.2151 (1.14); 5.1562 (0.38); 5.1395 (1.28); 5.1228 (1.28); 5.1059 (0.36); 5.0338 (0.37); 5.0172 (1.28); 5.0005 (1.31); 4.9837 (0.40); 4.4069 (0.34); 4.3922 (0.35); 4.3683 (1.69); 4.3520 (3.06); 4.3360 (1.70); 4.3115 (0.36); 4.2975 (0.33); 4.0065 (16.00); 3.9040 (12.93); 3.3319 (186.48); 3.1739 (0.87); 3.1609 (0.86); 2.6753 (0.68); 2.6710 (0.91); 2.6665 (0.68); 2.5063 (117.69); 2.5019 (150.80); 2.4974 (110.97); 2.4934 (56.74); 2.3330 (0.70); 2.3286 (0.94); 2.3240 (0.71); 1.5096 (5.04); 1.4929 (5.05); 1.4483 (5.26); 1.4315 (4.90); 0.0076 (1.62); -0.0002 (38.65); -0.0084 (1.67) |
| Beispiel Nr. 23, Lösungsmittel: DMSO-d6, NMR-Spektrometer: 399.95 MHz |
| 11.1938 (3.33); 8.3578 (0.75); 8.3434 (1.48); 8.3290 (0.81); 7.5591 (1.52); 7.5366 (3.88); 7.3734 (1.50); 7.3541 (2.95); 7.3346 (1.53); 7.0958 (1.99); 7.0767 (1.76); 4.2696 (4.53); 4.2546 (4.51); 4.0066 (16.00); 3.9034 (15.18); 3.5078 (0.63); 3.4945 (0.45); 3.3660 (742.89); 3.2623 (0.52); 3.1725 (0.86); 3.1648 (0.85); 2.6776 (0.77); 2.6732 (1.02); 2.6688 (0.78); 2.5434 (1.05); 2.5086 (126.74); 2.5042 (160.79); 2.4997 (119.54); 2.3353 (0.68); 2.3308 (0.93); 2.3263 (0.70); 2.1774 (1.71); 2.1583 (5.38); 2.1393 (5.55); 2.1203 (1.85); 1.2354 (0.39); 1.0528 (6.21); 1.0339 (12.70); 1.0148 (5.77); 0.0079 (1.27); -0.0002 (28.87); -0.0084 (1.20) |
| Beispiel Nr. 24, Lösungsmittel: DMSO-d6, NMR-Spektrometer: 399.95 MHz |
| 11.2540 (2.31); 8.5892 (0.56); 8.5750 (1.14); 8.5604 (0.55); 7.6346 (0.77); 7.6281 (1.17); 7.6124 (1.62); 7.6022 (0.81); 7.5918 (0.70); 7.5806 (0.70); 7.5737 (0.45); 7.2677 (1.06); 7.2443 (1.58); 7.2215 (0.92); 4.3313 (2.52); 4.3170 (2.51); 4.0046 (10.33); 3.9036 (8.87); 3.5080 (0.36); 3.4785 (0.48); 3.3653 (609.88); 3.2767 (0.40); 3.2634 (0.33); 3.1751 (0.60); 3.1624 (0.59); 3.1291 (9.04); 2.6775 (0.51); 2.6734 (0.69); 2.6692 (0.54); 2.5430 (0.59); 2.5087 (88.94); 2.5044 (114.14); 2.5001 (85.23); 2.3353 (0.50); 2.3309 (0.68); 2.3270 (0.51); 2.0964 (16.00); 0.0077 (1.01); -0.0002 (23.72); -0.0075 (1.28) |
| Beispiel Nr. 25, Lösungsmittel: DMSO-d6, NMR-Spektrometer: 399.95 MHz |
| 11.3021 (3.64); 8.4440 (1.91); 8.4266 (1.93); 7.7894 (3.30); 7.7836 (3.39); 7.5024 (1.10); 7.4964 (0.96); 7.4807 (2.89); 7.4748 (2.95); 7.4576 (5.51); 7.4361 (1.90); 5.1247 (1.24); 5.1073 (1.95); 5.0899 (1.24); 4.0946 (0.36); 4.0091 (16.00); 3.9041 (12.80); 3.3308 (120.31); 3.1742 (1.47); 3.1611 (1.42); 2.6756 (0.68); 2.6711 (0.91); 2.6669 (0.68); 2.5413 (1.04); 2.5106 (62.54); 2.5065 (116.78); 2.5021 (147.63); 2.4977 (107.47); 2.3333 (0.67); 2.3288 (0.89); 2.3244 (0.65); 2.0426 (5.74); 2.0249 (5.96); 1.3363 (7.68); 1.3188 (7.60); 1.2984 (0.35); 1.2585 (0.35); 1.2354 (0.59); 0.9925 (0.36); 0.9795 (0.70); 0.9730 (0.66); 0.9691 (0.59); 0.9610 (1.12); 0.9489 (0.71); 0.9417 (0.74); 0.9289 (0.40); 0.4205 (0.96); 0.4126 (1.27); 0.4070 (2.46); 0.4004 (1.91); 0.3922 (1.84); 0.3875 (2.38); 0.3719 (1.02); 0.1738 (0.50); 0.1667 (1.05); 0.1551 (1.45); 0.1467 (1.38); 0.1360 (0.78); 0.1305 (0.84); 0.1193 (1.29); 0.1127 (1.34); 0.1013 (1.02); 0.0906 (0.52); 0.0855 (0.41); 0.0078 (1.62); -0.0002 (37.68); -0.0084 (1.61) |
| Beispiel Nr. 26, Lösungsmittel: DMSO-d6, NMR-Spektrometer: 399.95 MHz |
| 11.2371 (3.43); 8.6247 (0.81); 8.6103 (1.69); 8.5959 (0.89); 7.6255 (0.56); 7.6188 (1.05); 7.6144 (0.85); 7.5995 (3.50); 7.5861 (3.26); 7.2663 (1.08); 7.2418 (2.05); 7.2199 (1.23); 4.3376 (3.71); 4.3232 (3.74); 4.0033 (16.00); 3.9036 (15.05); 3.8197 (3.32); 3.8037 (6.84); 3.7878 (3.41); 3.5081 (0.37); 3.4783 (0.37); 3.3620 (711.51); 3.2329 |
| (0.37); 3.1751 (1.00); 3.1621 (0.95); 2.6775 (0.78); 2.6730 (1.03); 2.6686 (0.85); 2.6569 (3.25); 2.6411 (6.35); 2.6251 (3.08); 2.5128 (60.87); 2.5085 (116.55); 2.5040 (148.75); 2.4994 (107.71); 2.4950 (53.65); 2.3352 (0.65); 2.3307 (0.89); 2.3262 (0.64); 1.2352 (0.33); 0.0078 (1.62); -0.0002 (36.74); -0.0084 (1.38) |
| Beispiel Nr. 27, Lösungsmittel: DMSO-d6, NMR-Spektrometer: 399.95 MHz |
| 8.3260 (0.54); 8.3116 (0.99); 8.2974 (0.55); 7.3289 (0.47); 7.3244 (0.47); 7.3122 (1.20); 7.2928 (2.47); 7.2734 (1.76); 7.1856 (1.68); 7.1664 (1.25); 7.1200 (2.39); 7.0879 (1.36); 7.0682 (1.09); 4.3090 (0.51); 4.2940 (0.51); 4.2070 (0.49); 4.1915 (0.48); 4.1687 (1.42); 4.1533 (1.43); 4.1339 (1.45); 4.1190 (1.44); 4.0957 (0.49); 4.0807 (0.49); 4.0614 (1.76); 4.0383 (1.16); 4.0205 (1.17); 4.0028 (0.41); 3.9504 (0.34); 3.9359 (11.86); 3.4362 (15.11); 3.3276 (28.79); 3.3241 (19.92); 3.2104 (2.11); 2.5238 (0.50); 2.5064 (18.15); 2.5021 (24.16); 2.4978 (18.62); 1.9887 (4.76); 1.8850 (2.26); 1.8432 (16.00); 1.2348 (0.49); 1.1926 (1.33); 1.1749 (2.58); 1.1570 (1.32); 0.0069 (0.46); - 0.0002 (12.62); -0.0081 (0.67) |
| Beispiel Nr. 28, Lösungsmittel: DMSO-d6, NMR-Spektrometer: 399.95 MHz |
| 11.3115 (3.70); 8.4692 (2.00); 8.4519 (2.03); 7.7107 (3.32); 7.7045 (3.54); 7.5449 (1.55); 7.5386 (1.43); 7.5233 (2.42); 7.5170 (2.37); 7.4564 (4.89); 7.4349 (3.05); 5.1044 (1.23); 5.0870 (1.92); 5.0696 (1.24); 4.4489 (0.33); 4.1070 (0.65); 4.0939 (0.68); 4.0113 (16.00); 3.9040 (14.85); 3.3940 (0.78); 3.3873 (0.61); 3.3806 (0.84); 3.3301 (139.59); 3.1740 (2.83); 3.1610 (2.79); 2.8901 (0.41); 2.7303 (0.38); 2.6748 (0.73); 2.6708 (1.00); 2.6663 (0.75); 2.5413 (1.15); 2.5102 (66.05); 2.5062 (126.77); 2.5018 (164.40); 2.4973 (121.89); 2.4929 (62.91); 2.3329 (0.74); 2.3284 (1.00); 2.3240 (0.77); 2.1662 (1.47); 2.1473 (4.81); 2.1284 (5.11); 2.1094 (1.74); 1.3502 (0.54); 1.3324 (7.78); 1.3149 (7.71); 1.2486 (0.37); 1.2351 (0.60); 1.0154 (5.69); 0.9965 (11.72); 0.9774 (5.38); 0.0080 (1.64); -0.0002 (40.92); -0.0084 (1.88) |
| Beispiel Nr. 29, Lösungsmittel: DMSO-d6, NMR-Spektrometer: 399.95 MHz |
| 8.3193 (0.77); 8.3051 (1.52); 8.2904 (0.80); 7.3633 (0.53); 7.3399 (0.43); 7.2326 (1.47); 7.2259 (1.57); 7.2128 (2.25); 7.1906 (2.68); 7.1674 (2.14); 7.1354 (1.01); 7.1243 (1.27); 7.1169 (1.12); 7.1067 (0.69); 7.1030 (0.68); 7.0960 (0.50); 4.3406 (0.53); 4.1920 (1.87); 4.1812 (2.48); 4.1687 (2.05); 4.0482 (2.34); 3.9750 (1.01); 3.9572 (1.40); 3.9369 (16.00); 3.9230 (1.95); 3.9050 (0.47); 3.8285 (0.40); 3.8104 (1.36); 3.7926 (1.61); 3.7760 (1.28); 3.7584 (1.02); 3.4762 (0.32); 3.3361 (82.29); 3.3326 (112.64); 3.3262 (118.72); 3.3244 (98.74); 2.6759 (0.32); 2.6711 (0.46); 2.6666 (0.35); 2.5414 (0.32); 2.5244 (1.04); 2.5109 (23.09); 2.5066 (47.20); 2.5021 (64.05); 2.4975 (48.44); 2.4932 (25.22); 2.3288 (0.42); 2.1402 (0.45); 2.1222 (0.97); 2.1015 (3.12); 2.0832 (5.73); 2.0739 (0.68); 2.0647 (3.65); 1.5602 (0.46); 1.5493 (0.69); 1.5418 (2.08); 1.5311 (0.86); 1.5233 (3.94); 1.5129 (0.68); 1.5049 (3.90); 1.4865 (2.06); 1.4682 (0.49); 1.3554 (0.61); 1.2362 (0.35); 1.1556 (4.62); 1.1377 (10.20); 1.1198 (4.51); 0.9403 (0.64); 0.9226 (1.40); 0.9048 (0.65); 0.8688 (0.98); 0.8511 (7.79); 0.8328 (13.37); 0.8143 (5.70); 0.0080 (0.42); -0.0002 (13.37); -0.0085 (0.62) |
| Beispiel Nr. 30, Lösungsmittel: DMSO-d6, NMR-Spektrometer: 399.95 MHz |
| 11.1312 (3.58); 8.5370 (0.86); 8.5239 (1.62); 8.5108 (0.83); 7.8700 (0.65); 7.8550 (0.81); 7.8479 (1.30); 7.8332 (1.31); 7.8260 (0.78); 7.8111 (0.65); 7.2062 (0.97); 7.1835 (1.86); 7.1608 (0.89); 4.3616 (3.41); 4.3488 (3.40); 4.0329 (16.00); 3.9041 (14.97); 3.3308 (139.87); 3.1735 (0.79); 3.1608 (0.75); 2.6754 (0.65); 2.6708 (0.88); 2.6662 |
| (0.66); 2.5408 (0.60); 2.5062 (112.63); 2.5018 (143.45); 2.4974 (105.37); 2.4932 (53.73); 2.3329 (0.67); 2.3285 (0.90); 2.3241 (0.65); 1.5842 (0.36); 1.5719 (0.80); 1.5651 (0.90); 1.5593 (0.74); 1.5532 (1.61); 1.5408 (0.94); 1.5341 (0.90); 1.5218 (0.43); 0.6826 (0.41); 0.6691 (1.79); 0.6622 (4.23); 0.6580 (3.64); 0.6497 (4.94); 0.6443 (5.25); 0.6373 (1.82); 0.6292 (2.13); 0.6244 (3.70); 0.6176 (1.54); 0.6048 (0.44); 0.0078 (1.55); -0.0002 (35.56); -0.0085 (1.68) |
| Beispiel Nr. 31, Lösungsmittel: DMSO-d6, NMR-Spektrometer: 399.95 MHz |
| 11.1215 (3.65); 8.2116 (0.86); 8.1986 (1.63); 8.1857 (0.84); 7.8576 (0.65); 7.8427 (0.81); 7.8356 (1.31); 7.8209 (1.31); 7.8138 (0.79); 7.7989 (0.65); 7.1967 (0.99); 7.1742 (1.86); 7.1541 (0.88); 7.1516 (0.89); 4.3452 (3.51); 4.3321 (3.48); 4.0314 (16.00); 3.9042 (7.09); 3.3318 (91.08); 3.1744 (0.44); 3.1613 (0.43); 2.6756 (0.42); 2.6711 (0.55); 2.6667 (0.41); 2.5066 (67.62); 2.5021 (86.09); 2.4977 (63.39); 2.3333 (0.39); 2.3287 (0.51); 2.3245 (0.38); 1.9847 (6.09); 1.9672 (6.26); 0.9539 (0.34); 0.9410 (0.69); 0.9349 (0.65); 0.9310 (0.58); 0.9228 (1.13); 0.9148 (0.58); 0.9106 (0.70); 0.9030 (0.74); 0.8913 (0.39); 0.4169 (1.10); 0.4062 (3.17); 0.4022 (3.28); 0.3969 (1.55); 0.3920 (1.64); 0.3860 (3.21); 0.3820 (3.05); 0.3719 (1.17); 0.0995 (1.14); 0.0861 (3.74); 0.0768 (3.41); 0.0737 (3.69); 0.0626 (0.99); 0.0079 (1.10); -0.0002 (24.39); -0.0084 (1.14) |
| Beispiel Nr. 32, Lösungsmittel: DMSO-d6, NMR-Spektrometer: 399.95 MHz |
| 8.3476 (0.85); 8.3328 (1.70); 8.3180 (0.86); 7.3177 (1.32); 7.3109 (1.57); 7.2998 (2.33); 7.2953 (1.66); 7.2771 (2.70); 7.2540 (2.08); 7.2130 (0.98); 7.2023 (1.25); 7.1946 (1.06); 7.1838 (0.76); 7.1733 (0.52); 5.2074 (3.22); 5.1631 (4.17); 4.8902 (4.15); 4.8460 (3.28); 4.3057 (0.36); 4.2893 (0.39); 4.1951 (1.90); 4.1855 (2.36); 4.1811 (2.34); 4.1720 (2.10); 4.0455 (0.51); 4.0084 (0.82); 3.9918 (16.00); 3.9437 (1.77); 3.4336 (0.34); 3.3438 (101.62); 3.3203 (0.86); 2.8915 (1.58); 2.7321 (1.22); 2.5263 (0.44); 2.5215 (0.64); 2.5128 (12.31); 2.5084 (26.73); 2.5038 (36.25); 2.4993 (26.13); 2.4948 (12.63); 2.4772 (0.70); 2.4586 (0.62); 2.1634 (0.32); 2.1482 (1.11); 2.1447 (1.37); 2.1292 (3.19); 2.1259 (3.43); 2.1099 (3.43); 2.1071 (3.49); 2.0906 (1.29); 2.0885 (1.35); 1.0521 (0.32); 1.0331 (0.92); 1.0255 (7.15); 1.0138 (1.01); 1.0066 (14.50); 0.9875 (6.53); 0.9660 (0.65); 0.0080 (0.38); -0.0002 (14.34); -0.0085 (0.55) |
| Beispiel Nr. 33, Lösungsmittel: DMSO-d6, NMR-Spektrometer: 399.95 MHz |
| 11.2495 (3.27); 8.5139 (0.82); 8.4989 (1.65); 8.4843 (0.82); 7.6081 (1.36); 7.6024 (1.96); 7.5964 (2.58); 7.5796 (2.91); 7.2581 (1.31); 7.2458 (0.48); 7.2340 (2.30); 7.2167 (0.43); 7.2099 (1.03); 4.3069 (3.57); 4.2924 (3.56); 4.0225 (1.23); 4.0081 (15.32); 3.9039 (16.00); 3.6610 (3.44); 3.6446 (7.25); 3.6282 (3.54); 3.4343 (0.35); 3.4118 (0.53); 3.3422 (417.11); 3.1678 (1.81); 2.6759 (0.85); 2.6717 (1.14); 2.6672 (0.85); 2.5415 (0.96); 2.5070 (147.21); 2.5026 (187.04); 2.4982 (137.26); 2.3256 (3.26); 2.3079 (4.50); 2.2890 (3.08); 2.0038 (0.83); 1.9873 (2.45); 1.9686 (3.18); 1.9514 (2.25); 1.9344 (0.64); 1.3507 (0.35); 1.2340 (0.57); 0.0077 (1.75); -0.0002 (43.83); -0.0085 (2.00) |
| Beispiel Nr. 34, Lösungsmittel: DMSO-d6, NMR-Spektrometer: 399.95 MHz |
| 8.2394 (0.66); 8.2260 (1.15); 8.2129 (0.71); 8.1851 (0.54); 8.1294 (0.58); 7.7363 (0.35); 7.7147 (0.42); 7.6317 (0.41); 7.6168 (0.54); 7.6100 (0.84); 7.5953 (0.83); 7.5883 (0.53); 7.5735 (0.49); 7.3382 (0.63); 7.3165 (1.33); 7.3019 (1.34); 7.2800 (1.29); 7.2581 (1.23); 7.2335 (0.58); 7.1698 (0.43); 7.0327 (0.45); 4.3646 (2.32); 4.3517 (2.46); 4.2924 (0.80); 4.1834 (0.54); 4.1071 (0.62); 4.0956 (0.68); 4.0597 (11.27); 4.0360 (2.35); 3.9505 (0.39); 3.9044 (16.00); 3.3918 (5.79); 3.3314 (154.32); 3.1905 (13.26); |
| 3.1743 (1.13); 3.1619 (0.99); 3.0610 (0.45); 2.8656 (0.48); 2.6761 (0.94); 2.6717 (1.21); 2.6673 (0.88); 2.5415 (0.95); 2.5111 (82.57); 2.5070 (156.18); 2.5026 (198.25); 2.4981 (144.42); 2.3337 (0.89); 2.3293 (1.18); 2.3248 (0.87); 1.9963 (3.96); 1.9788 (4.21); 1.9261 (2.57); 1.2586 (0.35); 1.2355 (0.97); 0.9490 (0.49); 0.9426 (0.50); 0.9304 (0.93); 0.9180 (0.85); 0.9106 (1.02); 0.8985 (1.00); 0.8933 (1.01); 0.8808 (0.91); 0.8540 (0.61); 0.4188 (0.77); 0.4081 (2.30); 0.4041 (2.62); 0.3987 (1.71); 0.3878 (4.33); 0.3841 (4.30); 0.3736 (2.89); 0.1057 (0.83); 0.0923 (2.53); 0.0799 (2.89); 0.0680 (2.48); 0.0562 (3.27); 0.0080 (2.20); -0.0002 (56.33); -0.0084 (2.57) |
| Beispiel Nr. 35, Lösungsmittel: DMSO-d6, NMR-Spektrometer: 399.95 MHz |
| 11.3198 (3.66); 8.3762 (0.93); 8.3613 (1.93); 8.3464 (0.95); 7.6573 (2.72); 7.6513 (3.29); 7.6069 (1.62); 7.6007 (1.33); 7.5853 (2.12); 7.5791 (1.92); 7.4944 (4.29); 7.4729 (2.99); 4.3319 (4.23); 4.3172 (4.28); 4.0060 (16.00); 3.9039 (14.06); 3.4035 (0.38); 3.3383 (413.75); 3.1740 (0.91); 3.1613 (0.88); 2.6757 (0.72); 2.6714 (0.99); 2.6669 (0.75); 2.5068 (122.62); 2.5024 (159.78); 2.4979 (120.45); 2.3335 (0.73); 2.3291 (0.98); 2.3247 (0.76); 2.0966 (6.04); 2.0790 (6.27); 1.2349 (0.68); 1.0410 (0.37); 1.0287 (0.73); 1.0220 (0.67); 1.0102 (1.14); 0.9981 (0.71); 0.9904 (0.78); 0.9784 (0.41); 0.4745 (1.01); 0.4636 (3.00); 0.4598 (3.21); 0.4548 (1.57); 0.4495 (1.57); 0.4435 (3.07); 0.4396 (3.03); 0.4294 (1.17); 0.1804 (1.08); 0.1669 (3.74); 0.1546 (3.76); 0.1434 (1.02); 0.0079 (1.35); -0.0002 (35.36); -0.0084 (1.76) |
| Beispiel Nr. 36, Lösungsmittel: DMSO-d6, NMR-Spektrometer: 399.95 MHz |
| 8.4511 (0.32); 8.3803 (0.93); 8.3662 (1.76); 8.3515 (0.88); 7.4092 (0.32); 7.3974 (0.32); 7.3536 (0.58); 7.3467 (0.35); 7.3303 (0.82); 7.3085 (0.43); 7.2348 (1.59); 7.2284 (1.89); 7.2184 (1.75); 7.2126 (2.56); 7.2026 (1.28); 7.1905 (2.33); 7.1807 (1.45); 7.1675 (2.21); 7.1554 (1.29); 7.1485 (1.09); 7.1436 (1.26); 7.1370 (1.17); 7.1261 (0.53); 7.1151 (0.42); 6.6783 (0.53); 6.6612 (0.57); 6.6402 (0.55); 6.6230 (0.55); 5.9366 (0.43); 5.9297 (0.82); 5.9254 (0.74); 5.9196 (0.87); 5.9116 (0.47); 5.9025 (0.49); 5.8940 (1.41); 5.8871 (0.87); 5.8766 (1.24); 5.8687 (0.50); 5.8594 (0.46); 5.8511 (0.84); 5.8338 (0.40); 5.1448 (0.32); 5.1405 (0.35); 5.1323 (0.59); 5.1284 (1.33); 5.1242 (1.47); 5.1204 (0.67); 5.1015 (0.35); 5.0915 (1.70); 5.0864 (2.07); 5.0814 (1.78); 5.0665 (1.37); 5.0622 (1.18); 4.3371 (0.47); 4.3236 (0.46); 4.2347 (0.76); 4.2200 (1.15); 4.2132 (1.11); 4.1785 (2.08); 4.1662 (2.11); 4.1088 (0.41); 4.0957 (0.40); 4.0540 (3.34); 3.9435 (14.29); 3.9325 (6.22); 3.9042 (16.00); 3.4343 (1.61); 3.4055 (18.91); 3.4023 (11.29); 3.3935 (2.25); 3.3895 (2.02); 3.3813 (0.78); 3.3326 (222.72); 3.2112 (0.35); 3.1894 (4.36); 3.1799 (0.50); 3.1744 (1.86); 3.1613 (1.55); 2.9644 (0.67); 2.9468 (0.89); 2.9377 (3.14); 2.9204 (3.04); 2.6758 (0.86); 2.6714 (1.15); 2.6670 (0.87); 2.6250 (0.37); 2.6161 (0.33); 2.5415 (0.88); 2.5111 (78.01); 2.5068 (149.63); 2.5024 (191.70); 2.4978 (140.18); 2.4935 (70.87); 2.3334 (0.87); 2.3291 (1.18); 2.3246 (0.87); 1.8063 (2.51); 1.8025 (2.56); 1.7892 (2.53); 1.7853 (2.52); 1.5012 (0.58); 1.4945 (0.69); 1.4848 (0.60); 1.4779 (0.62); 1.3508 (0.73); 1.2979 (0.44); 1.2582 (0.83); 1.2487 (0.93); 1.2349 (2.56); 0.8539 (0.55); 0.0079 (1.98); -0.0002 (52.89); -0.0084 (2.41) |
| Beispiel Nr. 37, Lösungsmittel: DMSO-d6, NMR-Spektrometer: 399.95 MHz |
| 11.2427 (3.24); 8.3933 (0.80); 8.3785 (1.62); 8.3639 (0.79); 7.6288 (0.61); 7.6221 (0.91); 7.6177 (0.79); 7.6105 (0.98); 7.6001 (1.19); 7.5880 (2.32); 7.5809 (1.20); 7.5706 (1.61); 7.5643 (1.11); 7.2544 (1.55); 7.2308 (2.28); 7.2082 (1.42); 5.5214 (1.49); 5.5169 (1.07); 5.5095 (3.80); 5.5000 (2.91); 5.4973 (2.01); 5.4870 (0.74); 4.2859 (3.41); 4.2714 (3.39); 4.0941 (0.33); 4.0082 (14.83); 3.9040 (16.00); 3.3946 (0.44); 3.3807 (0.48); 3.3301 (131.25); 3.1741 (1.37); 3.1610 (1.31); 2.8740 (3.78); 2.8638 (2.71); |
| 2.8606 (2.58); 2.6755 (0.69); 2.6710 (0.92); 2.6665 (0.70); 2.5412 (0.71); 2.5242 (3.18); 2.5109 (59.54); 2.5065 (116.83); 2.5019 (151.53); 2.4974 (111.68); 2.4930 (56.78); 2.3332 (0.69); 2.3286 (0.94); 2.3242 (0.69); 1.6312 (5.07); 1.6286 (4.66); 1.6216 (5.84); 1.6196 (6.15); 1.2343 (0.49); 0.0080 (1.61); -0.0002 (41.17); -0.0085 (1.76) |
| Beispiel Nr. 38, Lösungsmittel: DMSO-d6, NMR-Spektrometer: 601.6 MHz |
| 8.1739 (0.42); 8.1642 (0.72); 8.1544 (0.35); 7.1079 (0.65); 7.1036 (0.70); 7.0973 (0.67); 7.0929 (0.63); 7.0866 (0.58); 7.0717 (1.01); 7.0564 (0.67); 6.9942 (0.44); 6.9877 (0.52); 6.9820 (0.45); 6.9747 (0.36); 5.6384 (0.55); 4.0763 (1.30); 4.0670 (1.32); 3.9284 (0.98); 3.8449 (0.57); 3.8331 (0.78); 3.8221 (1.09); 3.8110 (6.92); 3.6754 (0.57); 3.6636 (0.68); 3.6526 (0.58); 3.6408 (0.43); 3.2190 (9.11); 3.1955 (0.35); 2.4166 (0.48); 2.3832 (7.43); 2.3804 (12.88); 2.3774 (16.00); 2.3744 (11.19); 2.3716 (5.10); 1.9308 (0.56); 1.9190 (0.62); 1.9074 (1.27); 1.9004 (1.38); 1.8957 (1.30); 1.8886 (1.17); 1.0652 (0.37); 1.0562 (0.39); 1.0270 (2.15); 1.0151 (4.15); 1.0032 (1.88); 0.8493 (0.40); 0.8444 (0.38); 0.8414 (0.33); 0.8363 (0.54); 0.8282 (0.35); 0.8242 (0.35); 0.8158 (0.44); 0.8039 (0.60); 0.3175 (0.66); 0.3110 (1.32); 0.3082 (1.47); 0.3042 (0.83); 0.3016 (0.83); 0.2977 (1.25); 0.2948 (1.36); 0.2883 (0.48); 0.0163 (0.39); 0.0085 (0.82); 0.0016 (1.56); -0.0002 (1.59); -0.0063 (1.43); -0.0083 (1.32); -0.0154 (0.39); -0.1268 (1.42) |
| Beispiel Nr. 39, Lösungsmittel: DMSO-d6, NMR-Spektrometer: 399.95 MHz |
| 11.3358 (2.09); 8.4100 (0.89); 8.3952 (1.87); 8.3807 (0.95); 7.6550 (1.35); 7.6487 (1.68); 7.6336 (1.73); 7.6271 (2.28); 7.5957 (3.32); 7.5896 (2.69); 7.4914 (4.04); 7.4699 (3.22); 4.3131 (4.37); 4.2984 (4.39); 4.0066 (16.00); 3.9709 (0.68); 3.9040 (0.62); 3.6494 (0.56); 3.5835 (0.36); 3.3944 (0.95); 3.3803 (1.91); 3.3382 (683.62); 3.2155 (0.42); 3.2003 (0.37); 3.1741 (0.79); 3.1610 (0.76); 2.6758 (1.24); 2.6713 (1.64); 2.6668 (1.22); 2.5415 (2.03); 2.5111 (105.61); 2.5068 (199.02); 2.5023 (253.22); 2.4977 (184.00); 2.4933 (91.94); 2.3335 (1.30); 2.3290 (1.71); 2.3245 (1.28); 2.2201 (1.81); 2.2010 (5.58); 2.1820 (5.76); 2.1630 (1.93); 1.9016 (0.71); 1.3504 (1.37); 1.3352 (0.34); 1.3131 (0.39); 1.2977 (1.45); 1.2581 (2.19); 1.2333 (2.52); 1.0750 (6.49); 1.0560 (13.29); 1.0370 (6.10); 0.8835 (0.34); 0.8753 (0.44); 0.8669 (0.57); 0.8531 (0.71); 0.8352 (0.45); 0.0077 (2.40); -0.0002 (48.12); -0.0084 (1.99) |
| Beispiel Nr. 40, Lösungsmittel: DMSO-d6, NMR-Spektrometer: 399.95 MHz |
| 11.3292 (2.82); 8.5439 (1.51); 8.5263 (1.54); 7.6615 (2.43); 7.6552 (2.87); 7.6060 (1.44); 7.5996 (1.16); 7.5844 (1.83); 7.5780 (1.62); 7.4605 (3.57); 7.4390 (2.72); 5.1149 (0.94); 5.0975 (1.47); 5.0800 (0.94); 4.0393 (0.41); 4.0180 (12.30); 3.9040 (4.74); 3.3825 (0.35); 3.3413 (133.55); 3.1751 (1.11); 3.1621 (1.10); 2.5117 (20.75); 2.5075 (39.89); 2.5030 (51.20); 2.4985 (37.45); 2.4942 (18.87); 1.8591 (16.00); 1.3307 (6.00); 1.3133 (5.97); 1.2352 (0.54); 0.0078 (0.59); -0.0002 (14.10); -0.0085 (0.57) |
| Beispiel Nr. 41, Lösungsmittel: DMSO-d6, NMR-Spektrometer: 399.95 MHz |
| 11.3218 (3.61); 8.4297 (0.91); 8.4153 (1.89); 8.4007 (0.92); 7.6214 (6.44); 7.6036 (2.52); 7.5973 (1.45); 7.4916 (2.90); 7.4865 (1.21); 7.4730 (0.93); 7.4683 (2.16); 4.3195 (4.30); 4.3049 (4.30); 4.0046 (16.00); 3.9041 (11.47); 3.3323 (166.34); 3.1742 (0.99); 3.1612 (1.01); 2.6753 (0.66); 2.6711 (0.89); 2.6668 (0.67); 2.5065 (114.01); 2.5022 (143.88); 2.4979 (105.74); 2.3332 (0.68); 2.3289 (0.89); 2.3248 (0.66); 2.1756 (2.95); 2.1573 (5.60); 2.1387 (3.34); 1.6145 (0.40); 1.5960 (1.85); 1.5776 (3.57); 1.5592 (3.57); 1.5408 (1.88); 1.5219 (0.42); 1.2353 (0.72); 0.8949 (5.84); 0.8765 (11.70); 0.8580 (5.30); 0.0078 (1.34); -0.0002 (31.95) |
| Beispiel Nr. 42, Lösungsmittel: DMSO-d6, NMR-Spektrometer: 399.95 MHz |
| 11.2055 (3.38); 11.1789 (0.60); 8.6326 (0.82); 8.6179 (1.57); 8.6033 (0.80); 7.5840 (1.64); 7.5643 (2.00); 7.5314 (3.19); 7.3829 (1.70); 7.3634 (3.19); 7.3438 (1.67); 7.1599 (0.34); 7.1047 (2.01); 7.0853 (1.74); 4.4941 (0.64); 4.4783 (0.65); 4.2947 (4.48); 4.2799 (4.41); 4.0094 (16.00); 4.0011 (3.45); 3.9714 (6.15); 3.9039 (7.54); 3.8168 (3.58); 3.8102 (3.51); 3.3450 (335.87); 3.3134 (0.74); 3.1740 (0.71); 3.1621 (0.66); 2.6765 (0.47); 2.6717 (0.63); 2.6676 (0.48); 2.5422 (0.59); 2.5074 (81.00); 2.5029 (104.18); 2.4985 (77.04); 2.3340 (0.44); 2.3299 (0.62); 2.3252 (0.46); 1.6361 (0.35); 1.6242 (0.81); 1.6170 (0.90); 1.6108 (0.74); 1.6052 (1.62); 1.5931 (0.95); 1.5858 (0.92); 1.5737 (0.44); 0.7237 (0.42); 0.7108 (1.87); 0.7037 (4.18); 0.6991 (3.53); 0.6919 (3.55); 0.6859 (3.24); 0.6804 (4.19); 0.6735 (1.72); 0.6662 (1.98); 0.6608 (3.51); 0.6538 (1.54); 0.6415 (0.47); 0.0079 (0.88); -0.0002 (22.21); -0.0083 (0.95) |
| Beispiel Nr. 43, Lösungsmittel: DMSO-d6, NMR-Spektrometer: 399.95 MHz |
| 11.2647 (3.04); 8.8262 (0.72); 8.8121 (1.40); 8.7980 (0.71); 7.6760 (0.67); 7.6692 (0.86); 7.6648 (0.82); 7.6576 (0.91); 7.6541 (0.85); 7.6470 (0.98); 7.6428 (0.83); 7.6358 (0.79); 7.5888 (1.39); 7.5822 (1.26); 7.5722 (1.43); 7.5656 (1.18); 7.2773 (1.55); 7.2538 (2.35); 7.2309 (1.39); 4.3519 (3.45); 4.3376 (3.43); 4.1185 (15.43); 4.1083 (0.52); 4.0946 (0.32); 4.0091 (14.24); 3.9040 (16.00); 3.3308 (164.76); 3.1741 (1.24); 3.1609 (1.20); 2.6797 (0.38); 2.6755 (0.74); 2.6710 (0.99); 2.6664 (0.74); 2.5107 (64.47); 2.5064 (125.46); 2.5019 (161.75); 2.4973 (118.11); 2.4929 (58.91); 2.3332 (0.72); 2.3286 (0.97); 2.3241 (0.70); 0.0079 (1.72); -0.0002 (44.49); -0.0085 (1.82) |
| Beispiel Nr. 45, Lösungsmittel: DMSO-d6, NMR-Spektrometer: 399.95 MHz |
| 11,1641 (2,28); 8,3737 (1,04); 7,5804 (1,16); 7,5613 (1,34); 7,5178 (2,32); 7,3724 (1,22); 7,3532 (2,16); 7,3335 (1,23); 7,1016 (1,5); 7,0826 (1,26); 4,2604 (3,44); 4,2454 (3,36); 4,0054 (11,12); 3,5339 (0,3); 3,5154 (0,35); 3,4683 (0,46); 3,4526 (0,57); 3,4263 (0,72); 3,3109 (707,599976); 3,2248 (0,6); 3,1769 (0,32); 2,6694 (0,83); 2,5392 (2,05); 2,5047 (89,040001); 2,5006 (105,18); 2,3316 (0,66); 2,3274 (0,8); 2,2155 (0,34); 2,0691 (0,73); 1,9079 (2,6); 1,8902 (1,05); 1,8753 (15); 1,2387 (0,43); 1,1777 (0,32); -0,0001 (3,49) |
| Beispiel Nr. 46, Lösungsmittel: DMSO-d6, NMR-Spektrometer: 399.95 MHz |
| 11.2829 (3.47); 9.4212 (0.77); 9.4069 (1.47); 9.3927 (0.75); 7.6875 (0.75); 7.6808 (0.97); 7.6765 (0.93); 7.6691 (1.04); 7.6659 (0.97); 7.6585 (1.11); 7.6549 (0.96); 7.6475 (0.92); 7.6004 (1.56); 7.5938 (1.44); 7.5838 (1.65); 7.5773 (1.36); 7.2904 (1.68); 7.2671 (2.67); 7.2440 (1.53); 6.4127 (1.72); 6.2788 (3.88); 6.1450 (1.90); 4.4029 (3.78); 4.3884 (3.77); 4.0095 (16.00); 3.9040 (12.44); 3.3332 (210.19); 3.1741 (0.68); 3.1612 (0.66); 2.6755 (0.64); 2.6712 (0.86); 2.6667 (0.63); 2.5415 (0.57); 2.5106 (58.53); 2.5066 (111.17); 2.5021 (141.78); 2.4977 (103.95); 2.3332 (0.66); 2.3288 (0.87); 2.3245 (0.65); 0.0078 (1.57); -0.0002 (38.61); -0.0083 (1.82) |
| Beispiel Nr. 47, Lösungsmittel: DMSO-d6, NMR-Spektrometer: 399.95 MHz |
| 8.4056 (0.73); 8.3915 (2.02); 8.3776 (2.09); 8.3638 (0.85); 7.3696 (0.90); 7.3547 (0.38); 7.3477 (0.72); 7.2396 (1.17); 7.2324 (2.36); 7.2237 (2.81); 7.2154 (2.66); 7.2103 (3.23); 7.2025 (3.62); 7.1870 (2.17); 7.1790 (2.18); 7.1640 (0.95); 7.1525 (1.20); 7.1443 (1.83); 7.1328 (1.69); 7.1251 (1.41); 7.1154 (0.73); 7.1114 (0.70); 7.1043 (0.52); |
| 6.7007 (0.34); 6.6837 (1.38); 6.6665 (1.42); 6.6542 (0.34); 6.6454 (1.56); 6.6282 (1.55); 6.6111 (0.42); 5.9403 (1.72); 5.9361 (1.90); 5.9204 (1.00); 5.9125 (0.59); 5.9022 (1.87); 5.8976 (1.76); 5.8950 (2.13); 5.8876 (0.39); 5.8775 (1.61); 5.8696 (0.66); 5.8601 (0.60); 5.8521 (1.14); 5.8347 (0.54); 5.1402 (0.61); 5.1360 (1.59); 5.1315 (1.90); 5.1276 (0.86); 5.0961 (2.14); 5.0930 (2.65); 5.0885 (2.51); 5.0847 (1.23); 5.0741 (0.82); 5.0708 (1.70); 5.0661 (1.43); 5.0627 (0.79); 4.4104 (0.34); 4.3967 (0.35); 4.3442 (0.38); 4.2521 (2.75); 4.2377 (2.92); 4.1991 (3.38); 4.1846 (3.34); 4.0575 (3.87); 3.9982 (0.54); 3.9804 (1.81); 3.9627 (2.40); 3.9462 (3.17); 3.9304 (16.00); 3.9204 (14.69); 3.8342 (0.47); 3.8274 (0.49); 3.8160 (1.47); 3.8098 (1.41); 3.7981 (1.77); 3.7919 (1.67); 3.7816 (1.44); 3.7755 (1.38); 3.7639 (1.16); 3.7577 (1.07); 3.7465 (0.46); 3.7398 (0.46); 3.5965 (0.39); 3.5907 (0.38); 3.5786 (0.34); 3.5044 (0.62); 3.4864 (0.70); 3.4685 (0.71); 3.4504 (0.74); 3.4135 (1.16); 3.3477 (1401.49); 3.2674 (0.68); 3.2403 (0.40); 2.9687 (0.65); 2.9512 (0.79); 2.9419 (3.48); 2.9248 (3.39); 2.6873 (0.47); 2.6828 (0.97); 2.6782 (1.39); 2.6736 (1.05); 2.6691 (0.56); 2.5484 (0.96); 2.5316 (2.76); 2.5268 (4.11); 2.5182 (67.33); 2.5137 (143.71); 2.5091 (196.75); 2.5045 (146.40); 2.5000 (73.87); 2.3450 (0.39); 2.3404 (0.91); 2.3358 (1.29); 2.3313 (0.96); 2.3267 (0.49); 2.0799 (1.40); 1.9951 (0.48); 1.8126 (5.87); 1.8086 (6.02); 1.7954 (5.92); 1.7914 (5.98); 1.2417 (1.17); 1.1811 (0.42); 1.1633 (6.27); 1.1457 (13.04); 1.1441 (12.91); 1.1276 (6.08); 0.9480 (1.07); 0.9303 (2.37); 0.9126 (1.05) |
| Beispiel Nr. 48, Lösungsmittel: DMSO-d6, NMR-Spektrometer: 399.95 MHz |
| 8.3197 (0.85); 8.3050 (1.78); 8.2899 (0.87); 7.3278 (1.28); 7.3209 (1.50); 7.3117 (1.43); 7.3048 (1.52); 7.2996 (1.54); 7.2770 (2.63); 7.2539 (2.01); 7.1991 (0.93); 7.1885 (1.19); 7.1808 (1.04); 7.1670 (0.75); 7.1595 (0.52); 5.2018 (3.27); 5.1576 (4.29); 4.8966 (4.21); 4.8524 (3.34); 4.2064 (2.33); 4.1957 (2.40); 4.0430 (0.56); 3.9887 (16.00); 3.3421 (123.62); 3.3183 (0.66); 2.6724 (0.33); 2.5258 (0.62); 2.5211 (0.95); 2.5124 (17.04); 2.5079 (37.35); 2.5034 (50.72); 2.4988 (36.23); 2.4942 (16.96); 2.3301 (0.33); 2.0427 (0.35); 2.0293 (5.63); 2.0117 (6.03); 0.9921 (0.37); 0.9819 (0.71); 0.9801 (0.74); 0.9742 (0.69); 0.9701 (0.56); 0.9676 (0.52); 0.9621 (1.28); 0.9542 (0.56); 0.9499 (0.72); 0.9441 (0.70); 0.9422 (0.78); 0.9300 (0.41); 0.4564 (1.31); 0.4477 (2.49); 0.4449 (2.51); 0.4427 (2.85); 0.4362 (1.72); 0.4330 (1.85); 0.4267 (2.52); 0.4224 (3.10); 0.4129 (1.42); 0.1377 (1.12); 0.1278 (3.38); 0.1243 (3.91); 0.1156 (3.52); 0.1121 (3.45); 0.1019 (0.98); 0.0080 (0.34); -0.0002 (12.53); -0.0085 (0.42) |
| Beispiel Nr. 49, Lösungsmittel: DMSO-d6, NMR-Spektrometer: 399.95 MHz |
| 8.7588 (0.77); 8.7442 (1.47); 8.7295 (0.76); 7.4160 (0.55); 7.4005 (0.36); 7.3692 (0.43); 7.3457 (0.50); 7.2641 (1.32); 7.2576 (1.51); 7.2478 (1.41); 7.2414 (1.43); 7.2281 (1.14); 7.2058 (2.67); 7.1826 (2.16); 7.1634 (1.08); 7.1565 (1.24); 7.1524 (1.35); 7.1451 (1.15); 7.1353 (0.68); 7.1237 (0.44); 4.3889 (0.72); 4.3751 (0.71); 4.2409 (3.92); 4.2262 (3.90); 4.1162 (3.45); 4.1062 (16.00); 4.0571 (3.09); 3.9438 (15.87); 3.9042 (10.63); 3.4344 (0.86); 3.4120 (21.13); 3.3946 (0.58); 3.3353 (204.13); 3.1996 (3.99); 3.1735 (0.45); 3.1621 (0.42); 2.6759 (0.61); 2.6716 (0.84); 2.6670 (0.61); 2.5418 (0.79); 2.5112 (56.08); 2.5070 (106.72); 2.5026 (136.37); 2.4981 (99.68); 2.4938 (50.67); 2.3336 (0.61); 2.3292 (0.81); 2.3248 (0.60); 1.2350 (1.15); 0.0078 (1.44); -0.0002 (36.39); -0.0084 (1.59) |
| Beispiel Nr. 50, Lösungsmittel: DMSO-d6, NMR-Spektrometer: 399.95 MHz |
| 11.2264 (3.53); 8.3994 (0.91); 8.3846 (1.81); 8.3699 (0.89); 7.6297 (1.35); 7.6235 (1.65); 7.6131 (1.40); 7.6065 (1.57); 7.5810 (0.89); 7.5737 (0.86); 7.5698 (1.05); 7.5592 |
| (1.11); 7.5519 (1.02); 7.5479 (1.06); 7.5412 (0.76); 7.2503 (1.71); 7.2269 (2.58); 7.2040 (1.52); 4.3011 (3.82); 4.2867 (3.77); 4.0008 (16.00); 3.9040 (12.85); 3.3310 (158.11); 3.1738 (0.64); 3.1613 (0.60); 2.6755 (0.65); 2.6711 (0.88); 2.6667 (0.65); 2.5107 (58.73); 2.5065 (111.01); 2.5020 (141.34); 2.4975 (103.38); 2.4934 (52.29); 2.3333 (0.66); 2.3287 (0.87); 2.3243 (0.65); 2.0128 (6.57); 1.9964 (1.01); 1.9799 (0.68); 0.8980 (0.40); 0.8791 (14.85); 0.8632 (14.59); 0.0079 (1.60); -0.0002 (36.51); - 0.0084 (1.64) |
| Beispiel Nr. 51, Lösungsmittel: DMSO-d6, NMR-Spektrometer: 399.95 MHz |
| 11.2677 (3.56); 8.7278 (0.81); 8.7134 (1.71); 8.6991 (0.87); 7.9533 (1.07); 7.6255 (0.94); 7.6194 (2.02); 7.6157 (1.84); 7.6033 (3.38); 7.5937 (1.21); 7.5863 (0.97); 7.5824 (1.18); 7.5756 (0.71); 7.2759 (1.56); 7.2531 (2.15); 7.2302 (1.21); 4.3333 (3.75); 4.3190 (3.77); 4.0094 (16.00); 3.3661 (84.84); 3.3630 (111.34); 2.9084 (2.31); 2.8916 (8.12); 2.8708 (4.76); 2.8332 (2.42); 2.7317 (6.65); 2.5270 (0.34); 2.5135 (9.43); 2.5093 (19.90); 2.5049 (26.76); 2.5004 (19.67); 1.7568 (3.99); 1.7086 (8.46); 1.6603 (4.26); 1.3553 (1.08); 1.2350 (0.32); 1.1911 (0.43); 1.1729 (0.83); 1.1547 (0.41); -0.0002 (3.11) |
| Beispiel Nr. 52, Lösungsmittel: DMSO-d6, NMR-Spektrometer: 399.95 MHz |
| 11.2273 (2.51); 8.3879 (0.91); 8.3735 (1.68); 8.3588 (0.83); 7.5988 (2.22); 7.5893 (1.74); 7.5832 (2.25); 7.5762 (2.14); 7.2507 (1.48); 7.2418 (0.36); 7.2362 (0.37); 7.2265 (2.75); 7.2132 (0.38); 7.2026 (1.35); 6.8704 (0.69); 6.6413 (0.48); 4.3009 (3.51); 4.2868 (3.48); 4.0202 (0.34); 4.0027 (16.00); 3.3473 (134.96); 3.3428 (227.33); 3.3063 (0.34); 3.3010 (0.34); 2.6720 (0.41); 2.5252 (0.68); 2.5071 (41.05); 2.5030 (56.17); 2.3296 (0.38); 2.1905 (0.53); 2.1835 (1.21); 2.1729 (1.00); 2.1545 (0.56); 2.1383 (3.29); 2.1202 (6.07); 2.1016 (3.73); 2.0733 (0.47); 1.9888 (1.03); 1.5931 (0.50); 1.5749 (2.14); 1.5563 (3.88); 1.5378 (3.93); 1.5195 (2.21); 1.5013 (0.58); 1.4979 (0.79); 1.4797 (0.43); 1.3555 (9.17); 1.3087 (0.42); 1.2357 (0.58); 1.1929 (0.44); 1.1804 (0.34); 1.1750 (0.67); 0.9285 (0.38); 0.9099 (0.77); 0.8937 (1.14); 0.8788 (7.06); 0.8755 (2.90); 0.8703 (1.19); 0.8604 (14.64); 0.8517 (1.02); 0.8418 (6.35); 0.8288 (0.68); 0.8102 (0.34); 0.7449 (0.34); -0.0002 (10.35) |
| Beispiel Nr. 53, Lösungsmittel: DMSO-d6, NMR-Spektrometer: 399.95 MHz |
| 11.1999 (2.16); 7.7638 (0.44); 7.7487 (0.83); 7.7335 (0.45); 7.5865 (0.78); 7.5663 (0.99); 7.5305 (1.67); 7.3767 (1.03); 7.3571 (1.93); 7.3374 (1.03); 7.1020 (1.10); 7.0831 (0.97); 4.1983 (2.44); 4.1829 (2.47); 4.0096 (10.00); 3.9041 (3.57); 3.5503 (16.00); 3.3316 (54.56); 3.1743 (0.36); 3.1613 (0.34); 2.6711 (0.36); 2.5414 (0.36); 2.5107 (24.22); 2.5065 (46.30); 2.5020 (59.22); 2.4975 (43.33); 2.4933 (21.93); 2.3288 (0.36); 0.0079 (0.67); -0.0002 (16.50); -0.0084 (0.71) |
| Beispiel Nr. 54, Lösungsmittel: DMSO-d6, NMR-Spektrometer: 399.95 MHz |
| 11,31 (2,64); 8,4362 (0,79); 8,4216 (1,43); 8,4065 (0,75); 7,7136 (1,25); 7,7076 (1,28); 7,692 (1,49); 7,686 (1,49); 7,5551 (2,58); 7,5493 (2,3); 7,4893 (2,78); 7,4677 (2,28); 4,3105 (3,79); 4,296 (3,65); 4,0378 (0,33); 4,0066 (12,18); 3,3025 (176,449997); 3,2792 (3,92); 2,673 (0,38); 2,6691 (0,45); 2,5037 (44,560001); 2,5 (50,560001); 2,3268 (0,32); 1,9559 (0,38); 1,913 (15); 1,3985 (1,64); 1,237 (0,39); -0,0001 (1,6); -0,0619 (0,8) |
| Beispiel Nr. 55, Lösungsmittel: DMSO-d6, NMR-Spektrometer: 399.95 MHz |
| 11.2438 (3.00); 7.7225 (0.60); 7.7073 (1.16); 7.6935 (0.61); 7.6482 (0.68); 7.6368 (0.78); 7.6294 (0.83); 7.6187 (0.72); 7.5878 (0.99); 7.5820 (0.91); 7.5710 (0.99); 7.2464 (1.79); 7.2226 (2.49); 7.1999 (1.64); 5.7549 (0.60); 4.2349 (2.53); 4.2202 (2.64); 4.0608 (0.33); 4.0563 (0.54); 4.0383 (1.88); 4.0177 (4.32); 4.0078 (16.00); 4.0002 (4.59); 3.9823 (1.39); 3.3284 (247.58); 3.3042 (0.35); 2.5244 (0.47); 2.5199 (0.70); 2.5105 (15.51); 2.5064 (30.07); 2.5020 (41.66); 2.4978 (28.49); 2.4935 (13.82); 2.0736 (0.47); 1.9887 (6.41); 1.3979 (9.37); 1.1928 (1.92); 1.1837 (2.79); 1.1750 (4.12); 1.1660 (5.60); 1.1572 (2.60); 1.1483 (2.87); -0.0002 (4.60) |
| Beispiel Nr. 56, Lösungsmittel: DMSO-d6, NMR-Spektrometer: 399.95 MHz |
| 11.1834 (3.36); 8.2417 (0.83); 8.2269 (1.62); 8.2122 (0.82); 7.5407 (1.78); 7.5252 (4.78); 7.3683 (1.62); 7.3608 (0.37); 7.3488 (2.19); 7.3343 (0.48); 7.3275 (1.48); 7.0786 (1.98); 7.0593 (1.77); 4.2618 (4.41); 4.2468 (4.39); 4.0052 (16.00); 3.9039 (13.28); 3.3828 (0.87); 3.3371 (256.86); 3.1738 (1.48); 3.1614 (1.40); 3.0781 (1.22); 3.0570 (1.91); 3.0360 (1.31); 3.0160 (0.34); 2.6757 (0.55); 2.6713 (0.75); 2.6669 (0.57); 2.5416 (0.78); 2.5110 (50.05); 2.5068 (96.07); 2.5023 (123.59); 2.4977 (90.65); 2.4934 (46.06); 2.3332 (0.56); 2.3290 (0.77); 2.3245 (0.58); 2.2087 (0.44); 2.1858 (1.39); 2.1802 (1.16); 2.1631 (1.86); 2.1568 (2.36); 2.1404 (1.47); 2.1352 (2.10); 2.1187 (0.50); 2.1131 (0.69); 2.0727 (0.54); 2.0658 (0.73); 2.0512 (1.35); 2.0431 (1.84); 2.0362 (1.17); 2.0301 (1.25); 2.0210 (1.75); 2.0068 (0.57); 1.9978 (0.59); 1.9925 (0.52); 1.9436 (0.34); 1.9213 (0.64); 1.8986 (1.11); 1.8733 (1.45); 1.8521 (0.77); 1.7971 (0.47); 1.7829 (0.55); 1.7731 (0.98); 1.7633 (0.61); 1.7598 (0.57); 1.7494 (0.74); 1.7405 (0.39); 1.2359 (0.37); 0.0079 (1.20); -0.0002 (28.72); -0.0085 (1.18) |
| Beispiel Nr. 57, Lösungsmittel: DMSO-d6, NMR-Spektrometer: 399.95 MHz |
| 11.2140 (3.38); 8.8181 (0.75); 8.8036 (1.41); 8.7897 (0.73); 7.5763 (3.24); 7.5634 (1.72); 7.5430 (1.84); 7.3993 (1.87); 7.3798 (3.19); 7.3602 (1.57); 7.1163 (1.99); 7.0973 (1.71); 4.3283 (4.49); 4.3136 (4.42); 4.0100 (16.00); 3.9041 (12.57); 3.3632 (2.68); 3.3353 (190.88); 3.3069 (5.27); 3.2786 (1.74); 3.1744 (0.82); 3.1614 (0.82); 2.6758 (0.63); 2.6712 (0.86); 2.6669 (0.63); 2.6628 (0.33); 2.5243 (3.41); 2.5110 (55.90); 2.5068 (106.51); 2.5023 (135.47); 2.4978 (98.61); 2.4936 (49.35); 2.3335 (0.60); 2.3290 (0.80); 2.3246 (0.59); 1.2317 (0.42); 0.0078 (1.41); -0.0002 (35.34); -0.0085 (1.52) |
| Beispiel Nr. 58, Lösungsmittel: DMSO-d6, NMR-Spektrometer: 399.95 MHz |
| 11.2064 (3.44); 8.8349 (0.81); 8.8201 (1.57); 8.8060 (0.82); 7.5733 (3.25); 7.5488 (1.57); 7.5285 (1.83); 7.3915 (1.70); 7.3719 (3.14); 7.3522 (1.58); 7.1122 (2.05); 7.0929 (1.77); 4.5762 (0.83); 4.5593 (2.74); 4.5424 (2.78); 4.5256 (0.86); 4.3208 (3.95); 4.3058 (3.93); 4.0080 (16.00); 3.9040 (12.15); 3.3308 (131.92); 3.1735 (0.67); 3.1609 (0.64); 2.6753 (0.67); 2.6710 (0.89); 2.6664 (0.66); 2.5061 (114.60); 2.5018 (145.64); 2.4975 (108.12); 2.3326 (0.69); 2.3284 (0.89); 2.3244 (0.66); 1.5730 (10.62); 1.5561 (10.51); 0.0069 (1.54); -0.0002 (33.47); -0.0083 (1.64) |
| Beispiel Nr. 59, Lösungsmittel: DMSO-d6, NMR-Spektrometer: 399.95 MHz |
| 11.1347 (2.55); 8.4733 (0.61); 8.4614 (1.16); 8.4488 (0.62); 7.8676 (0.44); 7.8526 (0.57); 7.8454 (0.92); 7.8307 (0.92); 7.8236 (0.58); 7.8088 (0.46); 7.2110 (0.70); 7.1885 (1.34); 7.1673 (0.65); 4.3735 (2.50); 4.3605 (2.49); 4.0311 (10.94); 3.9040 (6.42); 3.3354 (155.27); 3.1742 (0.48); 3.1610 (0.45); 3.0595 (8.94); 2.6715 (0.60); 2.5063 |
| (81.46); 2.5023 (98.72); 2.3290 (0.62); 2.0536 (16.00); -0.0002 (20.50) |
| Beispiel Nr. 60, Lösungsmittel: DMSO-d6, NMR-Spektrometer: 399.95 MHz |
| 11,3223 (3,42); 8,6312 (1); 8,617 (1,89); 8,6025 (0,99); 7,6965 (1,52); 7,6904 (1,61); 7,6751 (1,83); 7,669 (1,93); 7,5775 (3,23); 7,5716 (2,84); 7,4922 (3,57); 7,4705 (2,87); 4,3496 (4,53); 4,335 (4,42); 4,0383 (0,39); 4,0063 (15); 3,986 (0,94); 3,8432 (3,23); 3,8361 (0,99); 3,7788 (0,79); 3,7497 (0,42); 3,7389 (0,68); 3,7259 (0,6); 3,6404 (0,63); 3,6281 (0,66); 3,6163 (0,41); 3,3026 (186,630005); 2,6687 (0,43); 2,5392 (2,1); 2,5039 (43,049999); 2,5001 (50,330002); 2,0695 (0,69); 1,9868 (0,49); 1,6944 (0,41); 1,6833 (0,85); 1,6759 (0,96); 1,6643 (1,54); 1,6523 (0,99); 1,6447 (0,89); 1,6327 (0,43); 1,242 (0,34); 1,1753 (0,31); 0,7423 (0,71); 0,7294 (2,27); 0,7225 (4,31); 0,7181 (3,71); 0,7109 (3,71); 0,7045 (3,34); 0,6988 (3,95); 0,6922 (1,95); 0,679 (3,26); 0,6725 (1,54); 0,6598 (0,5); -0,0001 (11,06) |
| Beispiel Nr. 61, Lösungsmittel: DMSO-d6, NMR-Spektrometer: 399.95 MHz |
| 11.2563 (3.44); 8.8528 (0.86); 8.8383 (1.71); 8.8239 (0.84); 7.6265 (1.21); 7.6202 (1.74); 7.6098 (1.30); 7.6030 (2.53); 7.5956 (0.88); 7.5909 (1.19); 7.5804 (1.11); 7.5732 (0.98); 7.5691 (1.09); 7.5624 (0.72); 7.2769 (1.71); 7.2534 (2.46); 7.2307 (1.47); 4.5761 (0.89); 4.5592 (2.99); 4.5423 (3.04); 4.5254 (0.92); 4.3499 (3.81); 4.3355 (3.78); 4.0051 (15.93); 3.9039 (16.00); 3.4159 (0.38); 3.3411 (416.65); 3.1742 (0.70); 3.1615 (0.70); 2.6762 (0.72); 2.6717 (0.96); 2.6672 (0.71); 2.5417 (0.84); 2.5114 (61.48); 2.5071 (118.89); 2.5026 (153.15); 2.4981 (111.94); 2.4937 (56.21); 2.3339 (0.68); 2.3293 (0.94); 2.3247 (0.69); 1.5711 (11.09); 1.5542 (10.98); 0.0079 (1.69); -0.0002 (41.26); -0.0085 (1.75) |
| Beispiel Nr. 62, Lösungsmittel: DMSO-d6, NMR-Spektrometer: 399.95 MHz |
| 11.2232 (3.20); 8.3869 (0.86); 8.3720 (1.79); 8.3575 (0.86); 7.5971 (3.28); 7.5829 (2.58); 7.5672 (1.24); 7.5607 (0.54); 7.2500 (1.13); 7.2264 (2.38); 7.2178 (0.44); 7.2021 (1.24); 4.2952 (3.59); 4.2807 (3.61); 4.0384 (0.44); 4.0202 (0.63); 4.0048 (16.00); 3.3361 (236.47); 3.3128 (1.08); 3.0977 (0.45); 2.6715 (0.41); 2.6669 (0.32); 2.5249 (0.81); 2.5069 (50.72); 2.5027 (68.01); 2.4985 (46.90); 2.3294 (0.41); 2.1587 (2.99); 2.1403 (5.29); 2.1215 (3.31); 2.0733 (0.64); 1.9887 (1.74); 1.5448 (0.72); 1.5269 (1.85); 1.5080 (2.89); 1.4900 (2.05); 1.4704 (0.97); 1.3979 (9.39); 1.3148 (0.46); 1.2967 (1.69); 1.2777 (2.66); 1.2590 (2.68); 1.2407 (1.70); 1.2227 (0.51); 1.1928 (0.50); 1.1750 (0.99); 1.1572 (0.50); 0.8818 (0.39); 0.8714 (6.27); 0.8636 (0.92); 0.8531 (12.75); 0.8346 (5.48); -0.0002 (4.71) |
| Beispiel Nr. 63, Lösungsmittel: DMSO-d6, NMR-Spektrometer: 600.13 MHz |
| 7.4004 (0.39); 7.3900 (0.68); 7.3800 (0.36); 7.3220 (0.33); 7.2905 (0.62); 7.2860 (0.70); 7.2798 (0.70); 7.2754 (0.66); 7.1733 (0.59); 7.1584 (1.24); 7.1430 (0.96); 7.1126 (0.36); 7.1066 (0.49); 7.0997 (0.58); 7.0942 (0.51); 7.0866 (0.39); 5.7650 (0.55); 4.0778 (1.13); 4.0690 (1.34); 4.0561 (1.92); 4.0480 (0.89); 4.0361 (2.37); 4.0242 (2.37); 4.0124 (0.80); 3.9596 (6.74); 3.4412 (1.94); 3.4247 (0.72); 3.4111 (7.68); 3.3535 (6.04); 3.2135 (0.33); 3.1926 (1.46); 2.5122 (4.48); 2.5092 (9.94); 2.5062 (13.84); 2.5032 (10.07); 2.5003 (4.66); 1.9917 (10.21); 1.4102 (0.47); 1.3970 (4.10); 1.3894 (16.00); 1.3734 (1.27); 1.3677 (2.45); 1.2959 (0.50); 1.1877 (2.70); 1.1758 (5.45); 1.1640 (2.66); -0.0001 (1.18) |
| Beispiel Nr. 64, Lösungsmittel: DMSO-d6, NMR-Spektrometer: 399.95 MHz |
| 11.3447 (2.55); 8.4848 (0.65); 8.4702 (1.31); 8.4553 (0.64); 7.6485 (5.91); 7.6454 (6.40); 7.5257 (2.55); 4.2692 (3.30); 4.2545 (3.27); 4.0086 (11.38); 3.9040 (9.54); 3.3305 (102.54); 3.1740 (1.07); 3.1610 (1.05); 2.6755 (0.53); 2.6709 (0.71); 2.6665 (0.52); 2.5107 (44.71); 2.5064 (85.43); 2.5019 (109.31); 2.4974 (79.43); 2.4930 (39.52); 2.3331 (0.48); 2.3286 (0.66); 2.3242 (0.47); 1.9219 (16.00); 1.2352 (0.33); 0.0080 (1.14); -0.0002 (29.31); -0.0084 (1.28) |
| Beispiel Nr. 65, Lösungsmittel: DMSO-d6, NMR-Spektrometer: 399.95 MHz |
| 11.1922 (3.36); 8.3347 (0.83); 8.3202 (1.66); 8.3054 (0.83); 7.5975 (1.42); 7.5767 (1.72); 7.5037 (3.15); 7.3727 (1.71); 7.3532 (3.18); 7.3334 (1.70); 7.0989 (1.98); 7.0798 (1.74); 5.7121 (3.13); 5.7092 (2.48); 4.2990 (4.34); 4.2839 (4.29); 4.1247 (0.42); 4.1113 (0.44); 4.0033 (16.00); 3.9035 (13.77); 3.5081 (0.34); 3.4600 (0.56); 3.4306 (1.10); 3.3615 (712.08); 3.2834 (0.48); 3.2682 (0.35); 3.1747 (2.19); 3.1620 (2.04); 2.6774 (0.66); 2.6730 (0.90); 2.6684 (0.66); 2.5429 (0.75); 2.5124 (59.85); 2.5083 (115.70); 2.5038 (148.72); 2.4993 (109.28); 2.4950 (55.40); 2.3349 (0.68); 2.3305 (0.90); 2.3261 (0.67); 2.0908 (13.31); 2.0887 (13.45); 1.7905 (12.46); 1.7886 (12.54); 0.0079 (1.44); -0.0002 (36.61); -0.0084 (1.60) |
| Beispiel Nr. 66, Lösungsmittel: DMSO-d6, NMR-Spektrometer: 399.95 MHz |
| 11.2542 (3.14); 11.2426 (0.83); 8.5693 (0.88); 8.5550 (1.78); 8.5404 (0.89); 7.6419 (0.61); 7.6347 (0.84); 7.6309 (0.80); 7.6232 (0.91); 7.6124 (1.13); 7.6073 (1.25); 7.6023 (1.13); 7.5921 (0.73); 7.5825 (1.57); 7.5760 (1.40); 7.5659 (1.49); 7.5594 (1.10); 7.2572 (1.51); 7.2336 (2.47); 7.2109 (1.36); 4.3409 (0.38); 4.3091 (1.94); 4.3009 (2.28); 4.2876 (1.79); 4.0098 (14.07); 4.0002 (3.48); 3.9041 (16.00); 3.3332 (235.15); 3.1741 (1.02); 3.1611 (1.02); 2.6757 (0.73); 2.6712 (1.00); 2.6667 (0.75); 2.5414 (0.61); 2.5065 (127.66); 2.5021 (164.78); 2.4977 (122.50); 2.3332 (0.76); 2.3288 (1.03); 2.3244 (0.77); 1.3793 (0.62); 1.3685 (1.26); 1.3587 (1.39); 1.3487 (1.32); 1.3382 (0.79); 1.1551 (0.38); 1.1401 (0.69); 1.1300 (0.75); 1.1246 (0.85); 1.1186 (0.92); 1.1090 (0.79); 1.1040 (0.70); 1.0943 (0.54); 1.0662 (2.13); 1.0430 (9.23); 1.0282 (5.83); 0.9102 (0.76); 0.8999 (1.14); 0.8896 (1.42); 0.8789 (1.04); 0.8692 (0.75); 0.8276 (0.40); 0.8177 (0.39); 0.5333 (0.72); 0.5245 (0.80); 0.5187 (0.86); 0.5128 (1.11); 0.5046 (0.83); 0.4989 (0.80); 0.4900 (0.63); 0.0079 (1.71); -0.0002 (46.07); -0.0083 (1.92) |
| Beispiel Nr. 66, Lösungsmittel: DMSO-d6, NMR-Spektrometer: 399.95 MHz |
| 11.2542 (3.14); 11.2426 (0.83); 8.5693 (0.88); 8.5550 (1.78); 8.5404 (0.89); 7.6419 (0.61); 7.6347 (0.84); 7.6309 (0.80); 7.6232 (0.91); 7.6124 (1.13); 7.6073 (1.25); 7.6023 (1.13); 7.5921 (0.73); 7.5825 (1.57); 7.5760 (1.40); 7.5659 (1.49); 7.5594 (1.10); 7.2572 (1.51); 7.2336 (2.47); 7.2109 (1.36); 4.3409 (0.38); 4.3091 (1.94); 4.3009 (2.28); 4.2876 (1.79); 4.0098 (14.07); 4.0002 (3.48); 3.9041 (16.00); 3.3332 (235.15); 3.1741 (1.02); 3.1611 (1.02); 2.6757 (0.73); 2.6712 (1.00); 2.6667 (0.75); 2.5414 (0.61); 2.5065 (127.66); 2.5021 (164.78); 2.4977 (122.50); 2.3332 (0.76); 2.3288 (1.03); 2.3244 (0.77); 1.3793 (0.62); 1.3685 (1.26); 1.3587 (1.39); 1.3487 (1.32); 1.3382 (0.79); 1.1551 (0.38); 1.1401 (0.69); 1.1300 (0.75); 1.1246 (0.85); 1.1186 (0.92); 1.1090 (0.79); 1.1040 (0.70); 1.0943 (0.54); 1.0662 (2.13); 1.0430 (9.23); 1.0282 (5.83); 0.9102 (0.76); 0.8999 (1.14); 0.8896 (1.42); 0.8789 (1.04); 0.8692 (0.75); 0.8276 (0.40); 0.8177 (0.39); 0.5333 (0.72); 0.5245 (0.80); 0.5187 (0.86); 0.5128 (1.11); 0.5046 (0.83); |
| 0.4989 (0.80); 0.4900 (0.63); 0.0079 (1.71); -0.0002 (46.07); -0.0083 (1.92) |
| Beispiel Nr. 67, Lösungsmittel: DMSO-d6, NMR-Spektrometer: 399.95 MHz |
| 11.1834 (3.33); 8.3880 (0.80); 8.3728 (1.59); 8.3578 (0.80); 7.5647 (3.18); 7.5297 (1.47); 7.5097 (1.75); 7.3710 (1.74); 7.3515 (3.15); 7.3319 (1.63); 7.0993 (1.99); 7.0802 (1.72); 4.2754 (4.32); 4.2605 (4.28); 4.0033 (16.00); 3.9035 (12.93); 3.5087 (0.38); 3.4617 (0.45); 3.4456 (0.65); 3.3597 (646.56); 3.1748 (1.36); 3.1623 (1.28); 2.6773 (0.64); 2.6729 (0.86); 2.6684 (0.61); 2.5429 (0.66); 2.5258 (2.95); 2.5124 (58.76); 2.5083 (111.80); 2.5039 (141.58); 2.4994 (103.19); 2.4952 (52.21); 2.3350 (0.64); 2.3305 (0.86); 2.3263 (0.64); 2.0183 (5.21); 2.0125 (6.83); 2.0004 (1.23); 1.9877 (0.70); 1.2358 (0.41); 0.9072 (0.44); 0.8872 (14.01); 0.8712 (13.75); 0.8514 (0.55); 0.0078 (1.27); - 0.0002 (32.19); -0.0083 (1.42) |
| Beispiel Nr. 68, Lösungsmittel: DMSO-d6, NMR-Spektrometer: 399.95 MHz |
| 11.1011 (3.40); 8.2401 (0.83); 8.2260 (1.63); 8.2121 (0.82); 7.5205 (1.45); 7.5151 (1.64); 7.5003 (1.61); 7.4948 (1.93); 7.4505 (3.24); 7.4455 (2.76); 7.1962 (2.72); 7.1757 (2.39); 4.2292 (4.23); 4.2149 (4.21); 4.1083 (0.42); 4.0952 (0.46); 3.9924 (16.00); 3.9040 (12.65); 3.3943 (0.59); 3.3871 (0.44); 3.3807 (0.63); 3.3318 (191.66); 3.1741 (1.84); 3.1610 (1.78); 2.6753 (0.71); 2.6709 (0.95); 2.6663 (0.71); 2.5411 (0.80); 2.5104 (61.05); 2.5063 (116.82); 2.5018 (150.30); 2.4973 (110.02); 2.4930 (55.44); 2.3328 (0.75); 2.3285 (1.01); 2.3241 (0.77); 2.2691 (0.36); 2.2418 (14.25); 2.1861 (1.73); 2.1672 (5.38); 2.1481 (5.56); 2.1291 (1.85); 1.3505 (0.79); 1.3354 (0.33); 1.2579 (0.35); 1.2489 (0.44); 1.2291 (0.97); 1.0600 (6.16); 1.0411 (12.72); 1.0220 (5.82); 0.0078 (1.62); -0.0002 (39.85); -0.0084 (1.73) |
| Beispiel Nr. 69, Lösungsmittel: DMSO-d6, NMR-Spektrometer: 601.6 MHz |
| 11.4238 (2.73); 8.6402 (0.43); 8.6306 (0.82); 8.6208 (0.43); 8.5176 (0.76); 8.5077 (1.47); 8.4972 (0.79); 8.4848 (0.45); 8.4752 (0.84); 8.4650 (0.44); 7.5590 (1.23); 7.5447 (0.85); 7.5413 (1.29); 7.5127 (0.76); 7.4951 (0.75); 7.4819 (0.73); 7.4644 (0.74); 7.3069 (1.35); 7.2739 (1.48); 7.2360 (2.56); 6.9579 (0.56); 6.9413 (1.51); 6.9274 (1.13); 6.6996 (0.44); 6.6882 (1.85); 6.6768 (1.82); 6.6742 (0.57); 6.6627 (1.88); 6.6513 (1.97); 6.6399 (0.48); 5.9842 (1.97); 5.9814 (2.03); 5.9614 (0.62); 5.9588 (1.94); 5.9559 (1.85); 5.9412 (0.34); 5.9296 (0.71); 5.9244 (0.38); 5.9181 (0.34); 5.9127 (1.16); 5.9011 (1.11); 5.8958 (0.37); 5.8896 (0.35); 5.8842 (0.83); 5.8727 (0.35); 5.1465 (0.46); 5.1437 (1.16); 5.1406 (1.22); 5.1380 (0.48); 5.1179 (0.39); 5.1151 (1.07); 5.1121 (1.09); 5.1094 (0.45); 5.0972 (0.51); 5.0949 (1.13); 5.0918 (1.03); 5.0896 (0.42); 5.0803 (0.52); 5.0781 (1.09); 5.0749 (0.98); 5.0727 (0.42); 4.5014 (0.40); 4.4906 (0.47); 4.4872 (0.49); 4.4816 (0.44); 4.4764 (0.56); 4.4675 (0.44); 4.3440 (3.90); 4.3340 (3.94); 4.3181 (0.96); 4.3084 (1.79); 4.2982 (0.99); 4.2752 (2.39); 4.2652 (2.33); 4.0164 (10.65); 4.0135 (11.42); 4.0103 (16.00); 3.3770 (4.18); 3.3508 (1735.78); 3.3273 (12.16); 3.3158 (0.37); 2.9774 (1.52); 2.9751 (2.69); 2.9726 (1.55); 2.9659 (1.53); 2.9635 (2.68); 2.9611 (1.48); 2.6880 (0.52); 2.6791 (0.58); 2.6634 (1.41); 2.6546 (1.33); 2.6395 (1.48); 2.6254 (1.48); 2.6209 (0.68); 2.6180 (1.39); 2.6150 (2.38); 2.6119 (1.34); 2.6088 (0.61); 2.6008 (0.63); 2.5426 (1.24); 2.5292 (1.20); 2.5242 (3.20); 2.5211 (4.38); 2.5180 (5.09); 2.5093 (97.44); 2.5062 (210.40); 2.5032 (291.22); 2.5001 (207.61); 2.4971 (93.61); 2.3934 (0.58); 2.3904 (1.26); 2.3873 (1.79); 2.3843 (1.28); 2.3813 (0.55); 2.0766 (1.55); 1.9900 (0.47); 1.8113 (6.43); 1.8085 (6.47); 1.7999 (6.37); 1.7971 (6.34); 1.6910 (0.36); 1.6689 (0.38); 1.6443 (0.38); 1.6181 (0.42); 1.5103 (6.01); 1.4994 |
| (5.98); 1.3972 (3.90); 1.2342 (1.38); 1.1743 (0.45); 1.1706 (0.37); 1.1493 (0.42); 0.9400 (0.35); 0.0052 (2.03); -0.0002 (68.83); -0.0058 (1.99) |
| Beispiel Nr. 70, Lösungsmittel: DMSO-d6, NMR-Spektrometer: 399.95 MHz |
| 11.1937 (3.45); 8.4711 (0.84); 8.4563 (1.64); 8.4416 (0.81); 7.6055 (1.42); 7.5848 (1.70); 7.5067 (3.16); 7.3753 (1.72); 7.3557 (3.20); 7.3360 (1.71); 7.1009 (1.98); 7.0816 (1.73); 6.7032 (0.37); 6.6862 (1.37); 6.6690 (1.43); 6.6480 (1.56); 6.6308 (1.53); 6.6137 (0.41); 5.9888 (1.79); 5.9846 (1.80); 5.9505 (1.62); 5.9463 (1.61); 4.3406 (4.35); 4.3255 (4.31); 4.0027 (16.00); 3.9041 (15.21); 3.3310 (133.70); 3.1738 (0.74); 3.1610 (0.70); 2.6754 (0.62); 2.6709 (0.85); 2.6665 (0.64); 2.5411 (0.73); 2.5104 (55.31); 2.5063 (106.22); 2.5018 (136.67); 2.4973 (100.12); 2.4931 (50.18); 2.3328 (0.62); 2.3285 (0.84); 2.3240 (0.61); 1.8094 (6.01); 1.8056 (6.19); 1.7922 (5.99); 1.7884 (6.08); 1.2355 (0.35); 0.0078 (1.43); -0.0002 (34.57); -0.0081 (1.40) |
| Beispiel Nr. 71, Lösungsmittel: DMSO-d6, NMR-Spektrometer: 399.95 MHz |
| 10.7342 (2.79); 8.3439 (0.65); 8.3295 (1.32); 8.3150 (0.66); 7.6516 (1.22); 7.6426 (1.27); 7.6347 (1.72); 7.6219 (0.77); 7.6112 (0.73); 7.6045 (0.46); 7.2126 (1.14); 7.1895 (1.63); 7.1666 (0.97); 4.2839 (2.84); 4.2694 (2.84); 3.9039 (6.22); 3.8886 (16.00); 3.3863 (0.44); 3.3313 (60.71); 3.1726 (1.13); 3.1626 (1.10); 2.6708 (0.42); 2.5412 (0.39); 2.5106 (28.97); 2.5064 (56.20); 2.5020 (72.53); 2.4975 (53.58); 2.3333 (0.34); 2.3287 (0.46); 2.3241 (0.36); 2.1841 (1.25); 2.1651 (4.00); 2.1461 (4.13); 2.1271 (1.38); 1.4695 (0.99); 1.4490 (2.94); 1.4358 (1.73); 1.4071 (0.40); 1.3961 (0.41); 1.3675 (1.76); 1.3540 (2.94); 1.3338 (1.00); 1.0545 (4.60); 1.0355 (9.46); 1.0165 (4.33); 0.0078 (0.79); -0.0002 (19.73); -0.0083 (0.88) |
| Beispiel Nr. 72, Lösungsmittel: DMSO-d6, NMR-Spektrometer: 600.13 MHz |
| 11.2653 (0.46); 8.6190 (0.33); 7.2475 (0.46); 5.7618 (16.00); 4.3324 (0.68); 4.3228 (0.69); 4.0102 (3.05); 3.3526 (12.47); 2.6732 (0.49); 2.6613 (1.15); 2.6498 (0.68); 2.5168 (0.66); 2.5103 (1.64); 2.5074 (3.64); 2.5045 (5.45); 2.5015 (3.44); 2.4985 (1.59); 2.4935 (0.52); -0.0001 (0.60) |
| Beispiel Nr. 73, Lösungsmittel: DMSO-d6, NMR-Spektrometer: 399.95 MHz |
| 11.1920 (3.55); 9.1517 (0.90); 9.1371 (1.85); 9.1222 (0.91); 7.9930 (2.67); 7.9880 (1.20); 7.9791 (3.06); 7.9711 (3.10); 7.9624 (1.29); 7.9573 (2.78); 7.9343 (0.36); 7.9203 (0.41); 7.9124 (0.41); 7.8987 (0.36); 7.6156 (1.70); 7.5951 (2.24); 7.5804 (3.33); 7.4128 (0.35); 7.3915 (1.74); 7.3718 (3.16); 7.3519 (1.96); 7.3421 (3.05); 7.3372 (1.29); 7.3260 (1.92); 7.3200 (5.90); 7.3033 (1.24); 7.2980 (2.71); 7.2903 (0.41); 7.1769 (2.10); 7.1581 (1.88); 7.1416 (0.58); 4.4967 (4.19); 4.4819 (4.17); 4.4468 (0.44); 4.4324 (0.37); 4.0996 (2.11); 3.9967 (16.00); 3.9042 (7.84); 3.3943 (0.47); 3.3828 (0.53); 3.3364 (221.04); 3.1745 (0.56); 3.1621 (0.51); 2.6762 (0.59); 2.6717 (0.78); 2.6675 (0.57); 2.5416 (0.82); 2.5070 (101.30); 2.5027 (127.51); 2.4984 (93.90); 2.3338 (0.58); 2.3294 (0.77); 2.3250 (0.58); 1.2354 (0.59); 0.0078 (1.41); -0.0002 (30.46); -0.0084 (1.36) |
| Beispiel Nr. 74, Lösungsmittel: DMSO-d6, NMR-Spektrometer: 399.95 MHz |
| 11.3347 (1.52); 7.6581 (1.11); 7.6535 (1.20); 7.6081 (0.60); 7.5867 (0.73); 7.5808 (0.64); 7.5254 (0.39); 7.5106 (0.79); 7.4957 (0.41); 7.4789 (2.20); 7.4574 (1.61); 4.1943 (1.50); 4.1795 (1.58); 4.0041 (8.25); 3.9040 (7.06); 3.3811 (0.36); 3.3351 (155.30); 3.1741 (0.67); 3.1615 (0.65); 2.6759 (0.40); 2.6717 (0.53); 2.6670 (0.40); 2.5414 (0.39); |
| 2.5111 (35.97); 2.5069 (68.95); 2.5024 (88.48); 2.4980 (65.13); 2.4940 (33.36); 2.3336 (0.40); 2.3291 (0.54); 2.3246 (0.41); 1.4046 (16.00); 1.2975 (0.75); 1.2341 (0.44); 0.0079 (0.88); -0.0002 (21.62); -0.0083 (0.96) |
| Beispiel Nr. 75, Lösungsmittel: DMSO-d6, NMR-Spektrometer: 399.95 MHz |
| 11.4138 (3.72); 7.8043 (2.81); 7.7983 (3.30); 7.7415 (2.13); 7.7307 (2.84); 7.7195 (2.40); 7.7137 (2.83); 7.5099 (3.99); 7.4883 (3.37); 4.2343 (4.51); 4.2185 (4.48); 4.0177 (16.00); 3.9041 (8.87); 3.3875 (0.71); 3.3324 (137.80); 3.1729 (0.86); 3.1620 (0.80); 3.0977 (1.38); 3.0795 (4.58); 3.0611 (4.66); 3.0427 (1.43); 2.6755 (0.54); 2.6712 (0.73); 2.6668 (0.57); 2.5415 (0.70); 2.5065 (96.63); 2.5021 (124.82); 2.4977 (93.57); 2.3329 (0.56); 2.3290 (0.75); 2.3244 (0.59); 1.2384 (5.55); 1.2202 (10.90); 1.2017 (4.85); 0.0078 (1.15); -0.0002 (28.48); -0.0081 (1.31) |
| Beispiel Nr. 76, Lösungsmittel: DMSO-d6, NMR-Spektrometer: 399.95 MHz |
| 11.3898 (3.26); 8.3646 (0.87); 8.3499 (1.57); 8.3352 (0.85); 7.5115 (1.59); 7.4848 (1.60); 7.2738 (3.50); 6.9362 (1.45); 6.9126 (1.49); 4.2760 (4.69); 4.2610 (4.65); 4.0140 (16.00); 3.3327 (46.99); 2.5061 (20.02); 2.5017 (25.47); 2.4974 (18.22); 2.1912 (1.78); 2.1722 (5.48); 2.1532 (5.64); 2.1342 (1.92); 1.0557 (6.24); 1.0367 (12.51); 1.0177 (5.81); -0.0002 (3.93) |
| Beispiel Nr. 77, Lösungsmittel: DMSO-d6, NMR-Spektrometer: 399.95 MHz |
| 11.1562 (2.46); 8.7520 (0.57); 8.7391 (1.11); 8.7264 (0.54); 7.8800 (0.41); 7.8652 (0.53); 7.8579 (0.84); 7.8431 (0.85); 7.8358 (0.51); 7.8212 (0.40); 7.2241 (0.64); 7.2014 (1.21); 7.1788 (0.58); 4.4057 (1.19); 4.3950 (1.78); 4.3841 (1.19); 4.0317 (10.66); 3.9034 (8.46); 3.7140 (1.79); 3.6811 (3.02); 3.6036 (3.09); 3.5706 (1.91); 3.5096 (0.43); 3.4960 (0.39); 3.4758 (0.46); 3.4551 (0.70); 3.3705 (609.27); 3.2714 (0.38); 3.1753 (0.52); 3.1623 (0.57); 2.6777 (0.44); 2.6737 (0.59); 2.6691 (0.45); 2.5887 (16.00); 2.5436 (0.37); 2.5132 (39.27); 2.5090 (76.87); 2.5045 (99.37); 2.5000 (73.13); 2.4957 (36.96); 2.3354 (0.44); 2.3312 (0.61); 2.3267 (0.46); 0.0079 (0.88); -0.0002 (22.87); -0.0085 (0.99) |
| Beispiel Nr. 78, Lösungsmittel: DMSO-d6, NMR-Spektrometer: 399.95 MHz |
| 11.2422 (3.68); 9.3891 (0.91); 9.3748 (1.94); 9.3606 (0.93); 8.8938 (3.43); 8.8881 (3.43); 8.2953 (2.33); 8.2890 (2.30); 8.2744 (2.51); 8.2681 (2.48); 7.7158 (0.75); 7.7091 (1.00); 7.7046 (1.01); 7.6938 (4.56); 7.6869 (1.34); 7.6826 (1.15); 7.6732 (3.79); 7.6325 (1.53); 7.6260 (1.41); 7.6160 (1.62); 7.6094 (1.32); 7.2954 (1.70); 7.2719 (2.61); 7.2490 (1.58); 4.5501 (3.60); 4.5360 (3.63); 3.9900 (16.00); 3.9042 (12.14); 3.3952 (0.43); 3.3409 (318.08); 3.2937 (0.32); 3.1746 (0.45); 3.1619 (0.42); 2.6767 (0.51); 2.6719 (0.69); 2.6675 (0.50); 2.5419 (0.61); 2.5251 (2.55); 2.5118 (44.81); 2.5075 (87.29); 2.5030 (112.71); 2.4984 (82.68); 2.4940 (41.89); 2.3341 (0.51); 2.3296 (0.67); 2.3251 (0.51); 0.0079 (1.28); -0.0002 (31.57); -0.0085 (1.34) |
| Beispiel Nr. 79, Lösungsmittel: DMSO-d6, NMR-Spektrometer: 399.95 MHz |
| 10.8699 (0.84); 10.8257 (0.38); 10.7229 (2.63); 10.2355 (0.51); 8.3135 (0.64); 8.2984 (1.42); 8.2848 (0.80); 7.6968 (0.94); 7.6905 (1.05); 7.6799 (0.97); 7.6740 (0.97); |
| 7.6138 (0.52); 7.6025 (0.65); 7.5923 (0.71); 7.5838 (0.69); 7.5812 (0.69); 7.5740 (0.51); 7.3749 (0.33); 7.3485 (0.43); 7.3260 (0.33); 7.2162 (1.18); 7.1927 (1.86); 7.1698 (1.16); 4.3005 (2.73); 4.2860 (2.73); 4.1096 (0.50); 4.0966 (0.54); 3.9185 (2.09); 3.9126 (6.59); 3.9040 (15.41); 3.8889 (16.00); 3.3941 (0.45); 3.3873 (0.41); 3.3811 (0.59); 3.3346 (230.04); 3.1741 (2.47); 3.1611 (2.42); 2.6756 (0.70); 2.6712 (0.96); 2.6668 (0.71); 2.5414 (0.64); 2.5106 (65.73); 2.5066 (125.33); 2.5022 (160.41); 2.4977 (117.85); 2.4935 (60.22); 2.4593 (1.10); 2.3334 (0.74); 2.3288 (1.00); 2.3245 (0.76); 2.1234 (0.69); 2.1056 (0.71); 2.0645 (4.64); 2.0468 (4.79); 1.9374 (1.28); 1.9197 (1.32); 1.4738 (0.76); 1.4695 (1.21); 1.4505 (4.22); 1.4410 (1.75); 1.4359 (1.97); 1.4141 (0.37); 1.4073 (0.50); 1.4003 (0.43); 1.3963 (0.50); 1.3749 (1.19); 1.3670 (2.68); 1.3545 (3.91); 1.3335 (1.13); 1.2368 (0.43); 1.0212 (0.37); 1.0092 (0.67); 1.0026 (0.58); 0.9966 (0.52); 0.9907 (0.96); 0.9826 (0.49); 0.9786 (0.59); 0.9711 (0.66); 0.9592 (0.40); 0.9530 (0.38); 0.4698 (0.49); 0.4657 (0.88); 0.4550 (2.61); 0.4509 (2.55); 0.4455 (1.42); 0.4405 (1.50); 0.4347 (3.21); 0.4306 (2.90); 0.4204 (1.21); 0.4140 (0.76); 0.4098 (0.69); 0.1698 (0.43); 0.1632 (1.02); 0.1498 (2.79); 0.1407 (2.51); 0.1376 (2.78); 0.1264 (0.80); 0.1028 (0.87); 0.0986 (0.91); 0.0912 (0.84); 0.0872 (0.75); 0.0079 (1.57); -0.0002 (39.46); -0.0084 (1.69) |
| Beispiel Nr. 80, Lösungsmittel: DMSO-d6, NMR-Spektrometer: 399.95 MHz |
| 11.2695 (2.18); 8.7703 (0.40); 8.7566 (0.76); 8.7433 (0.41); 7.7013 (0.43); 7.6942 (0.53); 7.6901 (0.55); 7.6827 (0.56); 7.6798 (0.58); 7.6722 (0.61); 7.6685 (0.56); 7.6611 (0.52); 7.5631 (0.74); 7.5570 (0.73); 7.5467 (0.78); 7.5405 (0.68); 7.2630 (1.08); 7.2393 (1.65); 7.2165 (1.01); 4.3571 (2.35); 4.3426 (2.36); 4.0117 (9.71); 3.3453 (9.97); 3.3426 (10.35); 3.3360 (10.62); 3.3327 (11.69); 3.3290 (14.11); 2.5116 (4.26); 2.5074 (8.29); 2.5031 (11.59); 2.4988 (7.97); 2.4945 (3.92); 2.2271 (16.00); -0.0002 (2.04) |
| Beispiel Nr. 81, Lösungsmittel: DMSO-d6, NMR-Spektrometer: 399.95 MHz |
| 11.3940 (3.30); 8.6397 (0.89); 8.6250 (1.65); 8.6108 (0.88); 7.5340 (1.61); 7.5072 (1.60); 7.5022 (1.06); 7.2656 (3.50); 6.9406 (1.44); 6.9169 (1.47); 5.7461 (1.31); 4.3010 (4.68); 4.2861 (4.61); 4.0133 (16.00); 3.4874 (0.36); 3.3140 (837.07); 2.6744 (0.45); 2.6699 (0.56); 2.6651 (0.43); 2.5395 (0.99); 2.5094 (31.79); 2.5052 (57.79); 2.5008 (74.75); 2.4965 (53.07); 2.3320 (0.37); 2.3274 (0.51); 2.3229 (0.39); 2.0692 (0.46); 1.9868 (0.34); 1.6394 (0.43); 1.6270 (0.88); 1.6205 (1.02); 1.6150 (0.84); 1.6083 (1.78); 1.6000 (0.85); 1.5963 (1.00); 1.5893 (1.03); 1.5766 (0.49); 0.7322 (0.51); 0.7187 (2.04); 0.7115 (4.73); 0.7071 (4.14); 0.6992 (5.63); 0.6939 (5.99); 0.6868 (2.11); 0.6792 (2.38); 0.6741 (4.06); 0.6671 (1.71); 0.6541 (0.51); -0.0002 (1.18) |
| Beispiel Nr. 82, Lösungsmittel: DMSO-d6, NMR-Spektrometer: 399.95 MHz |
| 11.2602 (2.39); 9.0786 (0.56); 9.0639 (1.30); 9.0490 (0.61); 7.7041 (0.55); 7.6969 (0.73); 7.6927 (0.64); 7.6817 (0.79); 7.6749 (0.81); 7.6633 (0.70); 7.5291 (1.01); 7.5226 (0.99); 7.5124 (1.07); 7.5063 (0.95); 7.2579 (1.23); 7.2345 (1.85); 7.2112 (1.09); 4.9347 (0.39); 4.3139 (2.59); 4.2990 (2.65); 4.0094 (11.21); 3.9878 (1.19); 3.3230 (163.13); 3.2998 (0.54); 2.6707 (0.39); 2.5891 (1.26); 2.5057 (47.94); 2.5017 (61.82); 2.4977 (42.64); 2.3287 (0.35); 2.0737 (1.74); 1.9887 (0.85); 1.9837 (0.83); 1.9626 (16.00); 1.3979 (6.74); 1.1746 (0.40); -0.0002 (3.88) |
| Beispiel Nr. 83, Lösungsmittel: DMSO-d6, NMR-Spektrometer: 399.95 MHz |
| 10.9998 (3.71); 8.4162 (0.78); 8.4017 (1.49); 8.3872 (0.77); 7.5831 (3.16); 7.5786 (3.26); 7.5525 (3.48); 7.5318 (3.99); 7.2274 (1.79); 7.2227 (1.79); 7.2067 (1.61); 7.2019 (1.58); 4.2803 (4.60); 4.2654 (4.59); 4.0718 (16.00); 3.9040 (12.23); 3.3353 (278.39); 3.1738 (0.59); 3.1614 (0.57); 2.6756 (0.68); 2.6712 (0.91); 2.6668 (0.68); 2.5409 (0.60); 2.5066 (115.62); 2.5022 (148.58); 2.4977 (109.38); 2.3332 (0.68); 2.3289 (0.93); 2.3244 (0.69); 2.1750 (1.70); 2.1560 (5.44); 2.1370 (5.61); 2.1180 (1.88); 1.0494 (6.06); 1.0304 (12.20); 1.0113 (5.67); 0.0078 (1.45); -0.0002 (36.30); -0.0080 (1.53) |
| Beispiel Nr. 84, Lösungsmittel: DMSO-d6, NMR-Spektrometer: 399.95 MHz |
| 11.1009 (4.02); 8.0323 (0.96); 8.0216 (1.83); 8.0112 (0.96); 7.7725 (3.20); 7.7507 (4.14); 7.6056 (4.19); 7.5838 (3.31); 7.1259 (0.58); 7.1040 (0.64); 6.7752 (0.73); 6.7533 (0.65); 5.5475 (0.86); 4.5337 (4.47); 4.5224 (4.46); 4.4045 (0.90); 4.3934 (0.90); 4.0701 (16.00); 3.9041 (13.38); 3.3310 (153.01); 3.1739 (0.84); 3.1609 (0.82); 2.6753 (0.66); 2.6709 (0.89); 2.6664 (0.67); 2.5063 (115.14); 2.5019 (149.09); 2.4973 (110.31); 2.4930 (56.42); 2.3329 (0.69); 2.3285 (0.94); 2.3241 (0.71); 2.1159 (1.57); 2.0970 (5.12); 2.0780 (5.34); 2.0633 (1.21); 2.0591 (1.84); 2.0447 (0.34); 1.0084 (6.17); 0.9983 (1.53); 0.9896 (12.70); 0.9795 (2.59); 0.9705 (5.84); 0.9605 (1.10); 0.0080 (1.51); - 0.0002 (39.60); -0.0084 (1.71) |
| Beispiel Nr. 85, Lösungsmittel: DMSO-d6, NMR-Spektrometer: 399.95 MHz |
| 11.2712 (2.54); 11.2157 (0.47); 8.8171 (0.59); 8.8029 (1.13); 8.7895 (0.58); 7.9517 (2.25); 7.6735 (0.55); 7.6663 (0.67); 7.6542 (0.70); 7.6447 (0.79); 7.6331 (0.65); 7.6086 (1.11); 7.6022 (0.95); 7.5921 (1.14); 7.5856 (0.92); 7.2847 (1.27); 7.2612 (2.02); 7.2385 (1.15); 4.3353 (2.77); 4.3211 (2.74); 4.0110 (11.21); 3.6847 (9.57); 3.3500 (178.70); 3.3483 (177.85); 3.3431 (396.37); 3.3204 (2.30); 2.8909 (16.00); 2.7312 (14.43); 2.6720 (0.55); 2.6678 (0.37); 2.5247 (0.89); 2.5069 (60.29); 2.5028 (81.68); 2.4987 (57.73); 2.3342 (0.36); 2.3292 (0.53); 2.3251 (0.35); 2.0729 (0.35); 1.2349 (0.58); -0.0002 (9.59) |
| Beispiel Nr. 86, Lösungsmittel: DMSO-d6, NMR-Spektrometer: 399.95 MHz |
| 11.2016 (3.50); 9.3280 (0.86); 9.3133 (1.77); 9.2988 (0.89); 9.0685 (2.55); 9.0646 (2.58); 8.7275 (1.75); 8.7187 (1.74); 8.7157 (1.71); 8.2496 (1.12); 8.2445 (1.75); 8.2400 (1.18); 8.2297 (1.24); 8.2246 (1.87); 8.2201 (1.23); 7.6208 (1.66); 7.6034 (6.01); 7.5464 (1.49); 7.5345 (1.51); 7.5268 (1.50); 7.5146 (1.37); 7.4027 (1.31); 7.3813 (2.07); 7.3619 (1.23); 7.1969 (2.08); 7.1777 (1.81); 4.5276 (4.25); 4.5128 (4.24); 3.9997 (16.00); 3.9042 (12.93); 3.3885 (2.28); 3.3392 (184.06); 3.1682 (6.45); 2.6760 (0.57); 2.6717 (0.80); 2.6673 (0.60); 2.5421 (0.63); 2.5248 (2.33); 2.5071 (98.76); 2.5027 (127.92); 2.4982 (94.98); 2.3335 (0.57); 2.3294 (0.79); 2.3251 (0.60); 0.0079 (1.33); - 0.0002 (36.77); -0.0084 (1.55) |
| Beispiel Nr. 87, Lösungsmittel: DMSO-d6, NMR-Spektrometer: 399.95 MHz |
| 11.1771 (3.50); 8.3711 (0.85); 8.3566 (1.71); 8.3415 (0.89); 7.5811 (3.20); 7.5038 (1.40); 7.4825 (1.79); 7.3702 (1.80); 7.3507 (3.18); 7.3310 (1.62); 7.0931 (2.01); 7.0740 (1.77); 4.3253 (0.34); 4.3098 (0.36); 4.2867 (1.97); 4.2726 (3.68); 4.2588 (2.05); 4.2351 (0.36); 4.2205 (0.37); 4.0945 (0.33); 4.0035 (16.00); 3.9039 (13.26); 3.3306 (153.98); 3.1740 (1.28); 3.1609 (1.27); 2.6752 (0.69); 2.6709 (0.97); 2.6664 (0.72); 2.5062 (121.13); 2.5018 (158.03); 2.4973 (119.07); 2.3328 (0.73); 2.3284 (1.00); 2.3240 |
| (0.76); 2.2429 (0.68); 2.2259 (1.09); 2.2064 (1.16); 2.1902 (0.78); 1.5738 (0.61); 1.5545 (0.88); 1.5404 (0.93); 1.5352 (0.82); 1.5207 (1.13); 1.5015 (0.83); 1.3539 (0.73); 1.3388 (1.01); 1.3355 (0.99); 1.3205 (1.34); 1.3021 (0.99); 1.2872 (0.65); 1.2579 (0.35); 1.2344 (0.72); 1.0306 (10.08); 1.0135 (9.91); 0.8311 (5.23); 0.8127 (10.72); 0.7941 (4.62); 0.0080 (1.48); -0.0002 (39.80); -0.0084 (2.12) |
| Beispiel Nr. 88, Lösungsmittel: DMSO-d6, NMR-Spektrometer: 399.95 MHz |
| 11.3616 (3.60); 8.8446 (0.98); 8.8303 (2.01); 8.8158 (0.95); 7.6802 (1.44); 7.6740 (1.56); 7.6586 (1.79); 7.6524 (1.97); 7.5502 (3.15); 7.5442 (2.87); 7.4987 (4.11); 7.4771 (3.31); 4.3653 (4.09); 4.3509 (4.08); 4.0178 (16.00); 3.9041 (11.12); 3.3326 (150.28); 3.1717 (0.43); 3.1635 (0.41); 2.6758 (0.67); 2.6714 (0.89); 2.6671 (0.67); 2.5067 (113.15); 2.5023 (142.89); 2.4979 (104.72); 2.3335 (0.66); 2.3291 (0.88); 2.3245 (0.72); 1.6500 (1.31); 1.6443 (0.97); 1.6359 (3.01); 1.6273 (5.01); 1.6189 (2.56); 1.6068 (0.84); 1.5845 (0.86); 1.5723 (2.58); 1.5637 (4.92); 1.5551 (3.03); 1.5470 (0.99); 1.5410 (1.24); 1.2355 (0.36); 0.0079 (1.84); -0.0002 (40.85); -0.0078 (1.86) |
| Beispiel Nr. 89, Lösungsmittel: DMSO-d6, NMR-Spektrometer: 399.95 MHz |
| 11.2422 (2.82); 9.8595 (0.36); 8.5385 (0.98); 8.5232 (1.82); 8.5091 (0.82); 8.1140 (0.34); 7.6034 (1.88); 7.5911 (3.13); 7.5813 (1.20); 7.5698 (1.31); 7.5637 (0.67); 7.2569 (1.44); 7.2333 (1.85); 7.2106 (0.94); 4.3098 (3.45); 4.2954 (3.60); 4.2633 (0.34); 4.0077 (16.00); 3.5984 (0.35); 3.4892 (0.38); 3.4795 (0.40); 3.4693 (0.53); 3.4023 (0.83); 3.3416 (522.81); 3.3352 (1373.05); 3.2392 (0.48); 2.6758 (1.56); 2.6711 (1.89); 2.6670 (1.45); 2.6620 (0.80); 2.5414 (1.25); 2.5286 (4.82); 2.5246 (4.35); 2.5105 (130.79); 2.5065 (230.06); 2.5022 (308.81); 2.4981 (214.00); 2.4939 (107.69); 2.4635 (0.52); 2.4533 (0.41); 2.4463 (0.35); 2.3289 (1.99); 2.3242 (1.44); 2.2971 (2.72); 2.2785 (5.26); 2.2601 (3.08); 2.2457 (0.37); 2.0732 (1.73); 1.9888 (0.38); 1.8513 (0.68); 1.8333 (2.94); 1.8148 (3.95); 1.7965 (2.57); 1.7780 (0.83); 1.2347 (1.16); 0.8652 (0.35); 0.0658 (0.44); -0.0002 (9.77); -0.0085 (0.42); -0.0645 (1.51); -0.5511 (0.38) |
| Beispiel Nr. 90, Lösungsmittel: DMSO-d6, NMR-Spektrometer: 399.95 MHz |
| 11.1025 (3.45); 11.0193 (0.44); 7.9984 (0.85); 7.9877 (1.63); 7.9771 (0.84); 7.7908 (0.33); 7.7741 (2.71); 7.7523 (3.40); 7.6070 (3.48); 7.5852 (2.74); 7.4226 (0.35); 7.1280 (0.63); 7.1062 (0.71); 6.7768 (0.78); 6.7549 (0.70); 5.5501 (1.04); 4.5370 (3.75); 4.5258 (3.74); 4.4078 (1.02); 4.3967 (1.03); 4.3760 (0.50); 4.3616 (0.50); 4.1137 (0.42); 4.1008 (0.49); 4.0837 (2.22); 4.0705 (13.46); 3.9039 (16.00); 3.3413 (382.74); 3.1743 (1.74); 3.1614 (1.66); 2.6761 (0.67); 2.6716 (0.90); 2.6673 (0.66); 2.5070 (114.29); 2.5026 (144.55); 2.4982 (106.77); 2.3336 (0.66); 2.3293 (0.88); 2.3247 (0.66); 2.1074 (0.66); 2.0896 (0.69); 2.0047 (5.06); 1.9970 (1.81); 1.9872 (5.25); 1.9797 (1.63); 1.2354 (0.38); 0.9791 (0.40); 0.9669 (0.66); 0.9607 (0.71); 0.9488 (1.05); 0.9419 (0.69); 0.9365 (0.71); 0.9302 (0.76); 0.9167 (0.38); 0.4814 (0.36); 0.4772 (0.37); 0.4608 (0.40); 0.4569 (0.37); 0.4265 (0.94); 0.4159 (2.81); 0.4118 (2.94); 0.4068 (1.89); 0.4020 (1.83); 0.3956 (2.94); 0.3918 (2.86); 0.3819 (1.40); 0.1709 (0.46); 0.1591 (0.43); 0.1128 (1.02); 0.0992 (3.76); 0.0870 (3.70); 0.0761 (0.92); 0.0078 (1.14); -0.0002 (26.02); -0.0082 (1.31) |
| Beispiel Nr. 91, Lösungsmittel: DMSO-d6, NMR-Spektrometer: 399.95 MHz |
| 11.2390 (3.18); 8.1580 (0.84); 8.1432 (1.73); 8.1286 (0.81); 7.6356 (0.66); 7.6288 (0.86); 7.6248 (0.80); 7.6172 (0.90); 7.6066 (0.97); 7.5957 (0.76); 7.5421 (1.33); 7.5356 |
| (1.24); 7.5254 (1.37); 7.5188 (1.13); 7.2390 (1.53); 7.2156 (2.28); 7.1925 (1.37); 4.3151 (3.40); 4.3005 (3.34); 4.0080 (14.53); 3.9036 (14.92); 3.5087 (0.44); 3.4939 (0.43); 3.4783 (0.50); 3.3647 (730.71); 3.2970 (0.81); 3.2670 (0.40); 3.1744 (1.05); 3.1632 (1.02); 2.6777 (0.62); 2.6733 (0.83); 2.6689 (0.61); 2.5432 (0.63); 2.5128 (56.95); 2.5086 (109.28); 2.5042 (139.48); 2.4997 (101.39); 2.4956 (50.76); 2.3352 (0.63); 2.3309 (0.86); 2.3266 (0.64); 1.3056 (16.00); 1.0012 (1.40); 0.9923 (3.97); 0.9855 (4.20); 0.9771 (1.57); 0.5528 (1.78); 0.5440 (4.81); 0.5371 (4.70); 0.5279 (1.59); 0.0077 (1.24); -0.0002 (33.10); -0.0083 (1.45) |
| Beispiel Nr. 92, Lösungsmittel: DMSO-d6, NMR-Spektrometer: 399.95 MHz |
| 11.1069 (3.24); 8.1055 (0.75); 8.0947 (1.45); 8.0838 (0.75); 7.7762 (2.57); 7.7544 (3.31); 7.6112 (3.36); 7.5894 (2.63); 4.5264 (3.52); 4.5151 (3.53); 4.0714 (12.85); 3.9041 (9.29); 3.3300 (112.00); 3.1739 (0.48); 3.1609 (0.47); 2.6752 (0.58); 2.6708 (0.79); 2.6664 (0.61); 2.5062 (97.14); 2.5018 (126.31); 2.4973 (94.17); 2.3330 (0.53); 2.3286 (0.74); 2.3241 (0.57); 1.8187 (16.00); 1.8008 (1.18); 0.0079 (1.16); -0.0002 (31.40); -0.0085 (1.42) |
| Beispiel Nr. 93, Lösungsmittel: DMSO-d6, NMR-Spektrometer: 399.95 MHz |
| 11.3178 (3.20); 8.4347 (0.82); 8.4206 (1.67); 8.4060 (0.81); 7.6597 (2.57); 7.6535 (2.91); 7.5944 (1.39); 7.5880 (1.15); 7.5729 (1.99); 7.5665 (1.80); 7.4899 (4.21); 7.4684 (2.86); 4.3244 (3.74); 4.3098 (3.72); 3.9998 (14.41); 3.9036 (14.96); 3.5166 (0.35); 3.5086 (0.38); 3.4726 (0.52); 3.4615 (0.52); 3.3595 (751.97); 3.2822 (0.34); 3.2746 (0.32); 3.1744 (0.48); 3.1623 (0.46); 2.6773 (0.71); 2.6729 (0.98); 2.6684 (0.72); 2.5429 (0.82); 2.5259 (4.19); 2.5127 (63.79); 2.5083 (122.22); 2.5038 (155.85); 2.4993 (112.43); 2.4948 (55.52); 2.3394 (0.33); 2.3350 (0.69); 2.3305 (0.94); 2.3260 (0.67); 2.0698 (1.27); 2.0650 (2.00); 2.0509 (6.11); 2.0322 (0.95); 2.0181 (0.91); 2.0016 (0.61); 0.8959 (16.00); 0.8800 (15.53); 0.0079 (1.65); -0.0002 (39.26); -0.0085 (1.57) |
| Beispiel Nr. 94, Lösungsmittel: DMSO-d6, NMR-Spektrometer: 399.95 MHz |
| 11.5227 (0.52); 11.4535 (4.07); 8.9638 (0.87); 8.9497 (1.89); 8.9351 (0.94); 8.8029 (0.66); 8.7910 (0.67); 8.1151 (2.37); 7.8273 (0.41); 7.8114 (0.49); 7.8082 (0.48); 7.7926 (0.38); 7.7475 (1.41); 7.7408 (1.67); 7.7309 (1.63); 7.7242 (1.65); 7.6551 (0.95); 7.6481 (1.03); 7.6438 (1.14); 7.6368 (1.02); 7.6330 (1.13); 7.6259 (1.10); 7.6217 (1.13); 7.6149 (0.91); 7.3633 (0.42); 7.2888 (1.80); 7.2653 (2.79); 7.2424 (1.66); 4.3900 (3.32); 4.3759 (3.39); 4.0915 (0.33); 4.0760 (0.35); 4.0162 (16.00); 3.6015 (1.05); 3.5881 (3.03); 3.5737 (3.15); 3.5595 (1.22); 3.3947 (8.30); 2.9923 (0.33); 2.7371 (1.23); 2.7249 (1.11); 2.7093 (0.40); 2.6974 (0.38); 2.6716 (0.42); 2.5249 (0.72); 2.5116 (20.13); 2.5071 (42.79); 2.5025 (59.80); 2.4979 (44.88); 2.4934 (21.95); 2.3293 (0.39); 1.0005 (0.42); 0.9826 (0.88); 0.9647 (0.40); 0.0080 (0.34); -0.0002 (10.53); -0.0085 (0.38) |
| Beispiel Nr. 95, Lösungsmittel: DMSO-d6, NMR-Spektrometer: 399.95 MHz |
| 8.3690 (0.96); 7.6863 (0.73); 7.6795 (0.91); 7.6751 (0.92); 7.6679 (1.00); 7.6646 (0.99); 7.6572 (1.08); 7.6531 (0.99); 7.6461 (0.93); 7.5490 (1.58); 7.5423 (1.48); 7.5322 (1.60); 7.5256 (1.44); 7.2538 (1.77); 7.2300 (2.65); 7.2072 (1.67); 7.1991 (0.40); 7.1747 (0.38); 4.3468 (3.01); 4.3338 (3.09); 4.0389 (0.48); 4.0210 (0.77); 4.0074 (16.00); 3.7607 (1.12); 3.4638 (0.34); 3.4096 (1.22); 3.3923 (2.56); 3.3504 (18.00); 3.1492 (8.40); 2.5124 (7.34); 2.5080 (15.40); 2.5035 (21.09); 2.4989 (15.89); 2.4944 (8.25); 2.1841 (0.33); 2.0424 (0.91); 1.9893 (2.02); 1.3565 (1.82); 1.1933 (0.53); 1.1755 (1.03); 1.1577 (0.52); 1.1091 (0.41); 1.0916 (0.66); 1.0741 (0.33); -0.0002 (0.62) |
| Beispiel Nr. 96, Lösungsmittel: DMSO-d6, NMR-Spektrometer: 399.95 MHz |
| 11.2413 (2.51); 7.6533 (2.44); 7.6384 (0.86); 7.6225 (1.60); 7.6054 (1.65); 7.5828 (1.30); 7.4123 (1.24); 7.3928 (2.28); 7.3731 (1.21); 7.1900 (1.52); 7.1707 (1.26); 4.1816 (3.44); 4.1658 (3.39); 4.0158 (11.48); 3.9038 (6.99); 3.3434 (345.82); 3.1743 (0.81); 3.1614 (0.79); 2.9073 (16.00); 2.6759 (0.49); 2.6715 (0.64); 2.6675 (0.48); 2.5070 (83.64); 2.5026 (105.26); 2.4982 (77.61); 2.3336 (0.50); 2.3293 (0.65); 2.3249 (0.49); 1.2582 (0.33); 1.2332 (0.51); 0.0077 (1.12); -0.0002 (24.31); -0.0084 (1.26) |
| Beispiel Nr. 97, Lösungsmittel: DMSO-d6, NMR-Spektrometer: 399.95 MHz |
| 11.2371 (3.52); 9.3334 (0.96); 9.3175 (2.09); 9.3018 (1.06); 8.8383 (2.03); 8.8249 (2.14); 7.7153 (0.74); 7.7083 (0.98); 7.7045 (1.04); 7.6938 (1.13); 7.6857 (1.19); 7.6755 (0.97); 7.4885 (1.49); 7.4823 (1.62); 7.4720 (1.69); 7.4658 (1.59); 7.2579 (1.69); 7.2343 (2.78); 7.2113 (1.58); 6.8716 (0.38); 4.3781 (3.81); 4.3624 (3.96); 4.0386 (0.61); 4.0207 (0.67); 3.9975 (16.00); 3.3627 (192.22); 3.3559 (214.68); 3.3487 (235.27); 2.8916 (1.27); 2.8008 (0.53); 2.7932 (0.65); 2.7832 (1.15); 2.7704 (1.18); 2.7601 (0.68); 2.7534 (0.61); 2.7321 (1.09); 2.6731 (0.39); 2.5258 (0.87); 2.5081 (43.41); 2.5038 (59.43); 2.4994 (47.49); 2.3304 (0.36); 2.1834 (0.58); 2.0733 (0.39); 1.9890 (2.30); 1.3555 (4.09); 1.2352 (0.37); 1.1929 (0.63); 1.1751 (1.19); 1.1574 (0.60); 0.6767 (0.43); 0.6531 (3.06); 0.6476 (2.47); 0.6346 (4.23); 0.6308 (4.28); 0.6205 (4.52); 0.6166 (4.34); 0.5918 (0.43); -0.0002 (2.39) |
| Beispiel Nr. 98, Lösungsmittel: DMSO-d6, NMR-Spektrometer: 399.95 MHz |
| 10.7354 (2.40); 8.4151 (0.57); 8.4006 (1.11); 8.3861 (0.56); 7.6683 (0.38); 7.6620 (0.70); 7.6576 (0.63); 7.6441 (2.02); 7.6304 (1.97); 7.2161 (0.76); 7.1914 (1.38); 7.1692 (0.73); 4.2740 (2.63); 4.2595 (2.64); 3.9039 (7.26); 3.8910 (15.58); 3.3332 (99.53); 3.1738 (1.41); 3.1609 (1.35); 2.6755 (0.33); 2.6710 (0.45); 2.6664 (0.33); 2.5241 (1.57); 2.5107 (28.67); 2.5064 (55.49); 2.5019 (71.41); 2.4974 (52.18); 2.4929 (26.25); 2.3285 (0.43); 2.3243 (0.32); 1.8778 (16.00); 1.4704 (0.92); 1.4499 (2.65); 1.4367 (1.61); 1.4080 (0.37); 1.3970 (0.37); 1.3684 (1.69); 1.3549 (2.66); 1.3345 (0.93); 0.0079 (0.86); -0.0002 (21.48); -0.0085 (0.94) |
| Beispiel Nr. 99, Lösungsmittel: DMSO-d6, NMR-Spektrometer: 399.95 MHz |
| 11.3951 (2.16); 8.4362 (0.55); 8.4215 (0.96); 8.4068 (0.52); 7.5378 (0.73); 7.5330 (1.07); 7.5108 (0.76); 7.5062 (1.09); 7.2586 (2.33); 6.9446 (0.97); 6.9209 (0.97); 4.2676 (3.24); 4.2526 (3.17); 4.0135 (10.90); 3.3378 (155.45); 2.5110 (10.42); 2.5067 (18.69); 2.5023 (23.86); 2.4979 (16.69); 2.4938 (8.19); 1.8889 (16.00); 1.3985 (0.53); -0.0002 (0.53) |
| Beispiel Nr. 100, Lösungsmittel: DMSO-d6, NMR-Spektrometer: 399.95 MHz |
| 11.1803 (3.50); 8.3581 (0.90); 8.3434 (1.73); 8.3284 (0.88); 7.5501 (3.24); 7.5271 (1.59); 7.5071 (1.84); 7.3699 (1.73); 7.3505 (3.16); 7.3309 (1.61); 7.0834 (2.05); 7.0642 (1.78); 4.2726 (4.37); 4.2577 (4.32); 4.0069 (16.00); 3.9039 (13.26); 3.3309 (157.82); 3.1736 (0.80); 3.1610 (0.75); 2.6752 (0.69); 2.6708 (0.92); 2.6666 (0.70); 2.6315 (0.90); 2.6116 (1.37); 2.5928 (1.12); 2.5727 (0.43); 2.5062 (118.86); 2.5018 (150.82); 2.4974 (111.85); 2.3328 (0.70); 2.3285 (0.95); 2.3241 (0.72); 1.8066 (0.49); 1.7976 (0.57); 1.7783 (1.56); 1.7582 (1.79); 1.7432 (1.18); 1.6982 (0.49); 1.6804 (1.37); 1.6627 (1.73); 1.6484 (2.17); 1.6340 (3.31); 1.5913 (0.56); 1.5532 (0.38); 1.5420 (0.52); |
| 1.5368 (0.51); 1.5079 (1.79); 1.5018 (1.88); 1.4892 (1.47); 0.0079 (1.61); -0.0002 (35.45); -0.0083 (1.66) |
| Beispiel Nr. 101, Lösungsmittel: DMSO-d6, NMR-Spektrometer: 399.95 MHz |
| 11.3211 (2.53); 8.4002 (0.64); 8.3856 (1.35); 8.3710 (0.66); 7.6735 (2.08); 7.6673 (2.22); 7.5529 (0.90); 7.5467 (0.77); 7.5313 (1.71); 7.5250 (1.65); 7.4877 (3.65); 7.4662 (1.88); 4.3083 (3.01); 4.2936 (3.01); 4.0033 (11.54); 3.9040 (9.57); 3.3317 (118.91); 3.1741 (0.65); 3.1610 (0.64); 2.6756 (0.54); 2.6711 (0.72); 2.6667 (0.52); 2.5241 (2.69); 2.5108 (46.78); 2.5065 (89.81); 2.5020 (115.04); 2.4975 (83.82); 2.4931 (41.87); 2.4650 (1.37); 2.4479 (0.58); 2.3331 (0.52); 2.3288 (0.70); 2.3244 (0.52); 1.2351 (0.40); 1.0792 (16.00); 1.0620 (15.65); 0.0079 (1.21); -0.0002 (29.59); -0.0085 (1.20) |
| Beispiel Nr. 102, Lösungsmittel: DMSO-d6, NMR-Spektrometer: 399.95 MHz |
| 11.2569 (3.57); 8.3584 (0.91); 8.3437 (1.96); 8.3287 (0.94); 7.7051 (0.73); 7.6984 (0.89); 7.6940 (0.90); 7.6867 (0.94); 7.6835 (0.96); 7.6760 (1.04); 7.6718 (0.96); 7.6649 (0.87); 7.5196 (1.45); 7.5130 (1.44); 7.5029 (1.54); 7.4963 (1.39); 7.2584 (1.91); 7.2346 (2.72); 7.2119 (1.77); 5.7256 (2.42); 5.7226 (3.25); 5.7194 (2.51); 4.3258 (3.65); 4.3111 (3.69); 4.0017 (16.00); 3.3696 (99.69); 3.3639 (94.53); 3.3618 (89.81); 3.3535 (118.87); 2.6774 (0.38); 2.6729 (0.54); 2.6683 (0.41); 2.5432 (0.37); 2.5263 (0.97); 2.5216 (1.39); 2.5129 (27.89); 2.5084 (61.42); 2.5038 (83.20); 2.4992 (59.26); 2.4946 (27.75); 2.3351 (0.40); 2.3305 (0.55); 2.3259 (0.41); 2.0843 (13.35); 2.0819 (13.71); 1.7902 (12.59); 1.7877 (12.66); 0.0080 (0.42); -0.0002 (15.64); -0.0085 (0.57) |
| Beispiel Nr. 103, Lösungsmittel: DMSO-d6, NMR-Spektrometer: 399.95 MHz |
| 11.2287 (3.95); 11.2117 (0.83); 9.3809 (0.99); 9.3667 (2.12); 9.3519 (1.01); 8.1371 (3.70); 8.1164 (6.89); 8.0604 (7.08); 8.0393 (4.29); 7.9516 (2.33); 7.7083 (0.74); 7.7004 (0.94); 7.6898 (0.99); 7.6795 (1.08); 7.6689 (0.89); 7.6307 (1.57); 7.6245 (1.37); 7.6134 (1.60); 7.6077 (1.32); 7.2927 (1.69); 7.2690 (2.79); 7.2459 (1.49); 4.5597 (3.51); 4.5455 (3.52); 4.0381 (0.32); 3.9834 (15.80); 3.9528 (0.67); 3.4113 (0.94); 3.3313 (388.16); 3.3076 (2.64); 3.2642 (20.64); 2.8902 (16.00); 2.7305 (14.49); 2.6703 (0.62); 2.6663 (0.49); 2.5242 (1.15); 2.5057 (79.93); 2.5017 (108.28); 2.4978 (78.32); 2.3280 (0.64); 2.0734 (0.33); 1.9884 (1.05); 1.8365 (0.94); 1.3974 (2.07); 1.2344 (0.84); 1.1918 (0.35); 1.1746 (0.64); 1.1565 (0.39); 0.0078 (0.35); -0.0002 (12.76); -0.0085 (0.42) |
| Beispiel Nr. 104, Lösungsmittel: DMSO-d6, NMR-Spektrometer: 399.95 MHz |
| 11.2366 (3.66); 9.3314 (0.93); 9.3158 (2.01); 9.3001 (0.98); 8.8391 (0.91); 8.8238 (1.83); 8.8088 (0.96); 7.9526 (1.50); 7.7192 (0.74); 7.7122 (0.96); 7.7082 (0.98); 7.7007 (1.05); 7.6974 (1.03); 7.6900 (1.12); 7.6864 (1.04); 7.6792 (0.91); 7.4896 (1.48); 7.4832 (1.57); 7.4730 (1.63); 7.4665 (1.51); 7.2610 (1.80); 7.2374 (2.78); 7.2144 (1.67); 4.3856 (3.73); 4.3699 (3.82); 3.9975 (16.00); 3.3923 (0.58); 3.3747 (0.92); 3.3444 (93.46); 3.3417 (98.72); 3.3327 (110.07); 3.3305 (93.88); 3.1988 (0.78); 3.1810 (2.75); 3.1648 (3.42); 3.1475 (2.82); 3.1298 (0.87); 2.8913 (12.20); 2.7321 (10.01); 2.6718 (0.40); 2.5252 (0.77); 2.5117 (20.30); 2.5073 (42.74); 2.5028 (58.38); 2.4983 (44.65); 2.4939 (23.82); 2.3295 (0.38); 2.0737 (0.46); 1.9890 (0.82); 1.3557 (1.06); 1.2357 (0.35); 1.1750 (0.44); 1.0912 (0.62); 1.0684 (5.56); 1.0504 (12.00); 1.0325 (5.46); 0.0080 (0.53); -0.0002 (16.64) |
| Beispiel Nr. 105, Lösungsmittel: DMSO-d6, NMR-Spektrometer: 399.95 MHz |
| 11.2440 (2.22); 8.4752 (0.59); 8.4608 (1.22); 8.4461 (0.60); 7.8713 (0.46); 7.8577 (0.83); 7.8441 (0.47); 7.6496 (0.49); 7.6426 (0.63); 7.6383 (0.61); 7.6311 (0.68); 7.6276 (0.63); 7.6203 (0.74); 7.6165 (0.63); 7.6094 (0.62); 7.5687 (1.02); 7.5621 (0.93); 7.5520 (1.06); 7.5455 (0.88); 7.2545 (1.20); 7.2308 (1.77); 7.2081 (1.09); 6.8704 (0.63); 6.6408 (0.35); 4.3051 (2.37); 4.2906 (2.37); 4.0379 (0.38); 4.0200 (0.56); 4.0085 (10.66); 3.3335 (62.50); 3.3254 (41.49); 3.3228 (44.81); 3.2721 (0.91); 3.2543 (1.97); 3.2395 (1.96); 3.2219 (0.94); 2.6708 (0.35); 2.5241 (0.70); 2.5193 (1.06); 2.5107 (17.89); 2.5062 (37.80); 2.5017 (51.55); 2.4971 (38.16); 2.4926 (18.94); 2.3254 (2.06); 2.3075 (3.50); 2.2896 (1.77); 2.1830 (1.02); 1.9886 (1.59); 1.7583 (16.00); 1.3552 (7.93); 1.1924 (0.43); 1.1746 (0.85); 1.1568 (0.42); 0.0080 (0.50); -0.0002 (16.85); - 0.0084 (0.69) |
| Beispiel Nr. 106, Lösungsmittel: DMSO-d6, NMR-Spektrometer: 399.95 MHz |
| 11.1752 (0.94); 8.3093 (0.44); 7.5849 (0.86); 7.5193 (0.38); 7.4995 (0.47); 7.3692 (0.48); 7.3498 (0.86); 7.3300 (0.44); 7.1089 (0.55); 7.0900 (0.46); 4.2663 (1.18); 4.2515 (1.16); 4.0040 (4.36); 3.9040 (3.66); 3.3327 (49.96); 3.1738 (0.38); 3.1617 (0.36); 2.5107 (16.54); 2.5066 (31.36); 2.5022 (39.94); 2.4978 (29.11); 2.0254 (3.35); 0.9640 (16.00); 0.0078 (0.42); -0.0002 (9.90); -0.0081 (0.42) |
| Beispiel Nr. 107, Lösungsmittel: DMSO-d6, NMR-Spektrometer: 399.95 MHz |
| 11.2694 (3.37); 7.7157 (0.78); 7.7087 (0.98); 7.7047 (0.96); 7.6973 (1.03); 7.6940 (1.00); 7.6863 (1.11); 7.6825 (1.00); 7.6756 (0.87); 7.5073 (1.54); 7.5010 (1.52); 7.4908 (1.60); 7.4844 (1.44); 7.3003 (1.66); 7.2770 (2.73); 7.2539 (1.51); 4.4251 (7.91); 4.1076 (0.41); 4.0951 (0.41); 4.0149 (16.00); 3.9042 (10.50); 3.3310 (128.67); 3.3103 (3.00); 3.2928 (4.55); 3.2753 (2.73); 3.1741 (1.91); 3.1614 (1.81); 2.6755 (0.71); 2.6712 (0.94); 2.6669 (0.72); 2.5413 (0.72); 2.5066 (120.92); 2.5023 (151.38); 2.4979 (111.26); 2.3332 (0.68); 2.3290 (0.94); 2.3249 (0.73); 2.2999 (2.30); 2.2801 (4.50); 2.2596 (2.94); 1.9827 (0.76); 1.9644 (2.12); 1.9455 (2.81); 1.9264 (1.82); 1.9071 (0.56); 0.0078 (1.45); -0.0002 (34.69); -0.0079 (1.65) |
| Beispiel Nr. 108, Lösungsmittel: DMSO-d6, NMR-Spektrometer: 399.95 MHz |
| 11.1055 (1.83); 7.8487 (0.34); 7.8269 (0.73); 7.8118 (0.73); 7.7903 (0.35); 7.3616 (0.76); 7.1726 (0.68); 7.1502 (1.27); 7.1276 (0.64); 4.2277 (1.58); 4.2164 (1.69); 4.0291 (11.80); 3.9040 (8.86); 3.3298 (93.86); 3.1737 (1.24); 3.1609 (1.20); 2.6751 (0.54); 2.6707 (0.73); 2.6665 (0.55); 2.5409 (0.65); 2.5062 (95.03); 2.5018 (120.85); 2.4974 (89.91); 2.3330 (0.55); 2.3285 (0.73); 2.3242 (0.55); 1.3629 (16.00); 1.2981 (0.57); 1.2579 (0.33); 0.0077 (1.29); -0.0002 (29.31); -0.0084 (1.47) |
| Beispiel Nr. 109, Lösungsmittel: DMSO-d6, NMR-Spektrometer: 399.95 MHz |
| 11.3167 (3.87); 9.2297 (0.98); 9.2151 (2.06); 9.2006 (0.98); 7.9558 (5.61); 7.9516 (2.03); 7.9389 (2.04); 7.9344 (6.39); 7.9287 (0.92); 7.7696 (1.54); 7.7633 (1.63); 7.7480 (1.85); 7.7417 (1.97); 7.5970 (6.51); 7.5925 (2.15); 7.5754 (8.58); 7.5686 (3.60); 7.5282 (4.20); 7.5065 (3.56); 4.5409 (3.90); 4.5265 (3.87); 3.9761 (16.00); 3.9039 (11.61); 3.4474 (0.35); 3.4412 (0.34); 3.4229 (0.60); 3.4114 (0.78); 3.3502 (689.94); 3.2970 (0.77); 3.2813 (0.39); 3.1745 (0.54); 3.1621 (0.51); 2.6768 (0.68); 2.6723 (0.93); 2.6677 |
| (0.68); 2.5425 (0.77); 2.5118 (60.68); 2.5078 (116.54); 2.5033 (150.22); 2.4989 (110.62); 2.4947 (56.50); 2.3343 (0.68); 2.3299 (0.92); 2.3255 (0.68); 1.2357 (0.71); 0.0077 (1.49); -0.0002 (35.89); -0.0083 (1.81) |
| Beispiel Nr. 110, Lösungsmittel: DMSO-d6, NMR-Spektrometer: 399.95 MHz |
| 11.2424 (3.65); 9.3566 (0.87); 9.3406 (1.89); 9.3248 (0.89); 8.8830 (0.85); 8.8678 (1.74); 8.8523 (0.86); 7.9526 (0.52); 7.7207 (0.76); 7.7138 (0.92); 7.7095 (0.95); 7.7024 (0.99); 7.6986 (0.98); 7.6915 (1.05); 7.6874 (0.98); 7.6804 (0.87); 7.5003 (1.44); 7.4938 (1.45); 7.4836 (1.52); 7.4772 (1.37); 7.2636 (1.74); 7.2399 (2.66); 7.2170 (1.63); 4.3917 (3.47); 4.3759 (3.50); 4.0381 (0.60); 4.0203 (0.65); 3.9980 (16.00); 3.3319 (142.76); 3.3300 (163.50); 3.0243 (2.70); 3.0079 (4.66); 2.9915 (2.76); 2.8907 (4.43); 2.7309 (3.52); 2.6711 (0.38); 2.5244 (0.77); 2.5195 (1.24); 2.5111 (20.67); 2.5066 (43.10); 2.5020 (57.95); 2.4975 (42.63); 2.4930 (20.95); 2.3287 (0.39); 1.9888 (2.56); 1.3977 (0.42); 1.2353 (0.36); 1.1926 (0.69); 1.1748 (1.39); 1.1570 (0.69); 0.9972 (0.65); 0.9914 (0.64); 0.9878 (0.56); 0.9795 (1.13); 0.9712 (0.57); 0.9676 (0.69); 0.9598 (0.71); 0.9480 (0.38); 0.4157 (0.95); 0.4049 (2.88); 0.4006 (3.15); 0.3959 (1.52); 0.3904 (1.54); 0.3847 (3.01); 0.3804 (2.94); 0.3704 (1.19); 0.2141 (1.17); 0.2035 (3.38); 0.2003 (3.49); 0.1920 (3.06); 0.1883 (3.65); 0.1771 (0.89); -0.0002 (10.57); -0.0085 (0.39) |
| Beispiel Nr. 111, Lösungsmittel: DMSO-d6, NMR-Spektrometer: 399.95 MHz |
| 11.3186 (3.67); 9.1503 (0.94); 9.1359 (1.99); 9.1212 (0.94); 7.9426 (4.14); 7.9251 (4.54); 7.9213 (3.44); 7.7894 (1.51); 7.7832 (1.60); 7.7679 (1.79); 7.7615 (1.89); 7.5787 (0.73); 7.5601 (4.67); 7.5426 (2.20); 7.5285 (4.30); 7.5179 (3.84); 7.5067 (4.13); 7.5027 (2.87); 7.4987 (4.80); 7.4813 (1.71); 4.5464 (4.02); 4.5319 (4.01); 3.9734 (16.00); 3.9040 (10.19); 3.3381 (307.19); 3.3008 (0.48); 3.1745 (0.98); 3.1614 (0.95); 2.6757 (0.65); 2.6713 (0.87); 2.6668 (0.64); 2.5410 (0.67); 2.5108 (56.29); 2.5068 (107.29); 2.5024 (137.45); 2.4979 (100.82); 2.4939 (51.25); 2.3334 (0.62); 2.3291 (0.83); 2.3246 (0.60); 0.0077 (1.43); -0.0002 (35.85); -0.0084 (1.70) |
| Beispiel Nr. 112, Lösungsmittel: DMSO-d6, NMR-Spektrometer: 399.95 MHz |
| 11.2417 (3.62); 8.3043 (0.90); 8.2891 (1.88); 8.2746 (0.92); 7.7078 (0.75); 7.7007 (0.94); 7.6972 (0.95); 7.6890 (1.03); 7.6788 (1.08); 7.6678 (0.83); 7.5127 (1.46); 7.5065 (1.47); 7.4961 (1.56); 7.4897 (1.36); 7.2537 (1.62); 7.2303 (2.67); 7.2072 (1.51); 5.8615 (2.51); 4.3303 (3.70); 4.3158 (3.69); 3.9994 (16.00); 3.9040 (13.29); 3.9031 (13.65); 3.3300 (144.30); 3.1732 (1.17); 3.1604 (1.13); 2.6916 (1.38); 2.6744 (3.44); 2.6715 (3.42); 2.6601 (1.64); 2.5401 (0.89); 2.5055 (138.54); 2.5015 (174.45); 2.4973 (131.18); 2.3618 (1.53); 2.3443 (3.05); 2.3280 (2.61); 1.6623 (0.45); 1.6453 (1.66); 1.6276 (2.78); 1.6113 (2.19); 1.5950 (1.03); 1.5846 (1.03); 1.5689 (2.43); 1.5527 (2.97); 1.5351 (1.65); 1.5187 (0.39); 0.0066 (1.80); -0.0002 (37.06); -0.0012 (37.00); -0.0084 (1.91) |
| Beispiel Nr. 113, Lösungsmittel: DMSO-d6, NMR-Spektrometer: 399.95 MHz |
| 11.3497 (3.76); 8.6855 (0.94); 8.6710 (1.95); 8.6568 (0.94); 7.7071 (2.96); 7.7010 (3.31); 7.6258 (1.56); 7.6195 (1.38); 7.6042 (2.14); 7.5979 (1.98); 7.4976 (4.45); 7.4760 (3.28); 7.2908 (12.34); 7.2826 (7.25); 7.2781 (7.41); 7.2636 (0.60); 7.2570 (0.85); 7.2323 (1.00); 7.2247 (1.26); 7.2202 (1.05); 7.2166 (1.07); 7.2107 (1.39); 7.2039 (0.80); 7.1983 (0.57); 4.3413 (4.22); 4.3268 (4.20); 4.0174 (16.00); 3.9039 (15.70); 3.5221 (11.77); 3.3320 (227.03); 3.1733 (0.63); 3.1617 (0.62); 2.6752 (0.82); 2.6709 (1.12); |
| 2.6664 (0.85); 2.5412 (0.92); 2.5104 (73.48); 2.5063 (139.85); 2.5019 (179.94); 2.4974 (133.71); 2.3329 (0.79); 2.3286 (1.09); 2.3241 (0.81); 1.2358 (0.66); 0.0079 (1.87); - 0.0002 (44.09); -0.0084 (2.12) |
| Beispiel Nr. 114, Lösungsmittel: DMSO-d6, NMR-Spektrometer: 399.95 MHz |
| 11.4655 (2.85); 8.8818 (9.80); 8.2863 (0.92); 8.2717 (1.98); 8.2566 (0.95); 8.2466 (10.69); 7.7978 (1.45); 7.7918 (1.62); 7.7763 (1.67); 7.7704 (1.98); 7.7061 (2.85); 7.7006 (2.53); 7.5146 (3.22); 7.4933 (2.85); 4.1660 (4.07); 4.1512 (4.11); 4.0354 (15.80); 4.0202 (0.45); 3.3250 (349.58); 3.3013 (2.24); 2.6749 (0.36); 2.6705 (0.53); 2.6660 (0.39); 2.5240 (0.85); 2.5058 (56.14); 2.5016 (76.44); 2.4983 (50.62); 2.3329 (0.38); 2.3284 (0.59); 2.3237 (0.37); 2.1583 (1.76); 2.1393 (5.81); 2.1203 (6.00); 2.1013 (1.93); 1.9886 (1.23); 1.3978 (0.70); 1.1924 (0.36); 1.1747 (0.70); 1.1570 (0.34); 1.0342 (7.29); 1.0153 (16.00); 0.9962 (6.86); -0.0002 (8.65) |
| Beispiel Nr. 115, Lösungsmittel: DMSO-d6, NMR-Spektrometer: 399.95 MHz |
| 11.2590 (2.13); 8.5193 (0.53); 8.5045 (1.13); 8.4897 (0.54); 7.7112 (0.48); 7.7044 (0.57); 7.7000 (0.57); 7.6928 (0.60); 7.6892 (0.60); 7.6820 (0.65); 7.6779 (0.60); 7.6709 (0.52); 7.5308 (0.90); 7.5242 (0.90); 7.5142 (0.95); 7.5078 (0.84); 7.2668 (1.08); 7.2431 (1.64); 7.2203 (1.00); 6.6835 (1.29); 6.6673 (1.30); 6.6448 (1.40); 6.6286 (1.43); 5.9343 (1.53); 5.9307 (1.56); 5.8955 (1.38); 5.8920 (1.41); 4.3679 (2.20); 4.3533 (2.21); 4.0016 (9.94); 3.3455 (159.44); 2.6721 (0.34); 2.5255 (0.70); 2.5207 (1.08); 2.5120 (18.30); 2.5076 (39.53); 2.5031 (53.53); 2.4985 (38.89); 2.4941 (18.76); 2.4353 (0.42); 2.4320 (0.44); 2.4185 (0.69); 2.4153 (0.71); 2.4017 (0.69); 2.3986 (0.71); 2.3851 (0.44); 2.3819 (0.43); 2.3298 (0.35); 1.9900 (0.44); 1.3972 (2.08); 1.0082 (16.00); 0.9913 (15.63); -0.0002 (3.77) |
| Beispiel Nr. 116, Lösungsmittel: DMSO-d6, NMR-Spektrometer: 399.95 MHz |
| 11.3087 (0.98); 8.3509 (0.52); 7.6848 (0.81); 7.6787 (0.89); 7.5855 (0.40); 7.5792 (0.35); 7.5639 (0.60); 7.5576 (0.57); 7.4883 (1.26); 7.4667 (0.83); 4.3198 (1.17); 4.3053 (1.17); 4.0005 (4.42); 3.9041 (3.83); 3.3337 (63.51); 3.1739 (0.46); 3.1613 (0.43); 2.5105 (18.02); 2.5066 (34.00); 2.5022 (43.31); 2.4977 (31.56); 2.4934 (15.88); 2.0671 (3.27); 0.9713 (16.00); 0.0079 (0.48); -0.0002 (11.17); -0.0083 (0.47) |
| Beispiel Nr. 117, Lösungsmittel: DMSO-d6, NMR-Spektrometer: 399.95 MHz |
| 11.1802 (3.52); 9.4412 (0.85); 9.4250 (1.70); 9.4091 (0.83); 8.6712 (2.02); 8.6594 (1.97); 8.0689 (1.43); 8.0496 (3.09); 8.0283 (1.56); 8.0243 (1.59); 8.0095 (2.02); 8.0055 (2.01); 7.9904 (0.83); 7.9863 (0.82); 7.6317 (2.47); 7.6239 (2.50); 7.6201 (1.83); 7.6173 (1.71); 7.6127 (2.11); 7.6052 (2.82); 7.5588 (3.21); 7.3784 (1.62); 7.3589 (3.17); 7.3392 (1.72); 7.1849 (2.10); 7.1656 (1.70); 4.5188 (4.43); 4.5028 (4.36); 3.9888 (16.00); 3.9038 (12.66); 3.5081 (0.37); 3.5000 (0.32); 3.4799 (0.38); 3.4658 (0.41); 3.4524 (0.62); 3.4321 (0.94); 3.3603 (763.51); 3.2997 (0.98); 3.2750 (0.55); 3.2578 (0.35); 3.1752 (0.85); 3.1623 (0.80); 2.6774 (0.65); 2.6730 (0.89); 2.6687 (0.66); 2.5427 (0.60); 2.5084 (114.02); 2.5040 (146.21); 2.4996 (107.74); 2.3350 (0.67); 2.3306 (0.90); 2.3263 (0.69); 1.2292 (0.55); 0.0077 (1.41); -0.0002 (32.80); -0.0082 (1.33) |
| Beispiel Nr. 118, Lösungsmittel: DMSO-d6, NMR-Spektrometer: 399.95 MHz |
| 11.1633 (3.52); 8.8533 (0.86); 8.8408 (1.67); 8.8276 (0.84); 7.8899 (0.61); 7.8744 (0.75); 7.8675 (1.24); 7.8528 (1.22); 7.8459 (0.76); 7.8307 (0.59); 7.2356 (0.95); 7.2126 |
| (1.80); 7.1901 (0.87); 4.4160 (3.24); 4.4032 (3.23); 4.0738 (8.36); 4.0327 (15.59); 3.9036 (11.01); 3.5081 (0.33); 3.4774 (0.34); 3.4651 (0.40); 3.4535 (0.46); 3.3552 (650.84); 3.2679 (0.44); 3.1749 (0.90); 3.1617 (0.88); 3.0956 (16.00); 2.6764 (0.70); 2.6725 (0.93); 2.6682 (0.69); 2.5415 (0.70); 2.5076 (122.20); 2.5034 (154.46); 2.4990 (114.51); 2.3344 (0.72); 2.3301 (0.94); 2.3258 (0.71); 0.0069 (1.32); -0.0002 (33.57); -0.0083 (1.70) |
| Beispiel Nr. 119, Lösungsmittel: DMSO-d6, NMR-Spektrometer: 399.95 MHz |
| 11.1848 (1.71); 7.5518 (1.15); 7.5308 (0.62); 7.5096 (0.76); 7.4657 (0.35); 7.4497 (0.68); 7.4347 (0.36); 7.3693 (0.77); 7.3499 (1.41); 7.3302 (0.71); 7.0843 (1.04); 7.0653 (0.91); 4.1401 (1.50); 4.1247 (1.55); 4.0069 (8.17); 3.9037 (8.36); 3.4234 (0.36); 3.4060 (0.79); 3.3533 (335.58); 3.2872 (0.40); 3.1737 (0.47); 3.1628 (0.46); 2.6770 (0.39); 2.6723 (0.53); 2.6679 (0.40); 2.5424 (0.51); 2.5077 (67.85); 2.5033 (86.70); 2.4989 (63.82); 2.3342 (0.38); 2.3300 (0.52); 2.3256 (0.39); 1.3940 (16.00); 1.3220 (0.60); 1.2350 (0.32); 0.0078 (0.81); -0.0002 (19.78); -0.0084 (0.89) |
| Beispiel Nr. 120, Lösungsmittel: DMSO-d6, NMR-Spektrometer: 399.95 MHz |
| 11.2207 (3.31); 9.3531 (0.76); 9.3387 (1.57); 9.3236 (0.80); 7.6039 (4.15); 7.5879 (2.01); 7.5708 (0.41); 7.5511 (0.41); 7.5311 (0.34); 7.4078 (1.45); 7.4018 (0.50); 7.3884 (2.21); 7.3670 (1.69); 7.1734 (1.95); 7.1541 (1.63); 6.7411 (1.95); 6.7360 (4.26); 6.7307 (2.07); 5.1051 (7.00); 5.0999 (7.08); 4.4485 (3.93); 4.4336 (3.94); 4.0099 (16.00); 3.9042 (7.17); 3.4297 (0.33); 3.3882 (1.40); 3.3795 (0.38); 3.3336 (56.09); 3.1678 (2.46); 2.6756 (0.45); 2.6713 (0.63); 2.6668 (0.48); 2.5416 (0.60); 2.5108 (41.00); 2.5067 (80.42); 2.5022 (104.60); 2.4977 (77.51); 2.4934 (39.71); 2.3333 (0.49); 2.3289 (0.66); 2.3244 (0.50); 1.3941 (0.43); 1.3509 (0.53); 1.2583 (0.41); 1.2291 (0.68); 0.0080 (1.19); -0.0002 (31.38); -0.0085 (1.32) |
| Beispiel Nr. 121, Lösungsmittel: DMSO-d6, NMR-Spektrometer: 399.95 MHz |
| 11.1913 (3.42); 8.4523 (0.80); 8.4365 (1.57); 8.4215 (0.81); 7.5707 (1.54); 7.5501 (2.32); 7.5397 (3.33); 7.3687 (1.62); 7.3493 (3.10); 7.3298 (1.62); 7.1140 (2.06); 7.0948 (1.78); 4.3118 (4.56); 4.2962 (4.49); 4.0068 (16.00); 3.9040 (7.58); 3.8603 (13.17); 3.3331 (144.65); 3.1741 (0.44); 3.1612 (0.44); 2.6753 (0.48); 2.6709 (0.66); 2.6667 (0.48); 2.5407 (0.49); 2.5064 (87.11); 2.5021 (109.05); 2.4977 (79.69); 2.3330 (0.53); 2.3288 (0.68); 2.3244 (0.52); 0.0074 (1.43); -0.0002 (29.07); -0.0079 (1.34) |
| Beispiel Nr. 122, Lösungsmittel: DMSO-d6, NMR-Spektrometer: 399.95 MHz |
| 11.1765 (3.47); 8.3866 (0.88); 8.3715 (1.77); 8.3564 (0.87); 7.5823 (1.45); 7.5622 (1.79); 7.5147 (3.18); 7.3632 (1.70); 7.3437 (3.18); 7.3240 (1.69); 7.1022 (2.02); 7.0831 (1.74); 6.4186 (0.42); 6.4043 (1.33); 6.4010 (1.31); 6.3869 (1.34); 6.3836 (1.33); 6.3697 (0.43); 4.3268 (4.33); 4.3117 (4.28); 4.0046 (16.00); 3.9040 (11.33); 3.3330 (164.91); 3.1743 (0.75); 3.1612 (0.73); 2.6756 (0.62); 2.6711 (0.82); 2.6667 (0.60); 2.5412 (0.48); 2.5106 (54.93); 2.5066 (103.83); 2.5022 (131.79); 2.4978 (96.63); 2.3332 (0.59); 2.3290 (0.79); 2.3245 (0.59); 1.7775 (11.48); 1.7182 (5.89); 1.7009 (5.82); 1.2342 (0.32); 0.0078 (1.56); -0.0002 (36.47); -0.0082 (1.55) |
| Beispiel Nr. 123 , Lösungsmittel: DMSO-d6, NMR-Spektrometer: 399,95 MHz |
| 11,2453 (0,14); 8,7429 (0,04); 8,7301 (0,07); 8,7172 (0,04); 8,2882 (0,12); 8,2837 (0,13); 7,7656 (0,06); 7,7589 (0,07); 7,7444 (0,07); 7,7388 (0,07); 7,6584 (0,06); 7,6527 |
| (0,06); 7,6417 (0,06); 7,6359 (0,06); 7,5867 (0,04); 7,5753 (0,05); 7,5657 (0,05); 7,5574 (0,05); 7,4463 (0,13); 7,4264 (0,11); 7,2613 (0,06); 7,2377 (0,1); 7,2145 (0,06); 4,3244 (0,15); 4,3101 (0,15); 4,0161 (0,57); 3,5528 (0,36); 3,3684 (0,04); 3,3332 (16); 2,6711 (0,03); 2,6683 (0,02); 2,5022 (3,71); 2,3281 (0,02); 2,2411 (0,03); 2,0729 (5,65); 1,9008 (0,03); -0,0001 (0,3) |
| Beispiel Nr. 124, Lösungsmittel: DMSO-d6, NMR-Spektrometer: 399.95 MHz |
| 11.4794 (1.90); 8.8801 (6.73); 8.3531 (0.59); 8.3385 (1.31); 8.3238 (0.62); 8.2482 (6.91); 7.8599 (1.05); 7.8538 (1.10); 7.8383 (1.20); 7.8324 (1.28); 7.6649 (1.99); 7.6592 (1.90); 7.5187 (2.75); 7.4973 (2.48); 4.1615 (2.79); 4.1468 (2.79); 4.0383 (10.60); 4.0204 (0.33); 3.3366 (325.09); 3.3132 (1.55); 2.5246 (0.52); 2.5065 (31.80); 2.5024 (42.83); 2.0734 (2.69); 1.9887 (1.09); 1.8629 (16.00); 1.1748 (0.63); -0.0002 (2.51) |
| Beispiel Nr. 125, Lösungsmittel: DMSO-d6, NMR-Spektrometer: 399.95 MHz |
| 11.2428 (1.78); 8.5689 (0.57); 8.5547 (1.17); 8.5399 (0.57); 8.0806 (0.47); 8.0670 (0.89); 8.0540 (0.49); 7.6474 (0.49); 7.6404 (0.65); 7.6362 (0.57); 7.6293 (0.71); 7.6187 (0.83); 7.6141 (0.60); 7.6072 (0.79); 7.6004 (1.16); 7.5941 (0.84); 7.5838 (1.14); 7.5772 (0.80); 7.2629 (1.18); 7.2395 (1.71); 7.2166 (1.06); 4.3255 (2.26); 4.3111 (2.27); 4.0560 (1.18); 4.0381 (3.66); 4.0202 (4.69); 4.0150 (11.06); 4.0026 (1.33); 3.3360 (66.68); 3.3299 (164.55); 3.3064 (1.06); 3.0967 (8.15); 2.9575 (2.11); 2.9431 (2.76); 2.9267 (2.17); 2.6712 (0.41); 2.5245 (0.75); 2.5198 (1.06); 2.5104 (23.53); 2.5064 (44.23); 2.5021 (60.36); 2.4979 (40.84); 2.4936 (19.75); 2.3289 (0.40); 2.0734 (0.39); 1.9886 (16.00); 1.3978 (3.02); 1.1926 (4.23); 1.1748 (8.46); 1.1569 (4.12); 0.8915 (0.43); 0.8888 (0.45); 0.8836 (0.50); 0.8797 (0.38); 0.8716 (0.80); 0.8632 (0.40); 0.8597 (0.45); 0.8545 (0.49); 0.8513 (0.48); 0.4106 (0.77); 0.4002 (2.18); 0.3958 (2.25); 0.3908 (0.93); 0.3856 (1.05); 0.3799 (2.30); 0.3756 (2.01); 0.3657 (0.84); 0.1555 (0.88); 0.1450 (2.34); 0.1414 (2.33); 0.1332 (2.09); 0.1295 (2.42); 0.1185 (0.64); -0.0002 (8.63) |
| Beispiel Nr. 126, Lösungsmittel: DMSO-d6, NMR-Spektrometer: 399.95 MHz |
| 11.2200 (1.83); 8.3956 (0.60); 8.3807 (1.23); 8.3665 (0.59); 7.5950 (2.18); 7.5813 (2.24); 7.5628 (0.76); 7.5560 (0.42); 7.2473 (0.89); 7.2252 (1.40); 7.2015 (0.67); 5.7549 (2.17); 4.2903 (2.42); 4.2755 (2.41); 4.0559 (0.54); 4.0381 (1.72); 4.0203 (1.82); 4.0052 (10.80); 3.3268 (218.07); 2.5239 (0.51); 2.5057 (33.44); 2.5016 (45.71); 2.4975 (32.56); 2.1604 (1.89); 2.1413 (2.34); 2.1219 (2.06); 2.0859 (1.23); 2.0735 (0.43); 1.9885 (7.32); 1.5385 (0.34); 1.5218 (0.69); 1.5050 (0.93); 1.4885 (0.83); 1.4727 (0.53); 1.4503 (1.26); 1.4312 (1.75); 1.4122 (1.94); 1.3976 (4.12); 1.1926 (1.93); 1.1748 (3.83); 1.1570 (1.91); 0.8565 (16.00); 0.8404 (15.36); -0.0002 (3.97) |
| Beispiel Nr. 127, Lösungsmittel: DMSO-d6, NMR-Spektrometer: 399.95 MHz |
| 11.2413 (2.55); 8.4476 (0.66); 8.4329 (1.42); 8.4183 (0.72); 8.1216 (0.60); 8.1075 (1.20); 8.0933 (0.65); 7.6600 (0.54); 7.6533 (0.71); 7.6489 (0.73); 7.6417 (0.78); 7.6382 (0.76); 7.6309 (0.86); 7.6270 (0.78); 7.6199 (0.72); 7.5568 (1.11); 7.5504 (1.09); 7.5403 (1.22); 7.5338 (1.07); 7.2574 (1.25); 7.2338 (1.97); 7.2110 (1.16); 4.3186 (2.71); 4.3041 (2.78); 4.0152 (11.46); 3.7194 (3.94); 3.7048 (3.99); 3.3921 (0.70); 3.3746 (0.86); 3.3568 (0.70); 3.3310 (123.02); 3.3084 (1.31); 2.5109 (10.65); 2.5066 (21.69); |
| 2.5021 (29.21); 2.4976 (22.36); 2.4932 (12.02); 2.1832 (0.45); 1.8479 (16.00); 1.3555 (3.36); 1.1085 (0.59); 1.0910 (1.17); 1.0735 (0.57); 0.0079 (0.35); -0.0002 (9.13); - 0.0083 (0.54) |
| Beispiel Nr. 128, Lösungsmittel: DMSO-d6, NMR-Spektrometer: 399.95 MHz |
| 11.3318 (2.96); 7.6406 (0.88); 7.6346 (1.71); 7.6182 (5.11); 7.4729 (2.47); 7.4661 (0.63); 7.4566 (0.60); 7.4496 (1.91); 6.2934 (0.75); 6.2784 (1.53); 6.2631 (0.74); 5.9626 (1.75); 5.9431 (1.77); 4.2644 (3.37); 4.2492 (3.30); 4.0038 (12.77); 3.9040 (9.72); 3.7017 (0.65); 3.6846 (1.01); 3.6675 (0.98); 3.6499 (0.64); 3.3952 (0.45); 3.3859 (0.61); 3.3392 (297.43); 3.1744 (0.83); 3.1613 (0.78); 2.6758 (0.68); 2.6718 (0.86); 2.5069 (111.06); 2.5027 (136.25); 2.4986 (100.09); 2.3292 (0.81); 1.3510 (0.34); 1.2579 (0.61); 1.2354 (2.45); 1.0450 (16.00); 1.0287 (15.74); 0.8538 (0.46); 0.0076 (1.42); -0.0002 (27.81) |
| Beispiel Nr. 129, Lösungsmittel: DMSO-d6, NMR-Spektrometer: 399.95 MHz |
| 11.3562 (3.73); 8.7487 (4.05); 8.6499 (0.60); 7.7423 (1.50); 7.7361 (1.65); 7.7207 (1.80); 7.7145 (2.04); 7.6249 (3.28); 7.6189 (3.00); 7.5094 (3.93); 7.4878 (3.32); 7.4554 (0.77); 7.4355 (0.96); 7.4117 (3.95); 7.3919 (4.85); 7.2955 (0.59); 7.2766 (0.88); 7.2562 (0.55); 7.2345 (2.94); 7.2155 (4.58); 7.1953 (2.68); 6.9645 (0.45); 6.9118 (1.38); 6.8936 (2.44); 6.8754 (1.10); 6.7108 (1.03); 6.6960 (2.25); 6.6807 (1.08); 4.3742 (4.05); 4.3594 (4.02); 3.9913 (16.00); 3.9039 (12.54); 3.3321 (173.56); 3.1737 (1.07); 3.1611 (0.97); 2.6708 (1.07); 2.6667 (0.82); 2.5062 (132.52); 2.5020 (168.05); 2.4978 (126.55); 2.3328 (0.80); 2.3286 (1.05); 2.3247 (0.79); 1.2352 (0.67); 0.0073 (1.87); - 0.0002 (36.77); -0.0077 (2.19) |
| Beispiel Nr. 130, Lösungsmittel: DMSO-d6, NMR-Spektrometer: 399.95 MHz |
| 11.3572 (3.65); 7.6668 (6.85); 7.6469 (2.26); 7.6406 (1.45); 7.4814 (2.98); 7.4605 (2.41); 7.3091 (1.16); 7.2901 (3.90); 7.2729 (6.97); 7.2648 (6.81); 7.2487 (1.98); 7.2260 (1.06); 7.2218 (1.30); 7.2160 (0.77); 7.2048 (1.88); 7.1978 (0.53); 7.1941 (0.47); 7.1881 (0.63); 6.6389 (1.04); 6.6241 (2.10); 6.6092 (1.05); 6.5749 (0.99); 6.5598 (2.06); 6.5445 (0.99); 4.3114 (4.12); 4.2964 (4.11); 4.2414 (4.82); 4.2265 (4.77); 4.0116 (16.00); 3.9039 (13.44); 3.3333 (252.55); 3.1738 (0.84); 3.1612 (0.77); 2.6756 (0.92); 2.6712 (1.20); 2.6671 (0.91); 2.5064 (151.53); 2.5021 (189.16); 2.4978 (138.61); 2.3329 (0.86); 2.3287 (1.14); 2.3246 (0.84); 1.2355 (0.94); 0.0075 (2.09); -0.0002 (42.05); - 0.0076 (1.94); -0.0084 (1.94) |
| Beispiel Nr. 131, Lösungsmittel: DMSO-d6, NMR-Spektrometer: 601.6 MHz |
| 11.2664 (1.07); 8.6900 (0.51); 7.6064 (0.45); 7.6020 (0.36); 7.5954 (0.46); 7.5910 (0.33); 7.2681 (0.46); 7.2526 (0.72); 7.2374 (0.44); 4.3256 (1.07); 4.3160 (1.07); 4.0177 (4.76); 3.6104 (8.67); 3.3470 (23.76); 3.3043 (4.50); 2.5095 (2.11); 2.5066 (4.65); 2.5035 (6.44); 2.5005 (4.67); 2.4975 (2.12); 2.0772 (16.00); 1.3974 (1.09); -0.0002 (1.49) |
| Beispiel Nr. 132, Lösungsmittel: DMSO-d6, NMR-Spektrometer: 399.95 MHz |
| 11.2467 (1.58); 8.6236 (0.36); 8.6088 (0.75); 8.5943 (0.37); 7.6692 (0.33); 7.6621 (0.41); 7.6577 (0.39); 7.6507 (0.44); 7.6468 (0.39); 7.6400 (0.49); 7.6356 (0.39); 7.6288 (0.41); 7.5916 (0.68); 7.5850 (0.60); 7.5751 (0.70); 7.5683 (0.57); 7.2684 (0.79); 7.2449 (1.17); 7.2220 (0.73); 4.3267 (1.49); 4.3125 (1.51); 4.0566 (1.13); 4.0388 (3.52); |
| 4.0210 (3.71); 4.0115 (7.14); 4.0033 (1.38); 3.3300 (10.18); 3.1944 (6.38); 2.5066 (6.27); 2.5025 (8.55); 1.9889 (16.00); 1.9096 (0.70); 1.1931 (4.50); 1.1753 (9.00); 1.1575 (4.41); -0.0002 (2.57) |
| Beispiel Nr. 133, Lösungsmittel: DMSO-d6, NMR-Spektrometer: 399.95 MHz |
| 11.2440 (3.52); 8.5788 (0.87); 8.5644 (1.76); 8.5508 (0.86); 7.9261 (0.97); 7.9165 (0.96); 7.6470 (0.75); 7.6406 (0.92); 7.6357 (0.86); 7.6290 (1.05); 7.6180 (1.16); 7.6142 (0.91); 7.6070 (1.01); 7.5930 (1.58); 7.5861 (1.27); 7.5763 (1.64); 7.5696 (1.23); 7.2630 (1.81); 7.2393 (2.54); 7.2167 (1.71); 5.7542 (1.16); 4.3216 (3.47); 4.3071 (3.51); 4.0167 (16.00); 3.4604 (0.42); 3.3893 (0.38); 3.3359 (653.72); 3.3128 (3.39); 3.3002 (0.69); 3.0727 (12.29); 2.6759 (0.52); 2.6714 (0.74); 2.6667 (0.53); 2.5875 (12.14); 2.5759 (12.09); 2.5414 (0.38); 2.5246 (1.25); 2.5200 (1.94); 2.5064 (76.52); 2.5023 (104.28); 2.3337 (0.52); 2.3288 (0.79); 2.3242 (0.47); 2.0732 (3.90); 0.0080 (0.55); - 0.0002 (18.59); -0.0085 (0.59) |
| Beispiel Nr. 134, Lösungsmittel: DMSO-d6, NMR-Spektrometer: 399.95 MHz |
| 11.2572 (3.55); 9.0735 (0.87); 9.0580 (1.96); 9.0436 (0.94); 7.8101 (1.32); 7.8035 (1.47); 7.7932 (1.45); 7.7867 (1.44); 7.5871 (0.78); 7.5803 (0.86); 7.5757 (0.95); 7.5689 (0.95); 7.5650 (1.01); 7.5580 (1.03); 7.5539 (1.04); 7.5469 (0.90); 7.5329 (0.54); 7.5224 (1.66); 7.5191 (1.98); 7.5027 (4.37); 7.4995 (5.07); 7.4856 (2.49); 7.4812 (3.09); 7.4771 (1.78); 7.4723 (1.67); 7.4587 (2.99); 7.4538 (1.92); 7.4394 (1.93); 7.4342 (1.38); 7.4231 (2.49); 7.4193 (2.48); 7.4049 (2.62); 7.4012 (2.53); 7.3866 (0.90); 7.3831 (0.84); 7.2877 (1.87); 7.2641 (2.61); 7.2413 (1.67); 4.4953 (3.44); 4.4805 (3.51); 4.0169 (16.00); 3.3317 (43.97); 3.3264 (60.27); 2.6706 (0.33); 2.5238 (0.59); 2.5057 (33.63); 2.5016 (45.59); 2.4982 (30.85); 1.9886 (0.38); 1.3557 (0.34); -0.0002 (8.03) |
| Beispiel Nr. 135, Lösungsmittel: DMSO-d6, NMR-Spektrometer: 399.95 MHz |
| 11.2213 (3.58); 9.0259 (0.89); 9.0112 (1.91); 8.9969 (0.92); 7.8437 (0.39); 7.8223 (5.41); 7.8018 (5.72); 7.7271 (0.71); 7.7202 (0.88); 7.7159 (0.92); 7.7088 (0.92); 7.6977 (1.01); 7.6867 (0.84); 7.5799 (1.43); 7.5733 (1.37); 7.5635 (1.49); 7.5570 (1.34); 7.2955 (4.58); 7.2756 (6.03); 7.2518 (2.57); 7.2288 (1.70); 4.5181 (3.36); 4.5033 (3.42); 4.0387 (0.96); 4.0209 (0.98); 4.0030 (0.36); 3.9773 (16.00); 3.3438 (50.18); 3.3356 (82.73); 2.5069 (23.89); 2.5028 (32.10); 2.3705 (1.22); 2.3516 (15.03); 2.3294 (0.33); 2.0739 (0.71); 1.9890 (4.34); 1.3976 (4.12); 1.1930 (1.17); 1.1752 (2.41); 1.1574 (1.14); -0.0002 (2.83) |
| Beispiel Nr. 136, Lösungsmittel: DMSO-d6, NMR-Spektrometer: 399.95 MHz |
| 11.2250 (3.62); 9.2394 (0.87); 9.2246 (1.93); 9.2099 (0.92); 7.9531 (2.07); 7.9488 (3.81); 7.9441 (2.47); 7.9001 (0.54); 7.8952 (0.45); 7.8907 (0.37); 7.8750 (2.01); 7.8556 (2.23); 7.7238 (0.73); 7.7133 (0.98); 7.7064 (1.05); 7.6953 (1.13); 7.6841 (0.89); 7.6428 (1.15); 7.6405 (1.34); 7.6377 (1.22); 7.6354 (1.19); 7.6232 (1.75); 7.6205 (1.86); 7.6178 (2.03); 7.5954 (1.52); 7.5888 (1.42); 7.5784 (1.63); 7.5721 (1.36); 7.5619 (0.32); 7.5518 (2.84); 7.5415 (0.42); 7.5323 (4.09); 7.5125 (1.77); 7.2871 (1.79); 7.2633 (2.69); 7.2403 (1.70); 4.5305 (3.54); 4.5163 (3.53); 3.9854 (16.00); 3.3429 (79.18); 3.3334 (167.44); 3.2896 (0.45); 3.2778 (0.32); 2.6709 (0.45); 2.6666 (0.35); 2.5249 (0.74); 2.5066 (50.08); 2.5026 (66.48); 2.3338 (0.36); 2.3292 (0.51); 2.3250 (0.40); 2.0740 (0.37); -0.0002 (1.19) |
| Beispiel Nr. 137, Lösungsmittel: DMSO-d6, NMR-Spektrometer: 399.95 MHz |
| 11.5084 (3.09); 8.8912 (8.70); 8.5806 (1.04); 8.5659 (2.04); 8.5508 (1.02); 8.3152 (0.94); 8.2597 (8.50); 8.2045 (0.44); 8.0457 (0.32); 7.9225 (0.43); 7.9013 (0.93); 7.8724 (0.95); 7.8532 (1.78); 7.8480 (1.84); 7.8322 (2.00); 7.8264 (2.03); 7.8147 (0.34); 7.7052 (3.21); 7.6997 (2.84); 7.5316 (3.62); 7.5102 (3.31); 4.2052 (4.19); 4.1904 (4.25); 4.0505 (16.00); 3.4151 (0.49); 3.3692 (121.47); 3.3576 (285.99); 2.6842 (0.49); 2.5195 (56.23); 2.5156 (71.61); 2.3419 (0.41); 2.0988 (12.34); 1.6428 (0.40); 1.6238 (0.87); 1.6113 (1.56); 1.5977 (0.86); 1.5930 (0.92); 1.5798 (0.49); 1.3265 (0.47); 0.6969 (4.59); 0.6871 (6.59); 0.6683 (3.58); 0.6461 (0.34) |
| Beispiel Nr. 138, Lösungsmittel: DMSO-d6, NMR-Spektrometer: 399.95 MHz |
| 11.2572 (1.47); 8.5964 (0.56); 8.5821 (1.01); 8.5704 (0.57); 8.1598 (3.55); 8.1564 (3.51); 8.1322 (0.47); 7.6463 (0.69); 7.6399 (1.05); 7.6351 (0.83); 7.6284 (1.17); 7.6142 (2.52); 7.6069 (1.97); 7.5979 (1.72); 7.5913 (0.97); 7.2703 (1.46); 7.2467 (2.11); 7.2243 (1.41); 4.3974 (0.33); 4.3831 (0.35); 4.3506 (3.61); 4.3357 (3.67); 4.0081 (16.00); 3.3422 (55.72); 3.3389 (63.35); 3.3332 (69.53); 3.3310 (84.34); 3.3276 (103.77); 2.8909 (0.48); 2.7311 (0.38); 2.6709 (0.35); 2.5244 (0.58); 2.5063 (41.53); 2.5021 (55.30); 2.4982 (38.55); 2.0859 (0.66); 2.0734 (0.36); 1.9888 (0.43); 1.2358 (0.68); 0.0082 (0.36); -0.0002 (12.94); -0.0086 (0.39) |
| Beispiel Nr. 139, Lösungsmittel: DMSO-d6, NMR-Spektrometer: 399.95 MHz |
| 11.2621 (3.63); 9.0523 (6.93); 8.8085 (0.84); 8.7938 (1.78); 8.7794 (0.88); 8.6894 (13.86); 7.6674 (1.29); 7.6609 (1.60); 7.6508 (1.39); 7.6442 (1.58); 7.6089 (0.86); 7.6019 (0.81); 7.5976 (1.02); 7.5904 (0.90); 7.5870 (1.06); 7.5795 (1.00); 7.5756 (1.04); 7.5687 (0.81); 7.2672 (1.75); 7.2438 (2.63); 7.2292 (0.33); 7.2209 (1.58); 4.3400 (3.55); 4.3256 (3.59); 4.0213 (16.00); 3.5913 (10.26); 3.3365 (37.04); 2.5073 (17.19); 2.5034 (22.90); -0.0002 (0.90) |
| Beispiel Nr. 140, Lösungsmittel: DMSO-d6, NMR-Spektrometer: 399.95 MHz |
| 11.2488 (3.47); 9.5233 (0.81); 9.5079 (1.69); 9.4929 (0.83); 7.7094 (0.78); 7.7024 (0.94); 7.6980 (0.93); 7.6911 (0.99); 7.6873 (0.96); 7.6802 (1.08); 7.6760 (0.97); 7.6690 (0.91); 7.5454 (1.48); 7.5389 (1.47); 7.5288 (1.58); 7.5223 (1.39); 7.2735 (1.77); 7.2500 (2.68); 7.2270 (1.64); 4.3876 (3.75); 4.3725 (3.80); 4.2707 (2.10); 4.2530 (6.71); 4.2352 (6.79); 4.2174 (2.21); 4.2096 (0.37); 4.0032 (16.00); 3.5679 (0.55); 3.3299 (130.69); 3.2026 (0.34); 2.6753 (0.52); 2.6708 (0.72); 2.6663 (0.53); 2.5411 (0.48); 2.5243 (1.36); 2.5108 (38.05); 2.5063 (79.58); 2.5017 (108.11); 2.4971 (80.68); 2.4926 (41.10); 2.3330 (0.51); 2.3285 (0.71); 2.3238 (0.53); 2.0737 (0.65); 1.9885 (0.50); 1.3551 (0.63); 1.2853 (7.18); 1.2675 (15.25); 1.2589 (1.06); 1.2497 (7.16); 1.2409 (1.21); 1.2231 (0.45); 0.0080 (0.38); -0.0002 (14.01); -0.0085 (0.63) |
| Beispiel Nr. 141, Lösungsmittel: DMSO-d6, NMR-Spektrometer: 399.95 MHz |
| 11.2346 (3.55); 9.3377 (0.92); 9.3219 (1.99); 9.3061 (0.95); 8.7798 (0.52); 8.7685 (1.48); 8.7562 (1.50); 8.7448 (0.57); 8.2572 (0.35); 7.7235 (0.80); 7.7166 (0.96); 7.7125 (0.98); 7.7051 (1.02); 7.7019 (1.04); 7.6943 (1.09); 7.6907 (1.03); 7.6833 (0.91); 7.4783 (1.51); 7.4718 (1.56); 7.4617 (1.63); 7.4552 (1.49); 7.2605 (1.92); 7.2368 (2.83); 7.2140 (1.79); 4.3874 (3.75); 4.3717 (3.80); 4.3243 (1.14); 4.2789 (0.60); 4.2613 (1.11); 4.2438 (0.69); 4.0387 (0.71); 4.0209 (0.76); 3.9964 (16.00); 3.5689 (2.60); 3.4580 (0.37); 3.4424 (0.41); 3.3645 (89.30); 3.3627 (84.42); 3.3564 (83.98); 3.3499 (133.12); 2.6866 (11.27); 2.6742 (11.82); 2.6675 (8.37); 2.6612 (1.36); 2.6551 (7.64); 2.6458 |
| (0.92); 2.5264 (0.43); 2.5217 (0.67); 2.5131 (11.74); 2.5086 (24.75); 2.5040 (33.80); 2.4994 (25.19); 2.4949 (12.77); 2.4447 (0.38); 2.4251 (0.82); 2.4042 (0.53); 2.1627 (0.34); 2.1426 (0.39); 1.9891 (2.85); 1.2592 (0.50); 1.2413 (0.36); 1.1931 (0.77); 1.1753 (1.59); 1.1696 (0.80); 1.1575 (0.78); -0.0002 (0.47) |
| Beispiel Nr. 142, Lösungsmittel: DMSO-d6, NMR-Spektrometer: 399.95 MHz |
| 8.4968 (0.69); 8.4823 (1.38); 8.4678 (0.77); 8.4473 (0.33); 8.4329 (0.55); 8.4185 (0.34); 7.6239 (0.79); 7.6172 (1.13); 7.6129 (0.96); 7.6059 (1.26); 7.5935 (2.86); 7.5852 (1.91); 7.5763 (2.05); 7.2471 (1.46); 7.2246 (2.44); 7.2009 (1.49); 6.8716 (0.80); 4.3081 (3.59); 4.2941 (3.83); 4.0584 (0.60); 4.0045 (16.00); 3.5723 (0.32); 3.5682 (0.41); 3.3333 (23.93); 2.9950 (0.32); 2.9808 (0.34); 2.7855 (2.31); 2.7688 (4.98); 2.7523 (2.59); 2.6758 (0.50); 2.6714 (0.59); 2.6667 (0.41); 2.5245 (0.97); 2.5111 (33.90); 2.5067 (59.40); 2.5022 (68.65); 2.4976 (46.46); 2.4932 (20.25); 2.4129 (0.90); 2.3951 (1.60); 2.3772 (0.85); 2.3381 (0.40); 2.3334 (0.53); 2.3289 (0.63); 2.3243 (0.52); 2.2847 (0.33); 2.2524 (2.64); 2.2359 (5.04); 2.2192 (2.37); 2.1835 (1.41); 2.0953 (0.92); 2.0900 (1.13); 2.0860 (3.18); 2.0739 (0.48); 2.0416 (1.24); 1.9888 (0.83); 1.8559 (2.80); 1.8528 (3.83); 1.7558 (4.84); 1.3555 (9.08); 1.2354 (0.53); 1.1748 (0.44); 0.0038 (5.55); -0.0002 (17.52); -0.0085 (0.57) |
| Beispiel Nr. 143, Lösungsmittel: DMSO-d6, NMR-Spektrometer: 399.95 MHz |
| 11.2407 (3.58); 9.4872 (1.04); 9.4728 (1.97); 9.4589 (1.01); 9.3389 (5.91); 9.2112 (13.41); 7.7259 (0.82); 7.7188 (1.09); 7.7068 (1.20); 7.6970 (1.29); 7.6865 (0.97); 7.6520 (1.71); 7.6464 (1.61); 7.6356 (1.80); 7.6296 (1.47); 7.3037 (1.57); 7.2803 (2.67); 7.2572 (1.44); 4.5742 (4.23); 4.5602 (4.23); 3.9946 (16.00); 3.3319 (197.80); 2.6721 (0.39); 2.5418 (1.02); 2.5026 (60.22); 2.3298 (0.38); 1.2358 (1.07); -0.0002 (3.23) |
| Beispiel Nr. 144, Lösungsmittel: DMSO-d6, NMR-Spektrometer: 399.95 MHz |
| 11.2362 (3.45); 9.4120 (0.86); 9.3976 (1.81); 9.3835 (0.89); 9.0013 (4.16); 8.9968 (4.20); 8.8129 (3.83); 8.8070 (3.90); 8.3465 (2.74); 8.3412 (3.94); 8.3359 (2.63); 7.7215 (0.72); 7.7148 (0.93); 7.7104 (0.90); 7.7030 (0.99); 7.6996 (0.92); 7.6924 (1.08); 7.6884 (0.94); 7.6814 (0.89); 7.6328 (1.49); 7.6264 (1.39); 7.6162 (1.56); 7.6096 (1.30); 7.2988 (1.71); 7.2753 (2.59); 7.2523 (1.57); 4.5560 (3.63); 4.5418 (3.63); 3.9924 (16.00); 3.4301 (0.33); 3.4223 (0.35); 3.4128 (0.35); 3.3464 (495.14); 3.3004 (0.75); 2.6767 (0.32); 2.6725 (0.44); 2.6679 (0.32); 2.5426 (1.00); 2.5123 (26.01); 2.5079 (53.21); 2.5034 (70.38); 2.4988 (50.44); 2.4944 (24.12); 2.3346 (0.32); 2.3301 (0.45); 2.3256 (0.33); 1.2581 (0.37); 1.2351 (1.24); -0.0002 (2.87) |
| Beispiel Nr. 145, Lösungsmittel: DMSO-d6, NMR-Spektrometer: 399.95 MHz |
| 11.2299 (1.18); 9.4872 (0.47); 9.4732 (0.91); 9.4588 (0.47); 9.3385 (3.53); 9.2290 (0.97); 9.2108 (8.96); 9.2003 (1.05); 8.9503 (3.05); 8.9455 (3.02); 8.1373 (1.90); 8.1315 (1.86); 8.1171 (2.01); 8.1113 (1.97); 7.7147 (1.47); 7.7103 (1.32); 7.7033 (1.55); 7.6926 (1.65); 7.6520 (0.85); 7.6453 (0.78); 7.6354 (0.89); 7.6290 (0.75); 7.6100 (1.57); 7.6038 (1.54); 7.5936 (1.68); 7.5875 (1.41); 7.3866 (2.85); 7.3662 (2.75); 7.3034 (0.90); 7.2868 (1.86); 7.2802 (1.59); 7.2632 (2.78); 7.2402 (1.60); 4.5738 (1.99); 4.5593 (2.14); 4.5399 (3.78); 4.5257 (3.72); 4.0193 (0.55); 3.9938 (8.76); 3.9833 (16.00); 3.3331 (422.50); 2.6759 (0.66); 2.6714 (0.80); 2.6670 (0.60); 2.5416 (24.01); 2.5202 (22.24); 2.5067 (101.28); 2.5023 (130.63); 2.4979 (96.40); 2.4562 (0.75); 2.3333 (0.71); 2.3291 (0.91); 2.3246 (0.71); 2.2924 (0.93); 2.1003 (0.33); 2.0090 (0.51); 1.9899 |
| (0.58); 1.9757 (0.43); 1.4724 (0.34); 1.4573 (0.34); 1.2978 (0.62); 1.2581 (1.62); 1.2357 (5.78); 1.1876 (0.33); 0.8696 (0.45); 0.8537 (1.11); 0.8363 (0.55); -0.0002 (8.84); - 0.0083 (0.41) |
| Beispiel Nr. 146, Lösungsmittel: DMSO-d6, NMR-Spektrometer: 399.95 MHz |
| 11.2941 (2.09); 11.2486 (4.56); 9.3589 (1.23); 9.3443 (2.81); 9.3287 (2.01); 9.3129 (0.60); 8.7396 (3.52); 8.6378 (2.32); 8.6257 (2.68); 8.6193 (3.78); 8.6065 (3.80); 7.8486 (0.75); 7.8436 (0.75); 7.8325 (0.79); 7.7431 (0.91); 7.7363 (1.21); 7.7250 (1.31); 7.7136 (1.53); 7.7021 (5.11); 7.6288 (4.02); 7.6161 (4.21); 7.5986 (0.69); 7.5876 (0.64); 7.5328 (1.84); 7.5208 (1.79); 7.3092 (0.95); 7.2971 (2.00); 7.2859 (1.64); 7.2737 (3.24); 7.2630 (0.99); 7.2506 (1.78); 4.5630 (4.48); 4.5487 (5.92); 4.5343 (2.14); 4.0432 (7.83); 4.0103 (16.00); 3.3933 (2.07); 2.5505 (53.73); 2.5147 (3.89); 2.5104 (5.12); 2.5062 (3.99); 1.2394 (0.65) |
| Beispiel Nr. 147, Lösungsmittel: DMSO-d6, NMR-Spektrometer: 399.95 MHz |
| 11.2724 (3.48); 11.2287 (2.49); 9.3485 (0.62); 9.3342 (1.29); 9.3186 (1.39); 9.3030 (1.90); 9.2884 (0.90); 8.7291 (6.47); 8.6226 (4.44); 8.6146 (2.37); 8.6106 (4.78); 8.6025 (2.11); 7.8204 (1.39); 7.8140 (1.52); 7.8039 (1.47); 7.7973 (1.41); 7.7129 (0.58); 7.7058 (0.80); 7.6944 (3.16); 7.6839 (0.89); 7.6728 (0.63); 7.6184 (1.44); 7.6065 (2.20); 7.5964 (1.25); 7.5897 (1.70); 7.5826 (0.96); 7.5783 (0.97); 7.5709 (0.96); 7.5676 (1.01); 7.5603 (1.04); 7.5562 (1.00); 7.5494 (0.80); 7.5108 (3.62); 7.5099 (3.57); 7.4988 (3.54); 7.4979 (3.46); 7.3021 (1.77); 7.2909 (1.35); 7.2786 (2.68); 7.2676 (1.95); 7.2557 (1.61); 7.2444 (1.15); 7.2039 (1.03); 7.0761 (1.06); 6.9483 (0.99); 4.5388 (2.69); 4.5228 (5.72); 4.5074 (3.69); 4.0202 (16.00); 3.9881 (11.54); 3.4970 (0.37); 3.3598 (28.34); 2.9956 (0.47); 2.6757 (0.35); 2.6714 (0.47); 2.6667 (0.35); 2.5416 (67.66); 2.5245 (1.38); 2.5111 (30.23); 2.5068 (60.79); 2.5022 (79.54); 2.4976 (56.68); 2.4932 (27.01); 2.3335 (0.41); 2.3290 (0.52); 2.3245 (0.40); 2.2916 (0.34); 2.0098 (0.33); 1.9910 (0.34); 1.2586 (0.66); 1.2360 (2.79); 0.8540 (0.59); -0.0002 (7.08) |
| Beispiel Nr. 148, Lösungsmittel: DMSO-d6, NMR-Spektrometer: 399.95 MHz |
| 11.2312 (3.41); 9.4118 (0.87); 9.3971 (1.80); 9.3826 (0.87); 8.7572 (4.76); 8.7458 (3.24); 8.7422 (4.77); 7.8111 (5.77); 7.8071 (3.41); 7.7998 (3.50); 7.7959 (5.41); 7.7188 (0.74); 7.7119 (0.94); 7.7077 (0.90); 7.7004 (1.02); 7.6895 (1.08); 7.6788 (0.84); 7.6149 (1.52); 7.6084 (1.41); 7.5983 (1.60); 7.5917 (1.32); 7.2934 (1.65); 7.2702 (2.60); 7.2469 (1.50); 7.2027 (0.62); 7.0750 (0.61); 6.9471 (0.58); 4.5515 (3.79); 4.5372 (3.76); 3.9859 (16.00); 3.3305 (133.59); 2.6756 (0.37); 2.6712 (0.49); 2.6669 (0.35); 2.5414 (26.50); 2.5066 (61.12); 2.5022 (78.42); 2.4978 (55.73); 2.3333 (0.37); 2.3290 (0.49); 2.3243 (0.36); 1.2360 (2.09); 0.8542 (0.40); -0.0002 (5.39) |
| Beispiel Nr. 149, Lösungsmittel: DMSO-d6, NMR-Spektrometer: 399.95 MHz |
| 11.2352 (3.33); 7.6260 (1.33); 7.6197 (1.49); 7.6092 (1.38); 7.6029 (1.38); 7.4982 (1.00); 7.4831 (2.16); 7.4678 (1.79); 7.4569 (1.48); 7.4513 (4.42); 7.4465 (2.48); 7.4352 (3.03); 7.4300 (8.73); 7.3989 (1.34); 7.3935 (8.26); 7.3883 (2.00); 7.3770 (1.37); 7.3720 (3.74); 7.2110 (1.77); 7.1874 (2.53); 7.1644 (1.49); 4.2524 (3.55); 4.2377 (3.50); 4.0405 (16.00); 3.4109 (0.50); 3.3989 (0.70); 3.3446 (554.85); 3.3204 (5.37); 3.3000 (0.88); 3.2842 (0.44); 2.6766 (0.36); 2.6719 (0.50); 2.6674 (0.36); 2.5421 (13.46); 2.5116 (29.06); 2.5073 (59.48); 2.5028 (79.09); 2.4983 (57.01); 2.4940 (27.56); 2.3340 (0.36); 2.3295 (0.48); 2.3249 (0.38); 2.0101 (0.45); 1.9910 (0.45); 1.3999 (1.71); |
| 1.3900 (4.32); 1.3829 (4.70); 1.3738 (1.90); 1.2570 (0.77); 1.2357 (3.13); 1.0249 (1.89); 1.0155 (4.52); 1.0083 (4.49); 0.9984 (1.67); 0.8540 (0.72); -0.0002 (0.90) |
| Beispiel Nr. 150, Lösungsmittel: DMSO-d6, NMR-Spektrometer: 399.95 MHz |
| 11.2248 (3.19); 8.6333 (0.90); 8.6189 (1.87); 8.6044 (0.89); 7.6644 (1.36); 7.6578 (1.53); 7.6476 (1.44); 7.6411 (1.46); 7.5513 (0.82); 7.5443 (0.86); 7.5403 (0.97); 7.5294 (1.04); 7.5220 (1.04); 7.5182 (1.03); 7.5113 (0.78); 7.4592 (1.68); 7.4545 (1.89); 7.4403 (2.03); 7.4357 (2.22); 7.4142 (1.88); 7.4104 (1.79); 7.3953 (2.49); 7.3911 (2.50); 7.3090 (0.75); 7.3053 (0.91); 7.2908 (2.20); 7.2867 (2.16); 7.2723 (1.89); 7.2673 (3.10); 7.2619 (2.08); 7.2480 (2.13); 7.2430 (3.36); 7.2294 (0.85); 7.2245 (0.97); 7.2188 (2.65); 7.1959 (1.53); 4.3482 (0.33); 4.3239 (1.72); 4.3098 (3.18); 4.2960 (1.69); 4.0868 (0.62); 4.0691 (2.15); 4.0513 (2.20); 4.0336 (0.73); 4.0119 (16.00); 3.3932 (0.51); 3.3403 (487.98); 3.3160 (5.05); 3.2922 (0.78); 3.2681 (0.40); 2.6761 (0.37); 2.6717 (0.51); 2.6670 (0.36); 2.5417 (0.63); 2.5246 (1.27); 2.5113 (30.40); 2.5070 (61.78); 2.5025 (81.75); 2.4980 (58.83); 2.4937 (28.50); 2.3337 (0.40); 2.3292 (0.53); 2.3248 (0.39); 1.3998 (8.89); 1.3821 (8.82); 1.3554 (0.32); 1.2580 (0.39); 1.2359 (1.32); - 0.0002 (4.52) |
| Beispiel Nr. 151, Lösungsmittel: DMSO-d6, NMR-Spektrometer: 399.95 MHz |
| 11.2314 (2.60); 8.6280 (0.87); 8.6135 (1.74); 8.5989 (0.85); 7.6508 (1.31); 7.6444 (1.55); 7.6343 (1.44); 7.6277 (1.45); 7.5579 (0.85); 7.5509 (0.85); 7.5467 (0.98); 7.5361 (1.04); 7.5287 (1.02); 7.5250 (1.03); 7.5180 (0.79); 7.3581 (1.93); 7.3537 (3.58); 7.3391 (0.79); 7.3256 (0.86); 7.3157 (1.84); 7.3021 (3.74); 7.2873 (5.40); 7.2835 (4.89); 7.2728 (1.58); 7.2680 (1.44); 7.2646 (1.36); 7.2600 (0.98); 7.2479 (1.70); 7.2245 (2.58); 7.2015 (1.53); 4.3707 (0.69); 4.3556 (0.72); 4.3323 (1.29); 4.3169 (1.27); 4.2535 (1.26); 4.2400 (1.32); 4.2153 (0.70); 4.2010 (0.70); 4.0048 (16.00); 3.7320 (0.59); 3.7145 (2.03); 3.6968 (2.07); 3.6792 (0.62); 3.3420 (492.26); 3.3411 (477.77); 3.3179 (4.60); 3.2799 (0.36); 2.6762 (0.36); 2.6718 (0.49); 2.6674 (0.36); 2.5419 (0.43); 2.5248 (1.30); 2.5115 (29.24); 2.5072 (59.51); 2.5027 (78.67); 2.4982 (56.38); 2.4938 (27.07); 2.3342 (0.37); 2.3294 (0.50); 2.3250 (0.36); 2.2395 (0.51); 1.3731 (8.60); 1.3554 (8.53); 1.3283 (0.36); 1.2578 (0.41); 1.2356 (1.35); -0.0002 (4.74) |
| Beispiel Nr. 152, Lösungsmittel: DMSO-d6, NMR-Spektrometer: 399.95 MHz |
| 11.2246 (2.72); 8.5155 (0.88); 8.5011 (1.74); 8.4867 (0.85); 7.6416 (1.33); 7.6353 (1.52); 7.6251 (1.40); 7.6185 (1.42); 7.5559 (0.81); 7.5488 (0.83); 7.5447 (0.95); 7.5340 (1.02); 7.5266 (1.01); 7.5228 (1.01); 7.5158 (0.77); 7.3382 (0.73); 7.2407 (1.68); 7.2171 (2.84); 7.2086 (4.16); 7.1937 (3.01); 7.1885 (5.74); 7.0873 (4.79); 7.0674 (3.49); 4.3489 (0.66); 4.3335 (0.68); 4.3101 (1.36); 4.2951 (1.30); 4.2435 (1.29); 4.2298 (1.33); 4.2050 (0.66); 4.1912 (0.65); 4.0069 (16.00); 3.6557 (0.58); 3.6382 (1.89); 3.6206 (1.93); 3.6027 (0.61); 3.4129 (0.38); 3.3410 (469.93); 3.3170 (4.46); 2.6760 (0.35); 2.6715 (0.49); 2.6669 (0.36); 2.5417 (1.51); 2.5245 (1.23); 2.5112 (28.59); 2.5069 (58.23); 2.5024 (77.19); 2.4980 (55.71); 2.4937 (27.02); 2.3334 (0.36); 2.3291 (0.49); 2.3247 (0.37); 2.2559 (0.49); 2.2393 (14.97); 1.3458 (8.57); 1.3282 (8.29); 1.2357 (0.96); -0.0002 (4.83) |
| Beispiel Nr. 153, Lösungsmittel: DMSO-d6, NMR-Spektrometer: 399.95 MHz |
| 11.2260 (1.19); 8.6106 (0.43); 8.5960 (0.87); 8.5816 (0.42); 7.6501 (0.66); 7.6437 (0.75); 7.6333 (0.69); 7.6270 (0.69); 7.5356 (0.40); 7.5286 (0.43); 7.5246 (0.47); 7.5137 |
| (0.49); 7.5064 (0.50); 7.5025 (0.49); 7.4955 (0.39); 7.3384 (16.00); 7.2444 (0.82); 7.2210 (1.25); 7.1980 (0.71); 4.3118 (0.72); 4.2968 (0.71); 4.2673 (0.68); 4.2534 (0.68); 4.0069 (7.65); 3.7062 (1.10); 3.6885 (1.12); 3.6709 (0.33); 3.3949 (0.33); 3.3419 (230.30); 3.3184 (2.28); 3.3004 (0.43); 3.2926 (0.34); 2.5115 (13.80); 2.5072 (27.58); 2.5027 (36.11); 2.4982 (25.89); 2.4939 (12.48); 1.3635 (4.11); 1.3458 (4.07); 1.2354 (0.45); -0.0002 (2.09) |
| Beispiel Nr. 154, Lösungsmittel: DMSO-d6, NMR-Spektrometer: 399.95 MHz |
| 11.2188 (1.88); 8.3852 (0.87); 8.3704 (1.78); 8.3557 (0.89); 7.6209 (1.30); 7.6145 (1.62); 7.6043 (1.36); 7.5979 (1.58); 7.5751 (0.90); 7.5681 (0.84); 7.5638 (1.03); 7.5533 (1.07); 7.5458 (1.00); 7.5420 (1.06); 7.5351 (0.75); 7.2491 (1.78); 7.2256 (2.56); 7.2027 (1.55); 4.2955 (3.73); 4.2810 (3.70); 4.0023 (16.00); 3.3404 (412.24); 3.3170 (4.23); 3.2686 (0.33); 3.2508 (0.35); 2.6718 (0.45); 2.6676 (0.32); 2.5419 (1.46); 2.5249 (1.13); 2.5114 (26.04); 2.5071 (52.58); 2.5026 (69.29); 2.4981 (50.18); 2.4937 (24.54); 2.3340 (0.33); 2.3294 (0.44); 2.3252 (0.33); 2.1775 (0.51); 2.1618 (0.81); 2.1571 (0.86); 2.1419 (6.67); 2.1378 (5.93); 2.1224 (0.86); 1.7159 (1.01); 1.7030 (1.39); 1.6869 (1.39); 1.6739 (1.20); 1.6582 (0.63); 1.5775 (0.92); 1.5702 (1.19); 1.5574 (1.76); 1.5504 (1.18); 1.5462 (1.24); 1.5404 (1.47); 1.5335 (0.95); 1.5251 (0.63); 1.5185 (0.70); 1.5058 (1.04); 1.4911 (0.83); 1.4818 (1.44); 1.4725 (1.54); 1.4649 (1.70); 1.4529 (1.36); 1.4353 (0.60); 1.2583 (0.48); 1.2354 (1.72); 1.1634 (0.48); 1.1464 (1.14); 1.1283 (1.43); 1.1160 (1.38); 1.0976 (1.00); 1.0812 (0.40); 0.8537 (0.34); -0.0002 (4.20) |
| Beispiel Nr. 155, Lösungsmittel: DMSO-d6, NMR-Spektrometer: 399.95 MHz |
| 11.2509 (3.05); 8.6124 (0.90); 8.5978 (1.76); 8.5831 (0.87); 7.6565 (1.36); 7.6501 (1.67); 7.6400 (1.43); 7.6334 (1.58); 7.6102 (0.91); 7.6032 (0.89); 7.5988 (1.07); 7.5883 (1.09); 7.5808 (1.04); 7.5769 (1.07); 7.5702 (0.75); 7.4418 (1.94); 7.4343 (2.23); 7.4295 (2.12); 7.4221 (2.13); 7.2613 (1.92); 7.2519 (2.48); 7.2471 (2.37); 7.2383 (2.76); 7.2151 (1.51); 7.0412 (2.56); 7.0385 (2.44); 7.0289 (2.43); 7.0262 (2.26); 4.3178 (3.83); 4.3034 (3.82); 4.0138 (16.00); 3.5007 (10.18); 3.4112 (0.39); 3.3393 (461.21); 3.2709 (0.47); 3.2597 (0.41); 2.6757 (0.39); 2.6714 (0.50); 2.6669 (0.37); 2.5416 (6.23); 2.5068 (62.88); 2.5023 (80.95); 2.4979 (58.67); 2.4938 (29.05); 2.3335 (0.41); 2.3290 (0.53); 2.3246 (0.39); 1.2357 (1.53); -0.0002 (4.74) |
| Beispiel Nr. 156, Lösungsmittel: DMSO-d6, NMR-Spektrometer: 399.95 MHz |
| 11.2619 (2.88); 8.7831 (0.92); 8.7691 (1.68); 8.7551 (0.87); 7.6326 (3.04); 7.6187 (3.02); 7.6023 (1.21); 7.2766 (1.04); 7.2539 (1.86); 7.2296 (0.90); 6.1886 (4.51); 4.3396 (3.60); 4.3259 (3.56); 4.0155 (13.12); 3.7039 (8.91); 3.4661 (0.35); 3.4198 (0.81); 3.3391 (366.01); 3.2427 (0.48); 3.2236 (0.34); 2.6713 (0.49); 2.5413 (1.23); 2.5025 (78.23); 2.3299 (0.53); 2.1750 (16.00); 1.2360 (1.17); -0.0002 (2.95) |
| Beispiel Nr. 157, Lösungsmittel: DMSO-d6, NMR-Spektrometer: 399.95 MHz |
| 11.2530 (3.46); 8.6301 (0.85); 8.6155 (1.69); 8.6011 (0.84); 7.6411 (0.66); 7.6345 (1.21); 7.6302 (0.98); 7.6161 (3.52); 7.6025 (3.41); 7.5177 (2.79); 7.5160 (3.08); 7.5134 (3.06); 7.2679 (1.17); 7.2433 (2.13); 7.2214 (1.17); 6.3671 (2.05); 6.3623 (2.29); 6.3594 (2.46); 6.3546 (2.15); 6.2051 (2.55); 6.2037 (2.59); 6.1973 (2.34); 4.3244 (3.80); 4.3100 (3.83); 4.0121 (16.00); 3.5556 (10.70); 3.3389 (441.09); 2.6761 (0.36); 2.6714 (0.49); 2.6669 (0.36); 2.5417 (6.43); 2.5245 (1.38); 2.5112 (30.90); 2.5069 (61.93); |
| 2.5024 (81.32); 2.4979 (58.83); 2.4936 (28.71); 2.3335 (0.39); 2.3292 (0.52); 2.3249 (0.39); 2.0095 (0.41); 1.9909 (0.41); 1.2361 (2.57); 0.8540 (0.59); -0.0002 (4.87) |
| Beispiel Nr. 158, Lösungsmittel: DMSO-d6, NMR-Spektrometer: 399.95 MHz |
| 11.2435 (3.43); 8.6302 (0.85); 8.6160 (1.64); 8.6018 (0.81); 7.6347 (0.70); 7.6281 (1.17); 7.6237 (0.95); 7.6093 (3.41); 7.5958 (3.29); 7.2650 (1.16); 7.2405 (2.11); 7.2187 (1.20); 6.6751 (2.43); 6.6667 (2.89); 6.5960 (2.27); 6.5934 (2.31); 6.5878 (1.93); 6.5852 (1.76); 4.3085 (3.72); 4.2942 (3.69); 4.0084 (16.00); 3.9929 (0.76); 3.6011 (8.78); 3.4066 (0.85); 3.3992 (0.67); 3.3388 (460.80); 3.3159 (5.00); 2.6758 (0.40); 2.6714 (0.53); 2.6668 (0.38); 2.5501 (0.54); 2.5417 (7.13); 2.5111 (33.88); 2.5068 (66.28); 2.5024 (85.78); 2.4979 (61.46); 2.4936 (29.69); 2.3598 (13.88); 2.3357 (1.13); 2.3290 (0.66); 2.3247 (0.50); 2.1189 (0.35); 2.0096 (0.34); 1.9906 (0.34); 1.2360 (2.13); 0.8542 (0.52); -0.0002 (4.69) |
| Beispiel Nr. 159, Lösungsmittel: DMSO-d6, NMR-Spektrometer: 399.95 MHz |
| 11.2641 (3.50); 8.9271 (0.72); 8.9124 (1.40); 8.8988 (0.74); 7.6621 (0.68); 7.6554 (1.02); 7.6509 (0.87); 7.6438 (1.09); 7.6334 (1.41); 7.6237 (2.44); 7.6080 (1.77); 7.6017 (1.14); 7.2822 (1.58); 7.2589 (2.48); 7.2362 (1.51); 4.3596 (3.57); 4.3456 (3.56); 4.0145 (16.00); 3.9454 (10.51); 3.3303 (155.54); 2.6757 (0.35); 2.6712 (0.47); 2.6665 (0.33); 2.5413 (6.28); 2.5242 (1.27); 2.5109 (29.59); 2.5065 (59.54); 2.5020 (78.08); 2.4974 (55.74); 2.4930 (26.52); 2.3330 (0.36); 2.3286 (0.48); 2.3241 (0.36); 1.2355 (1.15); -0.0002 (4.98) |
| Beispiel Nr. 160, Lösungsmittel: DMSO-d6, NMR-Spektrometer: 399.95 MHz |
| 11.2481 (2.09); 8.6164 (0.56); 8.6019 (1.13); 8.5877 (0.54); 7.6827 (0.88); 7.6761 (0.99); 7.6661 (0.93); 7.6594 (0.95); 7.5566 (0.53); 7.5494 (0.54); 7.5454 (0.61); 7.5381 (0.59); 7.5346 (0.65); 7.5273 (0.64); 7.5233 (0.64); 7.5165 (0.51); 7.2619 (1.14); 7.2385 (1.66); 7.2155 (0.99); 4.3126 (2.30); 4.2982 (2.28); 4.0077 (10.33); 3.3992 (0.35); 3.3779 (0.73); 3.3390 (324.39); 3.3154 (2.57); 3.3039 (0.56); 3.2505 (6.93); 2.5417 (1.35); 2.5249 (0.78); 2.5114 (18.44); 2.5069 (37.67); 2.5024 (49.78); 2.4979 (35.56); 2.4934 (16.90); 2.3291 (0.32); 2.2993 (0.71); 2.2866 (14.14); 2.1081 (16.00); 1.2357 (0.91); -0.0002 (2.91) |
| Beispiel Nr. 161, Lösungsmittel: DMSO-d6, NMR-Spektrometer: 399.95 MHz |
| 11.2614 (0.94); 8.9191 (0.45); 8.9046 (0.91); 8.8903 (0.45); 7.6696 (0.72); 7.6630 (0.90); 7.6530 (0.75); 7.6464 (0.88); 7.6214 (0.49); 7.6144 (0.46); 7.6101 (0.57); 7.6027 (0.50); 7.5993 (0.60); 7.5922 (0.56); 7.5881 (0.58); 7.5813 (0.44); 7.2799 (0.96); 7.2565 (1.46); 7.2336 (0.87); 4.3530 (2.01); 4.3388 (2.00); 4.0124 (8.83); 3.8343 (7.39); 3.3698 (0.50); 3.3349 (171.71); 3.3113 (1.40); 2.5416 (0.39); 2.5244 (0.61); 2.5112 (12.51); 2.5068 (25.30); 2.5023 (33.25); 2.4977 (23.62); 2.4933 (11.11); 2.2996 (16.00); 1.2358 (0.45); -0.0002 (2.44) |
| Beispiel Nr. 162, Lösungsmittel: DMSO-d6, NMR-Spektrometer: 399.95 MHz |
| 11.2274 (3.16); 8.4337 (0.84); 8.4188 (1.69); 8.4045 (0.84); 7.5982 (2.16); 7.5913 (2.35); 7.5839 (2.01); 7.5751 (2.44); 7.2548 (1.39); 7.2437 (0.41); 7.2307 (2.57); 7.2215 (0.37); 7.2158 (0.38); 7.2066 (1.25); 4.3533 (0.48); 4.3388 (0.48); 4.3149 (1.52); 4.2999 (1.55); 4.2845 (1.57); 4.2704 (1.55); 4.2458 (0.49); 4.2315 (0.46); 4.1222 (0.39); |
| 4.1053 (1.50); 4.0884 (2.23); 4.0716 (1.52); 4.0548 (0.45); 4.0065 (16.00); 3.7556 (0.84); 3.7361 (1.49); 3.7196 (1.68); 3.7015 (1.08); 3.5961 (1.04); 3.5770 (1.89); 3.5604 (1.77); 3.5406 (0.88); 3.3833 (0.46); 3.3651 (0.73); 3.3320 (351.12); 3.3084 (3.38); 2.6755 (0.35); 2.6711 (0.47); 2.6669 (0.33); 2.5413 (7.11); 2.5109 (28.82); 2.5066 (57.50); 2.5021 (75.05); 2.4976 (53.26); 2.4932 (25.26); 2.4314 (1.46); 2.4142 (1.39); 2.3970 (2.16); 2.3798 (2.19); 2.3334 (0.37); 2.3288 (0.49); 2.3242 (0.37); 2.2847 (2.16); 2.2685 (2.11); 2.2504 (1.37); 2.2341 (1.38); 2.0087 (0.37); 1.9904 (0.38); 1.9748 (0.52); 1.9607 (0.64); 1.9545 (0.61); 1.9449 (0.92); 1.9400 (0.78); 1.9317 (0.68); 1.9241 (1.10); 1.9156 (0.55); 1.9106 (0.78); 1.8945 (0.65); 1.8380 (0.55); 1.8222 (1.15); 1.8171 (0.80); 1.8041 (1.85); 1.7861 (1.88); 1.7692 (1.24); 1.7561 (0.33); 1.7501 (0.42); 1.5294 (0.55); 1.5115 (1.06); 1.4992 (0.59); 1.4936 (0.72); 1.4895 (1.00); 1.4813 (1.08); 1.4718 (0.62); 1.4599 (0.97); 1.4420 (0.49); 1.2361 (2.21); 0.8543 (0.53); - 0.0002 (5.85) |
| Beispiel Nr. 163, Lösungsmittel: DMSO-d6, NMR-Spektrometer: 399.95 MHz |
| 11.2412 (2.85); 8.6834 (0.88); 8.6689 (1.79); 8.6545 (0.85); 7.6843 (1.34); 7.6778 (1.55); 7.6676 (1.41); 7.6611 (1.50); 7.5947 (0.86); 7.5878 (0.88); 7.5835 (0.99); 7.5762 (0.95); 7.5727 (1.04); 7.5655 (1.00); 7.5615 (1.01); 7.5547 (0.76); 7.4116 (1.68); 7.4027 (1.75); 7.3977 (1.62); 7.3884 (2.38); 7.3751 (1.43); 7.3662 (1.67); 7.3613 (1.56); 7.3518 (2.15); 7.3423 (0.36); 7.2856 (1.04); 7.2759 (5.45); 7.2671 (4.32); 7.2613 (5.54); 7.2526 (3.95); 7.2377 (2.76); 7.2147 (1.61); 4.3401 (3.53); 4.3257 (3.55); 4.0194 (16.00); 3.6558 (11.17); 3.5103 (0.49); 3.3269 (238.10); 3.3033 (3.07); 2.6752 (0.38); 2.6706 (0.51); 2.6661 (0.37); 2.5409 (0.42); 2.5239 (1.31); 2.5105 (30.79); 2.5061 (63.21); 2.5016 (83.78); 2.4970 (59.84); 2.4926 (28.44); 2.3329 (0.40); 2.3283 (0.53); 2.3238 (0.40); 1.2582 (0.33); 1.2355 (1.14); -0.0002 (8.16) |
| Beispiel Nr. 164, Lösungsmittel: DMSO-d6, NMR-Spektrometer: 399.95 MHz |
| 11.2490 (2.89); 8.6939 (0.82); 8.6794 (1.70); 8.6651 (0.86); 7.6623 (1.30); 7.6559 (1.58); 7.6458 (1.37); 7.6393 (1.54); 7.6106 (0.86); 7.6036 (0.81); 7.5992 (1.01); 7.5887 (1.05); 7.5812 (1.00); 7.5773 (1.03); 7.5705 (0.75); 7.3333 (0.95); 7.3234 (3.48); 7.3139 (2.72); 7.2958 (4.89); 7.2908 (3.82); 7.2862 (2.02); 7.2757 (0.75); 7.2706 (0.93); 7.2640 (1.88); 7.2408 (4.10); 7.2183 (2.68); 4.3174 (3.66); 4.3031 (3.64); 4.0104 (16.00); 3.5105 (10.47); 3.3314 (291.56); 3.3077 (3.05); 2.6754 (0.34); 2.6710 (0.47); 2.6666 (0.34); 2.5413 (5.23); 2.5242 (1.23); 2.5108 (28.40); 2.5064 (56.98); 2.5019 (74.55); 2.4974 (52.90); 2.4930 (24.97); 2.3331 (0.33); 2.3288 (0.45); 2.3242 (0.33); 1.2583 (0.46); 1.2355 (1.89); 0.8536 (0.38); -0.0002 (6.16) |
| Beispiel Nr. 165, Lösungsmittel: DMSO-d6, NMR-Spektrometer: 399.95 MHz |
| 11.2498 (2.41); 8.4547 (0.87); 8.4400 (1.84); 8.4256 (0.87); 7.6844 (1.33); 7.6780 (1.48); 7.6677 (1.39); 7.6613 (1.40); 7.5542 (0.78); 7.5471 (0.85); 7.5428 (0.94); 7.5355 (0.93); 7.5321 (1.03); 7.5249 (1.03); 7.5209 (1.02); 7.5140 (0.81); 7.2617 (1.76); 7.2381 (2.50); 7.2152 (1.59); 4.7326 (6.45); 4.7174 (6.92); 4.3624 (3.59); 4.3480 (3.58); 4.2947 (6.70); 4.2795 (6.40); 4.0039 (16.00); 3.3316 (373.85); 3.3078 (3.38); 2.6755 (0.35); 2.6711 (0.48); 2.6666 (0.36); 2.5413 (21.65); 2.5242 (1.23); 2.5110 (28.43); 2.5065 (57.98); 2.5020 (76.42); 2.4974 (54.21); 2.4930 (25.46); 2.3334 (0.35); 2.3289 (0.48); 2.3241 (0.36); 2.0086 (0.43); 1.9996 (1.09); 1.9812 (3.46); 1.9627 (3.54); 1.9443 (1.14); 1.2581 (0.55); 1.2361 (2.06); 0.8540 (0.53); 0.8476 (0.32); 0.7878 (4.16); 0.7694 (9.44); 0.7508 (3.98); -0.0002 (6.83) |
| Beispiel Nr. 166, Lösungsmittel: DMSO-d6, NMR-Spektrometer: 399.95 MHz |
| 11.2495 (3.52); 8.6821 (0.95); 8.6676 (1.83); 8.6533 (0.96); 7.6679 (0.78); 7.6611 (1.04); 7.6572 (1.01); 7.6495 (1.13); 7.6389 (1.20); 7.6280 (0.92); 7.5769 (1.64); 7.5707 (1.52); 7.5606 (1.69); 7.5543 (1.36); 7.2660 (1.62); 7.2427 (2.63); 7.2195 (1.47); 4.9155 (0.45); 4.9061 (0.56); 4.8999 (0.81); 4.8906 (0.85); 4.8842 (0.52); 4.8754 (0.41); 4.7497 (0.42); 4.7407 (0.54); 4.7346 (0.84); 4.7251 (0.83); 4.7185 (0.55); 4.7096 (0.44); 4.4134 (0.64); 4.3989 (0.67); 4.3752 (1.55); 4.3602 (1.55); 4.3282 (1.57); 4.3142 (1.57); 4.2892 (0.68); 4.2752 (0.62); 4.0049 (16.00); 3.3255 (117.94); 2.6707 (0.44); 2.5410 (10.18); 2.5059 (56.56); 2.5017 (70.34); 2.4974 (52.19); 2.3282 (0.42); 1.8635 (0.37); 1.8462 (0.89); 1.8406 (0.66); 1.8287 (1.17); 1.8238 (0.99); 1.8158 (0.73); 1.8101 (0.96); 1.7924 (0.45); 1.5846 (0.45); 1.5754 (0.55); 1.5679 (0.76); 1.5589 (0.82); 1.5501 (0.50); 1.5412 (0.47); 1.5267 (0.49); 1.5176 (0.56); 1.5092 (0.79); 1.5010 (0.79); 1.4929 (0.52); 1.4833 (0.46); 1.2362 (0.92); 1.0727 (0.41); 1.0570 (0.89); 1.0495 (0.59); 1.0421 (0.89); 1.0339 (0.95); 1.0273 (0.99); 1.0190 (0.85); 1.0116 (0.58); 1.0042 (0.84); 0.9884 (0.38); -0.0002 (5.15) |
| Beispiel Nr. 167, Lösungsmittel: DMSO-d6, NMR-Spektrometer: 399.95 MHz |
| 11.2109 (2.73); 8.8297 (0.81); 8.8155 (1.64); 8.8012 (0.80); 7.6793 (1.20); 7.6734 (1.31); 7.6626 (1.25); 7.6566 (1.23); 7.5517 (0.68); 7.5406 (0.84); 7.5300 (0.91); 7.5225 (0.92); 7.5119 (0.68); 7.2549 (1.42); 7.2316 (2.20); 7.2085 (1.21); 4.3951 (0.40); 4.3707 (1.49); 4.3546 (2.36); 4.3379 (1.47); 4.3137 (0.34); 3.9980 (13.42); 3.3241 (119.21); 3.3004 (2.60); 2.6704 (0.40); 2.5407 (0.49); 2.5058 (50.86); 2.5015 (65.47); 2.4972 (48.55); 2.3283 (0.45); 2.0974 (2.70); 2.0785 (2.80); 1.5934 (2.86); 1.5744 (2.83); 1.5585 (16.00); 1.2363 (0.62); -0.0002 (5.39) |
| Beispiel Nr. 168, Lösungsmittel: DMSO-d6, NMR-Spektrometer: 399.95 MHz |
| 11.2140 (3.05); 8.2716 (0.89); 8.2565 (1.84); 8.2417 (0.87); 7.6083 (1.22); 7.6021 (1.57); 7.5917 (1.27); 7.5855 (1.52); 7.5717 (0.89); 7.5603 (1.00); 7.5501 (1.01); 7.5422 (0.95); 7.5385 (0.99); 7.5316 (0.69); 7.2437 (1.67); 7.2202 (2.43); 7.1972 (1.53); 4.3872 (3.66); 4.3723 (3.62); 4.0074 (16.00); 3.3958 (0.35); 3.3322 (482.73); 3.3085 (4.57); 3.2929 (0.56); 3.2791 (0.32); 2.6755 (0.47); 2.6710 (0.65); 2.6667 (0.46); 2.5412 (5.76); 2.5241 (1.82); 2.5107 (39.82); 2.5064 (79.21); 2.5020 (103.59); 2.4975 (74.12); 2.4932 (35.58); 2.3333 (0.48); 2.3287 (0.67); 2.3241 (0.49); 2.0088 (0.46); 1.9900 (0.45); 1.4202 (0.37); 1.4073 (0.73); 1.3996 (0.79); 1.3871 (1.43); 1.3744 (0.84); 1.3667 (0.76); 1.3541 (0.40); 1.2574 (0.73); 1.2361 (2.80); 0.8526 (2.23); 0.8430 (4.71); 0.8359 (5.17); 0.8272 (1.91); 0.5435 (0.81); 0.5321 (2.65); 0.5285 (2.73); 0.5173 (1.36); 0.5118 (2.61); 0.5082 (2.67); 0.4973 (0.89); 0.4443 (1.92); 0.4354 (4.84); 0.4282 (4.67); 0.4188 (1.69); 0.1312 (0.97); 0.1177 (3.34); 0.1048 (3.24); 0.0929 (0.85); -0.0002 (5.61) |
| Beispiel Nr. 169, Lösungsmittel: DMSO-d6, NMR-Spektrometer: 399.95 MHz |
| 11.2393 (3.11); 8.5231 (0.85); 8.5086 (1.73); 8.4942 (0.83); 7.6278 (1.17); 7.6215 (1.66); 7.6113 (1.24); 7.6041 (2.32); 7.5958 (0.82); 7.5916 (1.14); 7.5812 (1.06); 7.5739 (0.95); 7.5700 (1.05); 7.5630 (0.70); 7.2631 (1.68); 7.2397 (2.44); 7.2169 (1.49); 4.3235 (2.29); 4.3143 (2.32); 4.0077 (16.00); 3.3702 (0.57); 3.3323 (335.84); 3.3084 (3.41); 3.2897 (0.37); 3.2839 (0.35); 2.6756 (0.33); 2.6711 (0.44); 2.6667 (0.32); 2.5414 (0.87); 2.5110 (26.82); 2.5066 (54.54); 2.5021 (71.84); 2.4975 (51.18); 2.4931 (24.32); |
| 2.4148 (0.53); 2.3968 (0.59); 2.3760 (1.20); 2.3580 (1.31); 2.3284 (1.25); 2.3081 (0.97); 2.3027 (0.89); 2.2894 (0.48); 2.2830 (0.38); 2.2702 (0.41); 2.2641 (0.40); 1.9329 (0.59); 1.9143 (0.66); 1.9041 (0.73); 1.8973 (0.72); 1.8857 (0.81); 1.8789 (0.61); 1.8684 (0.79); 1.8500 (0.65); 1.6140 (0.41); 1.6023 (0.47); 1.5946 (0.51); 1.5825 (1.00); 1.5700 (0.64); 1.5625 (0.65); 1.5533 (0.92); 1.5413 (0.45); 1.5336 (0.43); 1.5216 (0.42); 1.2395 (0.90); 1.2307 (0.84); 1.2207 (0.93); 1.2111 (0.96); 1.2014 (0.59); 1.1974 (0.65); 1.1874 (0.88); 1.1779 (0.84); 1.1681 (0.41); 1.1588 (0.39); -0.0002 (6.21) |
| Beispiel Nr. 170, Lösungsmittel: DMSO-d6, NMR-Spektrometer: 399.95 MHz |
| 11.2188 (2.64); 8.4000 (0.73); 8.3856 (1.48); 8.3709 (0.73); 7.6329 (1.10); 7.6263 (1.35); 7.6162 (1.16); 7.6097 (1.30); 7.5790 (0.73); 7.5720 (0.70); 7.5680 (0.85); 7.5573 (0.89); 7.5499 (0.83); 7.5461 (0.86); 7.5391 (0.61); 7.2489 (1.47); 7.2255 (2.18); 7.2026 (1.32); 4.3015 (3.11); 4.2869 (3.07); 4.0012 (13.67); 3.3309 (269.40); 3.3069 (2.97); 2.6711 (0.42); 2.5413 (0.86); 2.5108 (24.72); 2.5065 (49.96); 2.5020 (65.84); 2.4974 (47.27); 2.4930 (22.68); 2.3286 (0.40); 2.1591 (1.09); 2.1438 (1.17); 2.1251 (1.45); 2.1097 (1.58); 1.9642 (1.51); 1.9438 (1.83); 1.9302 (1.01); 1.9097 (1.46); 1.8164 (0.50); 1.7994 (0.79); 1.7823 (0.75); 1.7644 (0.46); 1.3402 (0.46); 1.3259 (0.67); 1.3215 (0.59); 1.3069 (1.13); 1.2925 (0.80); 1.2881 (0.94); 1.2739 (0.79); 1.2558 (0.41); 1.2358 (0.70); 1.1759 (0.78); 1.1573 (1.26); 1.1516 (0.39); 1.1391 (1.00); 1.1236 (0.89); 1.1052 (0.59); 0.8472 (13.99); 0.8296 (16.00); 0.8103 (3.89); -0.0002 (5.70) |
| Beispiel Nr. 171 , Lösungsmittel: DMSO-d6, NMR-Spektrometer: 399,95 MHz |
| 11,2006 (0,67); 8,1355 (0,2); 8,1207 (0,41); 8,1064 (0,2); 7,6101 (0,28); 7,6038 (0,31); 7,5932 (0,29); 7,5872 (0,29); 7,5142 (0,16); 7,5071 (0,19); 7,503 (0,2); 7,4927 (0,22); 7,485 (0,22); 7,4745 (0,16); 7,2362 (0,34); 7,2127 (0,5); 7,1897 (0,3); 4,3016 (0,8); 4,2871 (0,79); 3,9999 (3,25); 3,3223 (16); 3,2987 (0,42); 2,5407 (0,64); 2,5058 (10,46); 2,5014 (13,35); 2,497 (9,76); 1,2358 (0,16); 1,1515 (14,31); -0,0001 (1,4) |
| Beispiel Nr. 172, Lösungsmittel: DMSO-d6, NMR-Spektrometer: 399.95 MHz |
| 11.2398 (3.28); 9.0660 (1.08); 9.0522 (2.02); 9.0383 (1.07); 7.6373 (3.23); 7.6234 (3.77); 7.6048 (1.40); 7.2807 (1.45); 7.2586 (2.26); 7.2353 (1.10); 4.3923 (4.17); 4.3783 (4.19); 4.0041 (16.00); 3.3233 (107.12); 3.3002 (3.54); 2.7120 (1.53); 2.6897 (3.16); 2.6669 (2.14); 2.5403 (1.15); 2.5015 (79.31); 2.3286 (0.54); 1.9719 (0.58); 1.9530 (4.70); 1.9304 (5.45); 1.2358 (1.47); -0.0002 (5.45) |
| Beispiel Nr. 201, Lösungsmittel: DMSO-d6, NMR-Spektrometer: 399.95MHz |
| 11.2304 (1.15); 10.7879 (0.37); 8.3642 (0.86); 8.3502 (1.71); 8.3356 (0.93); 8.3161 (0.44); 7.6331 (0.82); 7.6260 (1.15); 7.6221 (1.00); 7.6146 (1.22); 7.6042 (1.33); 7.5930 (1.04); 7.5788 (1.67); 7.5724 (1.36); 7.5621 (1.70); 7.5558 (1.28); 7.2486 (1.66); 7.2252 (2.55); 7.2022 (1.50); 6.8703 (1.07); 6.6415 (0.51); 4.2952 (3.97); 4.2804 (4.34); 4.2641 (0.59); 4.1730 (1.05); 4.0556 (0.38); 4.0378 (1.15); 4.0200 (1.21); 3.9961 (16.00); 3.3213 (14.75); 2.6750 (0.53); 2.6706 (0.72); 2.6662 (0.54); 2.5058 (82.72); 2.5014 (107.65); 2.4971 (81.74); 2.3327 (0.61); 2.3282 (0.78); 2.3238 (0.60); 2.2211 (0.50); 2.2022 (1.44); 2.1825 (4.25); 2.1623 (5.96); 2.1432 (5.87); 2.1242 (1.99); 1.9887 (4.61); 1.3552 (11.55); 1.3354 (0.94); 1.2981 (0.47); 1.2586 (0.62); 1.2496 (0.57); 1.2355 (1.04); 1.1925 (1.32); 1.1748 (2.48); 1.1571 (1.23); 1.0522 (6.25); 1.0333 (12.68); 1.0142 |
| (5.96); 1.0000 (1.96); 0.9812 (3.64); 0.9624 (1.76); -0.0002 (1.12) |
| Beispiel Nr. 263, Lösungsmittel: DMSO-d6, NMR-Spektrometer: 399.95MHz |
| 11.2499 (2.14); 8.4379 (0.52); 8.4233 (1.04); 8.4088 (0.59); 7.6762 (0.52); 7.6693 (0.69); 7.6651 (0.67); 7.6576 (0.75); 7.6471 (0.98); 7.6360 (0.74); 7.5660 (0.99); 7.5594 (0.95); 7.5493 (1.04); 7.5427 (0.91); 7.2522 (1.13); 7.2287 (1.75); 7.2057 (1.03); 6.8709 (0.39); 4.2865 (2.59); 4.2717 (2.88); 4.2555 (0.42); 4.1762 (0.76); 4.1725 (0.76); 4.0560 (0.42); 4.0382 (1.22); 4.0204 (1.27); 3.9996 (10.70); 2.5245 (0.63); 2.5110 (12.19); 2.5067 (24.22); 2.5022 (32.30); 2.4978 (24.62); 2.4937 (12.91); 2.1837 (0.63); 1.9890 (5.03); 1.9092 (1.20); 1.8779 (16.00); 1.3559 (4.28); 1.1929 (1.36); 1.1751 (2.66); 1.1573 (1.32); -0.0002 (3.12) |
| Beispiel Nr. 298, Lösungsmittel: DMSO-d6, NMR-Spektrometer: 399.95MHz |
| 11.2378 (3.42); 10.8089 (0.45); 8.6324 (1.46); 8.6186 (2.34); 8.6030 (1.22); 8.5933 (0.46); 8.3159 (2.70); 7.6640 (0.94); 7.6532 (1.17); 7.6458 (1.21); 7.6355 (1.12); 7.6243 (1.03); 7.5705 (1.62); 7.5637 (1.55); 7.5536 (1.66); 7.5476 (1.46); 7.4685 (0.69); 7.4588 (0.66); 7.2581 (1.57); 7.2342 (2.63); 7.2111 (1.56); 7.1754 (0.38); 4.3207 (3.81); 4.3070 (4.09); 4.2890 (0.64); 4.2699 (0.50); 4.2553 (0.51); 4.1740 (1.03); 4.0363 (0.33); 4.0187 (0.42); 3.9981 (16.00); 3.5723 (0.32); 3.5575 (0.33); 3.5342 (0.35); 3.4844 (0.52); 3.4339 (1.10); 3.4250 (1.32); 3.3556 (95.67); 3.2387 (0.47); 3.1916 (0.36); 2.8031 (0.42); 2.7747 (0.35); 2.7232 (0.47); 2.6743 (9.62); 2.6701 (13.20); 2.6658 (10.13); 2.6346 (0.78); 2.6269 (0.78); 2.5053 (1498.13); 2.5010 (1992.64); 2.4966 (1545.36); 2.3937 (0.75); 2.3762 (0.69); 2.3320 (9.39); 2.3278 (12.77); 2.3234 (9.69); 1.9882 (0.93); 1.6507 (0.44); 1.6387 (0.88); 1.6305 (0.89); 1.6196 (1.71); 1.6071 (1.08); 1.6000 (1.11); 1.5883 (0.64); 1.3604 (1.13); 1.2345 (0.34); 1.1915 (0.32); 1.1739 (0.49); 0.7825 (0.77); 0.7739 (0.49); 0.7636 (0.41); 0.7027 (4.56); 0.6984 (3.93); 0.6906 (4.37); 0.6824 (4.91); 0.6761 (2.53); 0.6679 (2.31); 0.6628 (3.98); 0.6561 (2.22); -0.0002 (3.87) |
| Beispiel Nr. 312, Lösungsmittel: DMSO-d6, NMR-Spektrometer: 399.95MHz |
| 11.1648 (3.23); 8.3457 (0.77); 8.3311 (1.48); 8.3166 (0.82); 7.5602 (1.58); 7.5381 (4.53); 7.3706 (1.59); 7.3513 (3.00); 7.3318 (1.55); 7.0948 (2.04); 7.0756 (1.79); 4.2702 (4.58); 4.2553 (4.54); 4.0560 (0.67); 4.0382 (2.00); 4.0204 (2.08); 3.9988 (16.00); 3.3235 (14.13); 2.5061 (23.79); 2.5017 (30.73); 2.4973 (22.93); 2.1775 (1.68); 2.1585 (5.30); 2.1395 (5.48); 2.1205 (1.85); 1.9889 (8.36); 1.9090 (0.44); 1.3366 (0.34); 1.2498 (0.41); 1.1928 (2.26); 1.1751 (4.37); 1.1572 (2.15); 1.0537 (5.87); 1.0347 (11.72); 1.0157 (5.52); 0.0078 (0.94); -0.0002 (23.47); -0.0082 (0.99) |
| Beispiel Nr. 346, Lösungsmittel: DMSO-d6, NMR-Spektrometer: 399.95MHz |
| 11.3701 (2.72); 8.8268 (2.07); 8.8143 (2.08); 8.4756 (0.69); 8.4611 (1.40); 8.4465 (0.73); 8.3608 (0.48); 8.3415 (1.02); 8.3217 (0.60); 7.8723 (1.33); 7.8567 (1.61); 7.8540 (1.70); 7.8377 (1.29); 7.7217 (1.10); 7.7155 (1.22); 7.7001 (1.35); 7.6939 (1.51); 7.5672 (2.44); 7.5613 (2.32); 7.4902 (2.89); 7.4686 (2.42); 4.3062 (3.49); 4.2916 (3.54); 4.0001 (12.49); 2.5023 (41.99); 2.4981 (33.59); 2.1832 (0.49); 1.9157 (15.05); 1.9094 (16.00); 1.8760 (0.68); 1.3556 (3.13); -0.0002 (4.78) |
| Beispiel Nr. 390, Lösungsmittel: DMSO-d6, NMR-Spektrometer: 399.95MHz |
| 11.4126 (3.52); 8.7297 (6.27); 8.7182 (6.41); 8.4254 (0.91); 8.4107 (1.86); 8.3960 (0.95); 8.1347 (1.72); 8.1309 (1.08); 8.1155 (3.70); 8.0962 (2.05); 7.6812 (4.99); 7.6657 (6.95); 7.6627 (6.61); 7.6472 (5.81); 7.6177 (3.40); 7.6119 (2.73); 7.4863 (3.77); 7.4649 (3.07); 6.8721 (0.43); 4.3142 (4.37); 4.2995 (4.46); 4.0390 (0.45); 4.0211 (0.55); 4.0008 (16.00); 2.5085 (23.11); 2.5045 (30.96); 2.5004 (24.82); 2.2414 (0.51); 2.2262 (1.67); 2.2229 (1.90); 2.2074 (4.93); 2.1884 (5.22); 2.1695 (1.75); 1.9899 (1.42); 1.3571 (4.22); 1.1936 (0.38); 1.1759 (0.72); 1.1580 (0.36); 1.0788 (5.07); 1.0599 (10.27); 1.0408 (4.95); 1.0080 (1.49); 0.9892 (2.87); 0.9704 (1.41); -0.0002 (1.60) |
| Beispiel Nr. 458, Lösungsmittel: DMSO-d6, NMR-Spektrometer: 399.95MHz |
| 11.1803 (3.09); 8.6219 (0.82); 8.6075 (1.56); 8.5929 (0.80); 8.3161 (0.63); 7.5799 (1.70); 7.5608 (2.00); 7.5319 (3.17); 7.3799 (1.76); 7.3603 (3.07); 7.3405 (1.78); 7.1021 (1.98); 7.0835 (1.70); 4.3854 (0.34); 4.3693 (0.33); 4.2931 (4.44); 4.2782 (4.43); 4.0376 (0.34); 4.0197 (0.43); 3.9990 (16.00); 3.9809 (1.72); 3.3208 (175.79); 2.6748 (2.35); 2.6705 (3.09); 2.6660 (2.36); 2.5057 (331.34); 2.5013 (431.25); 2.4970 (326.35); 2.3325 (2.17); 2.3281 (2.89); 2.3237 (2.15); 1.9886 (1.11); 1.6354 (0.45); 1.6234 (0.97); 1.6163 (0.95); 1.6044 (1.64); 1.5922 (0.97); 1.5849 (0.89); 1.5733 (0.42); 1.3354 (1.03); 1.2979 (0.86); 1.2585 (1.16); 1.2496 (1.22); 1.2359 (0.40); 1.1748 (0.63); 0.9161 (0.59); 0.8968 (0.38); 0.7617 (0.38); 0.7577 (0.38); 0.7231 (0.50); 0.7100 (2.25); 0.7032 (4.40); 0.6985 (3.93); 0.6918 (3.85); 0.6848 (2.85); 0.6786 (4.22); 0.6722 (1.98); 0.6590 (3.38); 0.6521 (1.55); 0.6397 (0.49); 0.1462 (0.51); 0.0078 (4.93); -0.0002 (110.46); -0.0082 (5.05); -0.1497 (0.50) |
| Beispiel Nr. 910, Lösungsmittel: DMSO-d6, NMR-Spektrometer: 399.95 |
| MHzlO.9328 (2.87); 8.8827 (3.01); 8.8701 (3.02); 8.4875 (0.74); 8.4680 (1.58); 8.4486 (0.94); 8.4448 (0.70); 8.4362 (0.71); 8.4221 (1.33); 8.4075 (0.71); 7.9798 (2.05); 7.9636 (2.38); 7.9611 (2.42); 7.9447 (1.87); 7.6833 (0.52); 7.6765 (0.79); 7.6723 (0.72); 7.6646 (0.87); 7.6544 (1.13); 7.6469 (1.87); 7.6303 (1.40); 7.6244 (0.94); 7.2364 (1.21); 7.2131 (1.92); 7.1903 (1.14); 4.2827 (3.19); 4.2682 (3.21); 4.0148 (12.67); 3.8328 (0.36); 3.7633 (0.33); 3.7229 (0.33); 3.7146 (0.34); 3.7015 (0.34); 3.6766 (0.33); 3.6190 (0.32); 2.6719 (0.40); 2.5069 (41.71); 2.5026 (56.17); 2.4983 (43.72); 2.3296 (0.39); 1.9094 (1.98); 1.8821 (16.00); 1.3556 (1.01); 0.0078 (0.61); -0.0002 (14.16) |
| Beispiel Nr. 911, Lösungsmittel: DMSO-d6, NMR-Spektrometer: 399.95MHz |
| 10.9265 (1.30); 8.6071 (0.64); 8.5928 (0.33); 7.6609 (0.77); 7.6546 (0.92); 7.6480 (0.69); 7.6379 (0.97); 7.2434 (0.48); 7.2193 (0.89); 7.1951 (0.42); 4.3183 (1.36); 4.3038 (1.36); 4.0143 (5.71); 3.3207 (16.00); 2.5238 (0.76); 2.5059 (26.79); 2.5015 (35.93); 2.4971 (27.30); 1.9887 (0.86); 1.6221 (0.55); 1.6100 (0.33); 1.2497 (0.32); 1.1749 (0.45); 0.7156 (0.66); 0.7085 (1.43); 0.7040 (1.19); 0.6969 (1.19); 0.6901 (0.96); 0.6841 (1.41); 0.6772 (0.60); 0.6697 (0.71); 0.6644 (1.18); 0.6574 (0.55); -0.0002 (4.19) |
| Beispiel Nr. 912, Lösungsmittel: DMSO-d6, NMR-Spektrometer: 399.95MHz |
| 10.9051 (3.50); 8.3525 (0.84); 8.3381 (1.67); 8.3236 (0.84); 7.6498 (3.09); 7.6358 (3.25); 7.6173 (1.10); 7.6105 (0.58); 7.2349 (1.30); 7.2129 (2.01); 7.1883 (1.03); 4.2923 (3.78); 4.2778 (3.79); 4.0113 (16.00); 3.3219 (51.12); 2.6750 (0.62); 2.6705 (0.85); 2.6663 (0.62); 2.5237 (2.54); 2.5101 (46.73); 2.5059 (91.83); 2.5014 (121.48); 2.4970 (90.24); 2.4930 (45.22); 2.3326 (0.60); 2.3282 (0.83); 2.3237 (0.62); 2.1859 (1.64); 2.1669 (5.29); 2.1479 (5.45); 2.1289 (1.82); 1.3357 (1.52); 1.2982 (0.69); 1.2586 (0.97); 1.2494 (1.81); 1.0563 (5.96); 1.0373 (12.30); 1.0183 (5.62); -0.0002 (1.33) |
| Beispiel Nr. 913, Lösungsmittel: DMSO-d6, NMR-Spektrometer: 399.95MHz |
| 4.0585 (3.06); 4.0406 (7.38); 4.0227 (7.54); 4.0050 (3.26); 3.3142 (2.04); 2.5126 (0.74); 2.5084 (1.44); 2.5039 (1.90); 2.4994 (1.42); 2.4953 (0.72); 1.9891 (16.00); 1.1955 (8.17); 1.1774 (12.13); 1.1596 (8.29); 1.0676 (0.49) |
| Beispiel Nr. 914, Lösungsmittel: DMSO-d6, NMR-Spektrometer: 399.95MHz |
| 11.5379 (0.39); 10.8585 (3.28); 8.5081 (0.35); 8.4803 (0.46); 8.4532 (0.92); 8.4392 (1.53); 8.4266 (0.93); 8.3676 (0.38); 8.3159 (3.10); 8.0730 (0.34); 7.6097 (1.74); 7.5891 (3.63); 7.5683 (0.72); 7.5460 (0.46); 7.3579 (1.71); 7.3386 (3.18); 7.3184 (1.60); 7.0760 (2.44); 7.0561 (1.92); 7.0040 (0.39); 6.7047 (0.35); 6.6860 (1.11); 6.6693 (1.16); 6.6479 (1.26); 6.6312 (1.23); 6.6131 (0.34); 5.9903 (1.59); 5.9524 (1.41); 5.9482 (1.48); 5.9288 (0.32); 5.9203 (0.35); 5.9030 (0.34); 5.1434 (0.40); 5.1321 (0.33); 5.0932 (0.50); 5.0624 (0.38); 4.3359 (3.81); 4.3212 (3.76); 4.3021 (0.56); 4.2866 (0.48); 4.2833 (0.46); 4.2660 (1.09); 4.2514 (1.02); 4.0560 (0.33); 4.0379 (1.10); 4.0100 (14.91); 3.8759 (0.44); 3.7979 (0.33); 3.4776 (0.34); 3.4395 (0.39); 3.4185 (0.39); 3.3198 (801.60); 3.2702 (0.40); 3.2622 (0.35); 3.2373 (0.37); 3.0518 (0.48); 2.9680 (0.90); 2.9460 (0.84); 2.8474 (0.37); 2.8053 (0.38); 2.7604 (0.47); 2.6744 (12.18); 2.6702 (16.00); 2.6660 (12.12); 2.6432 (1.49); 2.6248 (1.48); 2.5950 (1.76); 2.5054 (1798.68); 2.5011 (2306.18); 2.4969 (1757.30); 2.3804 (0.97); 2.3322 (11.99); 2.3279 (15.91); 2.3237 (12.06); 2.2639 (0.50); 2.1750 (0.34); 1.9885 (3.88); 1.8106 (5.48); 1.8071 (5.29); 1.7935 (5.32); 1.7902 (5.39); 1.5136 (0.97); 1.4962 (1.10); 1.4674 (0.52); 1.4523 (0.61); 1.3355 (1.97); 1.2978 (2.12); 1.2583 (2.60); 1.2491 (2.36); 1.2365 (1.22); 1.2255 (0.59); 1.2089 (0.46); 1.1927 (1.16); 1.1745 (1.95); 1.1566 (0.94); 1.1045 (1.22); 0.8310 (0.40); -0.0002 (21.34) |
| Beispiel Nr. 915, Lösungsmittel: DMSO-d6, NMR-Spektrometer: 399.95MHz |
| 10.8565 (2.35); 8.8418 (15.91); 8.8287 (16.00); 8.3965 (2.78); 8.3795 (6.05); 8.3604 (3.67); 8.3192 (1.27); 8.3110 (0.63); 8.2957 (1.15); 8.2824 (0.64); 7.9029 (8.10); 7.8864 (12.31); 7.8702 (8.78); 7.6288 (2.11); 7.5687 (1.02); 7.5481 (1.23); 7.3587 (1.04); 7.3393 (2.00); 7.3197 (1.07); 7.0835 (1.45); 7.0642 (1.32); 6.8697 (0.59); 4.5377 (0.34); 4.5263 (0.34); 4.4791 (0.37); 4.4118 (0.44); 4.3679 (0.48); 4.2823 (3.56); 4.2674 (3.56); 4.2418 (0.80); 4.2062 (0.73); 4.1950 (0.78); 4.1747 (0.83); 4.1237 (0.96); 4.0505 (1.29); 4.0156 (12.16); 3.9328 (1.67); 3.8774 (2.03); 3.7805 (2.49); 3.7673 (2.56); 3.7420 (2.60); 3.7295 (2.62); 3.7007 (2.70); 3.5704 (2.26); 3.3719 (1.22); 3.3329 (1.06); 3.2978 (0.90); 3.1866 (0.93); 3.1346 (0.54); 3.0860 (0.45); 3.0530 (0.49); 2.9967 (0.36); 2.9900 (0.33); 2.9830 (0.33); 2.9442 (0.35); 2.6710 (5.07); 2.6669 (3.93); 2.5059 (592.35); 2.5018 (762.19); 2.4979 (595.24); 2.4034 (1.25); 2.3328 (4.37); 2.3287 (5.55); 2.2826 (0.54); 2.2540 (0.62); 2.2362 (0.45); 2.2275 (0.47); 2.1832 (1.18); 2.1613 (0.44); 2.1418 (0.51); 2.1234 (11.92); 2.1058 (12.15); 2.0603 (4.33); 2.0427 (4.48); 1.3551 (5.53); 1.2360 (0.48); 1.0231 (0.33); 1.0138 (0.58); 0.9936 (0.82); 0.9770 (0.70); 0.9650 (0.71); 0.9591 (0.81); 0.9450 (1.29); 0.9390 (1.11); 0.9274 (1.88); 0.9153 (1.14); 0.9082 (1.33); 0.8965 (0.73); 0.8899 (0.58); 0.4706 (2.43); 0.4600 (7.28); 0.4560 (7.74); 0.4457 (3.67); 0.4397 (7.39); 0.4360 (7.22); 0.4256 (2.68); 0.4086 (0.35); 0.1629 (0.79); 0.1499 (2.69); 0.1369 (2.66); 0.1196 (2.00); 0.1063 (6.44); 0.0939 (6.34); 0.0827 (1.68); -0.0002 (6.90) |
| Beispiel Nr. 916, Lösungsmittel: DMSO-d6, NMR-Spektrometer: 399.95MHz |
| 11.0048 (3.61); 10.9874 (1.60); 10.9644 (0.95); 8.6097 (0.46); 8.4936 (0.88); 8.4787 (1.83); 8.4633 (1.20); 8.4477 (0.80); 8.4328 (0.38); 8.3157 (0.57); 7.7420 (1.25); 7.7356 (1.47); 7.7206 (2.02); 7.7142 (1.70); 7.6799 (1.34); 7.6727 (0.87); 7.6505 (0.77); 7.6450 (0.61); 7.6342 (0.42); 7.6277 (0.38); 7.6104 (2.96); 7.6048 (2.76); 7.4804 (4.78); 7.4587 (4.07); 6.7224 (0.36); 6.7055 (1.29); 6.6883 (1.33); 6.6673 (1.45); 6.6502 (1.41); 6.6329 (0.41); 6.0426 (1.66); 6.0385 (1.69); 6.0042 (1.50); 6.0000 (1.52); 5.9625 (0.35); 5.9371 (0.53); 5.9198 (0.56); 5.8943 (0.40); 5.1629 (0.67); 5.1586 (0.73); 5.1199 (0.59); 5.1159 (0.66); 5.1053 (0.70); 5.1022 (0.65); 5.0796 (0.65); 5.0760 (0.61); 4.4879 (0.37); 4.4701 (0.36); 4.3831 (4.13); 4.3684 (4.03); 4.3418 (0.62); 4.3372 (0.63); 4.3181 (1.95); 4.3034 (1.80); 4.0152 (7.44); 4.0099 (5.89); 4.0024 (16.00); 3.3194 (107.61); 3.0098 (1.67); 2.9924 (1.67); 2.6836 (0.86); 2.6749 (2.43); 2.6702 (2.64); 2.6655 (2.52); 2.6608 (1.63); 2.5232 (7.86); 2.5055 (267.13); 2.5011 (356.02); 2.4967 (268.96); 2.3322 (1.75); 2.3278 (2.41); 2.3235 (1.82); 1.8220 (5.45); 1.8184 (5.67); 1.8048 (5.49); 1.8012 (5.59); 1.5173 (2.36); 1.5008 (2.35); 1.3977 (1.84); 1.3352 (1.91); 1.2979 (0.51); 1.2585 (0.72); 1.2493 (2.13); 1.2353 (0.44); 0.1460 (1.23); 0.0077 (9.36); -0.0002 (254.11); -0.0082 (11.38); -0.1497 (1.26) |
| Beispiel Nr. 917, Lösungsmittel: DMSO-d6, NMR-Spektrometer: 399.95MHz |
| 10.8960 (3.60); 8.3159 (1.53); 8.3060 (1.83); 8.2910 (0.93); 7.6921 (1.55); 7.6806 (1.39); 7.6754 (1.45); 7.6157 (0.83); 7.6048 (1.04); 7.5942 (1.09); 7.5858 (1.03); 7.5760 (0.77); 7.2378 (1.57); 7.2145 (2.53); 7.1914 (1.39); 4.3083 (3.91); 4.2937 (3.92); 4.0106 (16.00); 3.3206 (248.14); 2.6704 (4.66); 2.6395 (0.35); 2.5056 (519.56); 2.5013 (668.23); 2.4971 (507.37); 2.4428 (0.63); 2.3322 (3.44); 2.3281 (4.47); 2.3238 (3.38); 2.0658 (6.03); 2.0482 (6.17); 1.3977 (2.90); 1.3358 (0.57); 1.2981 (1.60); 1.2585 (2.04); 1.2496 (0.50); 1.2357 (0.35); 1.0216 (0.37); 1.0087 (0.69); 0.9905 (1.12); 0.9841 (0.61); 0.9787 (0.71); 0.9710 (0.78); 0.9591 (0.42); 0.4679 (1.07); 0.4570 (3.15); 0.4531 (3.32); 0.4425 (1.58); 0.4368 (3.20); 0.4330 (3.09); 0.4226 (1.15); 0.1631 (1.04); 0.1501 (3.95); 0.1381 (3.71); 0.1264 (0.93); 0.0076 (7.52); -0.0002 (155.03); - 0.1497 (0.74) |
| Beispiel Nr. 918, Lösungsmittel: DMSO-d6, NMR-Spektrometer: 399.95MHz |
| 11.1701 (3.21); 8.6356 (0.76); 8.6210 (1.48); 8.6063 (0.74); 7.6033 (1.32); 7.5831 (1.62); 7.5162 (2.94); 7.3944 (1.65); 7.3749 (3.00); 7.3552 (1.60); 7.1123 (1.86); 7.0932 (1.59); 4.3046 (4.12); 4.2897 (4.06); 4.0418 (16.00); 3.3232 (16.06); 2.5236 (1.10); 2.5103 (16.38); 2.5059 (32.04); 2.5014 (41.55); 2.4969 (30.15); 2.4926 (14.83); 2.3371 (0.91); 1.9886 (0.48); 1.6369 (0.36); 1.6249 (0.79); 1.6175 (0.88); 1.6109 (0.74); 1.6058 (1.55); 1.5971 (0.69); 1.5937 (0.91); 1.5864 (0.90); 1.5743 (0.43); 0.7259 (0.47); 0.7133 (1.91); 0.7062 (3.92); 0.7015 (3.30); 0.6947 (3.15); 0.6892 (2.09); 0.6852 (2.09); 0.6799 (3.82); 0.6728 (1.68); 0.6657 (1.96); 0.6600 (3.19); 0.6530 (1.53); 0.6409 (0.53); 0.0080 (0.67); -0.0002 (16.57); -0.0083 (0.68) |
| Beispiel Nr. 919, Lösungsmittel: DMSO-d6, NMR-Spektrometer: 399.95MHz |
| 11.2125 (2.50); 8.4526 (0.61); 8.4375 (1.18); 8.4226 (0.59); 8.3158 (0.41); 7.6934 (0.53); 7.6862 (0.67); 7.6824 (0.66); 7.6748 (0.69); 7.6644 (0.75); 7.6601 (0.69); 7.6532 (0.61); 7.5352 (1.05); 7.5285 (1.04); 7.5185 (1.08); 7.5120 (0.99); 7.2674 (1.22); 7.2440 (1.90); 7.2210 (1.12); 4.2928 (2.78); 4.2781 (2.76); 4.0391 (12.57); 4.0199 (0.50); 3.9269 (0.54); 3.3212 (132.87); 2.8905 (0.40); 2.7309 (0.35); 2.6748 (1.21); 2.6704 (1.67); 2.6659 (1.24); 2.5234 (5.88); 2.5101 (99.78); 2.5058 (197.02); 2.5014 (257.32); |
| 2.4969 (188.08); 2.4927 (93.13); 2.4390 (0.75); 2.3366 (1.46); 2.3326 (1.31); 2.3280 (1.67); 2.3237 (1.22); 1.9885 (1.85); 1.8767 (16.00); 1.1924 (0.48); 1.1746 (0.98); 1.1568 (0.48); 0.1458 (0.40); 0.0080 (3.40); -0.0002 (90.32); -0.0084 (3.56); -0.1496 (0.41) |
| Beispiel Nr. 920, Lösungsmittel: DMSO-d6, NMR-Spektrometer: 399.95MHz |
| 11.2062 (3.09); 8.3828 (0.76); 8.3681 (1.52); 8.3534 (0.75); 7.6476 (0.65); 7.6407 (0.85); 7.6364 (0.81); 7.6292 (0.90); 7.6259 (0.84); 7.6186 (0.99); 7.6145 (0.83); 7.6074 (0.82); 7.5714 (1.40); 7.5648 (1.24); 7.5547 (1.43); 7.5481 (1.15); 7.2635 (1.60); 7.2399 (2.29); 7.2171 (1.44); 4.3050 (3.37); 4.2904 (3.33); 4.0560 (0.44); 4.0388 (16.00); 4.0205 (0.85); 3.3356 (1.55); 2.8911 (0.62); 2.7319 (0.50); 2.5244 (0.63); 2.5110 (10.38); 2.5066 (20.73); 2.5021 (27.12); 2.4975 (19.58); 2.4930 (9.44); 2.3380 (2.54); 2.1842 (1.60); 2.1652 (5.19); 2.1461 (5.34); 2.1271 (1.76); 1.9890 (3.20); 1.1931 (0.89); 1.1753 (1.71); 1.1575 (0.84); 1.0534 (6.11); 1.0345 (12.62); 1.0154 (5.73); 0.0080 (0.49); -0.0002 (12.81); -0.0084 (0.45) |
| Beispiel Nr. 921, Lösungsmittel: DMSO-d6, NMR-Spektrometer: 399.95MHz |
| 11.2243 (3.14); 8.6534 (0.76); 8.6386 (1.56); 8.6239 (0.77); 7.6913 (0.68); 7.6845 (0.82); 7.6801 (0.82); 7.6729 (0.88); 7.6694 (0.85); 7.6621 (0.94); 7.6582 (0.84); 7.6510 (0.77); 7.5498 (1.34); 7.5432 (1.28); 7.5331 (1.38); 7.5265 (1.19); 7.2722 (1.61); 7.2487 (2.34); 7.2257 (1.47); 5.8632 (0.38); 4.3319 (3.28); 4.3173 (3.26); 4.0412 (16.00); 4.0202 (0.46); 3.9161 (0.55); 3.3333 (5.04); 2.8906 (0.92); 2.7311 (0.73); 2.5240 (0.79); 2.5192 (1.29); 2.5107 (14.71); 2.5062 (29.55); 2.5017 (38.66); 2.4971 (27.67); 2.4926 (13.16); 2.3373 (3.36); 1.9887 (1.68); 1.6512 (0.33); 1.6391 (0.74); 1.6319 (0.82); 1.6258 (0.67); 1.6200 (1.51); 1.6115 (0.64); 1.6078 (0.87); 1.6007 (0.85); 1.5885 (0.41); 1.1928 (0.46); 1.1750 (0.90); 1.1572 (0.44); 0.7261 (0.40); 0.7132 (1.74); 0.7061 (3.87); 0.7014 (3.23); 0.6943 (3.24); 0.6887 (3.09); 0.6834 (3.86); 0.6765 (1.56); 0.6693 (1.80); 0.6637 (3.14); 0.6567 (1.40); 0.6443 (0.45); 0.0080 (0.65); -0.0002 (18.05); -0.0085 (0.60) |
| Beispiel Nr. 922, Lösungsmittel: DMSO-d6, NMR-Spektrometer: 399.95MHz |
| 10.8626 (3.24); 8.6136 (0.79); 8.5992 (1.58); 8.5848 (0.89); 7.5942 (3.24); 7.3660 (1.35); 7.3456 (2.20); 7.3366 (0.65); 7.3254 (1.42); 7.0811 (2.02); 7.0623 (1.84); 4.2892 (4.30); 4.2744 (4.44); 4.1286 (3.74); 4.0555 (0.55); 4.0379 (1.67); 4.0218 (16.00); 4.0023 (0.99); 3.3434 (55.26); 2.8906 (1.39); 2.7310 (1.23); 2.6714 (0.50); 2.6671 (0.41); 2.5066 (54.14); 2.5024 (73.39); 2.4981 (58.91); 2.3290 (0.47); 1.9887 (6.07); 1.6379 (0.35); 1.6258 (0.77); 1.6185 (0.88); 1.6069 (1.55); 1.5948 (0.99); 1.5874 (0.93); 1.5752 (0.46); 1.1928 (1.63); 1.1750 (3.27); 1.1572 (1.64); 0.7269 (0.44); 0.7141 (1.87); 0.7069 (3.88); 0.7025 (3.44); 0.6955 (3.32); 0.6905 (2.44); 0.6862 (2.36); 0.6806 (3.93); 0.6737 (1.94); 0.6608 (3.32); 0.6538 (1.74); 0.6417 (0.63); 0.0060 (0.60); -0.0002 (11.53) |
| Beispiel Nr. 923, Lösungsmittel: DMSO-d6, NMR-Spektrometer: 399.95MHz |
| 10.8965 (3.22); 8.3514 (0.80); 8.3370 (1.61); 8.3226 (0.90); 8.3135 (0.45); 7.6419 (2.13); 7.6353 (2.46); 7.6267 (2.13); 7.6187 (2.62); 7.2343 (1.22); 7.2234 (0.46); 7.2102 (2.32); 7.1952 (0.42); 7.1861 (1.14); 4.2916 (3.75); 4.2770 (3.92); 4.0168 (16.00); 3.3411 (153.02); 3.3365 (164.52); 3.3322 (153.52); 2.8908 (0.72); 2.7313 (0.62); 2.6712 (0.72); 2.6670 (0.57); 2.5064 (76.32); 2.5021 (106.28); 2.4978 (87.84); 2.3368 (0.69); 2.3337 (0.61); 2.3290 (0.76); 2.3245 (0.62); 2.1861 (1.53); 2.1671 (4.91); 2.1481 |
| (5.14); 2.1291 (1.80); 1.9885 (0.36); 1.0557 (5.59); 1.0367 (11.42); 1.0177 (5.47); 0.0079 (0.78); -0.0002 (25.99); -0.0081 (2.21) |
| Beispiel Nr. 924, Lösungsmittel: DMSO-d6, NMR-Spektrometer: 399.95MHz |
| 10.9204 (3.49); 8.6235 (0.87); 8.6089 (1.74); 8.5949 (0.93); 7.6718 (0.56); 7.6655 (0.98); 7.6459 (3.17); 7.6319 (2.69); 7.2417 (1.08); 7.2180 (2.00); 7.1960 (1.24); 4.3178 (3.75); 4.3035 (3.86); 4.0200 (16.00); 3.3410 (149.30); 3.3323 (210.34); 3.2821 (0.32); 2.8911 (0.39); 2.7318 (0.33); 2.6718 (0.66); 2.5065 (71.46); 2.5026 (97.77); 2.4984 (78.39); 2.3295 (0.63); 1.6526 (0.34); 1.6405 (0.74); 1.6332 (0.83); 1.6217 (1.54); 1.6095 (1.00); 1.6024 (0.91); 1.5906 (0.46); 0.7280 (0.38); 0.7147 (1.72); 0.7076 (3.92); 0.7036 (3.48); 0.6957 (3.72); 0.6907 (3.60); 0.6856 (4.27); 0.6788 (2.07); 0.6659 (3.59); 0.6590 (1.75); 0.6466 (0.58); 0.0079 (0.67); 0.0001 (20.35); -0.0071 (1.35) |
| Beispiel Nr. 925, Lösungsmittel: DMSO-d6, NMR-Spektrometer: 399.95MHz |
| 10.8490 (2.00); 8.4040 (0.45); 8.3893 (0.84); 8.3749 (0.46); 7.6006 (0.99); 7.5836 (3.25); 7.3576 (0.85); 7.3380 (1.22); 7.3360 (1.24); 7.3203 (0.51); 7.3168 (0.78); 7.0771 (1.29); 7.0578 (1.20); 4.2557 (2.93); 4.2408 (2.92); 4.0377 (0.50); 4.0223 (10.65); 3.3228 (19.65); 2.5238 (0.73); 2.5104 (13.19); 2.5059 (26.09); 2.5013 (34.58); 2.4968 (25.35); 2.4923 (12.13); 1.9887 (1.48); 1.8766 (16.00); 1.1925 (0.42); 1.1747 (0.83); 1.1569 (0.40); 0.0080 (0.86); -0.0002 (22.15); -0.0085 (0.70) |
| Beispiel Nr. 926, Lösungsmittel: DMSO-d6, NMR-Spektrometer: 399.95MHz |
| 10.9408 (2.23); 8.4265 (0.53); 8.4120 (1.05); 8.3977 (0.57); 8.2536 (0.38); 7.9529 (0.54); 7.6858 (0.47); 7.6789 (0.65); 7.6744 (0.55); 7.6672 (0.70); 7.6569 (0.82); 7.6523 (0.59); 7.6450 (1.66); 7.6383 (0.75); 7.6279 (1.07); 7.6214 (0.70); 7.2392 (1.27); 7.2157 (1.58); 7.1929 (1.07); 6.8139 (0.42); 6.7923 (0.50); 6.7889 (0.45); 6.7673 (0.46); 6.4669 (0.36); 6.4505 (0.37); 4.2843 (2.42); 4.2698 (2.44); 4.1805 (0.35); 4.1462 (0.86); 4.1316 (0.87); 4.0547 (0.34); 4.0378 (0.40); 4.0259 (10.24); 3.9373 (1.38); 3.8945 (0.44); 3.3243 (21.79); 3.0563 (0.67); 2.9388 (0.71); 2.8908 (4.75); 2.7319 (3.55); 2.7310 (3.54); 2.6710 (0.32); 2.5244 (0.86); 2.5196 (1.40); 2.5110 (17.95); 2.5065 (36.36); 2.5019 (48.83); 2.4972 (35.62); 2.4927 (16.83); 2.3286 (0.35); 1.9888 (0.42); 1.8873 (2.50); 1.8812 (16.00); 1.8562 (5.85); 1.3557 (0.58); 1.2356 (0.39); 0.0079 (0.73); - 0.0002 (22.63); -0.0085 (0.72) |
| Beispiel Nr. 927, Lösungsmittel: DMSO-d6, NMR-Spektrometer: 399.95MHz |
| 11.1622 (2.12); 8.4275 (0.43); 8.4129 (0.82); 8.3983 (0.43); 7.6034 (0.81); 7.6008 (0.83); 7.5832 (0.93); 7.5805 (1.04); 7.5074 (1.93); 7.3870 (1.20); 7.3675 (2.16); 7.3478 (1.18); 7.1098 (1.19); 7.0898 (1.03); 4.2694 (2.82); 4.2544 (2.79); 4.0557 (0.33); 4.0414 (10.89); 4.0202 (0.65); 2.5239 (0.33); 2.5192 (0.52); 2.5105 (6.43); 2.5060 (12.96); 2.5014 (17.36); 2.4967 (12.62); 2.4922 (5.91); 1.9887 (2.72); 1.8768 (16.00); 1.3365 (0.73); 1.2497 (0.98); 1.1927 (0.76); 1.1749 (1.52); 1.1571 (0.74); -0.0002 (2.47) |
| Beispiel Nr. 928, Lösungsmittel: DMSO-d6, NMR-Spektrometer: 399.95MHz |
| 11.1551 (3.07); 8.3552 (0.67); 8.3406 (1.30); 8.3255 (0.67); 7.5710 (1.24); 7.5508 (1.64); 7.5259 (2.80); 7.3839 (1.69); 7.3644 (3.11); 7.3448 (1.63); 7.1036 (1.72); 7.0837 (1.50); 4.2780 (4.02); 4.2631 (3.97); 4.0393 (16.00); 4.0200 (0.56); 3.3218 (28.95); 2.5238 (0.90); 2.5190 (1.46); 2.5105 (17.00); 2.5059 (34.27); 2.5013 (45.85); 2.4967 (33.36); 2.4922 (15.74); 2.1782 (1.74); 2.1592 (5.65); 2.1402 (5.82); 2.1212 (1.91); 1.9886 (2.20); 1.3359 (1.40); 1.2588 (0.43); 1.2496 (1.85); 1.1926 (0.67); 1.1749 (1.25); |
| 1.1571 (0.61); 1.0535 (6.67); 1.0345 (14.01); 1.0154 (6.24); -0.0002 (5.97) |
| Beispiel Nr. 929, Lösungsmittel: DMSO-d6, NMR-Spektrometer: 399.95MHz |
| 10.8811 (2.12); 8.3354 (0.50); 8.3211 (0.95); 8.3068 (0.55); 8.2536 (0.33); 7.6018 (1.97); 7.5711 (1.11); 7.3571 (1.01); 7.3375 (1.92); 7.3180 (1.03); 7.0724 (1.31); 7.0533 (1.17); 4.2668 (2.84); 4.2519 (2.83); 4.1800 (0.41); 4.1459 (0.33); 4.0558 (1.52); 4.0380 (4.13); 4.0265 (10.00); 4.0203 (5.09); 4.0024 (1.46); 3.9371 (1.16); 3.8944 (0.52); 3.3237 (12.99); 3.0561 (0.68); 2.9386 (0.71); 2.8906 (1.47); 2.7311 (1.17); 2.5240 (0.85); 2.5106 (16.81); 2.5062 (33.17); 2.5016 (44.08); 2.4971 (32.78); 2.4927 (16.28); 2.3285 (0.33); 2.1795 (1.20); 2.1604 (3.78); 2.1414 (3.94); 2.1298 (0.36); 2.1224 (1.37); 1.9888 (16.00); 1.2353 (0.37); 1.1927 (4.50); 1.1749 (8.73); 1.1571 (4.36); 1.0558 (4.36); 1.0368 (9.04); 1.0178 (4.37); -0.0002 (4.56) |
| Beispiel Nr. 930, Lösungsmittel: DMSO-d6, NMR-Spektrometer: 399.95MHz |
| 10.8971 (3.46); 8.6135 (0.78); 8.5989 (1.55); 8.5845 (0.79); 8.3157 (0.34); 7.6054 (5.25); 7.5872 (1.74); 7.3672 (1.15); 7.3483 (2.13); 7.3268 (1.25); 7.0817 (2.16); 7.0625 (1.72); 6.9378 (0.32); 4.2898 (4.38); 4.2749 (4.32); 4.1248 (0.50); 4.1104 (0.50); 4.0555 (0.88); 4.0376 (3.02); 4.0276 (16.00); 4.0199 (3.20); 4.0020 (0.88); 3.3210 (94.72); 2.6795 (0.37); 2.6748 (0.79); 2.6703 (1.12); 2.6656 (0.81); 2.6611 (0.37); 2.5404 (0.50); 2.5237 (3.30); 2.5190 (5.15); 2.5104 (59.55); 2.5058 (119.60); 2.5012 (159.55); 2.4966 (115.05); 2.4920 (53.65); 2.3372 (0.36); 2.3326 (0.79); 2.3280 (1.06); 2.3234 (0.75); 2.3188 (0.33); 1.9885 (11.20); 1.6377 (0.43); 1.6258 (0.96); 1.6184 (1.05); 1.6119 (0.92); 1.6066 (1.90); 1.5981 (0.83); 1.5945 (1.16); 1.5871 (1.13); 1.5751 (0.62); 1.2354 (0.72); 1.1924 (3.17); 1.1746 (6.31); 1.1568 (3.09); 0.7262 (0.55); 0.7137 (2.28); 0.7065 (4.56); 0.7017 (3.72); 0.6951 (3.85); 0.6893 (2.51); 0.6845 (2.47); 0.6791 (4.53); 0.6719 (2.04); 0.6652 (2.29); 0.6631 (2.02); 0.6592 (3.86); 0.6524 (1.96); 0.6402 (0.91); 0.6337 (0.51); 0.0080 (0.56); -0.0002 (17.20); -0.0086 (0.49) |
| Beispiel Nr. 931, Lösungsmittel: DMSO-d6, NMR-Spektrometer: 399.95MHz |
| 10.8837 (2.12); 8.4062 (0.46); 8.3912 (0.84); 8.3765 (0.45); 7.6016 (1.32); 7.5932 (2.10); 7.5885 (2.27); 7.3594 (1.02); 7.3400 (1.40); 7.3188 (0.99); 7.0778 (1.27); 7.0585 (1.11); 4.2562 (2.81); 4.2412 (2.78); 4.0270 (9.81); 3.3215 (45.49); 2.6750 (0.33); 2.6704 (0.46); 2.6657 (0.33); 2.5237 (1.45); 2.5188 (2.29); 2.5103 (24.99); 2.5058 (49.54); 2.5012 (65.68); 2.4967 (47.92); 2.4922 (22.96); 2.3280 (0.44); 1.8765 (16.00); -0.0002 (6.06) |
| Beispiel Nr. 932, Lösungsmittel: DMSO-d6, NMR-Spektrometer: 399.95MHz |
| 10.9350 (3.62); 8.3534 (0.86); 8.3389 (1.74); 8.3244 (0.89); 8.3161 (0.37); 7.6529 (3.06); 7.6392 (3.41); 7.6321 (1.40); 7.6243 (0.96); 7.6200 (1.15); 7.6132 (0.62); 7.2361 (1.34); 7.2139 (1.95); 7.1896 (1.00); 4.2933 (3.84); 4.2787 (3.84); 4.0375 (0.37); 4.0226 (16.00); 3.3265 (72.51); 3.1952 (0.33); 2.6751 (0.52); 2.6706 (0.73); 2.6660 (0.52); 2.5239 (2.09); 2.5191 (3.50); 2.5105 (41.20); 2.5060 (81.68); 2.5014 (107.82); 2.4968 (78.18); 2.4923 (37.11); 2.3328 (0.56); 2.3282 (0.73); 2.3237 (0.58); 2.1860 (1.86); 2.1670 (6.05); 2.1480 (6.28); 2.1290 (2.12); 1.9886 (0.69); 1.1747 (0.41); 1.0562 (7.25); 1.0373 (15.43); 1.0261 (0.82); 1.0182 (6.95); 1.0071 (0.36); 1.0020 (0.42); 0.1460 (0.33); 0.0080 (3.12); -0.0002 (85.43); -0.0085 (2.83); -0.1496 (0.34) |
| Beispiel Nr. 933, Lösungsmittel: DMSO-d6, NMR-Spektrometer: 399.95MHz |
| 10.9579 (3.64); 8.6235 (0.93); 8.6094 (1.84); 8.5953 (0.97); 8.2535 (0.54); 7.6700 (1.40); 7.6635 (2.01); 7.6577 (2.65); 7.6409 (3.03); 7.2567 (0.37); 7.2457 (1.50); 7.2345 (0.63); 7.2215 (2.50); 7.2036 (0.61); 7.1975 (1.15); 7.1785 (0.44); 4.3192 (3.82); 4.3048 (3.96); 4.2746 (0.44); 4.1800 (1.13); 4.1660 (0.46); 4.0548 (0.72); 4.0377 (1.12); 4.0255 (16.00); 4.0022 (0.39); 3.9367 (2.13); 3.8938 (0.79); 3.8076 (0.34); 3.7259 (0.51); 3.3217 (74.88); 3.0557 (1.08); 2.9382 (1.14); 2.8906 (2.16); 2.7308 (1.68); 2.6751 (0.76); 2.6706 (1.01); 2.6661 (0.73); 2.5238 (3.12); 2.5104 (55.87); 2.5060 (110.99); 2.5014 (147.68); 2.4969 (109.48); 2.4925 (53.98); 2.3371 (0.42); 2.3328 (0.78); 2.3282 (1.05); 2.3236 (0.77); 2.1929 (0.36); 1.9886 (2.25); 1.6532 (0.40); 1.6410 (0.89); 1.6338 (1.03); 1.6275 (0.99); 1.6220 (1.84); 1.6097 (1.28); 1.6027 (1.20); 1.5950 (0.66); 1.5908 (0.72); 1.5768 (0.39); 1.3553 (0.58); 1.2588 (0.33); 1.2354 (0.62); 1.2282 (0.46); 1.1925 (0.70); 1.1747 (1.29); 1.1569 (0.65); 0.7281 (0.45); 0.7152 (2.09); 0.7081 (4.52); 0.7034 (4.18); 0.6963 (4.18); 0.6899 (4.06); 0.6840 (5.10); 0.6771 (2.62); 0.6699 (2.90); 0.6642 (4.42); 0.6572 (2.23); 0.6446 (1.19); 0.0079 (0.47); -0.0002 (14.37); - 0.0085 (0.57) |
| Beispiel Nr. 934, Lösungsmittel: DMSO-d6, NMR-Spektrometer: 399.95MHz |
| 10.8941 (2.21); 8.6273 (0.53); 8.6129 (1.11); 8.5988 (0.59); 7.6905 (0.41); 7.6837 (0.72); 7.6791 (0.56); 7.6719 (0.81); 7.6640 (2.24); 7.6503 (2.00); 7.2431 (0.71); 7.2188 (1.33); 7.1969 (0.93); 4.3209 (2.40); 4.3065 (2.41); 4.1835 (0.73); 4.1707 (0.46); 3.9877 (9.85); 3.3235 (40.02); 2.6750 (0.36); 2.6705 (0.50); 2.6658 (0.36); 2.5238 (1.35); 2.5191 (2.02); 2.5104 (27.46); 2.5059 (56.20); 2.5013 (75.48); 2.4967 (55.17); 2.4922 (26.43); 2.3328 (0.40); 2.3281 (0.53); 2.3235 (0.38); 2.2869 (16.00); 1.9885 (0.62); 1.6421 (0.54); 1.6349 (0.61); 1.6285 (0.50); 1.6231 (1.12); 1.6109 (0.66); 1.6037 (0.64); 1.5917 (0.32); 1.1747 (0.35); 0.7136 (1.36); 0.7065 (2.91); 0.7017 (2.34); 0.6949 (2.28); 0.6872 (1.73); 0.6817 (2.77); 0.6747 (1.14); 0.6677 (1.41); 0.6619 (2.29); 0.6548 (1.05); 0.6426 (0.37); 0.0080 (1.10); -0.0002 (36.82); -0.0085 (1.13) |
| Beispiel Nr. 935, Lösungsmittel: DMSO-d6, NMR-Spektrometer: 399.95MHz |
| 10.8782 (2.54); 8.4265 (0.62); 8.4121 (1.21); 8.3978 (0.66); 7.7027 (0.54); 7.6962 (0.75); 7.6918 (0.72); 7.6845 (0.83); 7.6741 (0.88); 7.6700 (0.73); 7.6628 (0.76); 7.6484 (1.19); 7.6420 (0.95); 7.6316 (1.19); 7.6251 (0.96); 7.5390 (0.34); 7.2379 (1.21); 7.2146 (1.91); 7.1915 (1.28); 4.2854 (2.89); 4.2711 (2.88); 4.1828 (1.09); 4.0377 (0.52); 4.0200 (0.53); 4.0019 (0.39); 3.9869 (10.65); 3.3211 (58.79); 2.6748 (0.47); 2.6704 (0.63); 2.6658 (0.46); 2.5236 (2.20); 2.5102 (36.47); 2.5058 (70.83); 2.5013 (92.77); 2.4968 (68.45); 2.4924 (33.88); 2.3326 (0.50); 2.3281 (0.66); 2.3235 (0.51); 2.2850 (16.00); 1.9886 (2.14); 1.8783 (15.88); 1.1924 (0.58); 1.1747 (1.14); 1.1569 (0.56); 0.0079 (0.73); -0.0002 (18.79); -0.0084 (0.79) |
| Beispiel Nr. 936, Lösungsmittel: DMSO-d6, NMR-Spektrometer: 399.95MHz |
| 10.8270 (2.31); 8.4102 (0.50); 8.3957 (0.95); 8.3812 (0.54); 7.6130 (1.95); 7.6074 (2.35); 7.6017 (2.13); 7.3602 (1.12); 7.3494 (0.35); 7.3407 (1.61); 7.3302 (0.37); 7.3195 (1.22); 7.0755 (1.37); 7.0563 (1.19); 4.2590 (3.18); 4.2441 (3.15); 4.1883 (0.55); 3.9915 (10.13); 3.3227 (25.27); 2.5236 (0.81); 2.5102 (13.51); 2.5058 (26.40); 2.5013 (34.61); 2.4967 (25.39); 2.4922 (12.37); 2.2908 (15.25); 1.9885 (1.07); 1.8770 (16.00); 1.1747 (0.57); -0.0002 (7.64) |
| Beispiel Nr. 937, Lösungsmittel: DMSO-d6, NMR-Spektrometer: 399.95MHz |
| 10.9008 (2.66); 8.4236 (0.63); 8.4097 (1.25); 8.3956 (0.69); 7.6792 (0.54); 7.6720 (0.78); 7.6682 (0.72); 7.6604 (0.87); 7.6502 (0.95); 7.6387 (0.92); 7.6321 (1.36); 7.6259 (1.07); 7.6153 (1.32); 7.6090 (1.02); 7.2360 (1.21); 7.2129 (1.96); 7.1897 (1.16); 4.2830 (3.10); 4.2684 (3.20); 4.0380 (0.52); 4.0200 (13.29); 3.3389 (51.63); 3.3298 (60.41); 2.8909 (0.38); 2.5063 (30.51); 2.5020 (39.66); 2.4976 (30.17); 1.9887 (1.39); 1.8810 (16.00); 1.8565 (0.34); 1.1928 (0.38); 1.1750 (0.76); 1.1572 (0.38); 0.0080 (0.32); - 0.0002 (10.01); -0.0084 (0.53) |
| Beispiel Nr. 938, Lösungsmittel: DMSO-d6, NMR-Spektrometer: 399.95MHzl0.8450 (3.19); 8.3342 (0.78); 8.3198 (1.51); 8.3061 (0.90); 7.5892 (5.17); 7.5702 (1.96); 7.3555 (1.20); 7.3363 (2.24); 7.3160 (1.24); 7.0719 (2.17); 7.0529 (1.91); 4.2668 (4.47); 4.2519 (4.64); 4.1278 (0.51); 4.0382 (0.63); 4.0219 (16.00); 3.3333 (18.51); 2.8908 (1.67); 2.7319 (1.44); 2.5063 (17.85); 2.5021 (24.45); 2.4979 (20.56); 2.3379 (1.01); 2.1802 (1.58); 2.1612 (5.02); 2.1422 (5.27); 2.1232 (1.88); 1.9888 (1.92); 1.1930 (0.55); 1.1751 (1.08); 1.1573 (0.54); 1.0562 (5.58); 1.0372 (11.25); 1.0182 (5.45); -0.0002 (6.44) |
| Beispiel Nr. 939, Lösungsmittel: DMSO-d6, NMR-Spektrometer: 399.95MHz |
| 10.8567 (3.91); 9.7151 (6.81); 8.3527 (0.92); 8.3384 (1.75); 8.3236 (0.91); 7.5654 (1.67); 7.5452 (2.14); 7.5068 (3.73); 7.3704 (2.09); 7.3508 (3.85); 7.3312 (2.02); 7.0812 (2.32); 7.0620 (2.02); 4.2749 (5.34); 4.2599 (5.27); 3.3233 (34.52); 2.6749 (0.39); 2.6704 (0.53); 2.6659 (0.39); 2.5236 (1.91); 2.5102 (31.89); 2.5059 (62.51); 2.5014 (81.28); 2.4969 (59.11); 2.4925 (28.99); 2.3324 (0.40); 2.3282 (0.53); 2.3236 (0.40); 2.1754 (2.11); 2.1564 (6.79); 2.1374 (7.01); 2.1184 (2.36); 1.9886 (0.84); 1.1747 (0.44); 1.0519 (7.80); 1.0329 (16.00); 1.0138 (7.32); 0.0079 (1.49); -0.0002 (35.94); -0.0084 (1.35) |
| Beispiel Nr. 940, Lösungsmittel: DMSO-d6, NMR-Spektrometer: 399.95MHz |
| 10.9356 (9.35); 9.7203 (16.00); 8.6519 (2.38); 8.6373 (4.81); 8.6224 (2.38); 8.3157 (0.42); 7.6738 (2.09); 7.6669 (2.53); 7.6627 (2.52); 7.6552 (2.70); 7.6522 (2.63); 7.6447 (2.86); 7.6407 (2.55); 7.6337 (2.30); 7.5329 (4.04); 7.5263 (3.88); 7.5162 (4.17); 7.5097 (3.61); 7.2557 (4.74); 7.2322 (7.11); 7.2092 (4.29); 4.3275 (10.22); 4.3129 (10.11); 4.2863 (0.50); 4.0377 (0.71); 4.0199 (0.72); 3.3237 (185.82); 3.0551 (1.07); 2.8961 (1.15); 2.8906 (1.43); 2.7307 (1.17); 2.6750 (1.33); 2.6705 (1.84); 2.6661 (1.36); 2.5236 (7.54); 2.5103 (111.73); 2.5060 (220.26); 2.5015 (288.48); 2.4969 (209.88); 2.4925 (102.83); 2.3368 (2.93); 2.3330 (1.58); 2.3283 (1.89); 2.3237 (1.35); 1.9886 (3.13); 1.6487 (1.00); 1.6366 (2.20); 1.6295 (2.46); 1.6234 (2.04); 1.6177 (4.47); 1.6055 (2.65); 1.5984 (2.54); 1.5862 (1.25); 1.2358 (0.37); 1.1925 (0.84); 1.1747 (1.66); 1.1609 (0.43); 1.1569 (0.86); 0.7219 (1.20); 0.7090 (5.16); 0.7019 (11.48); 0.6973 (9.78); 0.6900 (10.00); 0.6846 (9.51); 0.6794 (11.68); 0.6725 (4.95); 0.6652 (5.59); 0.6598 (9.86); 0.6527 (4.47); 0.6403 (1.49); 0.1459 (0.38); 0.0079 (3.41); -0.0002 (91.11); -0.0084 (3.44); -0.1498 (0.40) |
| Beispiel Nr. 941, Lösungsmittel: DMSO-d6, NMR-Spektrometer: 399.95MHz |
| 10.9283 (2.34); 9.7198 (3.98); 8.4509 (0.59); 8.4367 (1.13); 8.4219 (0.56); 7.6781 (0.55); 7.6714 (0.66); 7.6669 (0.67); 7.6598 (0.70); 7.6563 (0.70); 7.6489 (0.74); 7.6451 (0.66); 7.6379 (0.59); 7.5229 (1.05); 7.5163 (1.01); 7.5062 (1.06); 7.4996 (0.94); 7.2507 (1.23); 7.2270 (1.84); 7.2042 (1.11); 4.2904 (2.72); 4.2757 (2.68); 3.3228 (26.46); |
| 2.8903 (0.43); 2.7306 (0.37); 2.6705 (0.36); 2.5103 (21.76); 2.5060 (43.01); 2.5015 (56.41); 2.4969 (41.38); 2.4925 (20.50); 2.3283 (0.36); 1.9887 (0.59); 1.8744 (16.00); 1.8554 (1.55); 0.0080 (0.96); -0.0002 (24.92); -0.0084 (1.03) |
| Beispiel Nr. 942, Lösungsmittel: DMSO-d6, NMR-Spektrometer: 399.95MHz |
| 10.9174 (3.66); 9.7180 (6.50); 8.3819 (0.92); 8.3672 (1.85); 8.3526 (0.94); 8.1582 (0.57); 8.1443 (1.00); 8.1318 (0.58); 7.6328 (0.81); 7.6260 (1.06); 7.6216 (0.98); 7.6145 (1.12); 7.6112 (1.04); 7.6038 (1.22); 7.5996 (1.03); 7.5926 (1.03); 7.5545 (1.72); 7.5479 (1.54); 7.5377 (1.77); 7.5312 (1.45); 7.2473 (2.03); 7.2236 (2.86); 7.2008 (1.83); 6.8099 (1.65); 6.7882 (2.00); 6.7849 (1.85); 6.7633 (1.79); 6.4563 (1.14); 6.4493 (1.61); 6.4401 (1.17); 6.4330 (1.63); 6.4241 (1.15); 6.4133 (1.23); 6.4058 (0.83); 6.4027 (1.00); 6.3952 (0.84); 6.3918 (0.97); 6.3846 (0.66); 4.9204 (1.88); 4.3005 (4.20); 4.2859 (4.23); 4.1550 (3.59); 4.1405 (3.54); 3.3250 (61.74); 2.8905 (1.94); 2.7309 (1.63); 2.6752 (0.39); 2.6707 (0.55); 2.6661 (0.39); 2.5239 (1.79); 2.5106 (32.42); 2.5061 (65.41); 2.5016 (86.36); 2.4970 (62.72); 2.4926 (30.77); 2.3328 (0.42); 2.3283 (0.56); 2.3238 (0.42); 2.1799 (2.07); 2.1609 (8.24); 2.1420 (11.98); 2.1230 (7.65); 2.1041 (1.87); 1.9887 (0.50); 1.2589 (0.38); 1.2354 (0.57); 1.0503 (7.58); 1.0389 (6.78); 1.0314 (16.00); 1.0199 (13.21); 1.0123 (7.66); 1.0009 (6.00); 0.0080 (1.46); -0.0002 (42.17); -0.0085 (1.61) |
| Beispiel Nr. 943, Lösungsmittel: DMSO-d6, NMR-Spektrometer: 399.95MHz |
| 10.8721 (9.15); 9.7168 (16.00); 8.6311 (2.09); 8.6163 (4.16); 8.6014 (2.07); 8.3156 (1.14); 7.5973 (3.57); 7.5948 (3.59); 7.5771 (4.09); 7.5744 (4.55); 7.4966 (8.46); 7.3798 (5.33); 7.3602 (9.55); 7.3406 (5.20); 7.0902 (5.25); 7.0707 (4.51); 4.3005 (11.98); 4.2856 (11.83); 4.0555 (0.33); 4.0377 (1.03); 4.0199 (1.07); 4.0021 (0.40); 3.4383 (0.34); 3.3214 (291.88); 2.8907 (0.35); 2.7305 (0.33); 2.6796 (1.10); 2.6750 (2.34); 2.6704 (3.23); 2.6658 (2.30); 2.6612 (1.06); 2.5239 (8.73); 2.5191 (13.55); 2.5105 (166.62); 2.5059 (339.33); 2.5013 (455.66); 2.4967 (329.53); 2.4921 (153.16); 2.3372 (1.08); 2.3327 (2.27); 2.3281 (3.15); 2.3235 (2.21); 2.3190 (0.97); 1.9885 (4.60); 1.6343 (1.12); 1.6224 (2.44); 1.6150 (2.67); 1.6085 (2.06); 1.6032 (4.85); 1.5948 (1.94); 1.5911 (2.67); 1.5837 (2.82); 1.5717 (1.35); 1.2981 (0.66); 1.2586 (1.01); 1.2345 (1.06); 1.2249 (0.36); 1.1926 (1.34); 1.1748 (2.58); 1.1570 (1.28); 1.1473 (0.34); 0.7231 (1.40); 0.7105 (5.76); 0.7033 (11.73); 0.6985 (9.49); 0.6919 (9.09); 0.6863 (5.46); 0.6820 (5.65); 0.6766 (11.59); 0.6695 (4.73); 0.6627 (5.68); 0.6599 (5.01); 0.6567 (9.57); 0.6497 (4.34); 0.6377 (1.64); 0.1460 (0.91); 0.0164 (0.34); 0.0157 (0.41); 0.0150 (0.49); 0.0142 (0.54); 0.0134 (0.65); 0.0127 (0.83); 0.0120 (0.98); 0.0112 (1.15); 0.0080 (7.65); 0.0061 (3.68); -0.0002 (247.94); -0.0066 (2.54); -0.0086 (7.18); -0.0132 (0.50); -0.0139 (0.49); -0.0147 (0.47); -0.0154 (0.42); -0.0161 (0.36); -0.0169 (0.37); -0.0176 (0.36); -0.1497 (0.91) |
| Beispiel Nr. 944, Lösungsmittel: DMSO-d6, NMR-Spektrometer: 399.95MHz |
| 4.0918 (0.39); 4.0770 (0.39); 4.0559 (1.39); 4.0381 (4.13); 4.0203 (4.14); 4.0025 (1.42); 3.3237 (5.23); 2.5105 (2.41); 2.5062 (4.77); 2.5016 (6.24); 2.4971 (4.55); 2.4927 (2.24); 1.9888 (16.00); 1.8742 (1.14); 1.8465 (1.83); 1.1928 (4.90); 1.1750 (9.21); 1.1572 (4.70); -0.0002 (3.55) |

### Herstellung der Ausgangsverbindungen

### 1-Methyl-4-nitro-3-(pentafluorethyl)-1H-pyrazol-5-carbonsäure

1-Methyl-4-nitro-3-(pentafluorethyl)-1H-pyrazol-5-carbonsäure wird in Analogie zu J. Med. Chem. **1987,** *30*, 91-96 durch Nitrierung von 1-Methyl-3-(pentafluorethyl)-1H-pyrazol-5-carbonsäure hergestellt.
¹H-NMR (600 MHz, d₆-DMSO): δ = 4,12 (s, 3H) ppm
HPLC-MS^{a)}: logP = 1,41; Masse (m/z) = 290 [M+H]⁺.

### 1-Methyl-4-(methylsulfanyl)-3-(pentafluorethyl)-1H-pyrazol-5-carbonsäure

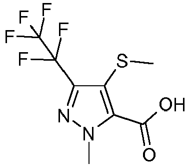

8,0 g (27,7 mmol) 1-Methyl-4-nitro-3-(pentafluorethyl)-1H-pyrazol-5-carbonsäure [Herstellung in Analogie zu J. Med. Chem. **1987,** *30*, 91-96] werden in 100 mL Dichlormethan gelöst. Die Lösung wird nacheinander mit 50 µL N,N-Dimehtylformamid und 10,5 g (83,0 mmol) Oxalylchlorid versetzt. Nach 0,5h bei Raumtemperatur, wird die Reaktion 0,5h unter Rückfluß erwärmt. Das Reaktiongemisch wird auf Raumtemperatur abgekühlt. Die Lösungsmittel und überschüssiges Oxalylchlorid werden am Rotationsverdampfer unter vermindertem Druck entfernt. Der Rückstand wird in Chloroform p.a. gelöst und langsam zu einer Suspension aus 5,56 g (41,5 mmol) Silber(I)cyanid, 100 mL Chloroform p.a. und 56 mL Methanol p.a. getropft. Die Mischung wird 8h unter Rückfluß erhitzt und anschließend auf Raumtemperatur abgekühlt. Das Reaktionsgemisch wird über eine kurze Kieselgelsäule filtriert und mit Dichlormethan nachgespült. Die Lösungsmittel werden am Rotationsverdampfer unter vermindertem Druck entfernt.

Man erhält 8,5 g Methyl-1-methyl-4-nitro-3-(pentafluorethyl)-1H-pyrazol-5-carboxylat. Das Rohprodukt wird ohne weitere Aufreinigung für die nächste Umsetzung verwendet.
¹H-NMR (600 MHz, d₆-DMSO): δ = 4,16 (s, 3H), 3,93 (s, 3H) ppm
HPLC-MS^{a)}: logP = 3,18; Masse (m/z) = 304 [M+H]⁺.

8,5 g (28,0 mmol) Methyl-1-methyl-4-nitro-3-(pentafluorethyl)-1*H*-pyrazol-5-carboxylat und 850 mg Palldium auf Kohle (10% Palladium) werden in 100 mL Methanol suspendiert. Nachdem Inertisieren des Autoklavs mit Stickstoff wird das Reaktionsgemisch unter 5 bar Wasserstoffatmosphäre gerührt. Nach 22h bei RT wird das Gemisch über Celite filtriert und das Lösungsmittel unter vermindertem Druck am Rotationsverdampfer entfernt. Das Rohprodukt wird in Dichlormethan aufgenommen und über Natriumsulfat filtriert. Das Dichlormethan wird anschließend unter vermindertem Druck am Rotationsverdampfer entfernt.

Man erhält 6,7 g (86%) Methyl-4-amino-1-methyl-3-(pentafluorethyl)-1*H-*pyrazol-5-carboxylat.
¹H-NMR (600 MHz, d₆-DMSO): δ = 5,32 (s, 2H), 4,07 (s, 3H), 3,86 (s, 3H) ppm
HPLC-MS^{a)}: logP = 2,52; Masse (m/z) = 274 [M+H]⁺.

2,0 g (7,32 mmol) Methyl-4-amino-1-methyl-3-(pentafluorethyl)-1*H-*pyrazol-5-carboxylat und 1,38 g (14,6 mmol) Dimethyldisulfid werden in 14 mL Acetonitril p.a. gelöst. Zu dieser Mischung wird langsam eine Lösung von 1,26 g (11,0 mmol) *tert*-Butylnitrit in 5 mL Acetonitril p.a. getropft. Nach Ende der Zugabe wird das Reaktionsgemisch noch 1h bei Raumtemperatur gerührt. Das Reaktionsgemisch wird anschließend auf 1 N Salzsäure gegossen. Die wässrige Phase wird dreimal mit Ethylacetat extrahiert. Die vereinigten organischen Phasen werden zweimal mit gesättigter Natriumchloridlösung gewaschen, über Magnesiumsulfat getrocknet und filtriert. Die Lösungsmittel werden am Rotationsverdampfer unter vermindertem Druck entfernt.

Man erhält 2,0 g (72%) Methyl-1-methyl-4-(methylsulfanyl)-3-(pentafluorethyl)-1H-pyrazol-5-carboxylat als 8:2 Gemisch aus dem gewünschten Produkt und dem Nebenprodukt Methyl-1-methyl-3-(pentafluorethyl)-1H-pyrazol-5-carboxylat.
¹H-NMR (400 MHz, d₆-DMSO): δ = 4,12 (s, 3H), 3,94 (s, 3H), 2,34 (s, 3H) ppm
HPLC-MS^{a)}: logP = 3,51; Masse (m/z) = 305 [M+H]⁺.

3,0 g Methyl-1-methyl-4-(methylsulfanyl)-3-(pentafluorethyl)-1H-pyrazol-5-carboxylat werden in 16 mL Methanol p.a. gelöst. Die Lösung wird anschließend mit 16,5 mL 2N wässriger Natronlauge versetzt und 16h bei Raumtemperatur gerührt. Das Reaktionsgemisch wird mit Ethylacetat verdünnt und dann mit 100 mL 1N Salzäure gewaschen. Die saure wässrige Phase wird zweimal mit zweimal mit Ethylacetat extrahiert. Die vereinigten organjschen Phasen werden mit gesättigter Natriumchloridlösung gewaschen, über Natriumsulfat getrocknet und filtriert. Die Lösungsmittel werden am Rotationsverdampfer unter vermindertem Druck entfernt.

Man erhält 2,5 g (90%) 1-Methyl-4-(methylsulfanyl)-3-(pentafluorethyl)-1H-pyrazol-5-carbonsäure als ein ca. 8:2 Gemisch aus dem gewünschten Produtk und dem Nebenprodukt 1-Methyl-3-(pentafluorethyl)-1H-pyrazol-5-carbonsäure.
¹H-NMR (400 MHz, d₆-DMSO): δ = 4,12 (s, 3H), 3,94 (s, 3H), 2,34 (s, 3H) ppm
HPLC-MS^{a)}: logP = 3,51; Masse (m/z) = 305 [M+H]⁺.

Mit Hilfe der oben beschriebenen Darstellungsverfahren A bis C wurden die in der Tabelle 1 und Tabelle 2 aufgeführten Verbindungen dargestellt.

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphomiumsalze wie beispielsweise Ammoniumsulfat oder Diammonium-hydrogenphosphat und /oder die Retention fördernde Mittel wie z. B. Dioctylsulfosuccinat oder Hydroxypropyl-guar polymers und/oder Humectants wie z.B. Glycerin und / oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR). Kapselkonzentrate (CS); diese und weitere mögliche Formuliertypen sind durch Crop Life International beschrieben: Catalog of Pesticide Formulation Types and International Coding System. Technical Monograph No. 2, 6th edition (http://www.croplife.org/files/documentspublished/1/en-us/PUB-TM/4147_PUB-TM_2008_05_01_Technical_Monograph 2_-_Revised_May_2008.pdf) gelesen June 17, 2010. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe, wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe, wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften, zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösemittel wie Dimethylformamid und Dimethylsulfoxid, sowie Wasser.

Grundsätzlich können alle geeigneten Lösemittel verwendet werden. Geeignete Lösemittel sind beispielsweise aromatische Kohlenwasserstoffe, wie z.B. Xylol, Toluol oder Alkylnaphthaline, chlorierte aromatische oder aliphatische Kohlenwasserstoffe, wie z.B. Chlorbenzol, Chlorethylen, oder Methylenchlorid, aliphatische Kohlenwasserstoffe, wie z.B. Cyclohexan, Paraffine, Erdölfraktionen, mineralische und pflanzliche Öle, Alkohole, wie z.B. Methanol, Ethanol, isoPropanol, Butanol oder Glykol sowie deren Ether und Ester, Ketone wie z.B. Aceton, Methylethylketon, Methylisobutylketon oder Cyclohexanon, stark polare Lösemittel, wie Dimethylsulfoxid, sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Nähr- und Spurennährstoffe, wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten, wie Gummiarabikum, Polyvinylalkohol, Polyvinylacetat, sowie natürliche Phospholipide, wie Kephaline und Lecithine, und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächespannung verringern oder die Visko-Elastizität erhöhen wie beispielsweise Dioctylsulfosuccinat und Hydroxypropyl-guar polymers beziehungsweise.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium und / oder Phosphonium-Salze wie beispielsweise Ammoniumsulfat oder Diammonium-hydrogenphosphat.

### Biologische Beispiele

An verschiedenen landwirtschaftlich relevanten Schadorganismen wurde die überlegene Wirkung beispielhaft ausgewählter Verbindungen gemäß Tabelle 1 gegenüber Verbindungen aus dem Stand der Technik belegt.

### Phaedon cochleariae - Sprühtest (PHAECO)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen die beispielhaft ausgewählten Verbindungen 1, 3, 4, 6, 7, 11 und 933 aus Tabelle 1 eine überlegene Wirksamkeit gegenüber ähnlichen Verbindungen aus dem Stand der Technik: siehe Tabelle 2.

### Myzus - Sprühtest (MYZUPE)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben *(Brassica pekinensis),* die von allen Stadien der Grünen Pfirsichblattlaus *(Myzus persicae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen die beispielhaft ausgewählten Verbindungen 1, 2, 3, 4, 7, 8, 9, 10, 201, 298 und 937 aus Tabelle 1 eine überlegene Wirksamkeit gegenüber ähnlichen Verbindungen aus dem Stand der Technik: siehe Tabelle 2.

### Tetranychus urticae - Sprühtest ; OP-resistent (TETRUR)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen die beispielhaft ausgewählten Verbindungen 1, 2, 3, 4, 7, 8, 9, 10, 11, 201, 926 und 933 aus Tabelle 1 eine überlegene Wirksamkeit gegenüber ähnlichen Verbindungen aus dem Stand der Technik: siehe Tabelle 2.

### Spodoptera frugiperda - Sprühtest (SPODFR)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Lösungsmittel: 78,0 Gewichtsteile Aceton 1,5 Gewichtsteile Dimethylformamid
Emulgator: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigt die beispielhaft ausgewählte Verbindungen 8 aus Tabelle 1 eine überlegene Wirksamkeit gegenüber einer ähnlichen Verbindungen aus dem Stand der Technik: siehe Tabelle 2.

**Tabelle 2**

| Substanz | Struktur | Objekt | Konzentration | % Wirkung dat | |
|---|---|---|---|---|---|
| Beispiel Ik-75 bekannt aus WO2010-051926 | 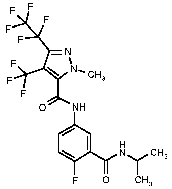 | PHAECO | 20 g/ha | 0 | 7 dat |
| | | MYZUPE | 500 g/ha | 0 | 6 dat |
| | | | 100 g/ha | 0 | 6 dat |
| | | TETRUR | 500 g/ha | 0 | 6 dat |
| | | | 100 g/ha | 0 | 6 dat |
| Beispiel Ik-30 bekannt aus WO2010-051926 | 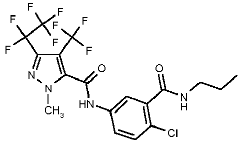 | MYZUPE | 20 g/ha | 0 | 6 dat |
| Beispiel 1 erfindungsgemäß | 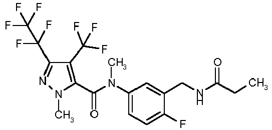 | PHAECO | 20 g/ha | 100 | 7 dat |
| | | MYZUPE | 20 g/ha | 90 | 6 dat |
| | | TETRUR | 500 g/ha | 100 | 6 dat |
| Beispiel 3 erfindungsgemäß | 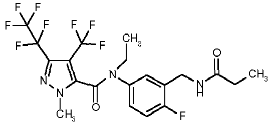 | PHAECO | 20 g/ha | 100 | 7 dat |
| | | MYZUPE | 20 g/ha | 100 | 6 dat |
| | | TETRUR | 500 g/ha | 100 | 6 dat |
| Beispiel 8 erfindungsgemäß | 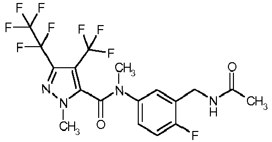 | MYZUPE | 500 g/ha | 100 | 6 dat |
| | | TETRUR | 500 g/ha | 100 | 6 dat |
| Beispiel 4 erfindungsgemäß | 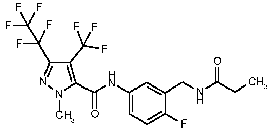 | PHAECO | 20 g/ha | 100 | 7 dat |
| | | MYZUPE | 500 g/ha | 100 | 6 dat |
| | | TETRUR | 500 g/ha | 100 | 6 dat |
| Beispiel 9 erfindungsgemäß | 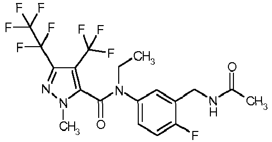 | MYZUPE | 20 g/ha | 100 | 6 dat |
| | | TETRUR | 500 g/ha | 100 | 6 dat |
| Beispiel 201 erfindungsgemäß | 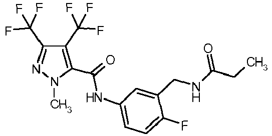 | MYZUPE | 20 g/ha | 100 | 6 dat |
| | | TETRUR | 100 g/ha | 100 | 6 dat |
| Beispiel Ik-155 bekannt aus WO2010-051926 | 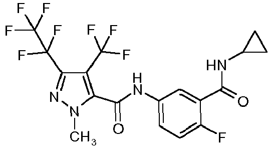 | MYZUPE | 100 g/ha | 0 | 6 dat |
| | | TETRUR | 100 g/ha | 0 | 6 dat |
| Beispiel Ik-132 bekannt aus WO2010-051926 | 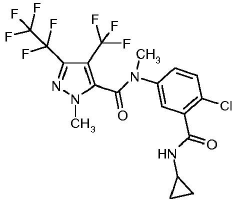 | MYZUPE | 100 g/ha | 0 | 6 dat |
| Beispiel 2 erfindungsgemäß | 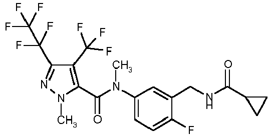 | MYZUPE | 100 g/ha | 90 | 6 dat |
| | | TETRUR | 100 g/ha | 100 | 6 dat |
| Beispiel Ik-13 bekannt aus WO2010-051926 | 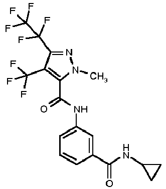 | MYZUPE | 500 g/ha | 0 | 6 dat |
| | | TETRUR | 500 g/ha | 0 | 6 dat |
| Beispiel Ik-132 bekannt aus WO2010-051926 | 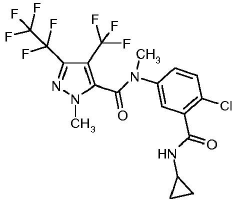 | MYZUPE | 100 g/ha | 0 | 6 dat |
| Beispiel 10 erfindungsgemäß | 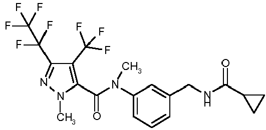 | MYZUPE | 100 g/ha | 100 | 6 dat |
| | | TETRUR | 500 g/ha | 100 | 6 dat |
| Beispiel Ik-74 bekannt aus WO2010-051926 | 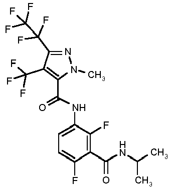 | PHAECO | 100 g/ha | 33 | 7 dat |
| | | SPODFR | 100 g/ha | 0 | 7 dat |
| | | MYZUPE | 500 g/ha | 0 | 6 dat |
| | | TETRUR | 500 g/ha | 0 | 6 dat |
| Beispiel 6 erfindungsgemäß | 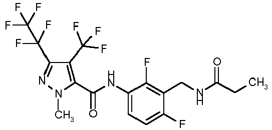 | PHAECO | 100 g/ha | 100 | 7 dat |
| | | SPODFR | 100 g/ha | 100 | 7 dat |
| Beispiel 7 erfindungsgemäß | 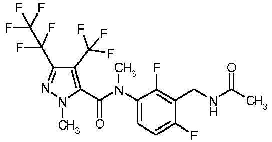 | PHAECO | 100 g/ha | 100 | 7 dat |
| | | MYZUPE | 500 g/ha | 100 | 6 dat |
| | | TETRUR | 500 g/ha | 100 | 6 dat |
| Beispiel 11 erfindungsgemäß | 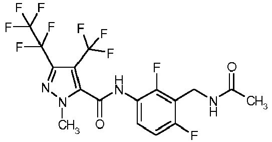 | PHAECO | 100 g/ha | 100 | 7 dat |
| | | TETRUR | 500 g/ha | 90 | 6 dat |
| Beispiel Ik-195 bekannt aus WO2010-051926 | 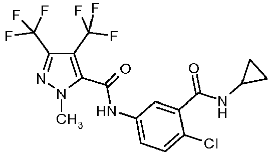 | MYZUPE | 20 g/ha | 0 | 6 dat |
| Beispiel 298 erfindungsgemäß | 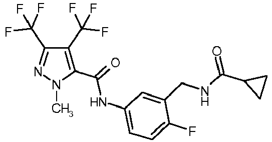 | MYZUPE | 20 g/ha | 90 | 6 dat |
| Substanz | Struktur | Objekt | Konzentration | % Wirkung dat | |
| Beispiel Ik-27 bekannt aus WO2010-051926 | 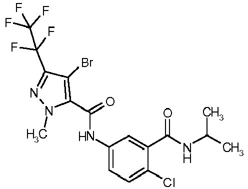 | TETRUR | 100 g/ha | 0 | 6 dat |
| Beispiel 926 erfindungsgemäß | 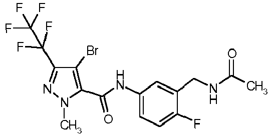 | TETRUR | 100 g/ha | 90 | 6 dat |
| Beispiel Ik-26 bekannt aus WO2010-051926 | 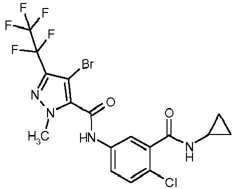 | TETRUR | 100 g/ha | 0 | 6 dat |
| | | PHAECO | 20 g/ha | 0 | 7 dat |
| Beispiel 933 erfindungsgemäß | 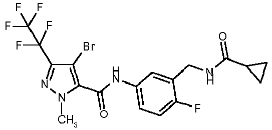 | TETRUR | 100 g/ha | 90 | 6 dat |
| | | PHAECO | 20 g/ha | 100 | 7 dat |
| Beispiel Ik-18 bekannt aus WO2010-051926 | 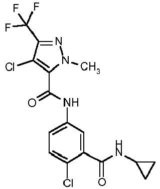 | MYZUPE | 100 g/ha | 0 | 6 dat |
| Beispiel 937 erfindungsgemäß | 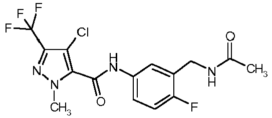 | MYZUPE | 100 g/ha | 90 | 6 dat |

**Tetranychus urticae - Sprühtest, OP-resistent (TETRUR)**

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen oder/und Penetrationsförderern werden diese jeweils in einer Konzentration von 1000 ppm der Präparatelösung zugefügt.

Bohnenpflanzen *(Phaseolus vulgaris),* die stark von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigt z. B. die Verbindung 13 aus Tabelle 1 eine überlegene Wirksamkeit gegenüber dem Stand der Technik: siehe Tabelle 3.

**Tetranychus urticae - Drenchtest, OP-resistent, systemische Behandlung (TETRUR sys.)**

| | | |
|---|---|---|
| Lösungsmittel: | 7 | Gewichtsteile Dimethylformamid |
| Emulgator: | 2 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Bei erforderlicher Zugabe von Ammoniumsalzen oder/und Penetrationsförderern werden diese jeweils in einer Konzentration von 1000 ppm der Präparatelösung zugefügt.

Bohnenpflanzen (*Phaseolus vulgaris*)*,* die stark von allen Stadien der Gemeinen Spinnmilbe *(Tetranychus urticae*) befallen sind, werden mit einer Wirkstoffzubereitung der gewünschten Konzentration angegossen.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigt z. B. die Verbindung 13 aus Tabelle 1 eine überlegene Wirksamkeit gegenüber dem Stand der Technik: siehe Tabelle 3.

**Tabelle 3**

| Substanz | Struktur | Objekt | Konzentration | % Wirkung dat | |
|---|---|---|---|---|---|
| Beispiel Ik-66 | 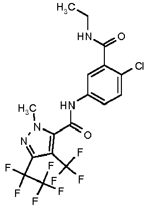 | TETRUR | 20 ppm | 0 | 7 dat |
| bekannt aus WO2010-051926 | | TETRUR sys | 20 ppm | 30 | 14 dat |
| Beispiel 13 | 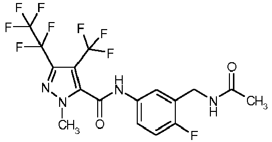 | TETRUR | 20 ppm | 100 | 7 dat |
| erfindungsgemäß | | TETRUR sys | 20 ppm | 90 | 14 dat |

## Patentansprüche

1. Verbindungen der allgemeinen Formel (Ia), 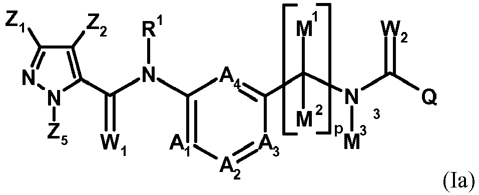 in denen
R¹ für Wasserstoff oder den gegebenenfalls substituierten Gruppierungen C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Cyano-C₁-C₂-alkyl, Aryl(C₁-C₃)-alkyl, Heteroaryl(C₁-C₃)-alkyl steht;
A₁ für CR²,
A₂ für CR³,
A₃ für CR⁴ oder Stickstoff, und
A₄ für CR⁵ stehen, wobei
R², R³, und R⁵ unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N,N*-Di-C₂-C₆alkylamino, *N*-C₂-C₇-Alkylaminocarbonyl, *N*-C₂-C₇-Cycloalkylaminocarbonyl oder C₂-C₄-Alkoxycarbonyl, stehen;
R⁴ für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N*,*N-*Di-C₂-C₆alkylamino, *N*-C₂-C₇-Alkylaminocarbonyl, *N-*C₂-C₇-Cycloalkylaminocarbonyl oder C₂-C₄-Alkoxycarbonyl oder *N*-Heteroaryl steht;
wenn die Gruppierung A₃ nicht für Stickstoff steht, können R³ und R⁴ gemeinsam mit dem Kohlenstoff, an den sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
R² und R³ können gemeinsam mit dem Kohlenstoff, an den sie gebunden sind, einen 6-gliedrigen Ring bilden, der 0, 1 oder 2 Stickstoffatome enthält und/oder 0 oder 1 Sauerstoffatom und/oder 0 oder 1 Schwefelatom enthält;
M¹ und M² jeweils unabhängig voneinander Wasserstoff, C₁-C₆-Alkyl, C₂-C₆-Alkenyl, C₂-C₆-Alkinyl, C₃-C₆-Cycloalkyl, C₂-C₇-Alkoxycarbonyl, C₁-C₆-Halogenalkyl, Cyano, oder Cyano-C₁-C₂-alkyl stehen, oder
M¹ und M² mit dem Kohlenstoffatom, an das sie gebunden sind, einen gegebenenfalls substituierten 3-, 4-, 5- oder 6-gliedrigen Ring bilden, der gegebenenfalls 0, 1 oder 2 Stickstoffatome und/oder 0, 1 oder 2 Sauerstoffatome und/oder 0, 1 oder 2 Schwefelatome enthält,
M³ für Wasserstoff, gegebenenfalls substituiertes C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Cyano-C₁-C₂-alkyl, Aryl(C₁-C₃)-alkyl oder Heteroaryl(C₁-C₃)-alkyl steht,
W¹ und W² unabhängig voneinander für Sauerstoff oder Schwefel stehen;
p den Wert 1 annimmt,
Q für Wasserstoff oder die gegebenenfalls substituierten Gruppierungen C₁-C₆-Alkyl, C₂-C₆-Alkenyl, C₂-C₆-Alkinyl, C₃-C₆-Cycloalkyl, Cyano-C₁-C₂-alkyl, C₁-C₅-Heterocycloalkyl, C₁-C₄-Alkoxy, C₄-C₇-Alkylcycloalkyl, C₄-C₇-Cycloalkylalkyl, C₂-C₇-Alkylcarbonyl, C₁-C₆-Alkylaldehyd, C₁-C₆-Hydroxyalkyl, C₂-C₇-Alkoxycarbonyl, C₁-C₆-Halogenalkyl, für Formyl, Hydroxy, Halogen, Cyano, Aryl, Heteroaryl oder für eine Gruppierung OR⁷, SR⁷, NR⁶R⁸ steht, wobei
R⁶ für Wasserstoff oder die gegebenenfalls substituierten Gruppierungen C₁-C₆-Alkyl, Aryl, Heteroaryl, C₂-C₆-Alkenyl, C₂-C₆-Alkinyl, C₃-C₆-Cycloalkyl, C₄-C₇-Alkylcycloalkyl und C₄-C₇-Cycloalkylalkyl, C₂-C₇-Alkylcarbonyl, C₂-C₇-Alkoxycarbonyl steht;
R⁷ ausgewählt ist aus den gegebenenfalls substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
R⁸ ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
wobei
Z¹ und Z² unabhängig voneinander für Wasserstoff, Halogen, Cyano, Nitro oder die gegebenenfalls substituierten Gruppierungen C₁-C₆-Alkyl, C₂-C₄-Alkenyl, C₂-C₄-Alkinyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆- Halogenalkylsulfinyl, C₁-C₆-Alkylsulfonyl, C₁-C₆-Halogenalkylsulfonyl, *N,N-*Di-(C₁-C₆)alkylamino, -S(O)₂NR¹³R¹⁴, -S(O)ₙR¹⁵, - S(O)(=NR¹⁶)R¹⁷ oder für gegebenenfalls substituiertes Phenyl oder Pyridinyl steht;
Z⁵ für Wasserstoff, gegebenenfalls substituiertes C₁-C₆-Alkyl, C₂-C₄-Alkenyl, C₂-C₄-Alkinyl, C₁-C₆-Halogenalkyl, C₃-C₆-Cycloalkyl, C₃-C₆-Halogencycloalkyl, oder gegebenenfalls substituiertes Phenyl und Pyridinyl steht;
R¹³ ausgewählt ist aus Wasserstoff oder einer der gegebenfalls substituierten Gruppierungen C₁-C₆-Alkyl, C₁-C₆-Halogenalkyl, C₂-C₆-Alkenyl, C₂-C₆-Alkinyl, C₃-C₆-Cycloalkyl, C₄-C₇-Alkylcycloalkyl, C₄-C₇-Cycloalkylalkyl, C₂-C₇-Alkylcarbonyl C₂-C₇-Alkoxycarbonyl, Aryl oder Heteroaryl;
R¹⁴ ausgewählt ist aus Wasserstoff oder einer der gegebenenfalls substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
R¹⁵ ausgewählt ist aus gegebenenfalls substituiertem C₁-C₆-Alkyl, C₂-C₆-Alkenyl, C₂-C₆-Alkinyl, C₃-C₆-Cycloalkyl, C₄-C₇-Alkylcycloalkyl, C₄-C₇-Cycloalkylalkyl, C₁-C₄-Haloalkyl, Aryl oder Heteroaryl;
R¹⁶ ausgewählt ist aus Wasserstoff oder einer der gegebenenfalls substituierten Gruppierungen C₁-C₆-Alkyl, C₁-C₆-Halogenalkyl, C₂-C₆-Alkenyl, C₂-C₆-Alkinyl, C₃-C₆-Cycloalkyl, C₄-C₇-Alkylcycloalkyl, C₄-C₇-Cycloalkylalkyl, C₂-C₇-Alkylcarbonyl, C₂-C₇-Alkoxycarbonyl, Cyano oder Nitro; und
R¹⁷ ausgewählt ist aus Wasserstoff, gegebenenfalls substituiertem C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl, Aryl oder Heteroaryl,
wobei für den Fall, dass die Gruppen R¹, R², R³, R⁴, R⁵, R⁶, R⁷, R⁸, R¹³, R¹⁴, R¹⁵, R¹⁶, R¹⁷, M¹, M², M³, Z¹, Z², Z⁵ und Q jeweils unabhängig voneinander substituiert sind, die Substituenten ein (1) Substituent oder mehrere Substituenten ausgewählt sind aus einer Gruppe bestehend aus Amino, Hydroxy, Halogen, Nitro, Cyano, Isocyano, Mercapto, Isothiocyanato, Carboxy, Carbonamid, SF₅, Aminosulfonyl, Alkyl, Cycloalkyl, Alkenyl, Cycloalkenyl, Alkinyl, *N-*Mono-alkyl-amino, *N,N*-Dialkylamino, *N*-Alkanoylamino, Alkoxy, Alkenyloxy, Alkinyloxy, Cycloalkoxy, Cycloalkenyloxy, Alkoxycarbonyl, Alkenyloxycarbonyl, Alkinyloxycarbonyl, Aryloxycarbonyl, Alkanoyl, Alkenylcarbonyl, Alkinylcarbonyl, Arylcarbonyl, Alkylthio, Cycloalkylthio, Alkenylthio, Cycloalkenylthio, Alkinylthio, Alkylsulfenyl und Alkylsulfinyl, wobei beide Enantiomere der Alkylsulfinylgruppe umfasst sind, Alkylsulfonyl, *N*-Mono-alkyl-aminosulfonyl, *N,N*-Dialkyl-aminosulfonyl, Alkylphosphinyl, Alkylphosphonyl, wobei für Alkylphosphinyl bzw. Alkylphosphonyl beide Enantiomere umfasst sind, *N*-Alkyl-aminocarbonyl, *N,N*-Dialkyl-amino-carbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl, Trialkylsilyl, Alkoxyalkyl, Alkylthioalkyl, Alkylthioalkoxy, Alkoxyalkoxy, Phenethyl, Benzyloxy, Halogenalkyl, Halogencycloalkyl, Halogenalkoxy, Halogenalkylthio, Halogenalkylsulfinyl, Halogenalkylsulfonyl, Halogenalkanoyl, Halogenalkylcarbonyl, Halogenalkoxycarbonyl, Halogenalkoxyalkoxy, Halogenalkoxyalkylthio, Halogenalkoxyalkanoyl und Halogenalkoxyalkyl.

2. Verbindungen der allgemeinen Formel (Ia) gemäß Anspruch 1,
in denen
R¹ für Wasserstoff, Methyl, Ethyl, n-Propyl, Isopropyl, n-Butyl, 2-Butinyl, Isobutyl, sec-Butyl, tert-Butyl, Methoxymethyl, Ethoxymethyl, Propoxymethyl, Methylcarbonyl, Ethylcarbonyl, n-Propylcarbonyl, Allyl, Propargyl, Isopropylcarbonyl, sec-Butylcarbonyl, tert-Butylcarbonyl, Methoxycarbonyl, Ethoxycarbonyl, n-Propoxycarbonyl, Isopropoxycarbonyl, sec-Butoxycarbonyl, tert-Butoxycarbonyl, Cyanomethyl, 2-Cyanoethyl steht;
A₁ für CR²,
A₂ für CR³,
A₃ für CR⁴ und
A₄ für CR⁵ stehen, wobei
R², R³, und R⁵ unabhängig voneinander für Wasserstoff, Fluor, Chlor, Brom, CN, NO₂, Methyl, Ethyl, Fluormethyl, Difluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Methylsulfonyl, Methylsulfinyl, Trifluormethylsulfonyl, Trifluormethylsulfinyl und *N*-Cyclopropylaminocarbonyl stehen;
R⁴ für Wasserstoff, Fluor, Chlor, Brom, CN, NO₂, Methyl, Ethyl, Fluormethyl, Difluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Methylsulfonyl, Methylsulfinyl, Trifluormethylsulfonyl, Trifluormethylsulfinyl, *N*-Cyclopropylaminocarbonyl stehen und *N*-Triazolyl;
R³ und R⁴ können gemeinsam mit dem Kohlenstoff, an den sie gebunden sind, einen 6-R² und R³ können gemeinsam mit dem Kohlenstoff, an den sie gebunden sind, einen 6-gliedrigen Ring bilden, der 0, 1 oder 2 Stickstoffatome enthält und/oder 0 oder 1 Sauerstoffatom und/oder 0 oder 1 Schwefelatom enthält;
M¹ für Wasserstoff steht,
M² für Wasserstoff, C₁-C₃-Alkyl, C₂-C₃-Alkenyl, C₂-C₃-Alkoxycarbonyl, Cyano, oder Cyano-C₁-C₂-alkyl steht,
M³ für Wasserstoff, Methyl, Ethyl, n-Propyl, Methoxymethyl, Allyl oder Cyanomethyl steht;
W¹ und W² jeweils für Sauerstoff stehen;
p den Wert 1 annimmt,
Q für Wasserstoff oder die gegebenenfalls substituierten Gruppierungen C₁-C₆-Alkyl, C₂-C₆-Alkenyl, C₂-C₆-Alkinyl, C₃-C₆-Cycloalkyl, Cyano-C₁-C₂-alkyl, C₁-C₅-Heterocycloalkyl, C₁-C₄-Alkoxy, C₄-C₇-Alkylcycloalkyl, C₄-C₇-Cycloalkylalkyl, C₂-C₇-Alkylcarbonyl, C₁-C₆-Alkylaldehyd, C₁-C₆-Hydroxyalkyl, C₂-C₇-Alkoxycarbonyl, C₁-C₆-Halogenalkyl, Cyano, Aryl, Heteroaryl, oder für eine Gruppierung OR⁷, SR⁷ oder NR⁶R⁸ steht, wobei
R⁶ für Wasserstoff oder C₁-C₃-Alkyl steht;
R⁷ ausgewählt ist aus den gegebenenfalls substituierten Gruppierungen C₁-C₄-Alkyl, C₂-C₄-Alkenyl, C₂-C₄-Alkinyl oder C₃-C₆-Cycloalkyl;
R⁸ Wasserstoff ist;
wobei
Z¹ und Z² unabhängig voneinander für Wasserstoff, Chlor, Brom, Iod, Cyano, Nitro oder die gegebenenfalls substituierten Gruppierungen C₁-C₄-Alkyl, C₁-C₄-Alkenyl, C₁-C₄-Halogenalkyl, C₃-C₆-Halogencycloalkyl, C₁-C₄-Alkylthio, C₁-C₄-Alkylsulfinyl, C₁-C₄-Alkylsulfonyl steht.
Z⁵ für Wasserstoff oder die gegebenenfalls substituierten Gruppierungen C₁-C₄-Alkyl, C₂-C₄-Alkenyl, C₂-C₄-Alkinyl, C₁-C₄-Halogenalkyl, C₃-C₄-Cycloalkyl oder C₁-C₃-Alkoxy steht,
wobei für den Fall, dass die Gruppen R⁷, Z¹, Z², Z⁵ und Q jeweils unabhängig voneinander substituiert sind, die Substituenten ein (1) Substituent oder mehrere Substituenten ausgewählt sind aus einer Gruppe bestehend aus Amino, Hydroxy, Halogen, Nitro, Cyano, Isocyano, Mercapto, Isothiocyanato, Carboxy, Carbonamid, SF₅, Aminosulfonyl, Alkyl, Cycloalkyl, Alkenyl, Cycloalkenyl, Alkinyl, *N*-Mono-alkyl-amino, *N,N*-Dialkylamino, *N*-Alkanoylamino, Alkoxy, Alkenyloxy, Alkinyloxy, Cycloalkoxy, Cycloalkenyloxy, Alkoxycarbonyl, Alkenyloxycarbonyl, Alkinyloxycarbonyl, Aryloxycarbonyl, Alkanoyl, Alkenylcarbonyl, Alkinylcarbonyl, Arylcarbonyl, Alkylthio, Cycloalkylthio, Alkenylthio, Cycloalkenylthio, Alkinylthio, Alkylsulfenyl und Alkylsulfinyl, wobei beide Enantiomere der Alkylsulfinylgruppe umfasst sind, Alkylsulfonyl, *N*-Mono-alkyl-aminosulfonyl, *N,N*-Dialkyl-aminosulfonyl, Alkylphosphinyl, Alkylphosphonyl, wobei für Alkylphosphinyl bzw. Alkylphosphonyl beide Enantiomere umfasst sind, *N*-Alkyl-aminocarbonyl, *N,N*-Dialkyl-amino-carbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl, Trialkylsilyl, Alkoxyalkyl, Alkylthioalkyl, Alkylthioalkoxy, Alkoxyalkoxy, Phenethyl, Benzyloxy, Halogenalkyl, Halogencycloalkyl, Halogenalkoxy, Halogenalkylthio, Halogenalkylsulfinyl, Halogenalkylsulfonyl, Halogenalkanoyl, Halogenalkylcarbonyl, Halogenalkoxycarbonyl, Halogenalkoxyalkoxy, Halogenalkoxyalkylthio, Halogenalkoxyalkanoyl und Halogenalkoxyalkyl.

3. Verbindungen der Formel (Ia) gemäß Anspruch 1 oder 2,
in denen
R¹ für Wasserstoff, Methyl, Ethyl, n-Propyl, Isopropyl, n-Butyl, 2-Butinyl, Isobutyl, sec-Butyl, Methoxymethyl, Ethoxymethyl, Methylcarbonyl, Ethylcarbonyl, Allyl, Propargyl, Isopropylcarbonyl, Methoxycarbonyl, Ethoxycarbonyl, Isopropoxycarbonyl, Cyanomethyl, 2-Cyanoethyl steht;
A₁ für CR²,
A₂ für CR³,
A₃ für CR⁴ und
A₄ für CR⁵ stehen, und wobei
R² für Wasserstoff oder Chlor steht,
R³ und R⁴ und R⁵ unabhängig voneinander für Wasserstoff, Fluor, Chlor, Brom, Methyl oder Ethyl stehen,
M¹ für Wasserstoff steht;
M² für Wasserstoff oder Methyl steht;
M³ für Wasserstoff steht;
W¹ und W² jeweils für Sauerstoff stehen;
p den Wert 1 annimmt,
Q für Wasserstoff, Methyl, Ethyl, *n*-Propyl, *n*-Butyl, *t*-Butyl, 1-Methylethyl, 1,1-Dimethylethyl, 1-Methylpropyl, 1,1-Dimethylpropyl, 2-Methylpropyl, 2-Methylbutyl, 3-Methylbutyl, 2-Hydroxyethyl, Cyanomethyl, 2-Cyanoethyl, 3-Cyanopropyl, 1,1-Difluormethyl, Trifluormethyl, 2-Fluorethyl, 2,2,2-Trifluorethyl, Chlormethyl, 1-Chlorethyl, 2-Chlorethyl, 3-Chlorpropyl, 2,2-Difluorpropyl, Cyclopropyl, 1-Methylcyclopropyl, 1-Cyanocyclopropyl, 2-Methylcyclopropyl, Cyclopropylmethyl, 2,2-Dichlorcyclopropyl, 2,2-Dichlor-1-methylcyclopropyl, 2,2-Difluorcyclopropyl, 2-Fluorcyclopropyl, 1,1'-Bi(cyclopropyl)-1-yl, (2,2-Difluorcyclopropyl)methyl, Cyclobutyl, 3-Ethyloxetan-3-yl, Cyclopentyl, Cyclopentylmethyl, 1-(Cyclopent-1-en-1-yl)methyl, (2-Methyl-1,3-dioxolan-2-yl)methyl, Tetrahydrofuran-2-ylmethyl, Cyclohexyl, 2-Trifluormethylcyclohexyl, 3-Trifluormethylcyclohexyl, 4-Trifluormethylcyclohexyl, 2-Chlorcyclohexyl, 3-Chlorcyclohexyl, 4-Chlorcyclohexyl, 5-Methyl-1,3-dioxan-5-yl, 1-Acetylpiperidin-4-yl, 1-Methylpiperidin-4-yl, Prop-1-en-1-yl, 1-Methylprop-1-en-1-yl, 2-Methylprop-1-en-1-yl, Prop-2-enyl, But-2-en-1-yl, 3-Methylbut-1-en-1-yl, Prop-1-in-1-yl, (4-Methyl-1,2,5-oxadiazol-3-yl)methyl, (3,5-Dimethyl-1,2-oxazol-4-yl)methyl, 1*H*-Tetrazol-5-ylmethyl, (5-Methyl-2-thienyl)methyl, 2-Furylmethyl, (3-Methyl-1,2-oxazol-5-yl)methyl, 3-Thienylmethyl, Benzyl, 4-Chlorbenzyl, 3-Chlorbenzyl, 2-Chlorbenzyl, 1-(4-Chlorphenyl)ethyl, 1-(4-Methylphenyl)ethyl, 1-(3-Chlorphenyl)ethyl, 1-(2-Chlorphenyl)ethyl, 1-(4-Chlorphenyl)cyclopropyl, Pyrimidin-2-ylmethyl, Methoxy, (Methylsulfanyl)methyl, (Methylsulfinyl)methyl, (Methylsulfonyl)methyl, Phenyl, 2-Chlorophenyl, 3-Chlorophenyl, 4-Chlorophenyl, 2-Trifluormethylphenyl, 3-Trifluormethylphenyl, 4-Trifluormethylphenyl, 2-Methylphenyl, 3-Methylphenyl, 4-Methylphenyl, Pyridin-2-yl, Pyridin-3-yl, Pyridin-4-yl, 3-Chlorpyridin-4-yl, 2-Chlorpyridin-4-yl, 2-Methylpyridin-4-yl, 6-Methylpyridin-3-yl, 5-Chlorpyridin-3-yl, 4-Chlropyridin-3-yl, Pyrimidin-5-yl, (6-Chlorpyridin-3-yl)methyl, Methoxycarbonyl, Ethoxycarbonyl, *N-*Methylcarboxamid, *N*-Ethylcarboxamid, *N*-Cyclopropylcarboxamid, *N-*Cyclopropylmethylcarboxamid, 3-Methoxy-3-oxopropanoyl, 3-(Methylamino)-3-oxopropanoyl, 3-(Cyclopropylamino)-3-oxopropanoyl, 3-(Cyclopropylmethylamino)-3-oxopropanoyl steht;
Z¹ für 1-Chlorcyclopropyl, Trifluormethyl oder Pentafluorethyl, und
Z² für Chlor oder Trifluormethyl steht; und
Z⁵ für Methyl und Ethyl.

4. Verbindungen der allgemeinen Formel (III), 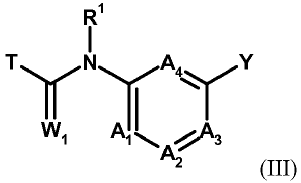 in denen
R¹ für Wasserstoff, gegebenenfalls substituiertes C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Cyano-C₁-C₂-alkyl, Aryl(C₁-C₃)-alkyl oder Heteroaryl(C₁-C₃)-alkyl steht,
die chemische Gruppierung
A₁ für CR²,
A₂ für CR³,
A₃ für CR⁴ oder Stickstoff, und
A₄ für CR⁵ stehen,
R², R³, R⁴ und R⁵ unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfmyl, C₁-C₆-Halogenalkylsulfinyl, C₁-C₆-Alkylsulfonyl, C₁-C₆-Halogenalkylsulfonyl, C₁-C₆-Alkylamino, *N,N*-Di-C₂-C₆-alkylamino, *N*-C₂-C₇-Alkylaminocarbonyl, *N*-C₂-C₇-Cycloalkylaminocarbonyl, C₂-C₄-Alkoxycarbonyl, Aryl, Heteroaryl oder N-Heteroaryl stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 5- oder 6-gliedrigen Ring bilden, der 0, 1 oder 2 Stickstoffatome enthält;
T für 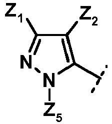 (T-6) steht,
wobei
Z¹ für Wasserstoff, Halogen, Cyano, Nitro, gegebenenfalls substituiertes C₁-C₆-Alkyl, C₂-C₄-Alkenyl, C₂-C₄-Alkinyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N,N*-Di-(C₁-C₆)alkylamino, - C(=W)NR¹¹R¹⁰, -C(=W)OR¹², -S(O)₂NR¹³R¹⁴, -S(O)ₙR¹⁵, -S(O)(=NR¹⁶)R¹⁷ oder gegebenenfalls substituiertes Phenyl und Pyridinyl steht;
Z² für C₁-C₆-Halogenalkyl steht
Z⁵ für Wasserstoff, gegebenenfalls substituiertes C₁-C₆-Alkyl, C₂-C₄-Alkenyl, C₂-C₄-Alkinyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N,N*-Di-(C₁-C₆)alkylamino, -C(=W)NR¹¹R¹⁰, -C(=W)OR¹², - S(O)₂NR¹³R¹⁴, -S(O)ₙR¹⁵, -S(O)(=NR¹⁶)R¹⁷ oder gegebenenfalls substituiertes Phenyl und Pyridinyl steht;
R¹⁰ ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, C₂-C₇-Alkylcarbonyl und C₂-C₇-Alkoxycarbonyl;
R¹¹ ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, C₂-C₇-Alkylcarbonyl und C₂-C₇-Alkoxycarbonyl, Aryl oder Heteroaryl;
R¹² ausgewählt ist aus Wasserstoff oder den gegebenenfalls substituierten Gruppierung C₁-C₆-Alkyl, C₁-C₆-Halogenalkyl, C₂-C₆-Alkenyl, C₂-C₆-Halogenalkenyl, C₂-C₆-Alkinyl, C₃-C₆-Cycloalkyl, C₃-C₆-Halogencycloalkyl, C₄-C₇-Alkylcycloalkyl und C₄-C₇-Cycloalkylalkyl, Aryl oder Heteroaryl;
R¹³ ausgewählt ist aus Wasserstoff oder den gegebenenfalls substituierten Gruppierungen C₁-C₆-Alkyl, C₁-C₆-Halogenalkyl, C₂-C₆-Alkenyl, C₂-C₆-Alkinyl, C₃-C₆-Cycloalkyl, C₄-C₇-Alkylcycloalkyl, C₄-C₇-Cycloalkylalkyl, C₂-C₇-Alkylcarbonyl und C₂-C₇-Alkoxycarbonyl, Aryl oder Heteroaryl;
R¹⁴ ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
R¹⁵ ausgewählt ist aus den gegebenenfalls substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl, C₁-C₄-Haloalkyl, Aryl oder Heteroaryl;
R¹⁶ ausgewählt ist aus Wasserstoff oder den gegebenenfalls substituierten Gruppierungen C₁-C₆-Alkyl, C₁-C₆-Halogenalkyl, C₂-C₆-Alkenyl, C₂-C₆-Alkinyl, C₃-C₆-Cycloalkyl, C₄-C₇-Alkylcycloalkyl, C₄-C₇-Cycloalkylalkyl, C₂-C₇-Alkylcarbonyl, C₂-C₇-Alkoxycarbonyl, Cyano oder Nitro;
R¹⁷ ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl, C₄-C₇-Cycloalkyl, Aryl oder Heteroaryl;
n die Werte 0, 1 oder 2 annehmen kann;
Y für CN oder CH₂NH₂ steht.

5. Verbindungen der allgemeinen Formel (III) gemäß Anspruch 4 in denen
R¹ für Wasserstoff oder den gegebenenfalls substituierten Gruppierungen C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Cyano-C₁-C₂-alkyl, Aryl(C₁-C₃)-alkyl, Heteroaryl(C₁-C₃)-alkyl steht;
die chemische Gruppierung
A₁ für CR²,
A₂ für CR³,
A₃ für CR⁴, und
A₄ für CR⁵ stehen,
R², R³, und R⁵ unabhängig voneinander für Wasserstoff, Fluor, Chlor, Brom, CN, NO₂, Methyl, Ethyl, Fluormethyl, Difluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Methylsulfonyl, Methylsulfinyl, Trifluormethylsulfonyl, Trifluormethylsulfinyl und *N-*Cyclopropylaminocarbonyl stehen;
R⁴ für Wasserstoff, Fluor, Chlor, Brom, CN, NO₂, Methyl, Ethyl, Fluormethyl, Difluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Methylsulfonyl, Methylsulfinyl, Trifluormethylsulfonyl, Trifluormethylsulfinyl, *N*-Cyclopropylaminocarbonyl stehen und *N-*Triazolyl;
R³ und R⁴ können gemeinsam mit dem Kohlenstoff, an den sie gebunden sind, einen 6-gliedrigen Ring bilden, der 0, 1 oder 2 Stickstoffatome und/oder 0 oder 1 Sauerstoffatom und/oder 0 oder 1 Schwefelatom enthält, oder
R² und R³ können gemeinsam mit dem Kohlenstoff, an den sie gebunden sind, einen 6-gliedrigen Ring bilden, der 0, 1 oder 2 Stickstoffatome enthält und/oder 0 oder 1 Sauerstoffatom und/oder 0 oder 1 Schwefelatom enthält;
T für 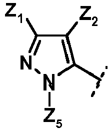 (T-6) steht,
wobei
Z¹ für Wasserstoff, Chlor, Brom, Iod, Cyano, Nitro oder die gegebenenfalls substituierten Gruppierungen C₁-C₄-Alkyl, C₁-C₄-Alkenyl, C₁-C₄-Halogenalkyl, C₃-C₆-Halogencycloalkyl, C₁-C₄-Alkylthio, C₁-C₄-Alkylsulfinyl, C₁-C₄-Alkylsulfonyl steht.
Z² für C₁-C₄-Halogenalkyl steht,
Z⁵ für Wasserstoff oder die gegebenenfalls substituierten Gruppierungen C₁-C₄-Alkyl, C₂-C₄-Alkenyl, C₂-C₄-Alkinyl, C₁-C₄-Halogenalkyl, C₃-C₄-Cycloalkyl oder C₁-C₃-Alkoxy steht;
n die Werte 0, 1 oder 2 annehmen kann;
Y für CN oder CH₂NH₂ steht.

6. Verbindungen der allgemeinen Formel (III) gemäß einem der Ansprüche 4 oder 5, in denen
R¹ für Wasserstoff, Methyl, Ethyl, n-Propyl, Isopropyl, n-Butyl, 2-Butinyl, Isobutyl, sec-Butyl, Methoxymethyl, Ethoxymethyl, Methylcarbonyl, Ethylcarbonyl, Allyl, Propargyl, Isopropylcarbonyl, Methoxycarbonyl, Ethoxycarbonyl, Isopropoxycarbonyl, Cyanomethyl, 2-Cyanoethyl steht;
die chemische Gruppierung
A₁ für CR²,
A₂ für CR³,
A₃ für CR⁴, und
A₄ für CR⁵ stehen,
R² für Wasserstoff oder Chlor steht,
R³ und R⁴ und R⁵ unabhängig voneinander für Wasserstoff, Fluor, Chlor, Brom, Methyl oder Ethyl stehen,
T für 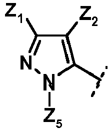 (T-6) steht,
wobei
Z¹ für Trifluormethyl oder Pentafluorethyl, und
Z² für Trifluormethyl steht;
Z⁵ für Methyl und Ethyl,
Y für CN oder CH₂NH₂ steht.

7. Verwendung von Verbindungen der allgemeinen Formel (Ia) gemäß einem der Ansprüche 1 bis 4 zur Bekämpfung von Insekten, Spinnentieren und Nematoden, wobei die chirurgische, therapeutische und diagnostische Behandlung des menschlichen oder tierischen Körpers ausgeschlossen ist..

8. Pharmazeutische Zusammensetzungen, enthaltend wenigstens eine Verbindung gemäß einem der Ansprüche 1 bis 4.

9. Verbindungen gemäß einem der Ansprüche 1 bis 4 zur Verwendung als Arzneimittel.

10. Verwendung von Verbindungen der allgemeinen Formel (Ia) gemäß einem der Ansprüche 1 bis 4 sowie Anspruch 7 zur Herstellung pharmazeutischer Zusammensetzungen zur Bekämpfung von Parasiten auf Tieren.

11. Verfahren zur Herstellung von Pflanzenschutzmitteln enthaltend Verbindungen der allgemeinen Formel (Ia) gemäß einem der Ansprüche 1 bis 4 sowie übliche Streckmittel und/oder oberflächenaktive Substanzen.

12. Verfahren zum Bekämpfen von Schädlingen, **dadurch gekennzeichnet, dass** man eine Verbindung der allgemeinen Formel (Ia) gemäß einem der Ansprüche 1 bis 4 auf die Schädlinge und/oder ihren Lebensraum einwirken lässt, wobei die chirurgische, therapeutische und diagnostische Behandlung des menschlichen oder tierischen Körpers ausgeschlossen ist..

13. Verwendung von Verbindungen der allgemeinen Formel (Ia) gemäß einem der Ansprüche 1 bis 4 zum Schutz des Vermehrungsmaterials von Pflanzen, insbesondere von Saatgut.

## Claims

1. Compounds of the general formula (Ia) 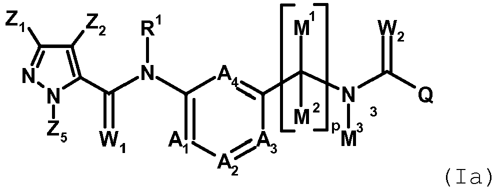 in which
R¹ represents hydrogen or the optionally substituted groupings C₁-C₆-alkyl, C₂-C₆-alkenyl, C₂-C₆-alkynyl, C₃-C₇-cycloalkyl, C₁-C₆-alkylcarbonyl, C₁-C₆-alkoxycarbonyl, cyano-C₁-C₂-alkyl, aryl-(C₁-C₃)-alkyl, heteroaryl-(C₁-C₃)-alkyl;
A₁ represents CR²,
A₂ represents CR³,
A₃ represents CR⁴ or nitrogen and
A₄ represents CR⁵, where
R², R³ and R⁵ independently of one another represent hydrogen, halogen, CN, NO₂, optionally substituted C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, C₁-C₆-alkylamino, *N,N*-di-C₂-C₆-alkylamino, *N-*C₂-C₇-alkylaminocarbonyl, *N*-C₂-C₇-cycloalkylaminocarbonyl or C₂-C₄-alkoxycarbonyl;
R⁴ represents hydrogen, halogen, CN, NO₂, optionally substituted C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, C₁-C₆-alkylamino, *N,N*-di-C₂-C₆-alkylamino, *N-*C₂-C₇-alkylaminocarbonyl, *N*-C₂-C₇-cycloalkylaminocarbonyl or C₂-C₄-alkoxycarbonyl or *N*-heteroaryl;
if the grouping A₃ does not represent nitrogen, R³ and R⁴ together with the carbon to which they are attached may form a 5- or 6-membered ring which contains 0, 1 or 2 nitrogen atoms and/or 0 or 1 oxygen atom and/or 0 or 1 sulphur atom, or
R² and R³ together with the carbon to which they are attached may form a 6-membered ring which contains 0, 1 or 2 nitrogen atoms and/or 0 or 1 oxygen atom and/or 0 or 1 sulphur atom;
M¹ and M² each independently of one another represent hydrogen, C₁-C₆-alkyl, C₂-C₆-alkenyl, C₂-C₆-alkynyl, C₃-C₆-cycloalkyl, C₂-C₇-alkoxycarbonyl, C₁-C₆-haloalkyl, cyano or cyano-C₁-C₂-alkyl, or
M¹ and M² with the carbon atom to which they are attached form an optionally substituted 3-, 4-, 5- or 6-membered ring which optionally contains 0, 1 or 2 nitrogen atoms and/or 0, 1 or 2 oxygen atoms and/or 0, 1 or 2 sulphur atoms,
M³ represents hydrogen, optionally substituted C₁-C₆-alkyl, C₂-C₆-alkenyl, C₂-C₆-alkynyl, C₃-C₇-cycloalkyl, C₁-C₆-alkylcarbonyl, C₁-C₆-alkoxycarbonyl, cyano-C₁-C₂-alkyl, aryl-(C₁-C₃)-alkyl or heteroaryl-(C₁-C₃)-alkyl,
W¹ and W² independently of one another represent oxygen or sulphur;
p assumes the value 1,
Q represents hydrogen or the optionally substituted groupings C₁-C₆-alkyl, C₂-C₆-alkenyl, C₂-C₆-alkynyl, C₃-C₆-cycloalkyl, cyano-C₁-C₂-alkyl, C₁-C₅-heterocycloalkyl, C₁-C₄-alkoxy, C₄-C₇-alkylcycloalkyl, C₄-C₇-cycloalkylalkyl, C₂-C₇-alkylcarbonyl, C₁-C₆-alkylaldehyde, C₁-C₆-hydroxyalkyl, C₂-C₇-alkoxycarbonyl, C₁-C₆-haloalkyl, represents formyl, hydroxy, halogen, cyano, aryl, heteroaryl or represents a grouping OR⁷, SR⁷, NR⁶R⁸, where
R⁶ represents hydrogen or the optionally substituted groupings C₁-C₆-alkyl, aryl, heteroaryl, C₂-C₆-alkenyl, C₂-C₆-alkynyl, C₃-C₆-cycloalkyl, C₄-C₇-alkylcycloalkyl and C₄-C₇-cycloalkylalkyl, C₂-C₇-alkylcarbonyl, C₂-C₇-alkoxycarbonyl;
R⁷ is selected from the optionally substituted groupings C₁-C₆-alkyl, C₂-C₆-alkenyl, C₂-C₆-alkynyl, C₃-C₆-cycloalkyl, C₄-C₇-alkylcycloalkyl and C₄-C₇-cycloalkylalkyl;
R⁸ is selected from hydrogen or the optionally substituted groupings C₁-C₆-alkyl, C₂-C₆-alkenyl, C₂-C₆-alkynyl, C₃-C₆-cycloalkyl, C₄-C₇-alkylcycloalkyl and C₄-C₇-cycloalkylalkyl;
where
Z¹ and Z² independently of one another represent hydrogen, halogen, cyano, nitro or the optionally substituted groupings C₁-C₆-alkyl, C₂-C₄-alkenyl, C₂-C₄-alkynyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, *N,N*-di-(C₁*-*C₆)*-*alkylamino, -S (O) ₂NR¹³R¹⁴, - S(O)ₙR¹⁵, -S(O)(=NR¹⁶)R¹⁷ or represent optionally substituted phenyl or pyridinyl;
Z⁵ represents hydrogen, optionally substituted C₁-C₆-alkyl, C₂-C₄-alkenyl, C₂-C₄-alkynyl, C₁-C₆-haloalkyl, C₃-C₆-cycloalkyl, C₃-C₆-halocycloalkyl, or optionally substituted phenyl or pyridinyl;
R¹³ is selected from hydrogen or one of the optionally substituted groupings C₁-C₆-alkyl, C₁-C₆-haloalkyl, C₂-C₆-alkenyl, C₂-C₆-alkynyl, C₃-C₆-cycloalkyl, C₄-C₇-alkylcycloalkyl, C₄-C₇-cycloalkylalkyl, C₂-C₇-alkylcarbonyl, C₂-C₇-alkoxycarbonyl, aryl or heteroaryl;
R¹⁴ is selected from hydrogen or one of the optionally substituted groupings C₁-C₆-alkyl, C₂-C₆-alkenyl, C₂-C₆-alkynyl, C₃-C₆-cycloalkyl, C₄-C₇-alkylcycloalkyl and C₄-C₇-cycloalkylalkyl;
R¹⁵ is selected from optionally substituted C₁-C₆-alkyl, C₂-C₆-alkenyl, C₂-C₆-alkynyl, C₃-C₆-cycloalkyl, C₄-C₇-alkylcycloalkyl, C₄-C₇-cycloalkylalkyl, C₁-C₄-haloalkyl, aryl or heteroaryl;
R¹⁶ is selected from hydrogen or one of the optionally substituted groupings C₁-C₆-alkyl, C₁-C₆-haloalkyl, C₂-C₆-alkenyl, C₂-C₆-alkynyl, C₃-C₆-cycloalkyl, C₄-C₇-alkylcycloalkyl, C₄-C₇-cycloalkylalkyl, C₂-C₇-alkylcarbonyl, C₂-C₇-alkoxycarbonyl, cyano or nitro; and
R¹⁷ is selected from hydrogen, optionally substituted C₁-C₆-alkyl, C₂-C₆-alkenyl, C₂-C₆-alkynyl, C₃-C₆-cycloalkyl, C₄-C₇-alkylcycloalkyl and C₄-C₇-cycloalkylalkyl, aryl or heteroaryl,
where, if the groups R¹, R², R³, R⁴, R⁵, R⁶, R⁷, R⁸, R¹³, R¹⁴, R¹⁵, R¹⁶, R¹⁷, M¹, M², M³. Z¹, Z², Z⁵ and Q are in each case substituted independently of one another, the substituents are one (1) substituent or a plurality of substituents selected from a group consisting of amino, hydroxy, halogen, nitro, cyano, isocyano, mercapto, isothiocyanato, carboxy, carboxamide, SF₅, aminosulfonyl, alkyl, cycloalkyl, alkenyl, cycloalkenyl, alkenyl, *N-*mono-alkyl-amino, *N,N*-dialkylamino, *N-*alkanoylamino, alkoxy, alkenyloxy alkinyloxy, cycloalkoxy, cycloalkenyloxy, alkoxycarbonyl, alkenyloxycarbonyl, alkinyloxycarbonyl, aryloxycarbonyl, alkanoyl, alkenylcarbonyl, alkinylcarbonyl, arylcarbonyl, alkylthio, cycloalkylthio, alkenylthio, cycloalkenylthio, alkinylthio, alkylsulfenyl and alkylsulfinyl, where both enantiomers of the alkylsulfinyl group are included, alkylsulfonyl, *N*-monoalkyl aminosulfonyl, *N-N*-dialkyl-aminosulfonyl, alkylphosphinyl, alkylphosphonyl, where for alkylphosphinyl and alkylphosphonyl both enantiomers are included, *N*-alkylaminocarbonyl, *N,N*-dialkylaminocarbonyl, *N*-alkanoylaminocarbonyl, *N*-alkanoyl *N*-alkylaminocarbonyl, aryl, aryloxy, benzyl, benzyloxy, benzylthio, arylthio, arylamino, benzylamino, heterocyclyl, trialkylsilyl, alkoxyalkyl, alkylthioalkyl, alkylthioalkoxy, alkoxyalkoxy, phenethyl, benzyloxy, halogenalkyl, halogencycloalkyl, halogenalkoxy, halogenalkylthio, halogenalkylsulfinyl, halogenalkylsulfonyl, halogenalkanoyl, halogenalkylcarbonyl, halogenalkoxycarbonyl, halogenalkoxyalkoxy, halogenalkoxyalkylthio, halogenalkoxyalkanoyl and halogenalkoxyalkyl.

2. Compounds of the general formula (Ia) according to Claim 1
in which
R¹ represents hydrogen, methyl, ethyl, n-propyl, isopropyl, n-butyl, 2-butynyl, isobutyl, sec-butyl, tert-butyl, methoxymethyl, ethoxymethyl, propoxymethyl, methylcarbonyl, ethylcarbonyl, n-propylcarbonyl, allyl, propargyl, isopropylcarbonyl, sec-butylcarbonyl, tert-butylcarbonyl, methoxycarbonyl, ethoxycarbonyl, n-propoxycarbonyl, isopropoxycarbonyl, sec-butoxycarbonyl, tert-butoxycarbonyl, cyanomethyl, 2-cyanoethyl;
A₁ represents CR²,
A₂ represents CR³,
A₃ represents CR⁴ and
A₄ represents CR⁵, where
R², R³ and R⁵ independently of one another represent hydrogen, fluorine, chlorine, bromine, CN, NO₂, methyl, ethyl, fluoromethyl, difluoromethyl, trifluoromethyl, 2,2,2-trifluoroethyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, methylsulphonyl, methylsulphinyl, trifluoromethylsulphonyl, trifluoromethylsulphinyl and *N-*cyclopropylaminocarbonyl;
R⁴ represents hydrogen, fluorine, chlorine, bromine, CN, NO₂, methyl, ethyl, fluoromethyl, difluoromethyl, trifluoromethyl, 2,2,2-trifluoroethyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, methylsulphonyl, methylsulphinyl, trifluoromethylsulphonyl, trifluoromethylsulphinyl, *N-*cyclopropylaminocarbonyl and *N*-triazolyl;
R³ and R⁴ together with the carbon to which they are attached may a 6-R² and R³ together with the carbon to which they are attached may form a 6-membered ring which contains 0, 1 or 2 nitrogen atoms and/or 0 or 1 oxygen atom and/or 0 or 1 sulphur atom;
M¹ represents hydrogen,
M² represents hydrogen, C₁-C₃-alkyl, C₂-C₃-alkenyl, C₂-C₃-alkoxycarbonyl, cyano or cyano-C₁-C₂-alkyl,
M³ represents hydrogen, methyl, ethyl, n-propyl, methoxymethyl, allyl or cyanomethyl;
W¹ and W² each represent oxygen;
p assumes the value 1,
Q represents hydrogen or the optionally substituted groupings C₁-C₆-alkyl, C₂-C₆-alkenyl, C₂-C₆-alkynyl, C₃-C₆-cycloalkyl, cyano-C₁-C₂-alkyl, C₁-C₅-heterocycloalkyl, C₁-C₄-alkoxy, C₄-C₇-alkylcycloalkyl, C₄-C₇-cycloalkylalkyl, C₂-C₇-alkylcarbonyl, C₁-C₆-alkylaldehyde, C₁-C₆-hydroxyalkyl, C₂-C₇-alkoxycarbonyl, C₁-C₆-haloalkyl, cyano, aryl, heteroaryl or represents a grouping OR⁷, SR⁷ or NR⁶R⁸, where
R⁶ represents hydrogen or C₁-C₃-alkyl;
R⁷ is selected from the optionally substituted groupings C₁-C₄-alkyl, C₂-C₄-alkenyl, C₂-C₄-alkynyl or C₃-C₆-cycloalkyl;
R⁸ represents hydrogen;
where
Z¹ and Z² independently of one another represent hydrogen, chlorine, bromine, iodine, cyano, nitro or the optionally substituted groupings C₁-C₄-alkyl, C₁-C₄-alkenyl, C₁-C₄-haloalkyl, C₃-C₆-halocycloalkyl, C₁-C₄-alkylthio, C₁-C₄-alkylsulphinyl, C₁-C₄-alkylsulphonyl;
Z⁵ represents hydrogen or the optionally substituted groupings C₁-C₄-alkyl, C₂-C₄-alkenyl, C₂-C₄-alkynyl, C₁-C₄-haloalkyl, C₃-C₄-cycloalkyl or C₁-C₃-alkoxy
where, if the groups R⁷, Z¹, Z², Z⁵ and Q are in each case substituted independently of one another, the substituents are one (1) substituent or a plurality of substituents selected from a group consisting of amino, hydroxy, halogen, nitro, cyano, isocyano, mercapto, isothiocyanato, carboxy, carboxamide, SF₅, aminosulfonyl alkyl, cycloalkyl, alkenyl, cycloalkenyl, alkenyl, *N-*mono-alkyl-amino, *N,N*-dialkylamino, *N-*alkanoylamino, alkoxy, alkenyloxy alkinyloxy, cycloalkoxy, cycloalkenyloxy, alkoxycarbonyl, alkenyloxycarbonyl, alkinyloxycarbonyl, aryloxycarbonyl, alkanoyl, alkenylcarbonyl, alkinylcarbonyl, arylcarbonyl, alkylthio, cycloalkylthio, alkenylthio, cycloalkenylthio, alkinylthio, alkylsulfenyl and alkylsulfinyl, where both enantiomers of the alkylsulfinyl group are included, alkylsulfonyl, *N*-monoalkyl aminosulfonyl, *N-N*-dialkyl-aminosulfonyl, alkylphosphinyl, alkylphosphonyl, where for alkylphosphinyl and alkylphosphonyl both enantiomers are included, *N*-alkylaminocarbonyl, *N,N*-dialkylaminocarbonyl, *N*-alkanoylaminocarbonyl, *N*-alkanoyl *N*-alkylaminocarbonyl, aryl, aryloxy, benzyl, benzyloxy, benzylthio, arylthio, arylamino, benzylamino, heterocyclyl, trialkylsilyl, alkoxyalkyl, alkylthioalkyl, alkylthioalkoxy, alkoxyalkoxy, phenethyl, benzyloxy, halogenalkyl, halogencycloalkyl, halogenalkoxy, halogenalkylthio, halogenalkylsulfinyl, halogenalkylsulfonyl, halogenalkanoyl, halogenalkylcarbonyl, halogenalkoxycarbonyl, halogenalkoxyalkoxy, halogenalkoxyalkylthio, halogenalkoxyalkanoyl and halogenalkoxyalkyl.

3. Compounds of the formula (Ia) according to Claim 1 or 2
in which
R¹ represents hydrogen, methyl, ethyl, n-propyl, isopropyl, n-butyl, 2-butynyl, isobutyl, sec-butyl, methoxymethyl, ethoxymethyl, methylcarbonyl, ethylcarbonyl, allyl, propargyl, isopropylcarbonyl, methoxycarbonyl, ethoxycarbonyl, isopropoxycarbonyl, cyanomethyl, 2-cyanoethyl;
A₁ represents CR²,
A₂ represents CR³,
A₃ represents CR⁴ and
A₄ represents CR⁵, and where
R² represents hydrogen or chlorine,
R³ and R⁴ and R⁵ independently of one another represent hydrogen, fluorine, chlorine, bromine, methyl or ethyl,
M¹ represents hydrogen;
M² represents hydrogen or methyl;
M³ represents hydrogen;
W¹ and W² each represent oxygen;
p assumes the value 1,
Q represents hydrogen, methyl, ethyl, *n*-propyl, *n*-butyl, t-butyl, 1-methylethyl, 1,1-dimethylethyl, 1-methylpropyl, 1,1-dimethylpropyl, 2-methylpropyl, 2-methylbutyl, 3-methylbutyl, 2-hydroxyethyl, cyanomethyl, 2-cyanoethyl, 3-cyanopropyl, 1,1-difluoromethyl, trifluoromethyl, 2-fluoroethyl, 2,2,2-trifluoroethyl, chloromethyl, 1-chloroethyl, 2-chloroethyl, 3-chloropropyl, 2,2-difluoropropyl, cyclopropyl, 1-methylcyclopropyl, 1-cyanocyclopropyl, 2-methylcyclopropyl, cyclopropylmethyl, 2,2-dichlorocyclopropyl, 2,2-dichloro-1-methylcyclopropyl, 2,2-difluorocyclopropyl, 2-fluorocyclopropyl, 1,1'-bi(cyclopropyl)-1-yl, (2,2-difluorocyclopropyl)methyl, cyclobutyl, 3-ethyloxetan-3-yl, cyclopentyl, cyclopentylmethyl, 1-(cyclopent-1-en-1-yl)methyl, (2-methyl-1,3-dioxolan-2-yl)methyl, tetrahydrofuran-2-ylmethyl, cyclohexyl, 2-trifluoromethylcyclohexyl, 3-trifluoromethylcyclohexyl, 4-trifluoromethylcyclohexyl, 2-chlorocyclohexyl, 3-chlorocyclohexyl, 4-chlorocyclohexyl, 5-methyl-1,3-dioxan-5-yl, 1-acetylpiperidin-4-yl, 1-methylpiperidin-4-yl, prop-1-en-1-yl, 1-methylprop-1-en-1-yl, 2-methylprop-1-en-1-yl, prop-2-enyl, but-2-en-1-yl, 3-methylbut-1-en-1-yl, prop-1-yn-1-yl, (4-methyl-1,2,5-oxadiazol-3-yl)methyl, (3,5-dimethyl-1,2-oxazol-4-yl)methyl, 1*H-*tetrazol-5-ylmethyl, (5-methyl-2-thienyl)methyl, 2-furylmethyl, (3-methyl-1,2-oxazol-5-yl)methyl, 3-thienylmethyl, benzyl, 4-chlorobenzyl, 3-chlorobenzyl, 2-chlorobenzyl, 1-(4-chlorophenyl)ethyl, 1-(4-methylphenyl)ethyl, 1-(3-chlorophenyl)ethyl, 1-(2-chlorophenyl)ethyl, 1-(4-chlorophenyl)cyclopropyl, pyrimidin-2-ylmethyl, methoxy, (methylsulphanyl)methyl, (methylsulphinyl)methyl, (methylsulphonyl)methyl, phenyl, 2-chlorophenyl, 3-chlorophenyl, 4-chlorophenyl, 2-trifluoromethylphenyl, 3-trifluoromethylphenyl, 4-trifluoromethylphenyl, 2-methylphenyl, 3-methylphenyl, 4-methylphenyl, pyridin-2-yl, pyridin-3-yl, pyridin-4-yl, 3-chloropyridin-4-yl, 2-chloropyridin-4-yl, 2-methylpyridin-4-yl, 6-methylpyridin-3-yl, 5-chloropyridin-3-yl, 4-chloropyridin-3-yl, pyrimidin-5-yl, (6-chloropyridin-3-yl)methyl, methoxycarbonyl, ethoxycarbonyl, *N-*methylcarboxamide, *N*-ethylcarboxamide, *N-*cyclopropylcarboxamide, *N-*cyclopropylmethylcarboxamide, 3-methoxy-3-oxopropanoyl, 3-(methylamino)-3-oxopropanoyl, 3-(cyclopropylamino)-3-oxopropanoyl, 3-(cyclopropylmethylamino)-3-oxopropanoyl;
Z¹ represents 1-chlorocyclopropyl, trifluoromethyl or pentafluoroethyl, and
Z² represents chlorine or trifluoromethyl; and
Z⁵ represents methyl or ethyl.

4. Compounds of the general formula (III) 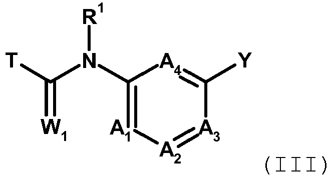 in which
R¹ represents hydrogen, optionally substituted C₁-C₆-alkyl, C₂-C₆-alkenyl, C₂-C₆-alkynyl, C₃-C₇-cycloalkyl, C₁-C₆-alkylcarbonyl, C₁-C₆-alkoxycarbonyl, cyano-C₁-C₂-alkyl, aryl-(C₁-C₃)-alkyl or heteroaryl-(C₁-C₃)-alkyl,
the chemical grouping
A₁ represents CR²,
A₂ represents CR³,
A₃ represents CR⁴ or nitrogen and
A₄ represents CR⁵,
R², R³, R⁴ and R⁵ independently of one another represent hydrogen, halogen, CN, NO₂, optionally substituted C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, C₁-C₆-alkylamino, *N,N*-di-C₂-C₆-alkylamino, *N-*C₂-C₇-alkylaminocarbonyl, *N*-C₂-C₇-cycloalkylaminocarbonyl, C₂-C₄-alkoxycarbonyl, aryl, heteroaryl or N-heteroaryl;
if none of the groupings A₂ and A₃ represents nitrogen, R³ and R⁴ together with the carbon atom to which they are attached may form a 5- or 6-membered ring which contains 0, 1 or 2 nitrogen atoms and/or 0 or 1 oxygen atom and/or 0 or 1 sulphur atom, or
if none of the groupings A₁ and A₂ represents nitrogen, R² and R³ together with the carbon atom to which they are attached may form a 5- or 6-membered ring which contains 0, 1 or 2 nitrogen atoms;
T represents 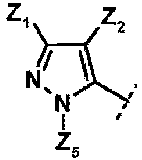 (T-6),
where
Z¹ represents hydrogen, halogen, cyano, nitro, optionally substituted C₁-C₆-alkyl, C₂-C₄-alkenyl, C₂-C₄-alkynyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, *N,N*-di-(C₁-C₆) *-* alkylamino, -C(=W)NR¹¹R¹⁰, -C(=W)OR¹², - S(O)₂NR¹³R¹⁴, -S(O)ₙR¹⁵, -S(O)(=NR¹⁶)R¹⁷ or optionally substituted phenyl or pyridinyl;
Z² represents C₁-C₆-haloalkyl;
Z⁵ represents hydrogen, optionally substituted C₁-C₆-alkyl, C₂-C₄-alkenyl, C₂-C₄-alkynyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, *N,N*-di-(C₁-C₆)-alkylamino, -C(=W)NR¹¹R¹⁰, - C(=W)OR¹², -S(O)₂NR¹³R¹⁴, -S(O)ₙR¹⁵, -S(O)(=NR¹⁶)R¹⁷ or optionally substituted phenyl or pyridinyl;
R¹⁰ is selected from hydrogen or the optionally substituted groupings C₁-C₆-alkyl, C₂-C₆-alkenyl, C₂-C₆-alkynyl, C₃-C₆-cycloalkyl, C₂-C₇-alkylcarbonyl and C₂-C₇-alkoxycarbonyl;
R¹¹ is selected from hydrogen or the optionally substituted groupings C₁-C₆-alkyl, C₂-C₆-alkenyl, C₂-C₆-alkynyl, C₃-C₆-cycloalkyl, C₂-C₇-alkylcarbonyl and C₂-C₇-alkoxycarbonyl, aryl or heteroaryl;
R¹² is selected from hydrogen or the optionally substituted grouping C₁-C₆-alkyl, C₁-C₆-haloalkyl, C₂-C₆-alkenyl, C₂-C₆-haloalkenyl, C₂-C₆-alkynyl, C₃-C₆-cycloalkyl, C₃-C₆-halocycloalkyl, C₄-C₇-alkylcycloalkyl and C₄-C₇-cycloalkylalkyl, aryl or heteroaryl;
R¹³ is selected from hydrogen or the optionally substituted groupings C₁-C₆-alkyl, C₁-C₆-haloalkyl, C₂-C₆-alkenyl, C₂-C₆-alkynyl, C₃-C₆-cycloalkyl, C₄-C₇-alkylcycloalkyl, C₄-C₇-cycloalkylalkyl, C₂-C₇-alkylcarbonyl and C₂-C₇-alkoxycarbonyl, aryl or heteroaryl;
R¹⁴ is selected from hydrogen or the optionally substituted groupings C₁-C₆-alkyl, C₂-C₆-alkenyl, C₂-C₆-alkynyl, C₃-C₆-cycloalkyl, C₄-C₇-alkylcycloalkyl and C₄-C₇-cycloalkylalkyl;
R¹⁵ is selected from the optionally substituted groupings C₁-C₆-alkyl, C₂-C₆-alkenyl, C₂-C₆-alkynyl, C₃-C₆-cycloalkyl, C₄-C₇-alkylcycloalkyl and C₄-C₇-cycloalkylalkyl, C₁-C₄-haloalkyl, aryl or heteroaryl;
R¹⁶ is selected from hydrogen or the optionally substituted groupings C₁-C₆-alkyl, C₁-C₆-haloalkyl, C₂-C₆-alkenyl, C₂-C₆-alkynyl, C₃-C₆-cycloalkyl, C₄-C₇-alkylcycloalkyl, C₄-C₇-cycloalkylalkyl, C₂-C₇-alkylcarbonyl, C₂-C₇-alkoxycarbonyl, cyano or nitro;
R¹⁷ is selected from hydrogen or the optionally substituted groupings C₁-C₆-alkyl, C₂-C₆-alkenyl, C₂-C₆-alkynyl, C₃-C₆-cycloalkyl, C₄-C₇-alkylcycloalkyl, C₄-C₇-cycloalkyl, aryl or heteroaryl;
n may assume the value 0, 1 or 2;
Y represents CN or CH₂NH₂.

5. Compounds of the general formula (III) according to Claim 4
in which
R¹ represents hydrogen or the optionally substituted groupings C₁-C₆-alkyl, C₂-C₆-alkenyl, C₂-C₆-alkynyl, C₃-C₇-cycloalkyl, C₁-C₆-alkylcarbonyl, C₁-C₆-alkoxycarbonyl, cyano-C₁-C₂-alkyl, aryl-(C₁-C₃)-alkyl, heteroaryl-(C₁-C₃)-alkyl;
the chemical grouping
A₁ represents CR²,
A₂ represents CR³,
A₃ represents CR⁴ and
A₄ represents CR⁵,
R², R³ and R⁵ independently of one another represent hydrogen, fluorine, chlorine, bromine, CN, NO₂, methyl, ethyl, fluoromethyl, difluoromethyl, trifluoromethyl, 2,2,2-trifluoroethyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, methylsulphonyl, methylsulphinyl, trifluoromethylsulphonyl, trifluoromethylsulphinyl and *N-*cyclopropylaminocarbonyl;
R⁴ represents hydrogen, fluorine, chlorine, bromine, CN, NO₂, methyl, ethyl, fluoromethyl, difluoromethyl, trifluoromethyl, 2,2,2-trifluoroethyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, methylsulphonyl, methylsulphinyl, trifluoromethylsulphonyl, trifluoromethylsulphinyl, *N*-cyclopropylaminocarbonyl and *N*-triazolyl;
R³ and R⁴ together with the carbon to which they are attached may form a 6-membered ring which contains 0, 1 or 2 nitrogen atoms and/or 0 or 1 oxygen atom and/or 0 or 1 sulphur atom; or
R² and R³ together with the carbon to which they are attached may form a 6-membered ring which contains 0, 1 or 2 nitrogen atoms and/or 0 or 1 oxygen atom and/or 0 or 1 sulphur atom;
T represents 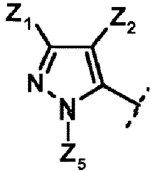 (T-6),
where
Z¹ represents hydrogen, chlorine, bromine, iodine, cyano, nitro or the optionally substituted groupings C₁-C₄-alkyl, C₁-C₄-alkenyl, C₁-C₄-haloalkyl, C₃-C₆-halocycloalkyl, C₁-C₄-alkylthio, C₁-C₄-alkylsulphinyl, C₁-C₄-alkylsulphonyl,
Z² represents C₁-C₄-haloalkyl,
Z⁵ represents hydrogen or the optionally substituted groupings C₁-C₄-alkyl, C₂-C₄-alkenyl, C₂-C₄-alkynyl, C₁-C₄-haloalkyl, C₃-C₄-cycloalkyl or C₁-C₃-alkoxy;
n may assume the value 0, 1 or 2;
Y represents CN or CH₂NH₂.

6. Compounds of the general formula (III) according to Claim 4 or 5 in which
R¹ represents hydrogen, methyl, ethyl, n-propyl, isopropyl, n-butyl, 2-butynyl, isobutyl, sec-butyl, methoxymethyl, ethoxymethyl, methylcarbonyl, ethylcarbonyl, allyl, propargyl, isopropylcarbonyl, methoxycarbonyl, ethoxycarbonyl, isopropoxycarbonyl, cyanomethyl, 2-cyanoethyl;
the chemical grouping
A₁ represents CR²,
A₂ represents CR³,
A₃ represents CR⁴ and
A₄ represents CR⁵,
R² represents hydrogen or chlorine,
R³ and R⁴ and R⁵ independently of one another represent hydrogen, fluorine, chlorine, bromine, methyl or ethyl,
T represents 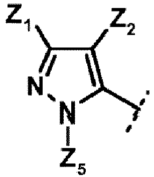 (T-6),
where
Z¹ represents trifluoromethyl or pentafluoroethyl, and
Z² represents trifluoromethyl;
Z⁵ represents methyl or ethyl,
Y represents CN or CH₂NH₂.

7. Use of compounds of the general formula (Ia) according to any of Claims 1 to 4 for controlling insects, arachnids and nematodes, where the surgical, therapeutic and diagnostic treatment of the human or animal body is excluded.

8. Pharmaceutical compositions comprising at least one compound according to any of Claims 1 to 4.

9. Compounds according to any of Claims 1 to 4 for use as medicaments.

10. Use of compounds of the general formula (Ia) according to any of Claims 1 to 4 and Claim 7 for preparing pharmaceutical compositions for controlling parasites on animals.

11. Process for preparing crop protection compositions comprising compounds of the general formula (Ia) according to any of Claims 1 to 4 and customary extenders and/or surfactants.

12. Method for controlling pests, **characterized in that** a compound of the general formula (Ia) according to any of Claims 1 to 4 is allowed to act on the pests and/or their habitat, where the surgical, therapeutic and diagnostic treatment of the human or animal body is excluded.

13. Use of compounds of the general formula (Ia) according to any of Claims 1 to 4 for protecting the propagation material of plants, in particular seed.

## Revendications

1. Composés de formule générale (Ia) 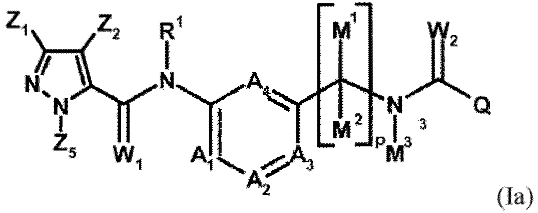 dans lesquels
R¹ représente hydrogène ou les groupes éventuellement substitués alkyle en C₁-C₆, alcényle en C₂-C₆, alcynyle en C₂-C₆, cycloalkyle en C₃-C₇, alkylcarbonyle en C₁-C₆, alcoxycarbonyle en C₁-C₆, cyano-alkyle en C₁-C₂, aryl-alkyle en C₁-C₃, hétéroaryl-alkyle en C₁-C₃ ;
A₁ représente CR²,
A₂ représente CR³,
A₃ représente CR⁴ ou azote, et
A₄ représente CR⁵,
R², R³ et R⁵ représentant indépendamment les uns des autres hydrogène, halogène, CN, NO₂, alkyle en C₁-C₆ éventuellement substitué, halogénoalkyle en C₁-C₆, cycloalkyle en C₃-C₆, halogénocycloalkyle en C₃-C₆, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, alkylamino en C₁-C₆, N,N-dialkylamino en C₂-C₆, N-alkylaminocarbonyle en C₂-C₇, N-cycloalkylaminocarbonyle en C₂-C₇ ou alcoxycarbonyle en C₂-C₄ ;
R⁴ représentant hydrogène, halogène, CN, NO₂, alkyle en C₁-C₆ éventuellement substitué, halogénoalkyle en C₁-C₆, cycloalkyle en C₃-C₆, halogénocycloalkyle en C₃-C₆, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, alkylamino en C₁-C₆, N,N-di-alkylamino en C₂-C₆, N-alkylaminocarbonyle en C₂-C₇, N-cycloalkylaminocarbonyle en C₂-C₇ ou alcoxycarbonyle en C₂-C₄ ou N-hétéroaryle ;
lorsque le groupe A₃ ne représente pas l'azote, R³ et R⁴ pouvant former ensemble avec le carbone auquel ils sont reliés un cycle à 5 ou 6 éléments, qui contient 0, 1 ou 2 atomes d'azote et/ou 0 ou 1 atome d'oxygène et/ou 0 ou 1 atome de soufre,
ou
R² et R³ pouvant former ensemble avec le carbone auquel ils sont reliés un cycle à 6 éléments qui contient 0, 1 ou 2 atomes d'azote et/ou 0 ou 1 atome d'oxygène et/ou 0 ou 1 atome de soufre ;
M¹ et M² représentent chacun indépendamment l'un de l'autre hydrogène, alkyle en C₁-C₆, alcényle en C₂-C₆, alcynyle en C₂-C₆, cycloalkyle en C₃-C₆, alcoxycarbonyle en C₂-C₇, halogénoalkyle en C₁-C₆, cyano ou cyano-alkyle en C₁-C₂, ou
M¹ et M² forment avec l'atome de carbone auquel ils sont reliés un cycle à 3, 4, 5 ou 6 éléments éventuellement substitué, qui contient éventuellement 0, 1 ou 2 atomes d'azote et/ou 0, 1 ou 2 atomes d'oxygène et/ou 0, 1 ou 2 atomes de soufre,
M³ représente hydrogène, alkyle en C₁-C₆ éventuellement substitué, alcényle en C₂-C₆, alcynyle en C₂-C₆, cycloalkyle en C₃-C₇, alkylcarbonyle en C₁-C₆, alcoxycarbonyle en C₁-C₆, cyano-alkyle en C₁-C₂, aryl-alkyle en C₁-C₃ ou hétéroaryl-alkyle en C₁-C₃,
W¹ et W² représentent indépendamment l'un de l'autre oxygène ou soufre ;
p prend la valeur 1,
Q représente hydrogène ou les groupes éventuellement substitués alkyle en C₁-C₆, alcényle en C₂-C₆, alcynyle en C₂-C₆, cycloalkyle en C₃-C₆, cyano-alkyle en C₁-C₂, hétérocycloalkyle en C₁-C₅, alcoxy en C₁-C₄, alkylcycloalkyle en C₄-C₇, cycloalkylalkyle en C₄-C₇, alkylcarbonyle en C₂-C₇, alkylaldéhyde en C₁-C₆, hydroxyalkyle en C₁-C₆, alcoxycarbonyle en C₂-C₇, halogénoalkyle en C₁-C₆, formyle, hydroxy, halogène, cyano, aryle, hétéroaryle, ou un groupe OR⁷, SR⁷, NR⁶R⁸, R⁶ représentant hydrogène ou les groupes éventuellement substitués alkyle en C₁-C₆, aryle, hétéroaryle, alcényle en C₂-C₆, alcynyle en C₂-C₆, cycloalkyle en C₃-C₆, alkylcycloalkyle en C₄-C₇ et cycloalkylalkyle en C₄-C₇, alkylcarbonyle en C₂-C₇, alcoxycarbonyle en C₂-C₇ ;
R⁷ étant choisi parmi les groupes éventuellement substitués alkyle en C₁-C₆, alcényle en C₂-C₆, alcynyle en C₂-C₆, cycloalkyle en C₃-C₆, alkylcycloalkyle en C₄-C₇ et cycloalkylalkyle en C₄-C₇ ;
R⁸ étant choisi parmi hydrogène ou les groupes éventuellement substitués alkyle en C₁-C₆, alcényle en C₂-C₆, alcynyle en C₂-C₆, cycloalkyle en C₃-C₆, alkylcycloalkyle en C₄-C₇ et cycloalkylalkyle en C₄-C₇ ; Z¹ et Z² représentent indépendamment l'un de l'autre hydrogène, halogène, cyano, nitro ou les groupes éventuellement substitués alkyle en C₁-C₆, alcényle en C₂-C₄, alcynyle en C₂-C₄, halogénoalkyle en C₁-C₆, cycloalkyle en C₃-C₆, halogénocycloalkyle en C₃-C₆, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, N,N-di-alkylamino en C₁-C₆, -S(O)₂NR¹³R¹⁴, -S(O)ₙR¹⁵, -S(O) (=NR¹⁶)R¹⁷ ou phényle ou pyridinyle éventuellement substitué ;
Z⁵ représente hydrogène, alkyle en C₁-C₆ éventuellement substitué, alcényle en C₂-C₄, alcynyle en C₂-C₄, halogénoalkyle en C₁-C₆, cycloalkyle en C₃-C₆, halogénocycloalkyle en C₃-C₆, ou phényle et pyridinyle éventuellement substitué ;
R¹³ étant choisi parmi hydrogène ou un des groupes éventuellement substitués alkyle en C₁-C₆, halogénoalkyle en C₁-C₆, alcényle en C₂-C₆, alcynyle en C₂-C₆, cycloalkyle en C₃-C₆, alkylcycloalkyle en C₄-C₇, cycloalkylalkyle en C₄-C₇, alkylcarbonyle en C₂-C₇, alcoxycarbonyle en C₂-C₇, aryle ou hétéroaryle,
R¹⁴ étant choisi parmi hydrogène ou un des groupes éventuellement substitué alkyle en C₁-C₆, alcényle en C₂-C₆, alcynyle en C₂-C₆, cycloalkyle en C₃-C₆, alkylcycloalkyle en C₄-C₇ et cycloalkylalkyle en C₄-C₇ ; R¹⁵ étant choisi parmi alkyle en C₁-C₆ éventuellement substitué, alcényle en C₂-C₆, alcynyle en C₂-C₆, cycloalkyle en C₃-C₆, alkylcycloalkyle en C₄-C₇, cycloalkylalkyle en C₄-C₇, haloalkyle en C₁-C₄, aryle ou hétéroaryle ;
R¹⁶ étant choisi parmi hydrogène ou un des groupes éventuellement substitués alkyle en C₁-C₆, halogénoalkyle en C₁-C₆, alcényle en C₂-C₆, alcynyle en C₂-C₆, cycloalkyle en C₃-C₆, alkylcycloalkyle en C₄-C₇, cycloalkylalkyle en C₄-C₇, alkylcarbonyle en C₂-C₇, alcoxycarbonyle en C₂-C₇, cyano ou nitro ; et
R¹⁷ étant choisi parmi hydrogène, alkyle en C₁-C₆ éventuellement substitué, alcényle en C₂-C₆, alcynyle en C₂-C₆, cycloalkyle en C₃-C₆, alkylcycloalkyle en C₄-C₇ et cycloalkylalkyle en C₄-C₇, aryle ou hétéroaryle, lorsque les groupes R¹, R², R³, R⁴, R⁵, R⁶, R⁷, R⁸, R¹³, R¹⁴, R¹⁵, R¹⁶, R¹⁷, M¹, M², M³, Z¹, Z², Z⁵ et Q sont substitués chacun indépendamment les uns des autres, les substituants étant un (1) substituant ou plusieurs substituants choisis dans un groupe constitué par amino, hydroxy, halogène, nitro, cyano, isocyano, mercapto, isothiocyano, carboxy, carbonamide, SF₅, aminosulfonyle, alkyle, cycloalkyle, alcényle, cycloalcényle, alcynyle, N-monoalkylamino, N,N-dialkylamino, N-alcanoylamino, alcoxy, alcényloxy, alcynyloxy, cycloalcoxy, cycloalcényloxy, alcoxycarbonyle, alcényloxycarbonyle, alcynyloxycarbonyle, aryloxycarbonyle, alcanoyle, alcénylcarbonyle, alcynylcarbonyle, arylcarbonyle, alkylthio, cycloalkylthio, alcénylthio, cycloalcénylthio, alcynylthio, alkylsulfényle et alkylsulfinyle, les deux énantiomères du groupe alkylsulfinyle étant compris, alkylsulfonyle, N-monoalkylaminosulfonyle, N,N-dialkylaminosulfonyle, alkylphosphinyle, alkylphosphonyle, les deux énantiomères étant compris pour alkylphosphinyle et alkylphosphonyle, N-alkylaminocarbonyle, N,N-dialkylaminocarbonyle, N-alcanoylaminocarbonyle, N-alcanoyl-N-alkylaminocarbonyle, aryle, aryloxy, benzyle, benzyloxy, benzylthio, arylthio, arylamino, benzylamino, hétérocyclyle, trialkylsilyle, alcoxyalkyle, alkylthioalkyle, alkylthioalcoxy, alcoxyalcoxy, phénéthyle, benzyloxy, halogénoalkyle, halogénocycloalkyle, halogénoalcoxy, halogénoalkylthio, halogénoalkylsulfinyle, halogénoalkylsulfonyle, halogénoalcanoyle, halogénoalkylcarbonyle, halogénoalcoxycarbonyle, halogénoalcoxyalcoxy, halogénoalcoxyalkylthio, halogénoalcoxyalcanoyle et halogénoalcoxyalkyle.

2. Composés de formule générale (Ia) selon la revendication 1, dans lesquels
R¹ représente hydrogène, méthyle, éthyle, n-propyle, isopropyle, n-butyle, 2-butinyle, isobutyle, sec-butyle, tert-butyle, méthoxyméthyle, éthoxyméthyle, propoxyméthyle, méthylcarbonyle, éthylcarbonyle, n-propylcarbonyle, allyle, propargyle, isopropylcarbonyle, sec-butylcarbonyle, tert-butylcarbonyle, méthoxycarbonyle, éthoxycarbonyle, n-propoxycarbonyle, isopropoxycarbonyle, sec-butoxycarbonyle, tert-butoxycarbonyle, cyanométhyle, 2-cyanoéthyle ;
A₁ représente CR²,
A₂ représente CR³,
A₃ représente CR⁴ et
A₄ représente CR⁵,
R², R³ et R⁵ représentant indépendamment les uns des autres hydrogène, fluoro, chlore, brome, CN, NO₂, méthyle, éthyle, fluorométhyle, difluorométhyle, trifluorométhyle, 2,2,2-trifluoroéthyle, méthoxy, éthoxy, n-propoxy, 1-méthyléthoxy, fluorométhoxy, difluorométhoxy, chlorodifluorométhoxy, dichlorofluorométhoxy, trifluorométhoxy, 2,2,2-trifluoroéthoxy, 2-chloro-2,2-difluoroéthoxy, pentafluoroéthoxy, méthylsulfonyle, méthylsulfinyle, trifluorométhylsulfonyle, trifluorométhylsulfinyle et N-cyclopropylaminocarbonyle ;
R⁴ représentant hydrogène, fluor, chlore, brome, CN, NO₂, méthyle, éthyle, fluorométhyle, difluorométhyle, trifluorométhyle, 2,2,2-trifluoroéthyle, méthoxy, éthoxy, n-propoxy, 1-méthyléthoxy, fluorométhoxy, difluorométhoxy, chloro-difluorométhoxy, dichloro-fluorométhoxy, trifluorométhoxy, 2,2,2-trifluoroéthoxy, 2-chloro-2,2-difluoroéthoxy, pentafluoroéthoxy, méthylsulfonyle, méthylsulfinyle, trifluorométhylsulfonyle, trifluorométhylsulfinyle, N-cyclopropylaminocarbonyle et N-triazolyle ;
R³ et R⁴ pouvant ensemble avec le carbone auquel ils sont reliés un 6-R² et R³ pouvant former ensemble avec le carbone auquel ils sont reliés un cycle à 6 éléments qui contient 0, 1 ou 2 atomes d'azote et/ou 0 ou 1 atome d'oxygène et/ou 0 ou 1 atome de soufre ;
M¹ représente hydrogène,
M² représente hydrogène, alkyle en C₁-C₃, alcényle en C₂-C₃, alcoxycarbonyle en C₂-C₃, cyano ou cyano-alkyle en C₁-C₂,
M³ représente hydrogène, méthyle, éthyle, n-propyle, méthoxyméthyle, allyle ou cyanométhyle,
W¹ et W² représentent chacun oxygène ;
p prend la valeur 1,
Q représente hydrogène ou les groupes éventuellement substitués alkyle en C₁-C₆, alcényle en C₂-C₆, alcynyle en C₂-C₆, cycloalkyle en C₃-C₆, cyano-alkyle en C₁-C₂, hétérocycloalkyle en C₁-C₅, alcoxy en C₁-C₄, alkylcycloalkyle en C₄-C₇, cycloalkylalkyle en C₄-C₇, alkylcarbonyle en C₂-C₇, alkylaldéhyde en C₁-C₆, hydroxyalkyle en C₁-C₆, alcoxycarbonyle en C₂-C₇, halogénoalkyle en C₁-C₆, cyano, aryle, hétéroaryle, ou un groupe OR⁷, SR⁷, NR⁶R⁸,
R⁶ représentant hydrogène ou alkyle en C₁-C₃ ;
R⁷ étant choisi parmi les groupes éventuellement substitués alkyle en C₁-C₄, alcényle en C₂-C₄, alcynyle en C₂-C₄ ou cycloalkyle en C₃-C₆ ;
R⁸ représentant hydrogène ;
Z¹ et Z² représentent indépendamment l'un de l'autre hydrogène, chlore, brome, iode, cyano, nitro ou les groupes éventuellement substitués alkyle en C₁-C₄, alcényle en C₁-C₄, halogénoalkyle en C₁-C₄, halogénocycloalkyle en C₃-C₆, alkylthio en C₁-C₄, alkylsulfinyle en C₁-C₄, alkylsulfonyle en C₁-C₄ ;
Z⁵ représente hydrogène ou les groupes éventuellement substitués alkyle en C₁-C₄, alcényle en C₂-C₄, alcynyle en C₂-C₄, halogénoalkyle en C₁-C₄, cycloalkyle en C₃-C₄ ou alcoxy C₁-C₃,
lorsque les groupes R⁷, Z¹, Z², Z⁵ et Q sont substitués chacun indépendamment les uns des autres, les substituants étant un (1) substituant ou plusieurs substituants choisis dans un groupe constitué par amino, hydroxy, halogène, nitro, cyano, isocyano, mercapto, isothiocyano, carboxy, carbonamide, SF₅, aminosulfonyle, alkyle, cycloalkyle, alcényle, cycloalcényle, alcynyle, N-monoalkylamino, N,N-dialkylamino, N-alcanoylamino, alcoxy, alcényloxy, alcynyloxy, cycloalcoxy, cycloalcényloxy, alcoxycarbonyle, alcényloxycarbonyle, alcynyloxycarbonyle, aryloxycarbonyle, alcanoyle, alcénylcarbonyle, alcynylcarbonyle, arylcarbonyle, alkylthio, cycloalkylthio, alcénylthio, cycloalcénylthio, alcynylthio, alkylsulfényle et alkylsulfinyle, les deux énantiomères du groupe alkylsulfinyle étant compris, alkylsulfonyle, N-monoalkylaminosulfonyle, N,N-dialkylaminosulfonyle, alkylphosphinyle, alkylphosphonyle, les deux énantiomères étant compris pour alkylphosphinyle et alkylphosphonyle, N-alkylaminocarbonyle, N,N-dialkylaminocarbonyle, N-alcanoylaminocarbonyle, N-alcanoyl-N-alkylaminocarbonyle, aryle, aryloxy, benzyle, benzyloxy, benzylthio, arylthio, arylamino, benzylamino, hétérocyclyle, trialkylsilyle, alcoxyalkyle, alkylthioalkyle, alkylthioalcoxy, alcoxyalcoxy, phénéthyle, benzyloxy, halogénoalkyle, halogénocycloalkyle, halogénoalcoxy, halogénoalkylthio, halogénoalkylsulfinyle, halogénoalkylsulfonyle, halogénoalcanoyle, halogénoalkylcarbonyle, halogénoalcoxycarbonyle, halogénoalcoxyalcoxy, halogénoalcoxyalkylthio, halogénoalcoxyalcanoyle et halogénoalcoxyalkyle.

3. Composés de formule (Ia) selon la revendication 1 ou 2, dans lesquels
R¹ représente hydrogène, méthyle, éthyle, n-propyle, isopropyle, n-butyle, 2-butinyle, isobutyle, sec-butyle, méthoxyméthyle, éthoxyméthyle, méthylcarbonyle, éthylcarbonyle, allyle, propargyle, isopropylcarbonyle, méthoxycarbonyle, éthoxycarbonyle, isopropoxycarbonyle, cyanométhyle, 2-cyanoéthyle ;
A₁ représente CR²,
A₂ représente CR³,
A₃ représente CR⁴ et
A₄ représente CR⁵,
R² représentant hydrogène ou chlore,
R³ et R⁴ et R⁵ représentant indépendamment les uns des autres hydrogène, fluor, chlore, brome, méthyle ou éthyle,
M¹ représente hydrogène,
M² représente hydrogène ou méthyle,
M³ représente hydrogène,
W¹ et W² représentent chacun oxygène ;
p prend la valeur 1,
Q représente hydrogène, méthyle, éthyle, n-propyle, n-butyle, t-butyle, 1-méthyléthyle, 1,1-diméthyléthyle, 1-méthylpropyle, 1,1-diméthylpropyle, 2-méthylpropyle, 2-méthylbutyle, 3-méthylbutyle, 2-hydroxyéthyle, cyanométhyle, 2-cyanoéthyle, 3-cyanopropyle, 1,1-difluorométhyle, trifluorométhyle, 2-fluoroéthyle, 2,2,2-trifluoroéthyle, chlorométhyle, 1-chloroéthyle, 2-chloroéthyle, 3-chloropropyle, 2,2-difluoropropyle, cyclopropyle, 1-méthylcyclopropyle, 1-cyanocyclopropyle, 2-méthylcyclopropyle, cyclopropylméthyle, 2,2-dichlorocyclopropyle, 2,2-dichloro-1-méthylcyclopropyle, 2,2-difluorocyclopropyle, 2-fluorocyclopropyle, 1,1'-bi(cyclopropyl)-1-yle, (2,2-difluorocyclopropyl)méthyle, cyclobutyle, 3-éthyloxétan-3-yle, cyclopentyle, cyclopentylméthyle, 1-(cyclopent-1-én-1-yl)méthyle, (2-méthyl-1,3-dioxolan-2-yl)méthyl, tétrahydrofuran-2-ylméthyle, cyclohexyle, 2-trifluorométhylcyclohexyle, 3-trifluorométhylcyclohexyle, 4-trifluorométhylcyclohexyle, 2-chlorocyclohexyle, 3-chlorocyclohexyle, 4-chlorocyclohexyle, 5-méthyl-1,3-dioxan-5-yle, 1-acétylpipéridin-4-yle, 1-méthylpipéridin-4-yle, prop-1-én-1-yle, 1-méthylprop-1-én-1-yle, 2-méthylprop-1-én-1-yle, prop-2-ényle, but-2-én-1-yle, 3-méthylbut-1-én-1-yle, prop-1-in-1-yle, (4-méthyl-1,2,5-oxadiazol-3-yl)méthyle, (3,5-diméthyl-1,2-oxazol-4-yl)méthyle, 1H-tétrazol-5-ylméthyle, (5-méthyl-2-thiényl)méthyle, 2-furylméthyle, (3-méthyl-1,2-oxazol-5-yl)méthyle, 3-thiénylméthyle, benzyle, 4-chlorobenzyle, 3-chlorobenzyle, 2-chlorobenzyle, 1-(4-chlorophényl)éthyle, 1-(4-méthylphényl)éthyle, 1-(3-chlorophényl)éthyle, 1-(2-chlorophényl)éthyle, 1-(4-chlorophényl)cyclopropyle, pyrimidin-2-ylméthyle, méthoxy, (méthylsulfanyl)méthyle, (méthylsulfinyl)méthyle, (méthylsulfonyl)méthyle, phényle, 2-chlorophényle, 3-chlorophényle, 4-chlorophényle, 2-trifluorométhylphényle, 3-trifluorométhylphényle, 4-trifluorométhylphényle, 2-méthylphényle, 3-méthylphényle, 4-méthylphényle, pyridin-2-yle, pyridin-3-yle, pyridin-4-yle, 3-chloropyridin-4-yle, 2-chloropyridin-4-yle, 2-méthylpyridin-4-yle, 6-méthylpyridin-3-yle, 5-chloropyridin-3-yle, 4-chloropyridin-3-yle, pyrimidin-5-yle, (6-chloropyridin-3-yl)méthyle, méthoxycarbonyle, éthoxycarbonyle, *N*-méthylcarboxamide, N-éthylcarboxamide, N-cyclopropylcarboxamide, N-cyclopropylméthylcarboxamide, 3-méthoxy-3-oxopropanoyle, 3-(méthylamino)-3-oxopropanoyle, 3-(cyclopropylamino)-3-oxopropanoyle, 3-(cyclopropylméthylamino)-3-oxopropanoyle ;
Z¹ représente 1-chlorocyclopropyle, trifluorométhyle ou pentafluoroéthyle, et
Z² représente chlore ou trifluorométhyle ; et
Z⁵ représente méthyle et éthyle.

4. Composés de formule générale (III) 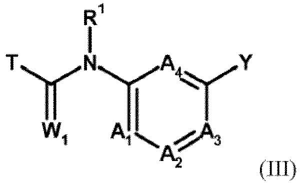 dans laquelle
R¹ représente hydrogène, alkyle en C₁-C₆ éventuellement substitué, alcényle en C₂-C₆, alcynyle en C₂-C₆, cycloalkyle en C₃-C₇, alkylcarbonyle en C₁-C₆, alcoxycarbonyle en C₁-C₆, cyano-alkyle en C₁-C₂, aryl-alkyle en C₁-C₃ ou hétéroaryl-alkyle en C₁-C₃,
le groupe chimique
A₁ représente CR²,
A₂ représente CR³,
A₃ représente CR⁴ ou azote, et
A₄ représente CR⁵,
R², R³, R⁴ et R⁵ représentant indépendamment les uns des autres hydrogène, halogène, CN, NO₂, alkyle en C₁-C₆ éventuellement substitué, halogénoalkyle en C₁-C₆, cycloalkyle en C₃-C₆, halogénocycloalkyle en C₃-C₆, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, alkylamino en C₁-C₆, N,N-di-alkylamino en C₂-C₆, N-alkylaminocarbonyle en C₂-C₇, N-cycloalkylaminocarbonyle en C₂-C₇, alcoxycarbonyle en C₂-C₄, aryle, hétéroaryle ou N-hétéroaryle ;
lorsqu'aucun des groupes A₂ et A₃ ne représente azote, R³ et R⁴ pouvant former ensemble avec l'atome de carbone auquel ils sont reliés un cycle à 5 ou 6 éléments, qui contient 0, 1 ou 2 atomes d'azote et/ou 0 ou 1 atome d'oxygène et/ou 0 ou 1 atome de soufre, ou
lorsqu'aucun des groupes A₁ et A₂ ne représente azote, R² et R³ pouvant former ensemble avec l'atome de carbone auquel ils sont reliés un cycle à 5 ou 6 éléments, qui contient 0, 1 ou 2 atomes d'azote ;
T représente 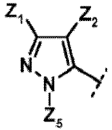 (T-6),
Z¹ représentant hydrogène, halogène, cyano, nitro, alkyle en C₁-C₆ éventuellement substitué, alcényle en C₂-C₄, alcynyle en C₂-C₄, halogénoalkyle en C₁-C₆, cycloalkyle en C₃-C₆, halogénocycloalkyle en C₃-C₆, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, N,N-di-alkylamino en C₁-C₆, -C(=W)NR¹¹R¹⁰, -C(=W)OR¹², -S(O)₂NR¹³R¹⁴,-S(O)ₙR¹⁵, -S(O)(=NR¹⁶)R¹⁷, ou phényle et pyridinyle éventuellement substitué ;
Z² représentant halogénoalkyle en C₁-C₆,
Z⁵ représentant hydrogène, alkyle en C₁-C₆ éventuellement substitué, alcényle en C₂-C₄, alcynyle en C₂-C₄, halogénoalkyle en C₁-C₆, cycloalkyle en C₃-C₆, halogénocycloalkyle en C₃-C₆, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, N,N-di-alkylamino en C₁-C₆, -C(=W)OR¹¹R¹⁰, -C(=W)OR¹², -S(O)₂NR¹³R¹⁴, -S(O)ₙNR¹⁵, -S(O)(=NR¹⁶)R¹⁷, ou phényle et pyridinyle éventuellement substitué ;
R¹⁰ étant choisi parmi hydrogène ou les groupes éventuellement substitués alkyle en C₁-C₆, alcényle en C₂-C₆, alcynyle en C₂-C₆, cycloalkyle en C₃-C₆, alkylcarbonyle en C₂-C₇ et alcoxycarbonyle en C₂-C₇,
R¹¹ étant choisi parmi hydrogène ou les groupes éventuellement substitués alkyle en C₁-C₆, alcényle en C₂-C₆, alcynyle en C₂-C₆, cycloalkyle en C₃-C₆, alkylcarbonyle en C₂-C₇ et alcoxycarbonyle en C₂-C₇, aryle ou hétéroaryle,
R¹² étant choisi parmi hydrogène ou les groupe éventuellement substitués alkyle en C₁-C₆, halogénoalkyle en C₁-C₆, alcényle en C₂-C₆, halogénoalcényle en C₂-C₆, alcynyle en C₂-C₆, cycloalkyle en C₃-C₆, halogénocycloalkyle en C₃-C₆, alkylcycloalkyle en C₄-C₇ et cycloalkylalkyle en C₄-C₇, aryle ou hétéroaryle,
R¹³ étant choisi parmi hydrogène ou les groupes éventuellement substitués alkyle en C₁-C₆, halogénoalkyle en C₁-C₆, alcényle en C₂-C₆, alcynyle en C₂-C₆, cycloalkyle en C₃-C₆, alkylcycloalkyle en C₄-C₇, cycloalkylalkyle en C₄-C₇, alkylcarbonyle en C₂-C₇ et alcoxycarbonyle en C₂-C₇, aryle ou hétéroaryle ;
R¹⁴ étant choisi parmi hydrogène ou les groupes éventuellement substitués alkyle en C₁-C₆, alcényle en C₂-C₆, alcynyle en C₂-C₆, cycloalkyle en C₃-C₆, alkylcycloalkyle en C₄-C₇ et cycloalkylalkyle en C₄-C₇ ; R¹⁵ étant choisi parmi les groupes éventuellement substitués alkyle en C₁-C₆, alcényle en C₂-C₆, alcynyle en C₂-C₆, cycloalkyle en C₃-C₆, alkylcycloalkyle en C₄-C₇ et cycloalkylalkyle en C₄-C₇, haloalkyle en C₁-C₄, aryle ou hétéroaryle ;
R¹⁶ étant choisi parmi hydrogène ou les groupes éventuellement substitués alkyle en C₁-C₆, halogénoalkyle en C₁-C₆, alcényle en C₂-C₆, alcynyle en C₂-C₆, cycloalkyle en C₃-C₆, alkylcycloalkyle en C₄-C₇, cycloalkylalkyle en C₄-C₇, alkylcarbonyle en C₂-C₇, alcoxycarbonyle en C₂-C₇, cyano ou nitro ;
R¹⁷ étant choisi parmi hydrogène ou les groupes éventuellement substitués alkyle en C₁-C₆, alcényle en C₂-C₆, alcynyle en C₂-C₆, cycloalkyle en C₃-C₆, alkylcycloalkyle en C₄-C₇, cycloalkyle en C₄-C₇, aryle ou hétéroaryle ;
n pouvant prendre les valeurs 0, 1 ou 2 ;
Y représente CN ou CH₂NH₂.

5. Composés de formule générale (III) selon la revendication 4, dans lesquels
R¹ représente hydrogène ou les groupes éventuellement substitués alkyle en C₁-C₆, alcényle en C₂-C₆, alcynyle en C₂-C₆, cycloalkyle en C₃-C₇, alkylcarbonyle en C₁-C₆, alcoxycarbonyle en C₁-C₆, cyano-alkyle en C₁-C₂, aryl-alkyle en C₁-C₃ ou hétéroaryl-alkyle en C₁-C₃,
le groupe chimique
A₁ représente CR²,
A₂ représente CR³,
A₃ représente CR⁴, et
A₄ représente CR⁵,
R², R³ et R⁵ représentant indépendamment les uns des autres hydrogène, fluore, chlore, brome, CN, NO₂, méthyle, éthyle, fluorométhyle, difluorométhyle, trifluorométhyle, 2,2,2-trifluoroéthyle, méthoxy, éthoxy, n-propoxy, 1-méthyléthoxy, fluorométhoxy, difluorométhoxy, chloro-difluorométhoxy, dichloro-fluorométhoxy, trifluorométhoxy, 2,2,2-trifluoroéthoxy, 2-chloro-2,2-difluoroéthoxy, pentafluoroéthoxy, méthylsulfonyle, méthylsulfinyle, trifluorométhylsulfonyle, trifluorométhylsulfinyle et N-cyclopropylaminocarbonyle ;
R⁴ représentant hydrogène, fluor, chlore, brome, CN, NO₂, méthyle, éthyle, fluorométhyle, difluorométhyle, trifluorométhyle, 2,2,2-trifluoroéthyle, méthoxy, éthoxy, n-propoxy, 1-méthyléthoxy, fluorométhoxy, difluorométhoxy, chloro-difluorométhoxy, dichloro-fluorométhoxy, trifluorométhoxy, 2,2,2-trifluoroéthoxy, 2-chloro-2,2-difluoroéthoxny, pentafluoroéthoxy, méthylsulfonyle, méthylsulfinyle, trifluorométhylsulfonyle, trifluorométhylsulfinyle, N-cyclopropylaminocarbonyle et N-triazolyle ;
R³ et R⁴ pouvant former ensemble avec le carbone auquel ils sont reliés un cycle à 6 éléments, qui contient 0, 1 ou 2 atomes d'azote et/ou 0 ou 1 atome d'oxygène et/ou 0 ou 1 atome de soufre, ou
R² et R³ pouvant former ensemble avec le carbone auquel ils sont reliés un cycle à 6 éléments, qui contient 0, 1 ou 2 atomes d'azote et/ou 0 ou 1 atome d'oxygène et/ou 0 ou 1 atome de soufre ;
T représente 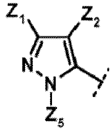 (T-6),
Z¹ représentant hydrogène, chlore, brome, iode, cyano, nitro, ou les groupes éventuellement substitués alkyle en C₁-C₄, alcényle en C₁-C₄, halogénoalkyle en C₁-C₄, halogénocycloalkyle en C₃-C₆, alkylthio en C₁-C₄, alkylsulfinyle en C₁-C₄, alkylsulfonyle en C₁-C₄ ;
Z² représentant halogénoalkyle en C₁-C₄,
Z⁵ représentant hydrogène ou les groupes éventuellement substitués alkyle en C₁-C₄, alcényle en C₂-C₄, alcynyle en C₂-C₄, halogénoalkyle en C₁-C₄, cycloalkyle en C₃-C₄ ou alcoxy en C₁-C₃ ;
n pouvant prendre les valeurs 0, 1 ou 2 ;
Y représente CN ou CH₂NH₂.

6. Composés de formule générale (III) selon l'une quelconque des revendications 4 ou 5, dans lesquels
R¹ représente hydrogène, méthyle, éthyle, n-propyle, isopropyle, n-butyle, 2-butinyle, isobutyle, sec-butyle, méthoxyméthyle, éthoxyméthyle, méthylcarbonyle, éthylcarbonyle, allyle, propargyle, isopropylcarbonyle, méthoxycarbonyle, éthoxycarbonyle, isopropoxycarbonyle, cyanométhyle, 2-cyanoéthyle ;
le groupe chimique
A₁ représente CR²,
A₂ représente CR³,
A₃ représente CR⁴, et
A₄ représente CR⁵,
R² représentant hydrogène ou chlore,
R³, R⁴ et R⁵ représentant indépendamment les uns des autres hydrogène, fluor, chlore, brome, méthyle ou éthyle,
T représente 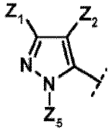 (T-6),
Z¹ représentant trifluorométhyle ou pentafluoroéthyle, et
Z² représentant trifluorométhyle,
Z⁵ représentant méthyle et éthyle,
Y représente CN ou CH₂NH₂.

7. Utilisation de composés de formule générale (Ia) selon l'une quelconque des revendications 1 à 4 pour lutter contre des insectes, des araignées et des nématodes, le traitement chirurgical, thérapeutique et diagnostic du corps humain ou animal étant exclu.

8. Compositions pharmaceutiques, contenant au moins un composé selon l'une quelconque des revendications 1 à 4.

9. Composés selon l'une quelconque des revendications 1 à 4, destinés à une utilisation en tant que médicament.

10. Utilisation de composés de formule générale (Ia) selon l'une quelconque des revendications 1 à 4 et selon la revendication 7 pour la fabrication de compositions pharmaceutiques pour lutter contre des parasites sur des animaux.

11. Procédé de fabrication d'agents phytoprotecteurs contenant des composés de formule générale (Ia) selon l'une quelconque des revendications 1 à 4, ainsi que des extendeurs et/ou substances tensioactives usuels.

12. Procédé de lutte contre des nuisibles, **caractérisé en ce qu'**un composé de formule générale (Ia) selon l'une quelconque des revendications 1 à 4 est laissé agir sur les nuisibles et/ou leur habitat, le traitement chirurgical, thérapeutique et diagnostic du corps humain ou animal étant exclu.

13. Utilisation de composés de formule générale (Ia) selon l'une quelconque des revendications 1 à 4 pour la protection du matériel de reproduction de plantes, notamment de graines.
